# EUROPÄISCHE PATENTSCHRIFT

(11) **EP 2 086 993 B1**
(45) Veröffentlichungstag und Bekanntmachung des Hinweises auf die Patenterteilung: **11.03.2015**
(21) Anmeldenummer: 07846304.9
(22) Anmeldetag: 08.11.2007
(51) Int. Cl.: C07K 5/08, C07K 5/10, C07K 7/06, C07K 1/02

(54) **MICHAELSYSTEME ALS TRANSGLUTAMINASEINHIBITOREN**
MICHAEL SYSTEMS AS TRANSGLUTAMINASE INHIBITORS
SYSTÈMES DE MICHAEL UTILISÉS COMME INHIBITEURS DE LA TRANSGLUTAMINASE

(30) Priorität: 08.11.2006 DE 102006052755; 12.12.2006 US 874246 P
(43) Veröffentlichungstag der Anmeldung: 12.08.2009
(73) Patentinhaber: Zedira GmbH, 64293 Darmstadt (DE)
(72) Erfinder: OERTEL, Kai, DE/ 60327 Frankfurt (DE)
(74) Vertreter: Arth, Hans-Lothar
(86) Internationale Anmeldenummer: PCT/DE2007/002014
(87) Internationale Veröffentlichungsnummer: WO 2008/055488

(56) Entgegenhaltungen:
- WO-A1-03/106437
- WO-A1-2005/063018
- WO-A1-2005/068421
- WO-A2-2005/025620
- US-A1- 2005 143 448
- US-A1- 2006 035 838
- US-A1- 2006 178 355
- PARIS M ET AL: "Post-synthesis modification of aspartyl or glutamyl residue side-chains on solid support" TETRAHEDRON LETTERS, ELSEVIER, AMSTERDAM, NL, Bd. 40, Nr. 28, 9. Juli 1999 (1999-07-09), Seiten 5179-5182, XP004170047 ISSN: 0040-4039
- LAZAROVA T ET AL: "Synthesis and biological evaluation of novel cyclosporin A analogues: Potential soft drugs for the treatment of autoimmune diseases" JOURNAL OF MEDICINAL CHEMISTRY 20030227 US, Bd. 46, Nr. 5, 27. Februar 2003 (2003-02-27), Seiten 674-676, XP002475699 ISSN: 0022-2623
- MEINKE P T ET AL: "Synthesis of side chain modified apicidin derivatives: Potent mechanism-based histone deacetylase inhibitors" TETRAHEDRON LETTERS 20001007 GB, Bd. 41, Nr. 41, 7. Oktober 2000 (2000-10-07), Seiten 7831-7835, XP002475700 ISSN: 0040-4039
- MARRANO C ET AL: "Evaluation of novel dipeptide-bound alpha,beta-unsaturated amides and epoxides as irreversible inhibitors of guinea pig liver transglutaminase." BIOORGANIC & MEDICINAL CHEMISTRY JUL 2001, Bd. 9, Nr. 7, Juli 2001 (2001-07), Seiten 1923-1928, XP002475701 ISSN: 0968-0896
- DE MACEDO PIERRE ET AL: "Synthesis of dipeptide-bound epoxides and alpha,beta-unsaturated amides as potential irreversible transglutaminase inhibitors" BIOORGANIC & MEDICINAL CHEMISTRY, ELSEVIER SCIENCE LTD, GB, Bd. 10, Nr. 2, Februar 2002 (2002-02), Seiten 355-360, XP002293620 ISSN: 0968-0896
- BYCROFT B W ET AL: "Convenient syntheses of (3S,5S)-carbapenam-3-carboxylates and their biosynthetic relevance" TETRAHEDRON LETTERS 20030127 GB, Bd. 44, Nr. 5, 27. Januar 2003 (2003-01-27), Seiten 973-976, XP002475702 ISSN: 0040-4039
- WEI Z -Y ET AL: "A regioselective tandem reduction - Wittig-Horner reaction involving the [alpha]-ester moiety of diethyl aspartate or glutamate" TETRAHEDRON LETTERS 1994 GB, Bd. 35, Nr. 15, 1994, Seiten 2305-2308, XP002475703 ISSN: 0040-4039
- DOYLE P M ET AL: "Peptides incorporating electrophilic glutamine analogues as potential transglutaminase inhibitors" BIOCHEMICAL SOCIETY TRANSACTIONS, COLCHESTER, ESSEX, GB, Bd. 18, Nr. 6, Dezember 1990 (1990-12), Seiten 1318-1320, XP008090045 ISSN: 0300-5127
- CSOSZ E ET AL: "Substrate Preference of Transglutaminase 2 Revealed by Logistic Regression Analysis and Intrinsic Disorder Examination", JOURNAL OF MOLECULAR BIOLOGY, ACADEMIC PRESS, UNITED KINGDOM, vol. 383, no. 2, 7 November 2008 (2008-11-07), pages 390-402, XP025471392, ISSN: 0022-2836, DOI: 10.1016/J.JMB.2008.08.026 [retrieved on 2008-08-22]

## Beschreibung

Die Erfindung betrifft Peptidderivate und Peptidomimetika als Inhibitoren für Transglutaminasen, Verfahren zu deren Herstellung, pharmazeutische Zusammensetzungen enthaltend diese Verbindungen sowie Verwendungen der Transglutaminaseinhibitoren.

Die Transglutaminasen gehören zur Klasse der Transferasen, nach EC-Nomenklatur korrekterweise "Protein-Glutamin: Amin-γ-Glutamyltransferasen" und besitzen die EC-Nummer EC 2.3.2.13.

Sie verknüpfen die ε-Aminogruppe der Aminosäure Lysin mit der γ-Glutamylgruppe der Aminosäure Glutamin unter Freisetzung von Ammoniak zu einer Isopeptidbindung.

Die Transglutaminasen spielen bei der Stabilisierung der extrazellulären Matrix (z. B. Aeschlimann und Paulsson, Thromb. Haemostasis, 71, S. 402-415, 1994) und beim programmierten Zelltod (Apoptose) in Säugerzellen ein Rolle (z. B. Fesus et al., FEBS Lett., 284, S. 109 -11, 1991).

Daneben spielen die Transglutaminasen eine große Rolle auf vielen Indikationsgebieten, wie zum Beispiel Herz-Kreislauf (Thrombose), Autoimmunkrankheiten (Zöliakie, Morbus Duhring), neurodegenerative Krankheiten (Alzheimer, Parkinson, Huntington Krankheit), dermatologische Krankheiten (Ichthyosis, Psoriasis, Akne), sowie auch bei Wundheilung und in Entzündungskrankheiten (Gewebsfibrose) (J.M. Wodzinska, Mini-Reviews in medical cheimistry, 2005, 5, 279-292).

Eine der wichtigsten Indikationen ist jedoch die Glutenunverträglichkeit Zöliakie. Zöliakie ist eine chronische Entzündung der Dünndarmschleimhaut. Die Darmepithelien werden bei den betroffenen Patienten, nach Aufnahme von glutenhaltigen Lebensmitteln, sukzessive zerstört, was eine gestörte Resorption von Nährstoffen nach sich zieht und massive Auswirkungen auf die betroffenen Patienten hat und zum Beispiel mit Gewichtsverlust, Anämie, Diarrhoe, Erbrechen, Appetitlosigkeit und Müdigkeit einhergeht. Auf Grund dieser Erkenntnisse entsteht somit ein großer Bedarf, Inhibitoren für diese Enzyme zu entwickeln.

Der vorliegenden Erfindung liegt die Aufgabe zugrunde neue Inhibitoren für Transglutaminasen zur Verfügung zu stellen sowie pharmazeutische Formulierungen enthaltend diese Inhibitoren und Synthesewege für diese Inhibitoren. Ferner werden neue Verwendungen dieser Inhibitoren aufgezeigt.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen.

Überraschend wurde gefunden, dass Verbindungen und insbesondere Peptidderivate und peptidähnliche Strukturen wie beispielsweise Peptidomimetika, welche mindestens eine akzeptorsubstituierte Doppelbindung aufweisen besonderes gute Inhibitoren für Transglutaminasen sind. Solche akzeptorsubstituierte Doppelbindungen sind vorzugsweise Michael-Systeme (MS) aus einer Carbonylfunktion (C=O) sowie einer damit konjugierten Kohlenstoff-Kohlenstoff-Doppelbindung (C=C), also ein Michael-Akzeptor-System der Art C=C-C=O. Grundsätzlich bedarf es jedoch keines konjugierten Systems. Verbindungen mit mindestens einer olefinischen Doppelbindung, wobei die Doppelbindung mindestens eine elektronenziehende Gruppe trägt, eignen sich hervorragend als Transglutaminase-Inhibitoren. Wahrscheinlich handelt es sich bei diesen Verbindungen mit akzeptorsubstituierter Doppelbindung bzw. mit Michael-System um Suizid-Inhibitoren, welche irreversibel an die Transglutaminasen binden. Zudem ist bevorzugt, wenn die akzeptorsubstituierte Doppelbindung über eine Ethylengruppe (-C₂H₄-) oder eine Carbonylethylengruppe (-CO-C₂H₄-) an ein Grundgerüst gebunden ist, wobei das Grundgerüst vorzugsweise eine peptidische, peptidähnliche oder peptidomimetische Struktur aufweist. Der erfindungsgemäße Rest des Grundgerüstes hat folgende Struktur:

akzeptorsubstituierte Doppelbindung-C₂H₄-Grundgerüst

oder akzeptorsubstituierte Doppelbindung-CO-C₂H₄-Grundgerüst.

Unter dem Begriff "akzeptorsubstituierte Doppelbindung" ist eine Doppelbindung zu verstehen, welche in Konjugation zu einer elektronenziehenden Gruppe (wie z.B. C=O, C=S, C=N, P=O, P=S, N=O, S=O, N=N, C=N) steht und in der Lage ist, mit Nukleophilen eine nukleophile Addition einzugehen und mit Atomen wie beispielsweise Sauerstoff, Schwefel und Stickstoff eine kovalente Bindung auszubilden.

Zu den humanen Transglutaminasen werden TG1, TG2, TG3, TG4, TG5, TG6, TG7 und FXIII bzw. FXIIIa gezählt. Zusätzlich können auch nicht-humane Transglutaminasen als Targets in der Parasitenbekämpfung interessant sein. ,

Die erfindungsgemäß als Transglutaminaseinhibitoren geeigneten Verbindungen können somit durch folgende allgemeine Struktur [TGI1] dargestellt werden:

akzeptorsubstituierte Doppelbindung-(CO)ₘ-C₂H₄-Grundgerüst

wobei
m für 0 oder 1 steht und
die akzeptorsubstituierte Doppelbindung mindestens einen zur Konjugation befähigten elektronenziehenden Rest vorzugsweise mit einer Elektronegativität ≥ 2,20, bevorzugt ≥ 2,50, insbesondere bevorzugt ≥ 2,80 trägt und
das Grundgerüst ein Peptid aus mindestens zwei Aminosäuren ist und/oder das Grundgerüst mindestens eine Amidbindung aufweist und das die akzeptorsubstituierte Doppelbindung tragende Grundgerüst über mindestens eine zum Kohlenstoffatom, das die Seitenkette mit der akzeptorsubstituierten Doppelbindung trägt, vicinale Carbonylgruppe verfügt. Das Grundgerüst wir hierin auch als Rest A bezeichet.

Ein weiterer Aspekt der vorliegenden Erfindung betrifft daher Verbindungen gemäß allgemeiner Formel [TGI1] als Arzneimittel sowie deren Verwendung in der Medizin.

Die akzeptorsubstituierte Doppelbindung wird hierin auch wie folgt bezeichnet: 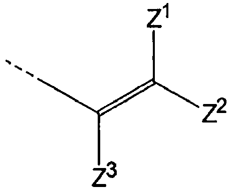 worin mindestends einer der Substituenten Z¹, Z² und Z³ eine elektronenziehende und zur Konjugation befähigte Gruppe darstellt und zwei der drei Substituenten Z¹, Z² und Z³ auch Wasserstoff bedeuten können oder für beliebige weitere elektronenziehende als auch elektronenschiebende Reste stehen. Somit kann die allgemeine Formel [TGI2] der Transglutaminaseinhibitoren auch wie folgt dargestellt werden:

(Z¹)(Z²)C=C(Z³HCO)ₘ-C₂H₄-Grundgerüst

worin m und Z¹, Z² und Z³ die hierin beschriebene Bedeutung haben, wobei jedoch zumindest einer der Reste Z¹, Z² und Z³ eine elektronenziehende und zur Konjugation befähigte Gruppe darstellt.

Weist die akzeptorsubstituierte Doppelbindung zwei oder drei von Wasserstoff verschiedene Reste auf, welche als Z¹, Z² und Z³ bezeichnet werden, so ist bevorzugt, wenn die gemittelte Elektronegativität der zwei bzw. drei Reste ≥ 2,20, bevorzugt ≥ 2,40 und insbesondere bevorzugt ≥ 2,60 beträgt. Bei der Berechnung der Elektronegativität wir der zum Gründgerüst führende Linker in Form der Ethylengruppe oder der Carbonylethylengruppe nicht berücksichtigt.

Das Grundgerüst ist vorzugsweise ein Peptid aus 2 bis 20 Aminosäuren, wobei die Aminosäuren auch nicht-proteinogene Aminsäuren umfassen oder ein Peptidomimetikum aus 2 bis 20 Aminosäuren, wobei im Peptidomimetikum 1 bis 10 vorzugsweise 1 bis 5 und insbesondere bevorzugt 1 bis 3 Aminosäuremimetika enthalten sein können. Ferner enthält das Gründgerüst vorzugsweise 1 bis 19 Amidbindungen, weiter bevorzugt 1 bis 10 Amidbindungen, noch weiter bevorzugt 1 bis 6 Amidbindungen und insbesondere bevorzugt 1 bis 4 Amidbindungen.

Die mit mindestens einer Elektronen wegziehenden Gruppe substituierte Vinylgruppe (Michael-Akzeptor-Gruppe) scheint eine wesentliche Einheit der TransglutaminaseInhibitoren zu sein und führt in Kombination mit dem peptidischen oder zumindest peptidähnlichen Grundgerüst zu potenten Transglutaminase-Inhibitoren. Es hat sich überraschend herausgestellt, dass diese Kombination von einem vorzugsweise peptidischen, peptidähnlichen oder peptidomimetischen Gründgerüst aus vorzugsweise nur wenigen Aminosäuren oder Aminosäurenderivaten oder -analoga zusammen mit der die akzeptorsubstituierte Doppelbindung tragenden Seitengruppe eine höhere Aktivität als auch Selektivität im Vergleich zu bekannten Inhibitoren mit Michael-Systemen aufweist. Somit scheint nicht nur die Anwesenheit eines Michael-Akzeptorsystems bzw. einer akzeptorsubstituierten Doppelbindung wichtig zu sein, sondern auch dessen konkreter Aufbau. Es hat sich als sehr vorteilhaft erwiesen, wenn die Seitengruppe mit akzeptorsubstituierter Doppelbindung bzw. mit Michael-System bioisoster zum Glutamin ist.

Als Grundgerüst für das mindestens eine Michael-System sind Peptide, Peptidderivate sowie Peptidomimetika geeignet. Das peptidische oder peptidähnliche Grundgerüst kann aus zwei bis 20 Aminosäuren oder Mimetika für Aminosäuren bestehen, welche über vorzugsweise Peptidbindungen oder beispielsweise auch Esterbindungen, Carbonatbindungen, Urethanbindungen, Carbamatbindungen und/oder Harnstoffbindungen miteinander verknüpft sind. Das das Michael-System tragende Grundgerüst sollte mindestens über eine zum Kohlenstoffatom, der die Seitenkette mit dem Michael-System trägt, vicinale Carbonylgruppe verfügen.

Wie in der nachfolgenden Struktur gezeigt, ist überraschend gefunden worden, dass für die inhibitorische Potenz der erfindungsgemäßen Transglutaminaseinhibitoren anscheinend drei Merkmale wesentlich sind: zum einen das Vorhandensein einer elektrophilen akzeptorsubstituierten Doppelbindung, welche zudem über einen Ethylen-Linker oder einen Carbonylethylen-Linker an ein Gründgerüst gebunden ist und zum dritten das Grundgerüst eine peptidähnliche oder proteinogene Struktur aufweist.

Die erfindungsgemäße Gruppe mit akzeptorsubstituierter Doppelbindung hat folgende allgemeine Struktur: 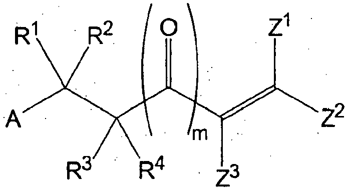 wobei die Verbindung mindestens ein akzeptorsubstituiertes Olefin mit den Resten Z¹, Z² und Z³ aufweist, welches über eine Ethylengruppe mit den Resten R¹, R², R³ und R⁴ oder über eine Carbonylethylengruppe mit den Resten R¹, R², R³ und R⁴ an eine mindestens sekundär substituierte Gruppe A gebunden ist, wobei
A einen Peptidrest, ein Peptidderivat oder einen peptidomimetischen Rest darstellt, und mindestens zwei Aminosäuren und/oder eine Amidbindung aufweist.
**m** für 0 oder 1 steht;
die Reste **Z¹**, **Z²**, **Z³** unabhängig voneinander für folgende Gruppen stehen: -H, -CO-(C₁-C₆-Alkyl), -CO-R⁶, -CO-R⁷, -CO-(C₁-C₆-Halogenalkyl), -CO-(C₃-C₁₀-Heteroaryl), -CO-(C₆-C₁₅-Aryl), -COO-(C₁-C₆-Halogenalkyl), -COO-(C₃-C₁₀-Heteroaryl), -COO-(C₆-C₁₅-Aryl), -COO-(C₁-C₆-Alkyl), -COO-R⁸, -COO-R⁹, -CN, -COOH, -CO-NH(C₁-C₆-Alkyl), -CO-N(C₁-C₆-Alkyl)(C₁-C₆-Alkyl), -CO-NR¹⁰R¹¹, -CO-NH₂, -CO-N(CR¹²R¹³R¹⁴)(CR¹⁵R¹⁶R¹⁷), -NO₂, -CS-(C₁-C₆-Alkyl), -CS-R¹⁸, -CS-R¹⁹, -CS-O-(C₁-C₆-Alkyl), -CS-O-R²⁰, -CS-O-R²¹, -CS-N(C₁-C₆-Alkyl)(C₁-C₆-Alkyl), -CS-NR²²R²³, -C^{S}₋NH₂, -CS-N(CR²⁴R²⁵R²⁶)(CR²⁷R²⁸R²⁹), -SO-R³⁰, -SO-R³¹, -SO₂-R³² -SO₂-R³³, -SO-CR³⁴R³⁵R³⁶, -SO-CR³⁷R³⁸R³⁹, -SO₂-CR⁴⁰R⁴¹R⁴², -SO₂-CR⁴³R⁴⁴R⁴⁵, -SO-N(C₁-C₆-Alkyl)(C₁-C₆-Alkyl), -SO-NR⁴⁶R⁴⁷, -SO-NH₂, -SO-N(CR⁴⁸R⁴⁹R⁵⁰)(CR⁵¹R⁵²R⁵³), -SO₂-N(C₁-C₆-Alkyl)(C₁-C₆-Alkyl), -SO₂-NR⁵⁴R⁵⁵, -SO₂-NH₂, -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, -O-P(O)(OH)₂, -O-P(O)(OR⁶⁹)(OR⁷⁰), -O-P(O)(O-C₁-C₆-Alkyl)(O-C₁-C₆-Alkyl), -P(O)(OR⁷¹)(OR⁷²), -P(O)(O-C₁-C₆-Alkyl)(O-C₁-C₆-Alkyl), -CF₂-P(O)(OR⁷³)(OR⁷⁴), -CF₂-P(O)(O-C₁-C₆-Alkyl)(O-C₁-C₆-Alkyl), wobei zumindest einer der Reste Z¹, Z², Z³ von Wasserstoff verschieden ist;
die Reste **Z¹** und **Z²** zusammen auch einen Rest -CO-O-CO-CH₂-, -CO-O-CH₂-CH₂- bedeuten können.
die Reste **Z²** und **Z³** zusammen auch einen Rest -CO-Z'-CH₂-, -CO-O-CH₂-, -CO-CH₂-CH₂-, -CO-O-CO-, oder -CO-NH-CO- bedeuten können, worin
Z**'** für eine der folgenden Gruppen steht: -CH₂-, -CF₂-, -C₂H₄-, -CF₂-CH₂-, -CH₂-CH₂-, -O-, -O-CH₂-, -NH- oder -NH-CH₂-;
und die Reste **R¹ - R⁷⁴** unabhängig voneinander eine der weiter unter beschriebenen Gruppen bedeuten.

R¹, R², R³ und R⁴ bedeuten Wasserstoff so dass sich folgende allgemeinen Formel [G] ergibt, worin die Reste A und Z¹ bis Z³ die obige Bedeutung haben und m für 0 oder 1 steht.

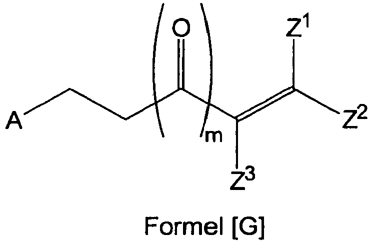

Für den Fall, dass m = 1 ist, ist nicht zwingend, dass Z¹ oder Z² oder Z³ eine zur Konjugation befähigte Gruppe darstellt, da bereits eine Carbonylgruppe in Konjugation zur Doppelbindung steht. In einem solchen Fall ist bevorzugt, wenn zumindest einer der Reste Z¹, Z², Z³ eine elektronenziehende Gruppe bedeutet. Bevorzugte Verbindungen sind jedoch auch insbesondere solche, worin m = 1 ist und zumindest einer der Reste Z¹, Z², Z³ vorzugsweise Z¹ oder Z² eine weitere zur Konjugation befähigte Gruppe bedeutet.

Eine bevorzugte Form der akzeptorsubstituierten Doppelbindung ist ein Michael-System aus einer Carbonylfunktion in Konjugation mit einer Doppelbindung, welches die folgende allgemeine Struktur [A] hat: 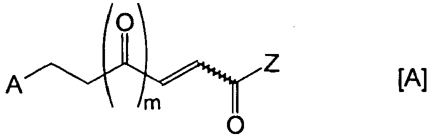 worin
Z eine Hydroxygruppe, Aminogruppe, C₁-C₆-Alkylaminogruppe (-NH(C₁-C₆-Alkyl)), C₁-C₆-Dialkylaminogruppe (-N(C₁-C₆-Alkyl)(C₁-C₆-Alkyl)), C₁-C₆-Alkoxygruppe (-O-(C₁-C₆-Alkyl)), C₁-C₆-Alkylgruppe, C₁-C₆-Halogenalkylgruppe, C₃-C₁₀-Heteroarylgruppe oder eine C₆-C₁₅-Arylgruppe bedeutet und
m für 0 oder 1 steht, und
A den Rest der Verbindung bezeichnet, an den das Michael-System gebunden ist.

A ist also das peptidische oder das daraus abgeleitete peptidähnliche Grundgerüst, wobei A jedoch keine Aminosäuren oder Peptidbindungen enthalten muss, sondern auch ein Kohlenstoffgrundgerüst optional mit Heteroatomen, Aromaten, Heteroaromaten, Heterocyclen oder Carbocyclen sein kann und optional Aminosäureanaloga oder Peptidanaloga aufweisen kann. Der Rest A kann bis zu 30 vorzugsweise 20 Heteroatome wie N, S, O, P, F, Cl, Br enthalten und bis zu 80, vorzugsweise bis zu 70, weiter bevorzugt bis zu 60 und insbesondere bevorzugt bis zu 50 Kohlenstoffatome umfassen, welche in linearen, verzweigten, gesättigten, ungesättigten sowie substituierten Kohlenstoffketten als auch Kohlenstoffcyclen oder Heterocyclen enthalten sein können.

Der Rest A umfasst bevorzugt ein sekundär substituiertes Atom, vorzugsweise ein Kohlenstoffatom, woran die olefinische Seitenketten gebunden ist. An dieses vorzugsweise sekundär substituierte Kohlenstoffatom ist vorzugsweise eine Carbonylgruppe (C=O) gebunden. Das die Seitenkette tragende Kohlenstoffatom des Restes A kann auch tertiär substituiert sein und ist vorzugsweise an drei oder auch vier Kohlenstoffatome gebunden, wobei eines dieser Kohlenstoffatome zur Seitenkette gehört. Ferner kann die Seitenkette auch an eine Schwefel oder Stickstoffatom des Restes A gebunden werden.

Die Zick-Zack-Bindung bedeutet, dass beide isomere Formen (Z-Isomer, E-Isomer) durch die allgemeine Formel [A] abgedeckt werden, also folgende beide Isomere umfasst sind:

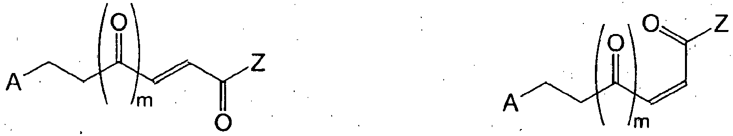

Unter dem hierin verwendeten Begriff "C₁-C₆-Alkylgruppe" werden folgende Reste verstanden: -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₆H₁₃, -C₂H₄-CH(CH₃)₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃. -CH(CH₃)-C(CH₃)₃, -cyclo-C₃H₅, -cyclo-C₄H₇, -cyclo-C₅H₉ und -cyclo-C₆H₁₁.

Bevorzugt sind die Reste -CH₃, -C₂H₅, -C₃H₇, -CH(CH₃)₂, -C₄H₉, -CH₂-CH(CH₃)₂, -CH(CH₃)-C₂H₅, -C(CH₃)₃, und -C₅H₁₁. Insbesondere bevorzugt sind die Reste -CH₃, -C₂H₅, -C₃H₇ und -CH(CH₃)₂.

Als C₁-C₆-Alkoxygruppe wird eine -O-(C₁-C₆-Alkylgruppe) bezeichnet und C₁-C₆-Alkylaminogruppe steht für -NH-(C₁-C₆-Alkylgruppe). C₁-C₆-Dialkylaminogruppe bezeichnet eine -N[(C₁-C₆-Alkylgruppe)(C₁-C₆-Alkylgruppe)] Gruppe, wobei die beiden Alkylreste gleich oder verscheiden voneinander sein können. Als C₁-C₆-Alkylgruppe sind -CH₃, -C₂H₅, -C₃H₇, -CH(CH₃)₂ und -C₄H₉ bevorzugt. Insbesondere bevorzugt sind -O-CH₃, -O-C₂H₅, -O-C₃H₇, -O-CH(CH₃)₂, -O-C(CH₃)₃

Als C₁-C₆-Halogenalkylgruppe wir eine C₁-C₆-Alkylgruppe bezeichnet, worin ein, zwei, drei, vier, fünf bis hin zu sämtlichen Wasserstoffatomen durch Halogenatome (-F, -Cl, -Br, -I) ersetzt sind. Bevorzugt sind: -NHCH₃, -NHC₂H₅, -NHC₃H₇, -NH-cyclo-C₃H₅, -NHCH(CH₃)₂, -NHC(CH₃)_{3,} -N(CH₃)₂, -N(C₂H₅)₂, -N(C₃H₇)₂, -N(cyclo-C₃H₅)₂.

C₆-C₁₅-Arylgruppe bezeichnet vorzugsweise eine Phenyl-, Tolyl-, Benzyl-, Styryl-, Dimethylphenyl-, Trimethylphenyl-, Dimethylbenzyl-, Trimethylbenzyl-, oder Naphthylgruppe, welche mit bis zu 5 Substituenten ausgewählt aus der Gruppe der Reste R⁶ bis R⁵⁰ substituiert sein kann. Die Substituenten R⁶ bis R⁵⁰ werden weiter unten definiert.

Die Gruppe Z kann folgende Reste bedeuten: -CR⁷R⁸R⁹, -CR¹⁰R¹¹-CR¹²R¹³R¹⁴, -CR¹⁵R¹⁶-CR¹⁷R¹⁸-CR¹⁹R²⁰R²¹, , -O-CR²²R²³R²⁴, -O-CR²⁵R²⁶-CR²⁷R²⁸R²⁹, -O-CR³⁰R³¹-CR³²R³³-CR³⁴R³⁵R³⁶, -NH-CR³⁷R³⁸R³⁹ , -NH-CR⁴⁰R⁴¹-CR⁴²R⁴³R⁴⁴, -NH-CR⁴⁵R⁴⁶-CR⁴⁷-CR⁴⁸-CR⁴⁹R⁵⁰R⁵¹ wobei die Substituenten R⁷ bis R⁴⁵ unabhängig voneinander aus der unter angegebenen Liste ausgewählt werden können.

C₃-C₁₀-Heteroarylgruppe steht für cyclische, bicyclische oder tricyclische aromatische Reste mit mindestens drei Kohlenstoffatomen und mindestens einem Heteroatom ausgewählt aus Stickstoff, Sauerstoff und/oder Schwefel.

Bevorzugte Beispiele für C₃-C₁₀-Heteroarylgruppen sind: 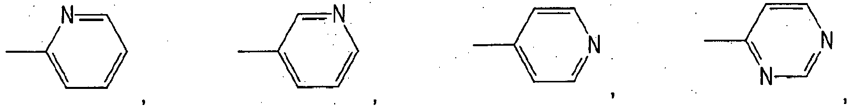 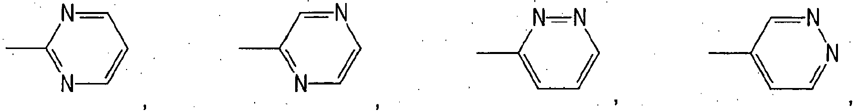 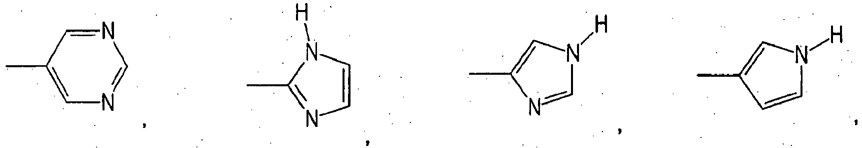 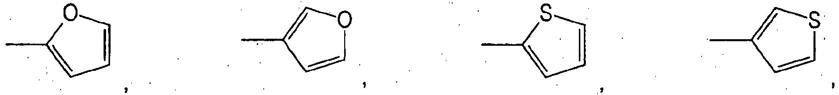 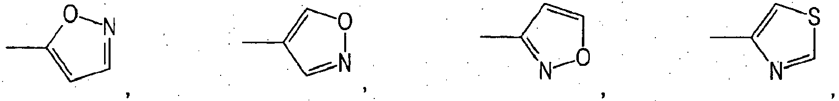 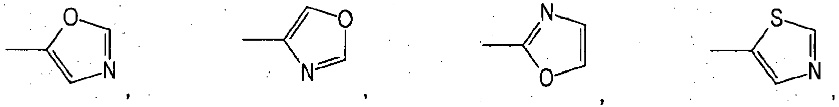 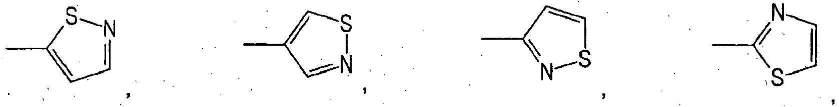 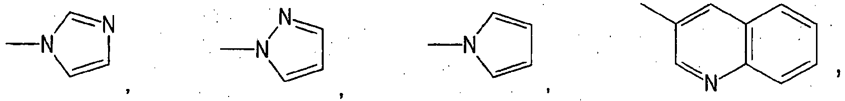 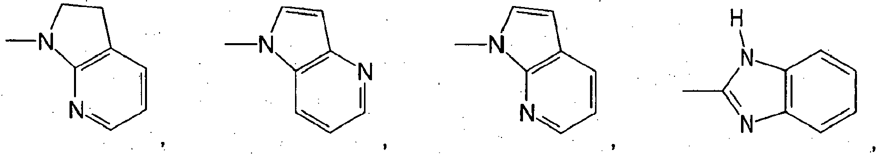 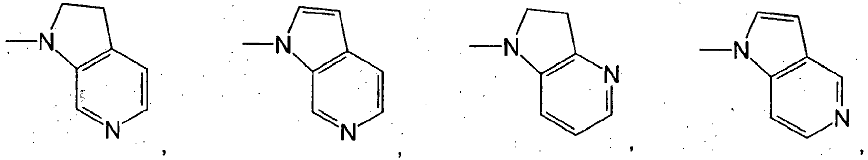 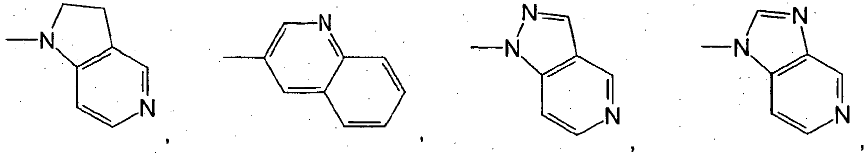 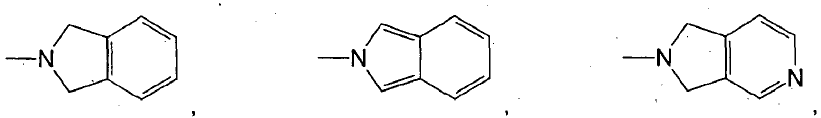 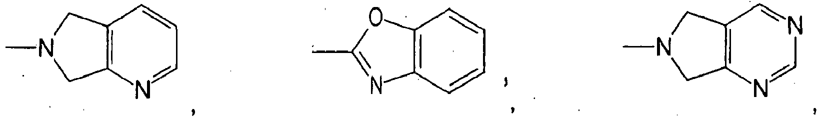 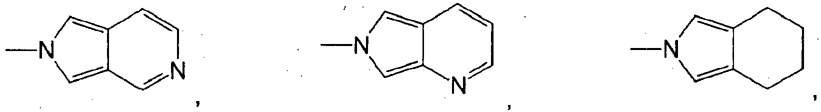 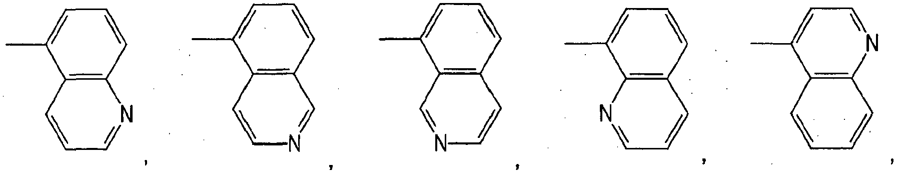 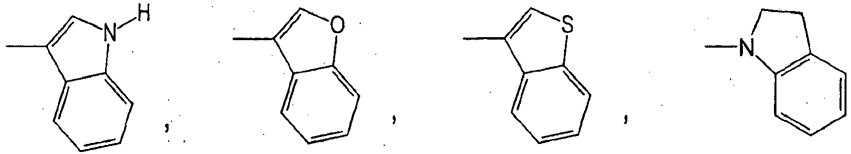 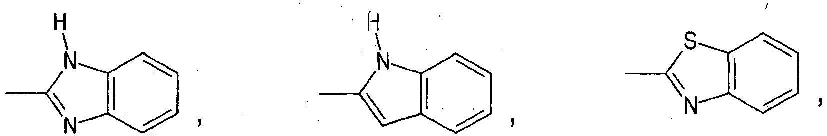 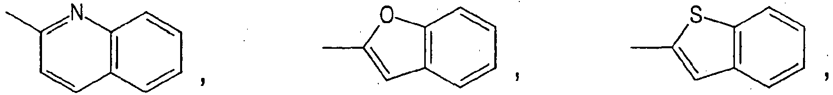 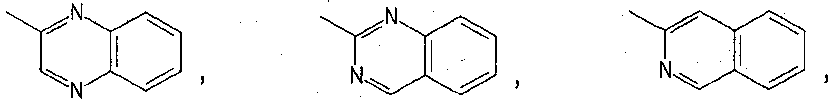 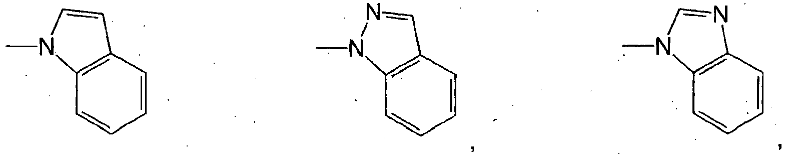 wobei diese Gruppen mit bis zu 5 Substituenten R⁵² bis R⁵⁶ oder R⁸⁰ bis R⁸⁴ ausgewählt aus der unter bezeichneten Liste substituiert sein können.

Weitere bevorzugte Michael-Systeme haben folgende Struktur: 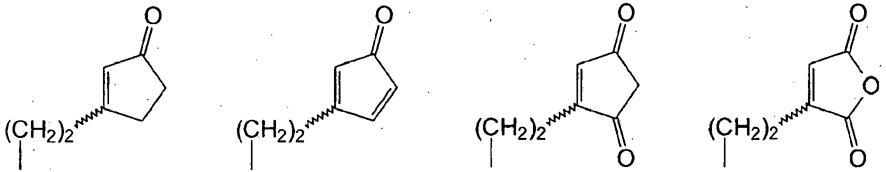 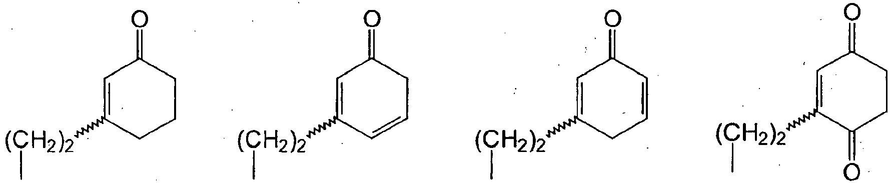 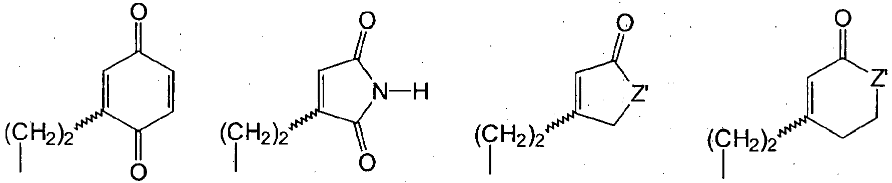 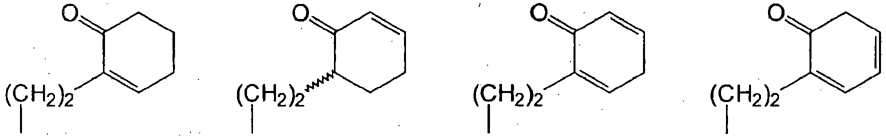 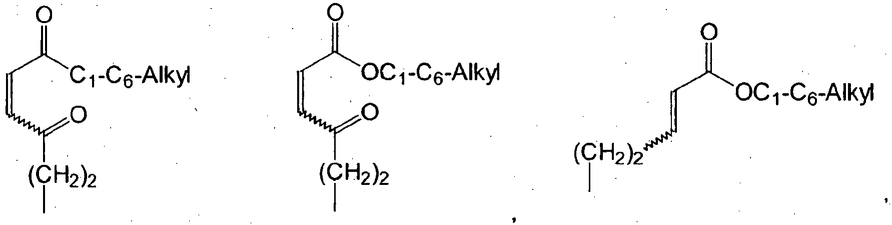 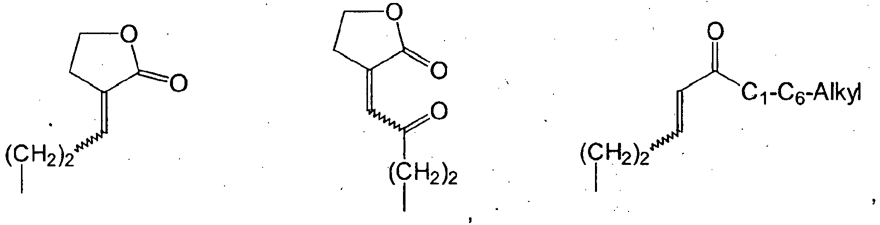 worin
Z' für eine der folgenden Gruppen steht: -CH₂-, -CF₂-. -C₂H₄-, -CF₂-CH₂-, -CH₂-CH₂-, -O-, -O-CH₂-, -NH- oder -NH-CH₂-;

Die gezackte Bindung soll symbolisieren, dass die Konfiguration der Doppelbindung sowohl zusammen "Z" als auch entgegen "E" sein kann.

Betrachtet man die besonders bevorzugten Michael-Systeme der folgenden Struktur: 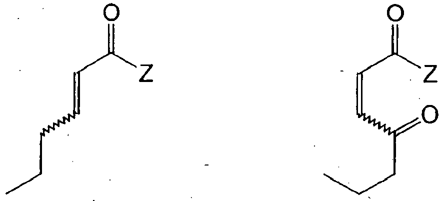 worin Z die hierin definierte Bedeutung hat, so stellt das Michael-System die Seitenkette einer Aminosäure oder eines Aminosäurederivates oder -analogons dar, wobei die Aminosäure einen Teil des peptidischen oder peptidähnlichen Rests des Moleküls bildet.

Das gesamte Molekül kann schematisch wie folgt dargestellt werden: 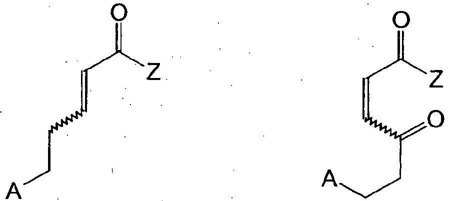 worin A das Peptid, Peptidderivat oder Peptidomimetikum darstellt und .die Verbindung wie folgt wiedergegeben werden könnte:
R⁷ oder X oder 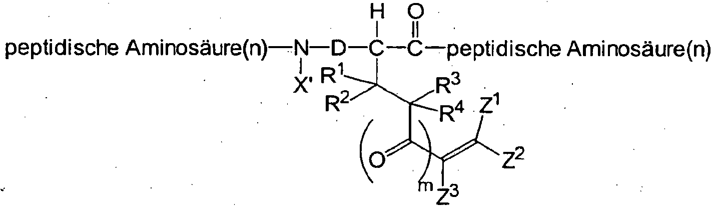 oder
R7 oder X oder 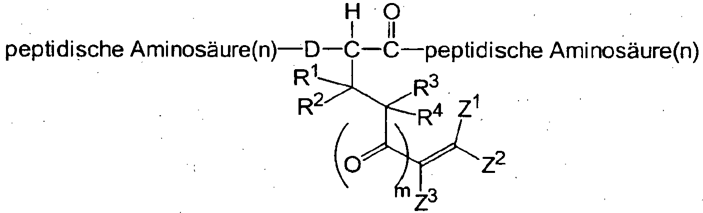 oder Y
oder
R⁷ oder X oder 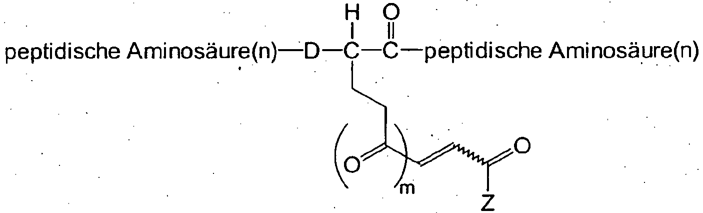 oder
X oder 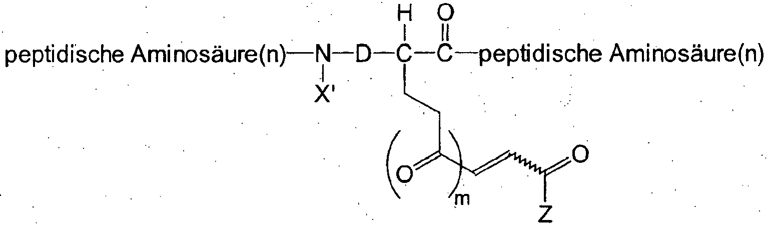 oder Y worin D für eine chemische Bindung oder für eine der folgenden Gruppen stehen kann: -CH₂-, -CL¹L²-, -CF₂-, -C₂H₄-, -CH₂-CF₂-, -CF₂-CH₂-, -CH=CH-, -CH(OH)-CH₂-, -C(=O)-CH₂-, -CH₂-NH-, -CH₂-O-, -CH(OH)-CH₂₋NH-, -CHQ-C₂H₄-, -CHQ-CH₂-CF₂-, -CHQ-CF₂-CH₂-, -CHQ-CH=CH-, -CHQ-CH(OH)-CH₂-, -CHQ-C(=O)-CH₂-, -CHQ-CH₂-NH-, -CHQ-CH₂-O--CHQ-P(=O)(OH)-NH-, -CHQ-P(=O)(OH)-O-, -CHQ-P(=O)(OH)-S-, -CHQ-P(=O)(OH)-CH₂-, oder -CHQ-CH(OH)-CH₂-NH-, wobei L¹, L² und Q unabhängig voneinander einen Seitenkettenrest einer natürlichen Aminosäure oder einen Rest -R⁵⁹, -R⁶⁰, -R⁶¹ bedeuten, und die Reste R¹ bis R⁴ und R⁵⁹, R⁶⁰ R⁶¹, X, X' und Y die Bedeutung haben, wie weiter unter definiert. Die Variable m kann für 0 oder 1 stehen.

Für den Fall dass D eine chemische Bindung darstellt, ergibt sich unter anderem folgende Formel:
X oder 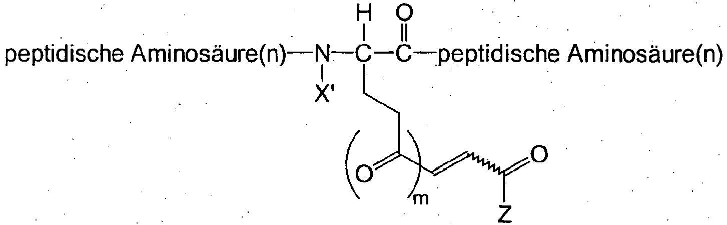 oder Y

Die Gruppe A stellt den Grundkörper des. Peptids, Peptidderivats oder Peptidomimetikums dar, an welchen zumindest ein akzeptorsubstituiertes Olefin (MS) bzw. ein Michael-System (MS) der hierin bezeichneten Art gebunden ist.

Es ist durchaus möglich, dass eine peptidische oder peptidähnliche Verbindung auch zwei oder drei gleiche oder unterschiedliche akzeptorsubstituierte Olefine oder Michael-Systeme aufweist. Die Michaelakzeptor-tragende(n) Seitenkette(n) bzw. die Seitenkette(n) mit akzeptorsubstituiertem Olefin kann/können an beliebiger Position von A angebunden sein.

A stellt in der einfachsten Ausführungsform vorzugsweise ein Dipeptid bzw. Dipeptidderivat dar, symbolisiert durch die beidem Aminosäuren AS1 und AS2, welche über eine Peptidbindung (Amidbindung) miteinander verbunden sind: AS1-AS2

AS1 oder AS2 oder AS1 und AS2 tragen als Seitenkette ein akzeptorsubstituiertes Olefin (MS) vorzugsweise ein Michaelsystem und das Dipeptid (Dipeptidderivat) weist am C-terminalen Ende vorzugsweise die Gruppe Y und am N-Terminus vorzugsweise die Gruppen X und X' auf.

Die Peptide, Peptidderivate und auch die Peptidomimetika werden in der üblichen Schreibweise vom N-Terminus in Richtung C-Terminus wiedergegeben.

Das Dipeptid kann daher wie folgt dargestellt werden:

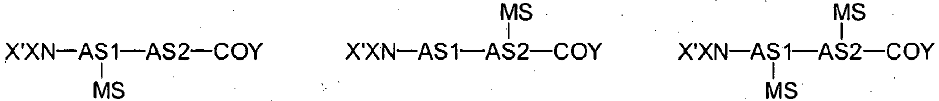

Erfindungsgemäß ist auch die Verwendung von Tri-, Tetra-, Penta-, Hexa-, Septa-, Octa-, Nona- und Decapeptiden sowie bis hin zu Peptiden aus bis zu 20 Aminosäuren oder aminosäurenähnlichen Verbindungen möglich. Bevorzugt sind jedoch Tripeptide, Tetrapeptide und Pentapeptide und insbesondere bevorzugt sind Tetrapeptide.

Die Tripeptide (Tripeptidderivate) können folgende Struktur aufweisen:

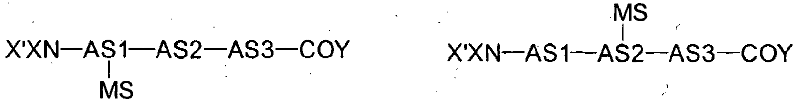

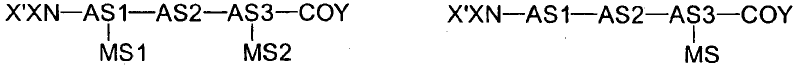

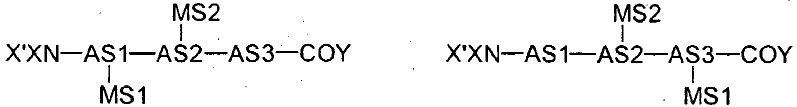

Die Tetrapeptide (Tetrapeptidderivate) können folgende Struktur aufweisen:

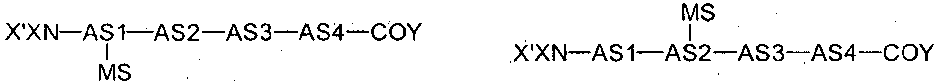

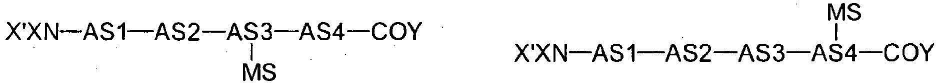

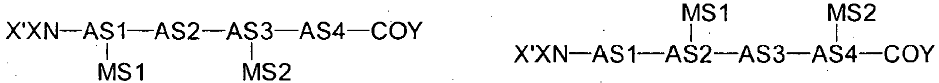

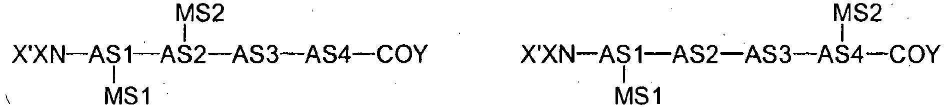

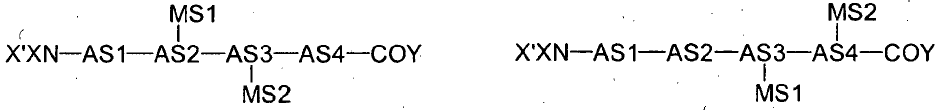

Die Pentapeptide (Pentapeptidderivate) können folgende Struktur aufweisen:

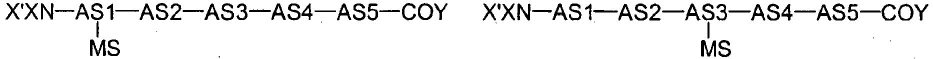

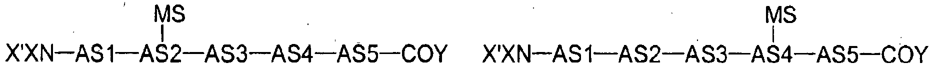

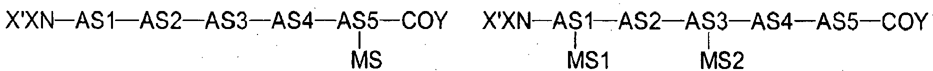

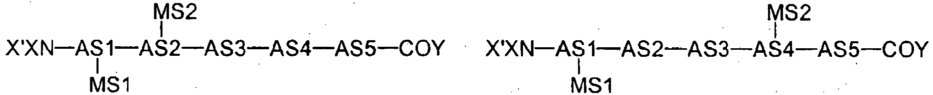

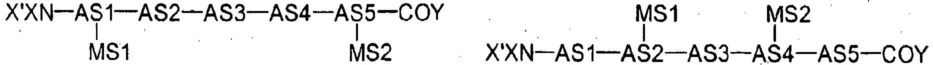

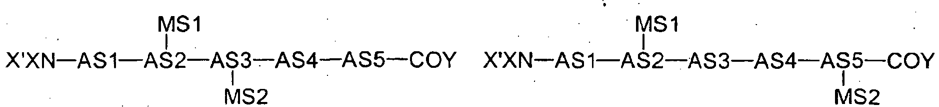

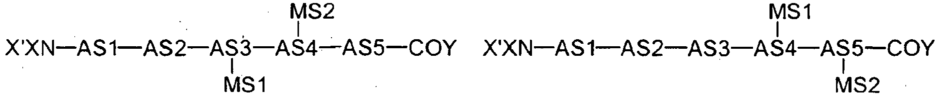

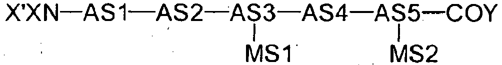

MS1 und MS2 können zwei gleiche oder auch zwei unterschiedliche akzeptorsubstituierte Olefine sein und die Tri-, Tetra- und Pentapeptide können natürlich auch mehr als zwei akzeptorsubstituierte Olefine und vorzugsweise mehr als zwei Michael-Systeme tragen.

Pro Aminosäure ist jedoch bei allen Peptiden, Peptidderivaten oder Peptidomimetika vorzugsweise maximal ein akzeptorsubstituiertes Olefin oder Michael-System vorhanden.

Als Aminosäuren (AS, AS1, AS2, .... AS19, AS20) können die natürlichen Aminosäuren wie beispielsweise Alanin (Ala, A), Cystein (Cys, C), Asparaginsäure (Asp, D), Glutaminsäure (Glu, E), Phenylalanin (Phe, F), Glycin (Gly, G), Histidin (His, H), Isoleucin (Ile, I), Lysin (Lys, K), Leucin (Leu, L), Methionin (Met, M), Asparagin (Asn, N), Prolin (Pro, P), Glutamin (Gln, Q), Arginin (Arg, R), Serin (Ser, S), Threonin (Thr, T), Valin (Val, V), Tryptophan (Trp, W) und Tyrosin (Tyr, Y) verwendet werden als auch Aminosäurederivate wie beispielsweise 4-Hydroxyprolin, N,N,N-Trimethyllysin, 3-Methylhistidin, 5-Hydroxylysin, O-Phosphoserin, γ-Carboxyglutamat, ε-N-Acetyllysin, ω-N-Methylarginin, Citrullin oder Ornithin.

Neben den L-Aminosäuren können zudem auch die D-Aminosäuren sowie Kombinationen aus L- und D-Aminosäuren in einem Peptid oder Peptidderivat verwendet werden.

Unter dem Begriff "Aminosäure" oder "Aminosäuren" sollen nicht nur die natürlichen Aminosäuren oder Derivate davon verstanden werden, sondern ganz allgemein eine chemische Verbindung, welche zumindest eine Aminofunktion oder zumindest eine Ammoniumfunktion und zumindest eine Carbonsäurefunktion oder zumindest eine Carboxylatfunktion aufweist. Derartige Verbindungen sollten oder müssen in der Lage sein, eine Betain-Struktur zu bilden und/oder sollten oder müssen in der Lage sein, Peptidbindungen bzw. Amidbindungen auszubilden.

Somit fallen unter den Begriff "Aminosäure" oder "Aminosäuren" beispielsweise auch Verbindungen der Formel 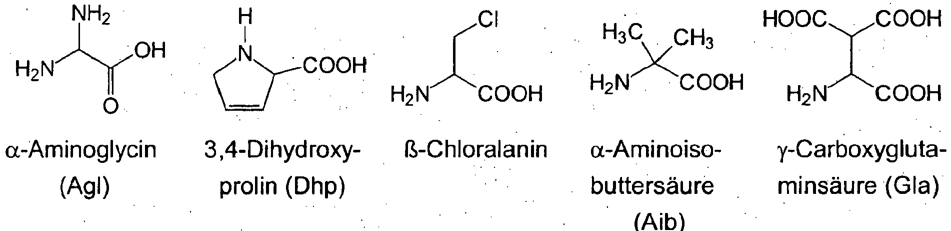 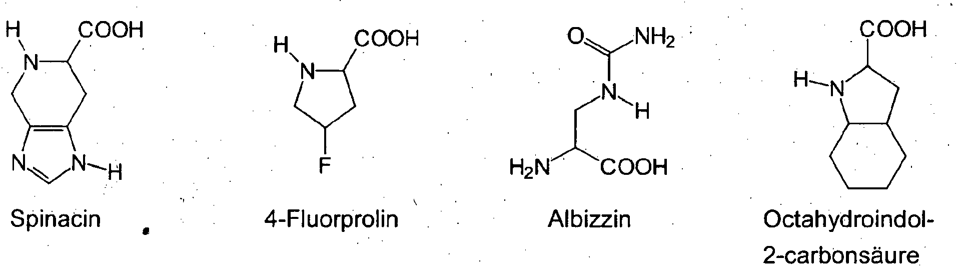 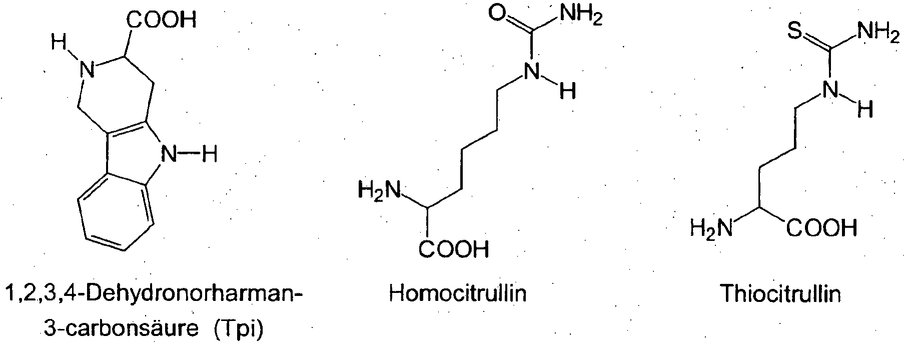 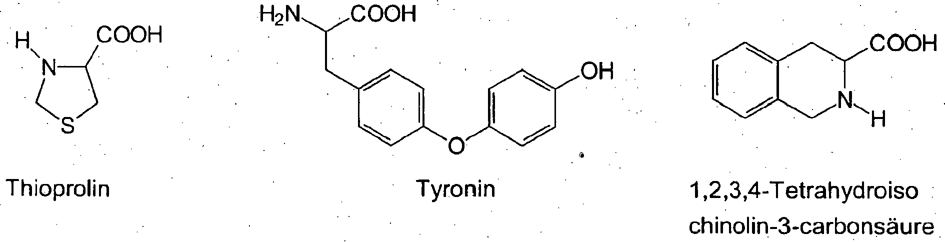 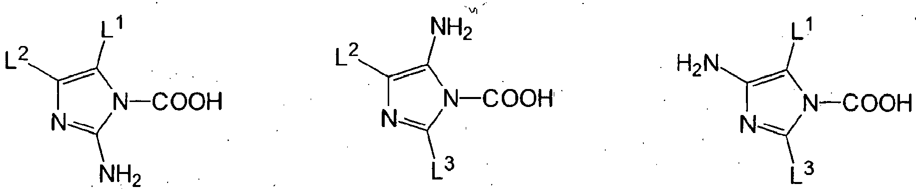 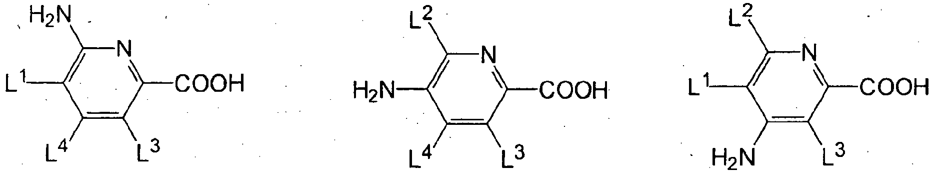 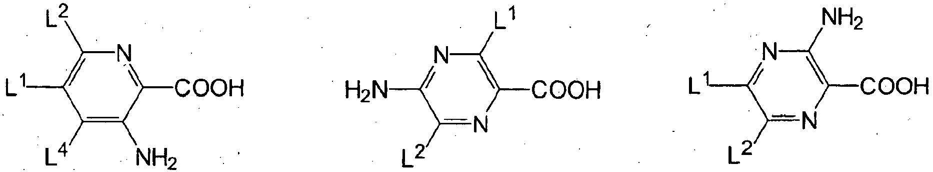 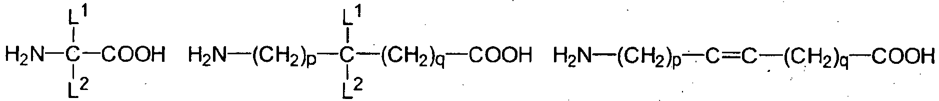 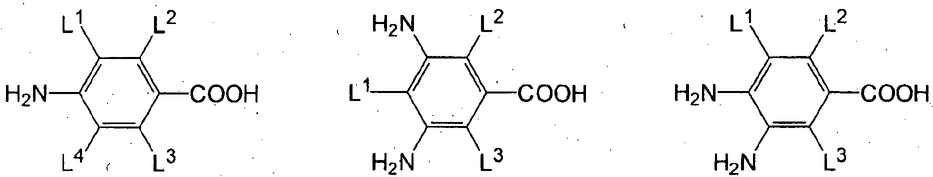 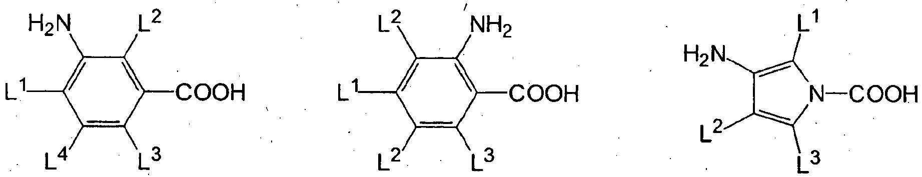 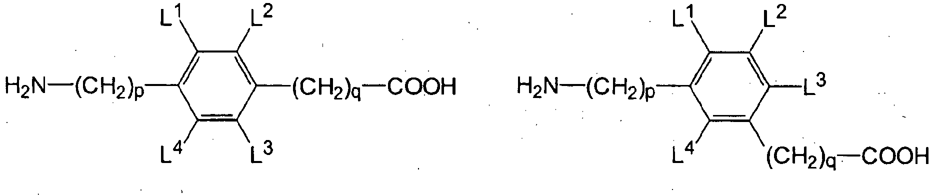 worin
p und q unabhängig voneinander 0, 1, 2, 3, 4, 5 bedeuten,
L¹, L², L³, L⁴ unabhängig voneinander einen Seitenkettenrest einer natürlichen Aminosäure oder auch einen Rest -R⁵⁷, -R⁵⁹, -R⁶⁰, -R⁶¹, -CR⁶²R⁶³R⁶⁴, -CR⁶⁵R⁶⁶-CR⁶⁷R⁶⁸R⁶⁹, -CR⁷⁰R⁷¹-CR⁷²R⁷³-CR⁷⁴R⁷⁵R⁷⁶ bedeuten, worin R⁵⁷ bis R⁷⁶ die unten angegebenen Bedeutungen haben. In einer Ausführungsform bedeuten die Reste L¹ bis L⁴ Wasserstoff.

Bei den Peptidomimetika wird vorzugsweise die Amidbindung (-NH-CO-) durch eine andere Funktionalität ersetzt. Beispielsweise kann der Amidstickstoff alkyliert werden, so dass die Peptidomimetika folgendes Strukturfragment enthalten:

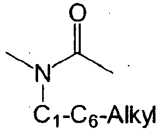

Als Animosäureanaloga, welche als Aminosäurebaustein im peptidischen Grundgerüst eingesetzt werden können, sind z.B. Thiocarbonylaminosäuren, β-Aminosäuren, γ-Aminosäuren oder δ-Aminosäuren zu nennen, wobei grundsätzlich nicht proteinogene Aminosäuren als Animosäureanaloga bezeichnet werden. Weitere Beispeile für Animosäureanaloga sind:

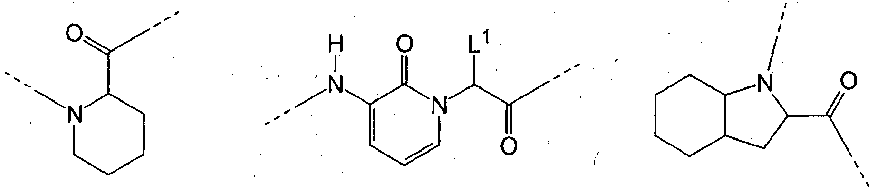

Des weiteren können die Peptidomimetika Aminosäuren der folgenden Form enthalten, welche auch als Dipeptid-Analoga bezeichnet werden:

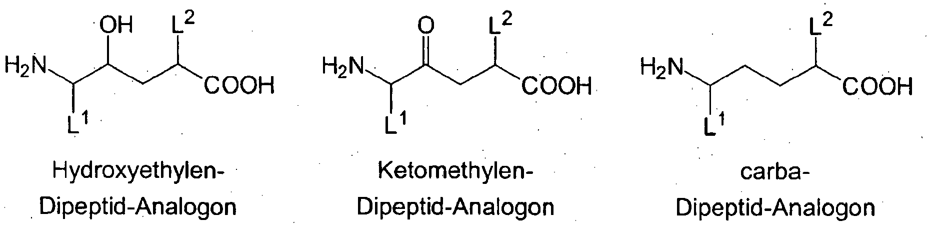

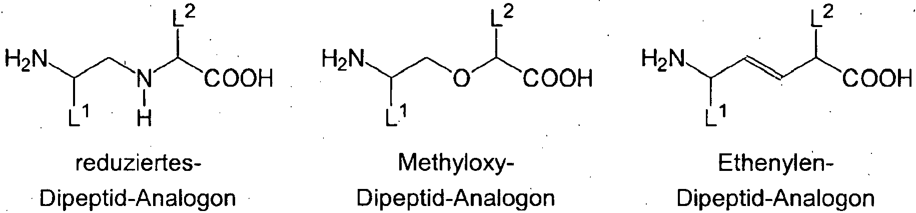

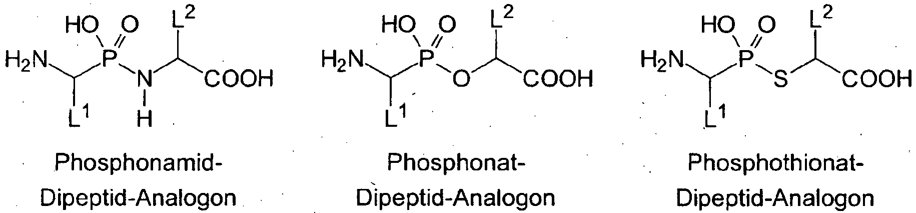

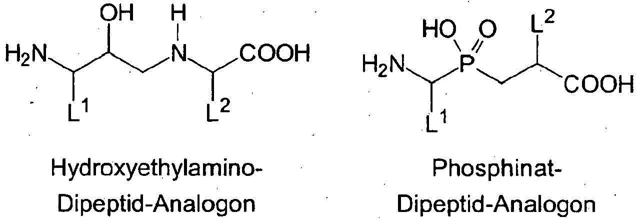

Wobei die Reste L¹ und L² unabhängig voneinander die oben angegebene Bedeutung wie bei den "Aminosäuren" haben. Die vorgenannten Verbindungen werden als Dipeptid-Analoga bezeichnet und sollen hierin jedoch als eine Aminosäure (AS) gerechnet werden. Ein hierin definierter Rest aus 4 Aminosäuren kann somit 4 der vorgenannten Dipeptid-Analoga umfassen und nicht nur maximal 2 dieser Dipeptid-Analoga, da ein Dipeptid-Analogon nicht als 2 Aminosäuren sondern nur als eine Aminosäure zu zählen ist.

Die Stereochemie bei den natürlichen Aminosäuren, den unnatürlichen Aminosäuren als auch bei den Peptid-Analoga bzw. danach die Stereochemie in den erfindungsgemäßen Peptiden oder Peptidomimetika kann L- als auch D- bzw. R als auch S sein, so dass enantiomere Formen als auch diastereomere Formen als auch Gemische von Enantiomeren und/oder Diastereomeren unter die Definition der hierin offenbarten erfindungsgemäßen Peptide, Peptidderivate und Peptidomimetika fallen. Erfindungsgemäße Peptide sowie die Verwendung folgender erfindungsgemäßer Peptide als Transglutaminaseinhibitoren sind bevorzugt, wobei es sich bei dem Peptid oder Peptidomimetikum um eine Verbindung der folgenden allgemeinen Formel (I), (II) oder (III) handelt: 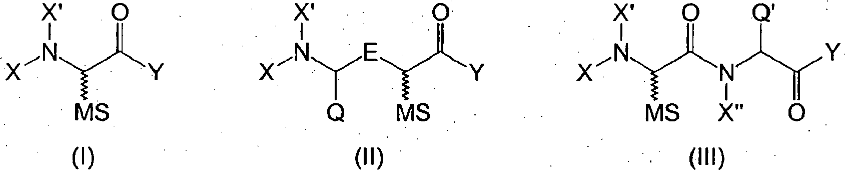 worin
MS das akzeptorsubstituierte Olefin vorzugsweise Michael-System folgender Struktur ist: 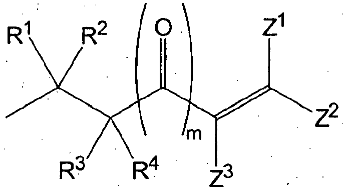 und
E folgende Gruppe bedeutet -CH₂-, -CF₂-, -C₂H₄-, -CH₂-CF₂-, -CF₂-CH₂-, -CH=CH-, -CH(OH)-CH₂-, -C(=O)-CH₂-, -CH₂-NH-, -CH₂-O-, -CH(OH)-CH₂-NH-, -P(=O)(OH)-NH-, -P(=O)(OH)-O-, -P(=O)(OH)-S-P(=O)(OH)-CH₂-, -CH(OH)-CH₂-NH-, -C(=O)-NH-, -C(=O)-O- oder -C(=O)-NX"-;
Q und Q' unabhängig voneinander einen Seitenkettenrest einer natürlichen Aminosäure darstellen, oder Q zusammen mit X' einen Propylenylrest bilden, oder Q' zusammen mit X" einen Propylenylrest bilden;
Y eine Hydroxygruppe, Aminogruppe, C₁-C₆-Alkylaminogruppe, C₁-C₆-Dialkylaminogruppe, C₁-C₆-Alkoxygruppe, C₆-C₁₉-Aryloxygruppe, C₁-C₆-Alkylgruppe, C₁-C₆-Halogenalkylgruppe, C₃-C₁₀-Heteroarylgruppe oder eine C₆-C₁₅-Arylgruppe bedeutet oder Y für einen über eine Amidbindung gebundenen Peptidrest aus bis zu 6 Aminosäuren steht, dessen C-terminale Carbonylfunktion eine Hydroxygruppe, Aminogruppe, C₁-C₆-Alkylaminogruppe, C₁-C₆-Dialkylaminogruppe, C₁-C₆-Alkoxygruppe, C₁-C₆-Alkylgruppe, C₁-C₆-Halogenalkylgruppe, C₃-C₁₀-Heteroarylgruppe oder eine C₆-C₁₅-Arylgruppe trägt oder Y für einen peptidomimetischen Rest aus bis zu 60 Kohlenstoffatomen, bevorzugt aus bis zu 30 Kohlenstoffatomen steht und
X" für Wasserstoff oder eine C₁-C₆-Alkylgruppe steht; und
-NXX' eine Aminogruppe, -NH-CHO, C₁-C₁₀-Alkylaminogruppe, C₁-C₈-Alkyloxycarbonylaminogruppe, C₆-C₁₂-Aralkyloxycarbonylaminogruppe, C₁-C₁₀-Dialkylaminogruppe, C₂-C₆-Stickstoffheterocyclus oder eine C₃-C₅-Stickstoffheteroarylgruppe bedeutet oder die Gruppe -NXX' Teil eines peptidomimetischen Restes aus bis zu 60 Kohlenstoffatomen, bevorzugt aus bis zu 30 Kohlenstoffatomen ist
oder
X' für Wasserstoff oder eine C₁-C₆-Alkylgruppe steht; und
X für einen über eine Amidbindung gebundenen Peptidrest aus bis zu 6 Aminosäuren steht, dessen N-Terminus eine Aminogruppe, -NH-CHO, C₁-C₁₀-Alkylaminogruppe, C₁-C₈-Alkyloxycarbonylaminogruppe, C₆-C₁₂-Aralkyloxycarbonylaminogruppe, C₁-C₁₀-Dialkylaminogruppe, C₂-C₆-Stickstoffhetero-cyclus oder eine C₃-C₅-Stickstoffheteroarylgruppe;
wobei jede der C₁-C₆-Alkoxygruppen, C₁-C₆-Alkylgruppen, C₁-C₁₀-Alkylaminogruppen, C₁-C₈-Alkyloxycarbonylaminogruppe, C₆-C₁₂-Aralkyloxydarbonylaminogruppe, C₁-C₁₀-Dialkylaminogruppen, C₂-C₆-Stickstoffheterocyclen sowie C₃-C₅-Stickstoffheteroarylgruppen unabhängig voneinander mit bis zu 5 Resten ausgewählt aus R⁸⁰, R⁸¹, R⁸², R⁸³, R⁸⁴ substituiert sein kann.

Die Reste R¹ bis R⁸⁴ sowie die Reste L¹, L², L³, L⁴ bedeuten unabhängig voneinander folgende Gruppen:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂. -SC(CH₃)₃, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-Cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C2H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, . -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃; -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅. -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂; -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₅H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CHz-C(CH₃)₂-CH=CH₂, -C(CH₃)₂-CH₂-CH=CH₂, -CH₂-C(CH₃)=C(CH₃)₂. -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -C₆H₄-OCH₃, -C₆H₄-OH, -CH₂CH=CH-CH₂-CH=CH₂, -CH=CH-C₂H₄-CH=CH₂, -C≡C-CH(CH₃)-C≡CH, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH₂-C₆H₄-OCH₃, -CH=CH-CH(CH₃)-CH=CH₂, -CH=C(CH₃)-CH₂-CH=CH₂, -CH₂-CH(C≡CH)₂, -C(CH₃)=CH-CH₂-CH=CH₂, -CH=CH-CH=C(CH₃)₂, -CH(C=CH)-CH₂-C≡CH. -CH=CH-C(CH₃)=CH-CH₃, -CH=C(CH₃)-CH=CH-CH₃, -CH₂-C₆H₄-OH, -C(CH₃)=CH-CH=CH-CH₃, -CH=C(CH₃)-C(CH₃)=CH₂, -CH(C≡CH)-C≡C-CH₃, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, , -C₂H₄-C≡CH, -CH₂-C≡C-CH₃, -C≡C-C₂H₅, -C₃H₆-C≡CH, -C₂H₄-C≡C-CH₃, ₋CH₂-C≡C-C₂H₅, -C≡C-C₃H₇, -CH(CH₃)-C≡CH. -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -CH(CH₃)-C≡C-C≡CH, -C(C≡CH)₂-CH₃;
sowie stereoisomere Formen, E/Z-Isomere, Enantiomere, Enantiomerengemische, Diastereomere, Diastereomerengemische, Racemate, Tautomere, Anomere, Keto-Enol-Formen, Betainformen, Solvate, Hydrate als auch pharmakologisch verträgliche Salze der vorgenannten Verbindungen.

Absoluter Substanzschutz wird nicht beansprucht für die Verbindungen gemäß allgemeiner Formel (I), worin X und X' Wasserstoff bedeuten und Y eine Hydroxygruppe darstellt sowie für die Verbindung H-Phenylalanyl-[2-(4-ethoxycarbonyl-3-buten)-glycyl]-leucin-amid.

Insbesondere ist für die Reste R¹, R², R³, R⁴ bevorzugt, wenn keiner, einer, zwei, drei oder alle vier Reste Fluor bedeuten und die restlichen Reste Wasserstoff bedeuten.

Die erfindungsgemäßen Verbindungen mit m = 0 lassen sich herstellen, indem die Aminosäure Glu (Glutaminsäure) am C-Terminus und N-Terminus mit einer Schutzgruppe (PG1 und PG2) versehen wird (1), danach die Carboxylfunktion der Seitenkette zum Aldehyd reduziert wird (2) und der erhaltene Aldehyd in eine akzeptorsubstituierte elektrophile Doppelbindung überführt wird (3). Anschließend werden die Schutzgruppen entfernt und der N-Terminus falls gewünscht (optional) mit einem Peptidfragment oder Peptidomimetikum verlängert (4). Nach Aktivierung der C-terminalen Carbonylfunktion kann auch der C-Terminus optional mit einem Peptidfragment oder Peptidomimetikum verlängert werden (5). Der systematische Syntheseablauf ist in folgendem Schema dargestellt.

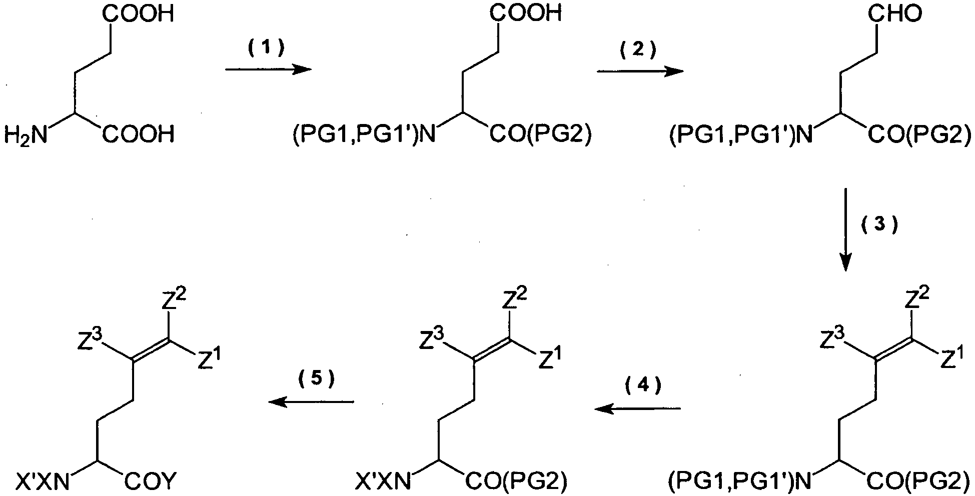

Alternativ kann auch zuerst das Grundgerüst ausgebildet werden und danach die akzeptorsubstituierte elektrophile Doppelbindung an der Seitenkette der Glutaminsäure erzeugt werden. Dazu wird ein C-terminal und N-terminal geschütztes Peptid oder Peptidomimetikum hergestellt (1), die Carboxylfunktion der Seitenkette der Glutaminsäure zum Aldehyd reduziert (2) und der erhaltene Aldehyd in eine akzeptorsubstituierte elektrophile Doppelbindung überführt (3). Abschließend kann noch die Abspaltung der Schutzgruppen erfolgen (4).

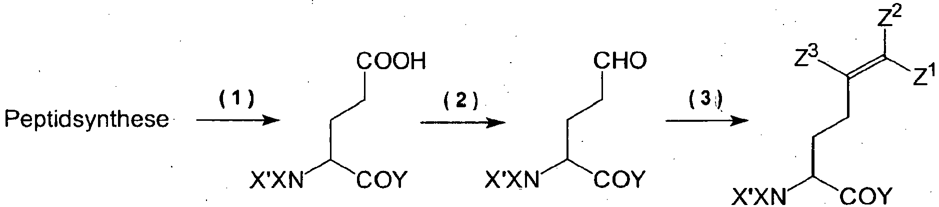

Die erfindungsgemäßen Verbindungen mit m = 1 lassen sich herstellen, indem die Aminosäure Glu (Glutaminsäure) am C-Terminus und N-Terminus mit einer Schutzgruppe (PG1 und PG2) versehen wird (1), danach die Carboxylfunktion der Seitenkette in eine Diketogruppe überführt wird (2) und die endständige Carbonylfunktion der erhaltenen Diketogruppe in eine akzeptorsubstituierte elektrophile Doppelbindung überführt wird (3). Anschließend werden die Schutzgruppen entfernt und der N-Terminus falls gewünscht (optional) mit einem Peptidfragment oder Peptidomimetikum verlängert (4). Nach Aktivierung der C-terminalen Carbonylfunktion kann auch der C-Terminus optional mit einem Peptidfragment oder Peptidomimetikum verlängert werden (5). Der systematische Syntheseablauf ist in folgendem Schema dargestellt.

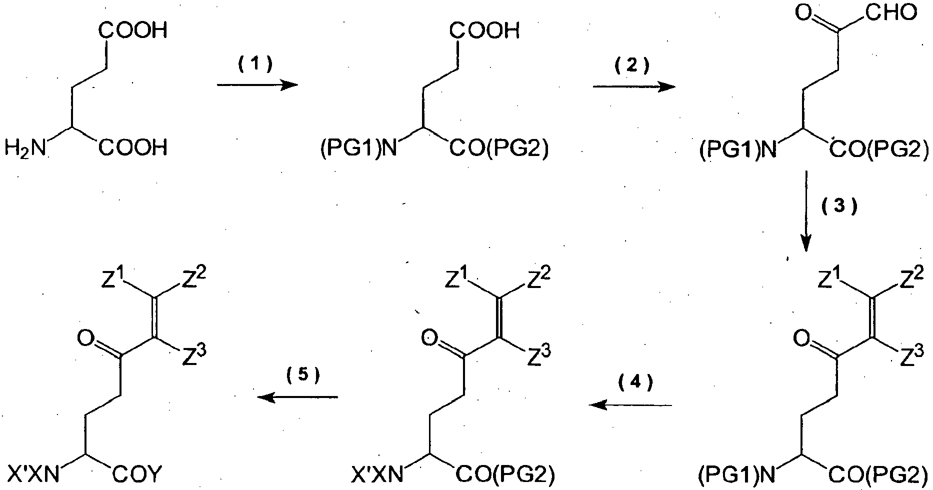

Alternativ kann auch zuerst das Grundgerüst ausgebildet werden und danach die akzeptorsubstituierte elektrophile Doppelbindung an der Seitenkette der Glutaminsäure erzeugt werden. Dazu wird ein C-terminal und N-terminal geschütztes Peptid oder Peptidomimetikum hergestellt (1), die Carboxylfunktion der Seitenkette der Glutaminsäure zur Diketogruppe umgesetzt (2) und die endständige Carbonylfunktion der erhaltenen Diketogruppe in eine akzeptorsubstituierte elektrophile Doppelbindung überführt (3). Abschließend kann noch die Abspaltung der Schutzgruppen erfolgen (4).

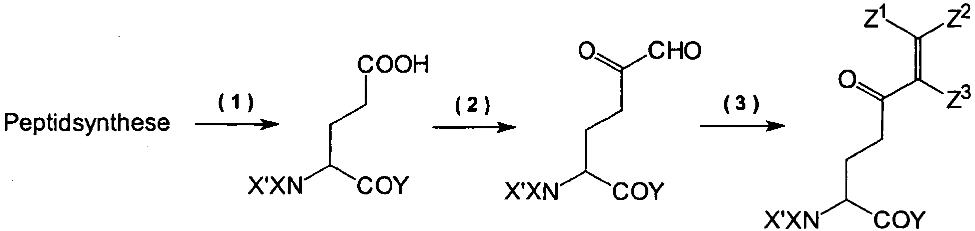

Als akzeptorsubstituierte Olefine sind insbesondere Michael-Systeme aus zumindest einer konjugierten Doppelbindung und einer Carbonylfunktion bevorzugt und insbesondere solche, welche aus den oben genannten Typen von Michaelsystemen ausgewählt werden.

Weitere erfindungsgemäß bevorzugte akzeptorsubstituierte Olefine (MS) besitzen folgende Struktur:

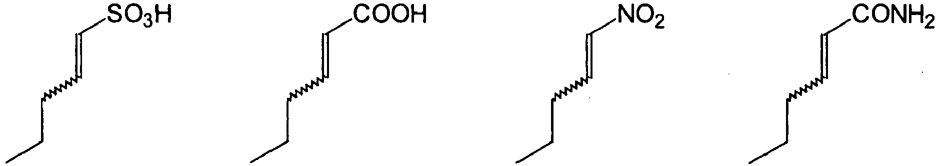

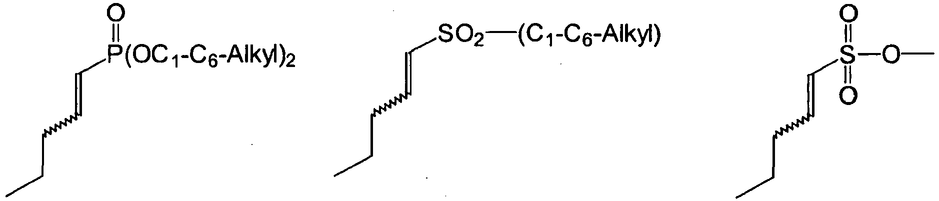

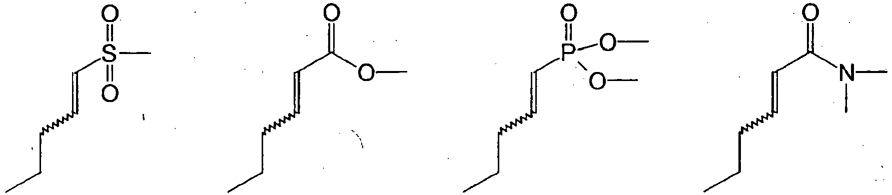

Die zur Konjugation befähigten Sulfoxid-, Sulfon-, Sulfonsäure-, Ester-, Amid- und Phosphonatgruppen können mit beliebigen weiteren Resten versehen sein, wobei Alkylreste, Arylreste und Alkarylreste bevorzugt sind. Das Vorhandensein weiterer Reste inklusive Wasserstoff wird in den obigen chemischen Strukturen durch eine weitergehende Bindung am Schwefel, Stickstoff- und Sauerstoffatom angedeutet.

Die peptidomimetischen Rest können Heteroatome wie S, N, O, P sowie Heterocyclen, Carbocyclen, Aromaten, Heteroaromaten als auch funktionelle Gruppen ausgewählt aus der Liste von R¹ bis R⁸⁴ enthalten und aus bis zu 80, vorzugsweise bis zu 60, weiter bevorzugt bis zu 50 und insbesondere bevorzugt bis zu 40 Kohlenstoffatomen bestehen.

Insbesondere bevorzugt ist, wenn die Gruppen L¹ - L⁴, R⁶ - R⁷⁶ und Q, Q', Q", Q'", Q"" unabhängig voneinander eine Seitenkette einer natürlichen Aminosäure oder ein davon abgeleitetes Derivat darstellen.

Bei diesen insbesondere bevorzugten Seitenketten handelt es sich um: -H, -CH₃, -CH(CH₃)₂, -CH₂-CH(CH₃)₂, -CH(CH₃)-CH₂-CH₃, -CH₂-CH₂-S-CH₃, -CH₂-CH₂-CH₂- (Prolinkette), -CH₂-Ph, -CH₂-OH, -CH(OH)-CH₃, -CH₂-CO-NH₂, -CH₂-CH₂-CO-NH₂, -CH₂-SH, -CH₂-CH₂-CH₂-CH₂-NH₂, -CH₂-CH₂-CH₂-NH-C(=NH)-NH₂, -CH₂-COOH, -CH₂-CH₂-COOH,

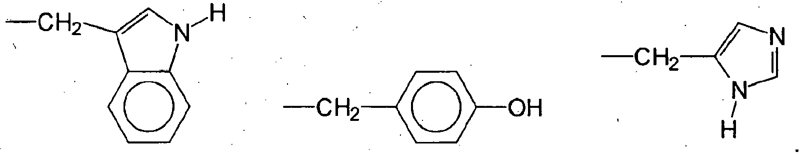

Die erfindungsgemäßen Peptide oder Peptidomimetika haben die allgemeine Formel (I), (II) oder (III) 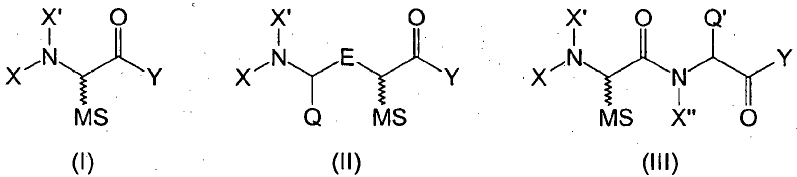 wobei

Formel (I) zumindest eine Aminosäure umfasst, welche das Michael-Akzeptor-System bzw. die akzeptorsubstituierte Doppelbindung (MS) trägt. Der Rest Y kann eine Aminosäurekette (Peptidrest) aus 1 bis 6 Aminosäuren oder Aminosäuren-Analoga, bevorzugt 1 bis 4, weiter bevorzugt 1 bis 3 und insbesondere bevorzugt 1 bis 2 Aminosäuren oder Aminosäuren-Analoga umfassen. Als Aminosäuren-Analoga werden auch die oben beschriebenen Dipeptid-Analoga bezeichnet. Wird zumindest ein Aminosäuren-Analogon oder Aminosäuren-Derivat verwendet, so erhält man den peptidomimetischen Rest Y, der 2 bis 40 Kohlenstoffatome, bevorzugt 3 bis 30, weiter bevorzugt 4 bis 25 und insbesondere bevorzugt 5 bis 20 Kohlenstoffatome umfassen kann. Andererseits kann Y auch eine Hydroxygruppe, Aminogruppe, C₁-C₆-Alkylaminogruppe, C₁-C₆-Dialkylaminagruppe, C₁-C₆-Alkoxygruppe, C₆-C₁₉-Aryloxygruppe, C₁-C₆-Alkylgruppe, C₁-C₆-Halogenalkylgruppe, C₃-C₁₀-Heteroarylgruppe oder eine C₆-C₁₅-Arylgruppe wie oben definiert darstellen und vorzugsweise eine C₁-C₄-Alkoxygruppe.

Als C₆-C₁₉-Aryloxygruppe werden vorzugsweise folgende Reste bezeichnet: -Ph, -CL⁵L⁶-Ph, worin L⁵ und L⁶ unabhängig voneinander ausgewählt werden aus: -H, -CH₃, -C₂H₅, -C₃H₇, -CH(CH₃)₂, -C₄H₉, -Ph und -CH₂-Ph.

Falls Y für einen Peptidrest steht, so kann das C-terminale Ende des Peptidrestes auch eine der vorgenannten Gruppen tragen.

Wie bereits ausgeführt, umfasst der Begriff "Aminosäure" wie hierin verwendet, nicht nur natürliche Aminosäuren, sondern auch Derivate von natürlichen Aminosäuren sowie Aminosäure-Analoga wie die oben beschriebenen Dipeptid-Analoga.

Als Aminosäure-Derivate oder Aminosäure-Analoga werden zum einen insbesondere natürliche Aminosäuren bezeichnet, deren Seitenkette modifiziert worden ist als auch Aminosäuren mit unnatürlichen Seitenketten wie beispielsweise Allyl- oder Propinyl-Seitenkette. Weitere Beispiele für modifizierte Seitenketten sind -CH₂-CH₂-S-(C₂-C₆-Alkyl), -CH₂-S-(C₁-C₆-Alkyl), Ersatz der cyclischen Prolin -CH₂-CH₂-CH₂-Kette durch eine -CH₂-CH₂-Kette oder -CH₂-CH₂-CH₂-CH₂-Kette, Substitution am Phenylrest von Phenylalanin durch ein oder mehrere Nucleophile, Veretherung oder Veresterung der Hydroxygruppe von Tyrosin, Threonin und Serin zu -CH₂-O-(C₁-C₆-Alkyl), -CH₂-O-CO-(C₁-C₆-Alkyl), -CH₂-O-CO-O-(C₁-C₆-Alkyl), -CH₂-O-CO-NH-(C₁-C₆-Alkyl), -CH(CH₃)-O-(C₁-C₆-Alkyl), -CH(CH₃)-O-CO-(C₁-C₆-Alkyl), -CH(CH₃)-O-CO-O-(C₁-C₆-Alkyl), -CH(CH₃)-O-CO-NH-(C₁-C₆-Alkyl), ortho, meta oder para-CH₂-C₆H₄-O-(C₁-C₆-Alkyl), ortho, meta oder para-CH₂-C₆H₄-O-CO-(C₁-C₆-Alkyl), ortho, meta oder para-CH₂-C₆H₄-O-CO-O-(C₁-C₆-Alkyl), ortho, meta oder para-CH₂-C₆H₄-O-CO-NH-(C₁-C₆-Alkyl), Substitution am Imidazolring von Histidin, Substitution am Phenylring von Trytophan oder Addition am Heterocyclus von Tryptophan, Reduktion von Asparagin oder Glutamin zu -CH₂-CH₂-NH₂, -CH₂-CH₂-CH₂-NH₂, Derivatisierung von Asparagin oder Glutamin zu -CH₂-CO-NH(C₁-C₆-Alkyl), -CH₂-CH₂-CO-NH(C₁-C₆-Alkyl), -CH₂-CO-N(C₁-C₆-Alkyl)(C₁-C₆-Alkyl), -CH₂-CH₂-CO-N(C₁-C₆-Alkyl)(C₁-C₆-Alkyl), Derivatisierung von Lysin zu -CH₂-CH₂-CH₂-CH₂-NH(C₁-C₆-Alkyl), -CH₂-CH₂-CH₂-CH₂-N(C₁-C₆-Alkyl)(C₁-C₆-Alkyl), -CH₂-CH₂-CH₂-CH₂-NH-CO-(C₁-C₆-Alkyl), -CH₂-CH₂-CH₂-CH₂-NH-CO-O-(C₁-C₆-Alkyl), Derivatisierung von Arginin zu -CH₂-CH₂-CH₂-NH-C(=NH)-NH(C₁-C₆-Alkyl), -CH₂-CH₂-CH₂-NH-C(=NH)-N(C₁-C₆-Alkyl)(C₁-C₆-Alkyl), -CH₂-CH₂-CH₂-NH-C(=O)-NH₂, -CH₂-CH₂-CH₂-NH-C(=O)-NH(C₁-C₆-Alkyl), -CH₂-CH₂-CH₂-NH-C(=O)-N(C₁-C₆-Alkyl)(C₁-C₆-Alkyl), -CH₂-CH₂-CH₂-NH₂, oder Cyclisierung der Guanidinogruppe zum Imidazol, Veresterung oder Redudtion von Asparaginsäure und Glutaminsäure zu -CH₂-CH₂-OH, -CH₂-CH₂-CH₂-OH, -CH₂-CH₂-O-(C₁-C₆-Alkyl), -CH₂-CH₂-CH₂-O-(C₁-C₆-Alkyl), -CH₂-CH₂-O-CO-(C₁-C₆-Alkyl), -CH₂-CH₂-CH₂-O-CO-(C₁-C₆-Alkyl), -CH₂-CH₂-O-CO-NH(C₁-C₆-Alkyl), -CH₂-CH₂-CH₂-O-CO-NH(C₁-C₆-Alkyl), -CH₂-CH₂-O-CO-N(C₁-C₆-Alkyl)(C₁-C₆-Alkyl), -CH₂-CH₂-CH₂-O-CO-N(C₁-C₆-Alkyl)(C₁-C₆-Alkyl), -CH₂-COO(C₁-C₆-Alkyl), -CH₂-CH₂-COO(C₁-C₆-Alkyl).

Ähnliches wie für die Gruppe Y, welche das C-terminale Ende der erfindungsgemäßen Verbindungen repräsentiert gilt auch für die Gruppe -NXX', welche das N-terminale Ende der erfindungsgemäßen Verbindungen darstellt.

Die Gruppen X, X', X", X"' und X"" können Wasserstoffatome oder eine C₁-C₆-Alkylgruppe darstellen und die Gruppe -NXX' kann eine Aminogruppe, C₁-C₁₀-Alkylaminogruppe, C₆-C₁₂-Aralkyloxy-carbonylaminogruppe, C₁-C₁₀-Dialkylaminogruppe, C₂-C₆-Stickstoffheterocyclus oder eine C₃-C₅-Stickstoffheteroarylgruppe bedeuten, wobei die Reste die oben definierte Bedeutung haben und die Definitionen für z.B. die C₁-C₆-Alkylreste umfassen. Als Rest C₇ ist ein Heptylrest, als Rest C₈ ist ein Octylrest, als Rest C₉ ist ein Nonylrest und als Rest C₁₀ ist ein Decylrest bevorzugt, wobei auch andere Alkylreste sowie Cycloalkylreste und verzweigte Alkylreste möglich sind.

Bevorzugt für die Gruppe -NXX' sind Amide als auch Aminoschutzgruppen wie beispielsweise Acetylamide, Benzoesäureamide oder Heterocyclen-tragende Carbonsäuren. X kann ferner für einen Benzyloxycarbonyl- (Z), einen tert-Butyloxycarbonyl- (Boc), Cyclopentyloxycarbonyl-, Ethylthiocarbonyl-, Isopropylthiocarbonyl-, Benzylthiocarbonyl-, Methylthiocarbonyl-, Ethylthiocarbonyl-, Isopropylcarbonyl-, Cyclopropylcarbonyl-, 2-Pyridylthiomethylcarbonyl-, Hydroxyethylcarbonyl-, Thiophen-3-carbonyl-, Isoxazol-5-carbonyl- oder einen Nicotinsäurerest stehen.

Ferner ist bevorzugt, wenn X für folgende Reste steht: 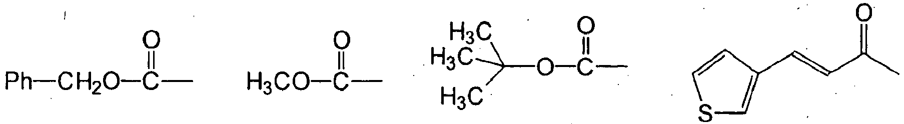 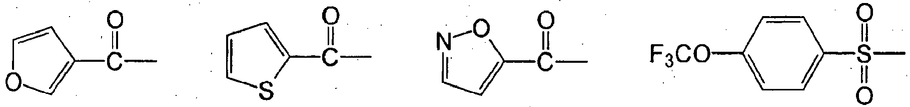 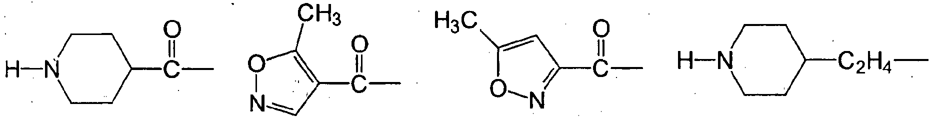 ist die Gruppe -NXX' ein Stickstoffheterocyclus so sind die folgenden Stickstoffheterocyclen bevorzugt:

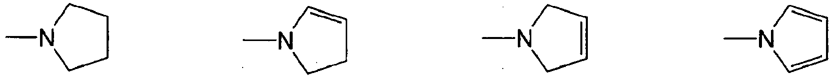

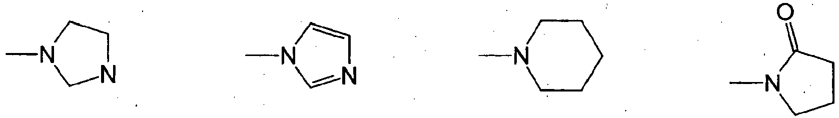

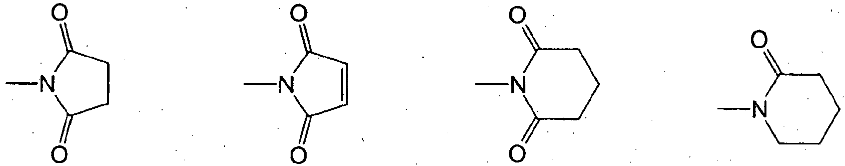

Es wurde überraschend gefunden, dass die Gruppe -NXX' keine unsubstituierte Aminogruppe (-NH₂) sein sollte, sofern sie sich in unmittelbarer Nähe zum Michaelsystem befindet, d.h. wenn die Aminogruppe (-NXX') und der zur elektrophilen Doppelbindung führende Ethylenlinker an demselben Kohlenstoffatom einer Aminosäure oder eines Aminosäureanalogons gebunden sind. Befinden sich Ethylenlinker und Aminogruppe -NXX' an demselben Kohlenstoffatom, so sollte zumindest einer der Reste X und X' von Wasserstoff verschieden sein und vorzugsweise eine Alkylgruppe oder Acylgruppe wie z.B. oben für X gezeigt darstellen.

Darüber hinaus kann die Gruppe -NXX' Teil eines peptidomimetischen Restes, der 2 bis 30 Kohlenstoffatomen, bevorzugt 2 bis 40, weiter bevorzugt 3 bis 30, noch weiter bevorzugt 4 bis 25 und insbesondere bevorzugt 5 bis 20 Kohlenstoffatomen bestehen kann. Darüber hinaus kann X für einen über eine Amidbindung gebundenen Peptidrest aus 1 bis 6 Aminosäuren oder Aminosäuren-Analoga, bevorzugt 1 bis 4, weiter bevorzugt 1 bis 3 und insbesondere bevorzugt 1 ,bis 2 Aminosäuren oder Aminosäuren-Analoga bestehen, wobei der N-Terminus dieses Peptidrestes oder peptidomimetischen Restes eine Aminogruppe, -NH-CHO, C₁-C₁₀-Alkylaminogruppe, C₁-C₈-Alkyloxycarbonylaminogruppe, C₆-C₁₂-Aralkyloxycarbonyl-aminogruppe, C₁-C₁₀-Dialkylaminogruppe, C₂-C₆-Stickstoffheterocyclus oder eine C₃-C₅-Stickstoffheteroarylgruppe tragen kann, wobei jede der C₁-C₆-Alkoxygruppen, C₁-C₆-Alkylgruppen, C₁-C₁₀-Alkylaminogruppen" C₁-C₈-Alkyloxycarbonylaminogruppe, C₆-C₁₂-Aralkyloxycarbonyl-aminogruppe, C₁-C₁₀-Dialkylaminogruppen, C₂-C₆-Stickstoffheterocyclen sowie C₃-C₅-Stickstoffheteroarylgruppen unabhängig voneinander mit bis zu 5 Resten ausgewählt aus R⁸⁰, R⁸¹, R⁸², R⁸³, R⁸⁴ wie oben definiert substituiert sein kann.

Sofern Y und X Peptidreste oder peptidomimetische Reste darstellen, ist eine Gesamtzahl der in X und Y enthaltenen Aminosäuren von 1 bis 5, weiter 1 bis 4 insbesondere 2 bis 3 bevorzugt.

Weitere bevorzugte allgemeine Formeln für die erfindungsgemäßen Verbindungen sind: 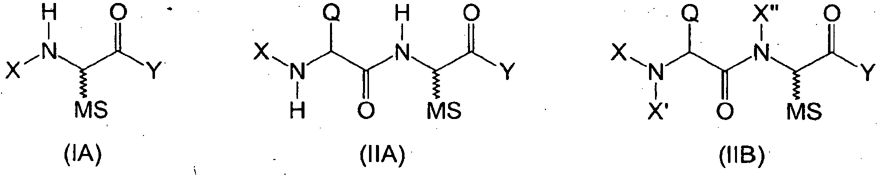 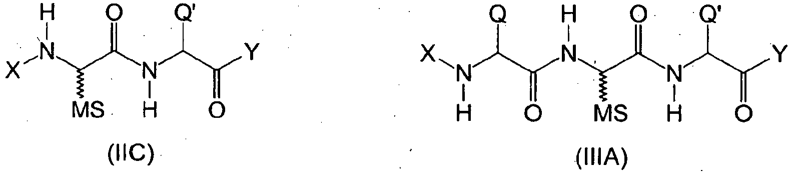 worin MS, E, Q, Q', X und Y die hierin offenbarte Bedeutung haben.

Bei einer besonderen Ausführungsform bilden die Seitenkettenreste Q bzw. Q', Q", Q"' und/oder Q"" mit dem vincinalen Stickstoffatom einen Pyrrolidinring, so dass in der erfindungsgemäßen Verbindung zumindest eine Aminosäure Prolin oder ein Prolin-Derivat oder Prolin-Mimetikum vorhanden ist. Es können auch zwei, drei, vier oder mehr Prolin-Aminosäuren in einer erfindungsgemäßen Verbindung enthalten sein, wobei eine AS als Prolin bevorzugt ist.

Insbesondere sind Tri- und Tetrapeptide bevorzugt, so dass ferner die vorliegende Anmeldung auch vorzugsweise Peptide der allgemeinen Formel (IV) sowie daraus abgeleitete Tetrapeptide betrifft. 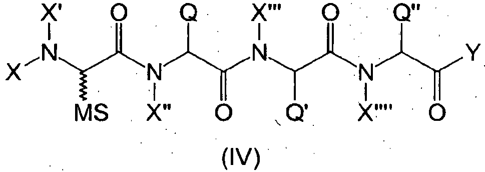 worin
MS ein akzeptorsubstituiertes Olefin wie hierin beschrieben ist und bevorzugt MA ein Michael-System aus zumindest einer konjugierten Doppelbindung und einer Carbonylfunktion wie hierin beschrieben ist;
Q, Q' und Q" unabhängig voneinander einen Seitenkettenrest einer natürlichen Aminosäure darstellen, oder Q zusammen mit X" einen Propylenylrest bilden, oder Q' zusammen mit X"' einen Propylenylrest bilden, oder Q" zusammen mit X"" einen Propylenylrest bilden;
Y eine Hydroxygruppe, Aminogruppe, C₁-C₆-Alkylaminogruppe, C₁-C₆-Dialkylaminogruppe, C₁-C₆-Alkoxygruppe, C₆-C₁₉-Aryloxygruppe, C₁-C₆-Alkylgruppe, C₁-C₆-Halogenalkylgruppe, C₃-C₁₀-Heteroarylgruppe oder eine C₆-C₁₅-Arylgruppe bedeutet oder Y für einen über eine Amidbindung gebundenen Peptidrest aus bis zu 6 Aminosäuren steht, dessen C-terminale Carbonylfunktion eine Hydroxygruppe, Aminogruppe, C₁-C₆-Alkylaminogruppe, C₁-C₆-Dialkylaminogruppe, C₁-C₆-Alkoxygruppe, C₁-C₆-Alkylgruppe, C₁-C₆-Halogenalkylgruppe, C₃-C₁₀-Heteroarylgruppe oder eine C₆-C₁₅-Arylgruppe trägt oder Y für einen peptidomimetischen Rest aus bis zu 60 Kohlenstoffatomen, bevorzugt aus bis zu 30 Kohlenstoffatomen steht und
X", X"', X"" unabhängig voneinander für Wasserstoff oder eine C₁-C₆-Alkylgruppe stehen; und
-NXX' eine Aminogruppe, -NH-CHO, C₁-C₁₀-Alkylaminogruppe, C₁-C₈-Alkyloxycarbonylaminogruppe, C₆-C₁₂-Aralkyloxycarbonylaminogruppe, C₁-C₁₀-Dialkylaminogruppe, C₂-C₆-Stickstoffheterocyclus oder eine C₃-C₅-Stickstoffheteroarylgruppe bedeutet oder die Gruppe -NXX' Teil eines peptidomimetischen Restes aus bis zu 60 Kohlenstoffatomen, bevorzugt aus bis zu 30 Kohlenstoffatomen ist
oder
X' für Wasserstoff oder eine C₁-C₆-Alkylgruppe steht; und
X für einen über eine Amidbindung gebundenen Peptidrest aus bis zu 6 Aminosäuren steht, dessen N-Terminus eine Aminogruppe, -NH-CHO, C₁-C₁₀-Alkylaminogruppe, C₁-C₈-Alkyloxycarbonylaminogruppe, C₆-C₁₂-Aralkyloxycarbonylaminogruppe, C₁-C₁₀-Dialkylaminogruppe, C₂-C₆-Stickstoffheterocyclus oder eine C₃-C₅-Stickstoffheteroarylgruppe;
wobei jede der C₁-C₆-Alkoxygruppen, C₁-C₆-Alkylgruppen, C₁-C₁₀-Alkylaminogruppen, C₁-C₈-Alkyloxycarbonylaminogruppe, C₆-C₁₂-Aralkyloxycarbonylaminogruppe, C₁-C₁₀-Dialkylaminogruppen, C₂-C₆-Stickstoffheterocyclen sowie C₃-C₅-Stickstoffheteroarylgruppen unabhängig voneinander mit bis zu 5 Resten ausgewählt aus R⁸⁰, R⁸¹, R⁸², R⁸³, R⁸⁴ substituiert sein kann,
wobei die Reste R⁸⁰, R⁸¹, R⁸², R⁸³ , R⁸⁴ unabhängig voneinander die obige Bedeutung haben.

Für den Fall, dass weder X noch Y einen Peptidrest oder Aminosäurenrest (umfassend Aminosäure-Analoga und peptidomimetische Reste) darstellen, ergeben sich aus Formel (IV) Tetrapeptide mit einem bevorzugten Aufbau.

Gemäß Formel (IV) ist nur ein akzeptorsubstituiertes Olefin (MS) bzw. nur ein Michael-System (MS) anwesend, welches sich in terminaler Position am N-terminalen Ende der erfindungsgemäßen Verbindung befindet. Es können jedoch auch zwei, drei oder vier gleiche oder unterschiedliche akzeptorsubstituierte Olefine bzw. Michael-Akzeptor-Systeme anwesend sein. Zudem kann sich das akzeptorsubstituierte Olefin bzw. das Michael-System auch an jede der anderen Aminosäuren befinden wie die folgenden allgemeinen Formeln IVA - IVD zeigen:

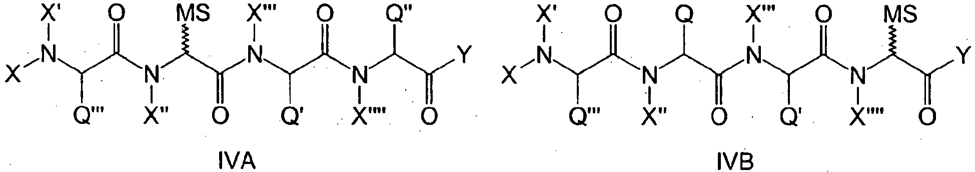

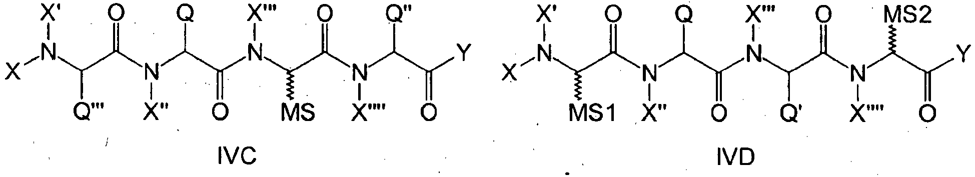

Die Seitenkette Q kann zusammen mit dem Rest X", dem an X" gebundenen Stickstoff und dem an Q gebundenen Kohlenstoff einen Pyrrolidinring ausbilden, so dass die erfindungsgemäßen Verbindungen eine Prolin-Aminosäure enthalten.

Gleichfalls kann die Seitenkette Q' zusammen mit der Gruppe X'" und dem an X"' gebundenen Stickstoff und dem an Q' gebundenen Kohlenstoff einen Pyrrolidinring ausbilden.

Die Seitenkette Q" kann zusammen mit dem Rest X"", dem an X"" gebundenen Stickstoff und dem an Q" gebundenen Kohlenstoff einen Pyrrolidinring ausbilden so wie auch Q'" mit dem an Q'" gebundenen chiralen Kohlenstoffatom und der Gruppe X sowie dem an X gebundenen Stockstoffatom einen Heterocyclylrest bilden können.

Es ergeben sich folgende Tetrapeptide VA bis VD mit einer Prolin-Aminosäure:

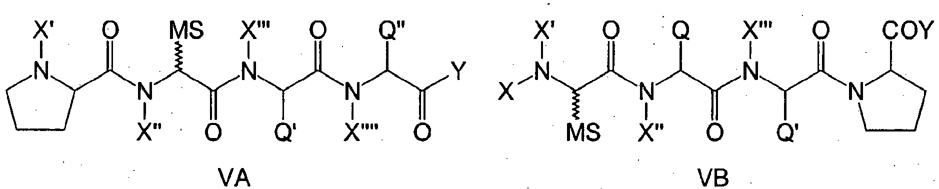

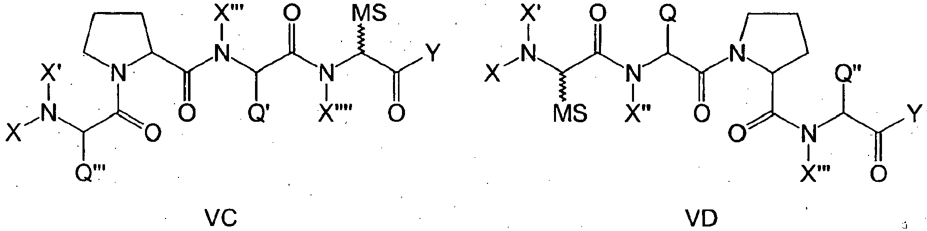

Des weiteren sind insbesondere Tetrapeptide der allgemeinen Formel (V) bevorzugt: 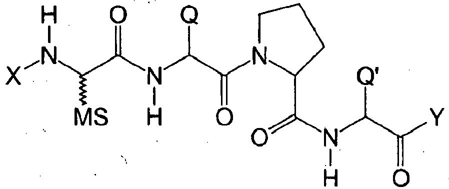 worin
MS, Q, Q', X, X' und Y die hierin beschriebene Bedeutung haben.

Besonders bevorzugt sind Michael-Systeme (MS) mit folgender Struktur: 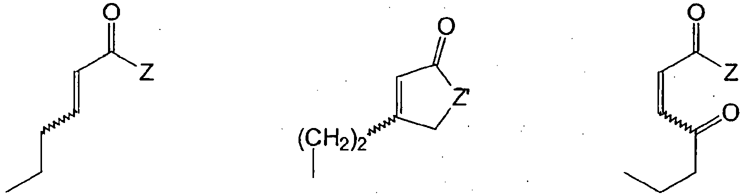 worin Z und Z' die hierin beschriebene Bedeutung haben.

Weitere bevorzugte Strukturen sind:

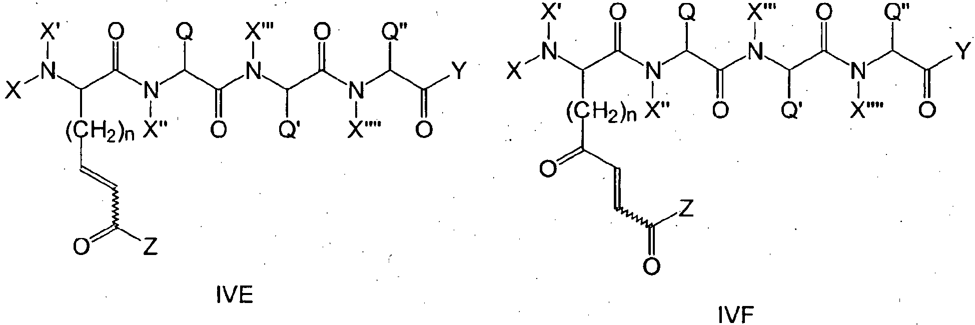

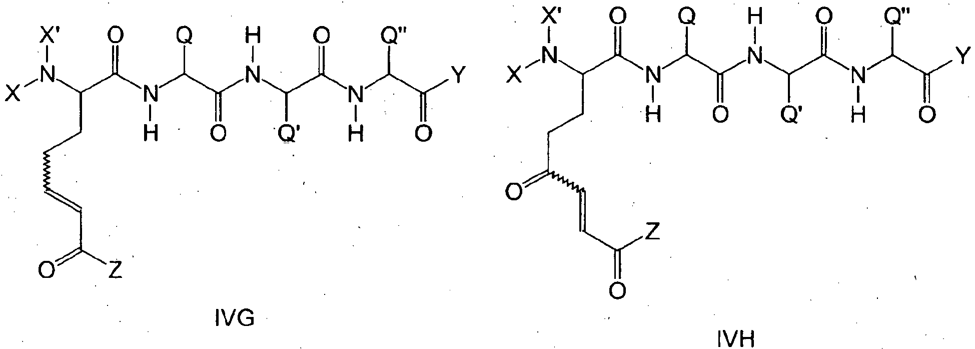

Zudem ist bevorzugt, wenn die Peptide, Peptidderivate oder Peptidomimetika die Aminosäure Glutamin, Prolin, Valin und/oder Leucin und insbesondere die Sequenz Prolin-Leucin enthalten.

Folgende Substrukturen der allgemeinen Formeln VE bis V sind bevorzugt:

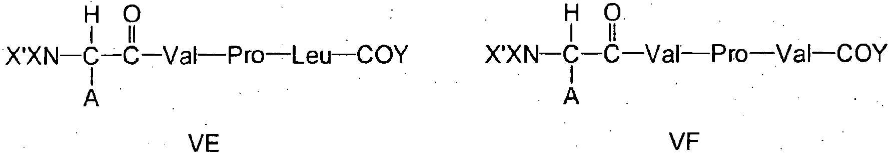

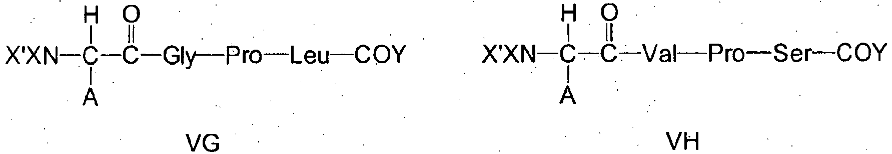

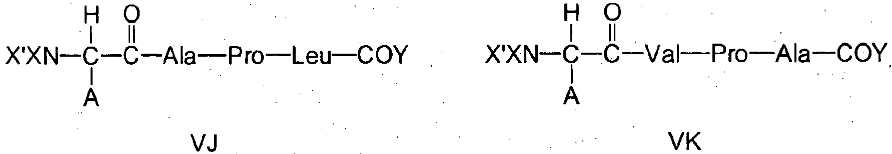

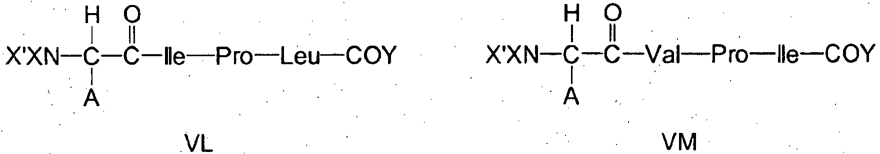

Y ist vorzugsweise eine Hydroxygruppe, C₁-C₆-Alkoxygruppe oder eine C₆-C₁₉-Aryloxygruppe. X' ist vorzugsweise Wasserstoff und X ist vorzugsweise eine Phenyl- oder Alkyl-oxycarbonylgruppe.

Insbesondere bevorzugt sind die Verbindungen der Formeln VN und VO sowie die Verbindungen 1 und 2:

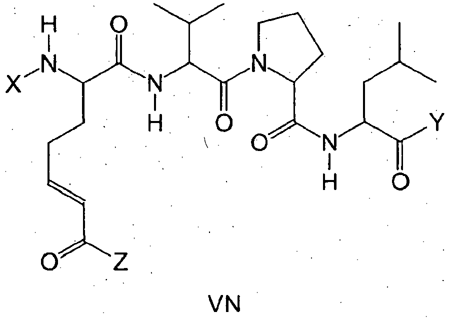

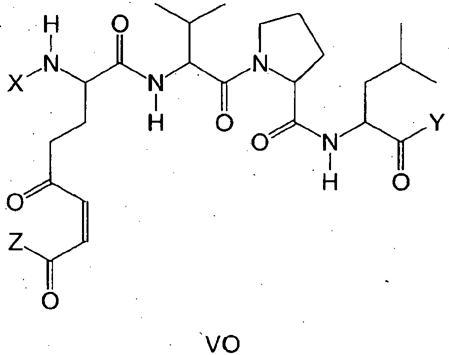

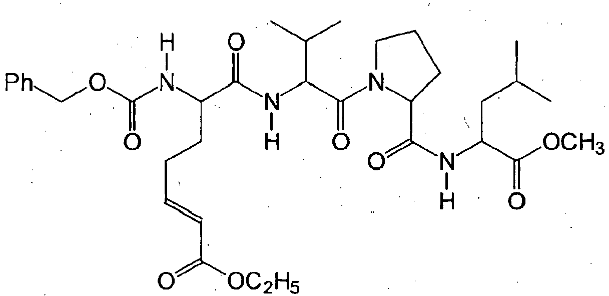

### Verbindung 1

### N^{α}-Benzyloxycarbonyl-{[(E)-(L)-6-Amino-hept-2-endicarbonsäure]-1-ethanoyl}-L-Valinyl-L-Prolinyl-L-Leucinmethylester

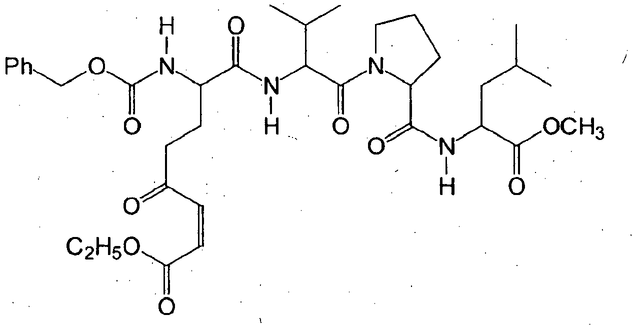

### Verbindung 7

### N^{α}-Acetyl-L-Asparaginyl-{[(E)-(L)-6-Amino-hept-2-endicarbonsäure]-1-methanoyl}-L-Glutamyl-L-Alaninyl-L-Valinmethylester

Weitere erfindungsgemäße Verbindungen sind im Folgenden namentlich genannt:
N^{α}-Benzyloxycarbonyl-{[(E)-(L)-6-Amino-hept-2-endicarbonsäure]-1-ethanoyl}-L-Glutaminyl-L-Prolinyl-L-Leucinmethylester (Verbindung 2);
N^{α}-Benzyloxycarbonyl-{[(E)-(L)-6-Amino-hept-2-endicarbonsäure]-1-ethanoyl}-L-phenylalaninmethylester (Verbindung 3);
N^{α}-Benzyloxycarbonyl-{[(E)-(L)-6-Amino-hept-2-endicarbonsäure]-1-methanoyl}-L-Glutaminyl-L-Prolinmethylester (Verbindung 4);
N^{α}-Benzyloxycarbonyl-{[(E)-(L)-6-Amino-hept-2-endicarbonsäure]-1-ethanoyl}-L-Glutaminyl-L-Prolinyl-Isopentylamid (Verbindung 5);
[(E)-(L)-6-(2-Oxo-pyrrblidon-1-yl)-hept-2-endicarbonsäure-1-ethanoyl]-L-Valinyl-L-Prolinmethylester (Verbindung 6);
N^{α}-Acetyl-L-Leucinyl-Glycinyl-L-Prolinyl-Glycinyl-{[(E)-(L)-6-Amino-hept-2-endicarbonsäure]-1ethanoyl}-L-Serinyl-L-Leucinyl-L-Valinyl-L-Isoleucinyl-Glycinmethylester (Verbindung 8);
N^{α}-Benzyloxycarbonyl-{[L-7-Amino-4-oxo-oct-2-endicarbonsäure]-1-ethanoyl}-L-Valinyl-L-Prolinyl-Leucin-methylester (Verbindung 9);
N^{α}-Acetyl-{[(E)-(L)-6-Amino-hept-2-endicarbonsäure]-1-methanoyl}-L-Glutaminyl-L-Glutamyl-L-Alaninnmethylester (Verbindung 10);
N^{α}-Acetyl-{[(E)-(L)-6-Amino-hept-2-endicarbonsäure]-1-methanoyl}-L-Glutaminyl-L-Glutamylmethylester (Verbindung 11);
N^{α}-Acetyl-{[(E)-(L)-6-Amino-hept-2-endicarbonsäure]-1-methanoyl}-L-Phenylalaninyl-L-Prolinyl-L-Leucinmethylester (Verbindung 12);
N^{α}-Benzyloxycarbonyl-{[(E)-(L)-6-Amino-hept-2-endicarbonsäure]-1-methanoyl}-L-Phenylalaninyl-L-Prolinyl-L-Leucinmethylester (Verbindung 13);
N^{α}-Acetyl-{[(E)-(L)-6-Amino-hept-2-endicarbonsäure]-1-methanoyl}-L-(p-Fluoro)-Phenylalaninyl-L-Prolinyl-L-Leucinmethylester (Verbindung 14);
N^{α}-Benzyloxycarbonyl-{[(E)-(L)-6-Amino-hept-2-endicarbonsäure]-1-methanoyl}-L-(p-Fluoro)-Phenylalaninyl-L-Prolinyl-L-Leucinmethylester (Verbindung 15);
[(E)-(L)-6-(2-Oxo-pyrrolidon-1-yl)-hept-2-endicarbonsäure-1-ethanoyl]-L-Valinyl-L-Homoprolinyl-L-Leucinmethylester (Verbindung 16);
[(E)-(L)-6-(2-Oxo-pyrrolidon-1-yl)-hept-2-endicarbonsäure-1-ethanoyl]-L-Cyclohexylglycinyl-L-Homoprolinyl-L-Leucinmethylester (Verbindung 17);
[(E)-(L)-6-(2-Oxo-pyrrolidon-1-yl)-hept-2-endicarbonsäure-1-ethanoyl]-L-Cyclohexylglycinyl-L-Prolinyl-L-Leucinmethylester (Verbindung 18);
N^{α}-Benzyloxycarbonyl-{[(E)-(L)-6-Amino-hept-2-endicarbonsäure]-1-ethanoyl}-L-Glutaminyl-L-Prolin-L-Tyrosinmethylester (Verbindung 19);
N^{α}-Benzyloxycarbonyl-L-Penylalaninyl-{[(E)-(L)-6-Amino-hept-2-endicarbonsäure]-1-ethanoyl}-L-Glutaminyl-L-Prolinyl-L-Leucinmethylester (Verbindung 20);
N^{α}-Benzyloxycarbonyl-L-Glutaminyl-{[(E)-(L)-6-Amino-hept-2-endicarbonsäure]-1-ethanoyl}-L-Leucinyl-L-Prolinyl-L-Glutaminmethylester (Verbindung 21);
N^{α}-Acetyl-{[L-7-Amino-4-oxo-oct-2-endicarbonsäure]-1-ethanoyl}-L-Glutaminyl-L-Prolinyl-L-Leucinmethylester (Verbindung 22);
N^{α}-(5-Methylisoxazol-3-carbonyl)-{[L-7-Amino-4-oxo-oct-2-endicarbonsäure]-1-isopropanoyl}-L-Valinyl-L-Prolinyl-Leucin-methylester (Verbindung 23);
N^{α}-(2-Fluorbenzoyl)-{[L-7-Amino-4-oxo-oct-2-endicarbonsäure]-1-methanoyl}-L-Valinyl-L-4-Fluoroprolinyl-Leucin-isopropylester (Verbindung 24).
N^{α}-Benzyloxycarbonyl-{[(E)-(L)-6-Amino-hept-2-endicarbonsäure]-1-ethanoyl}-L-Phenylalaninyl-L-Prolinyl-L-Leucinmethylester (Verbindung 25);
N^{α}-Benzyloxycarbonyl-{[(E)-(L)-6-Amino-hept-2-endicarbonsäure]-1-ethanoyl}-Glycinyl-L-Prolinyl-L-Leucinmethylester (Verbindung 26);
N^{α}-Benzyloxycarbonyl-{[(E)-(L)-6-Amino-hept-2-endicarbonsäure]-1-ethanoyl}-L-Alaninyl-L-Prolinyl-L-Leucinmethylester (Verbindung 27);
N^{α}-tert.-Butyloxycarbonyl-{[(E)-(L)-6-Amino-hept-2-endicarbonsäure]-1-ethanoyl}-L-Glutaminyl-L-Prolinyl-L-Leucinmethylester (Verbindung 28);
N^{α}-Thiophen-2-carbonyl-{[(E)-(L)-6-Amino-hept-2-endicarbonsäure]-1-ethanoyl}-L-Glutaminyl-L-Prolinyl-L-Leucinmethylester (Verbindung 29);
N^{α}-Furan-3-carbonyl-{[(E)-(L)-6-Amino-hept-2-endicarbonsäure]-1-ethanoyl}-L-Glutaminyl-L-Prolinyl-L-Leucinmethylester (Verbindung 30);
N^{α}-Isoxazol-5-carbonyl-{[(E)-(L)-6-Amino-hept-2-endicarbonsäure]-1-ethanoyl}-L-Glutaminyl-L-Prolinyl-L-Leucinmethylester (Verbindung 31);
N^{α}-(5-Methyl-Isoxazol-3-carbonyl)-{[(E)-(L)-6-Amino-hept-2-endicarbonsäure]-1-ethanoyl}-L-Glutaminyl-L-Prolinyl-L-Leucinmethylester (Verbindung 33);
N^{α}-(trans-3-(3-Thienyl)acryloyl)-{[(E)-(L)-6-Amino-hept-2-endicarbonsäure]-1-ethanoyl}-L-Glutaminyl-L-Prolinyl-L-Leucinmethylester (Verbindung 34);
N^{α}-Acetyl-{[(E)-(L)-6-Amino-hept-2-endicarbonsäure]-1-ethanoyl}-L-Glutaminyl-L-Prolinyl-L-Leucinmethylester (Verbindung 35);
N^{α}-(4-Trifluormethoxy-benzolsulfonyl)-{[(E)-(L)-6-Amino-hept-2-endicarbonsäure]-1-ethanoyl}-L-Glutaminyl-L-Prolinyl-L-Leucinmethylester (Verbindung 36);
N^{α}-Benzyloxycarbonyl-{[(E)-(L)-6-Amino-hept-2-endicarbonsäure]-1-ethanoyl}-L-Cyclohexylglycin-L-Prolinyl-L-Leucinmethylester (Verbindung 37);
N^{α}-Benzyloxycarbonyl-{[(E)-(L)-6-Amino-hept-2-endicarbonsäure]-1-ethanoyl}-L-Valinyl-L-Homoprolinyl-L-Leucinmethylester (Verbindung 38);
(E)-(S)-6-Benzyloxycarbonylamino-6-[3-((R)-2-phenylcarbamoyl-pyrrolidin-1carbonyl)-phenylcarbamoyl]-hex-2-ensäureethylester (Verbindung 39);
(E)-(S)-6-Benzyloxycarbonylamino-6-{1-[(S)-3-carboxy-1-(3-methyl-butylcarbamoyl)-propyl]-2-oxo-1,2-dihydro-pyridin-3-ylcarbamoyl}-hex-2-enoylsäureisopropylester (Verbindung 40);
N^{α}-Benzyloxycarbonyl-{[(E)-(L)-2-Amino-6-methansulfonyl]-hex-5-enyl}-L-Glutaminyl-L-Prolinyl-L-Leucinmethylester (Verbindung 3.2.1);
N^{α}-Benzyloxycarbonyl-[(E)-(L)-2-Amino-6-dimethylsulfamoyl}-hex-5-enyl]-L-Glutaminyl-L-Prolinyl-L-Leucinmethylester (Verbindung 3.2.2);
N^{α}-Benzyloxycarbonyl-[(L)-2-Amino-4-(3)-oxo-cyclopent-1-enyl]-butyryl-L-Glutaminyl-L-Prolinyl-L-Leucinmethylester (Verbindung 3.2.3);
N^{α}-Benzyloxycarbonyl[(L)-2-Amino-5-(2-oxo-dihydrofuran-(3E)-yliden)]-pentanoyl-L-Glutaminyl-L-Prolinyl-L-Leucinmethylester (Verbindung 3.2.4);
N^{α}-Benzyloxycarbonyl-[(E)-(L)-6-Amino-hept-2-endicarbonsäure]-L-Glutaminyl-L-Prolinyl-L-Leucinmethylester (Verbindung 3.2.5);
N^{α}-Acetyl-{[(E)-(L)-6-Amino-hept-2-endicarbonsäure]-1-pentylamido}-L-Glutaminyl-L-Asparatyl-L-Prolinmethylester (Verbindung 3.2.6);
N^{α}-Benzyloxycarbonyl-{[(E)-(L)-6-Amino-hept-2-endicarbonsäure]-1-isopropanoyl}-L-(p-Fluor-phenylalaninyl)-L-Prolin (Verbindung 3.2.7);
N^{α}-Benzyloxycarbonyl-{[(E)-(L)-6-Amino-hept-2-endicarbonsäure]-1-benzoyl}-L-Phenylalaninyl-L-Homoprolinyl-L-Leucinylamid (Verbindung 3.2.8);
N^{α}-Benzyloxycarbonyl-{[(E)-(L)-7-Amino-2-oxo-oct-3-endicarbonsäure]-1-ethanoyl}-LPhenylalaninmethylester (Verbindung 3.2.9);
N^{α}-Benzyloxycarbonyl-[(Z)-(L)-2-Amino-7-oxo-oct-5-ensäure]-L-Glutaminyl-L-Prolinyl-L-Leucin-methylester (Verbindung 3.2.10);
N^{α}-Benzyloxycarbonyl-[(Z)-(L)-2-Amino-6-cyano-hex-5-ensäure]-L-Glutaminyl-L-Prolinyl-L-Leucin-methylester (Verbindung 3.2.11);
N^{α}-Benzyloxycarbonyl-{[(E)-(L)-6-Amino-hept-2-endicarbonsäure]-1-ethanoyl}-L-Valinyl-L-(Octahydroindol-2-carboxyl)-L-Leucinylamid (Verbindung 4.1);
N^{α}-(Piperidinyl-4-carbonyl)-{[(E)-(L)-2-Amino-6-phenylsulfonyl]-hex-5-enyl}-L-Phenylalaninyl-L-Prolinyl-L-1-Cyclopentylmethyl-2-oxo-2-(1H-tetrazol-5-yl)-ethylamid (Verbindung 4.2);
N^{α}-Benzyloxycarbonyl-[(E)-(L)-2-Amino-6-benzyloxysulfonyl-hex-5-enyl]-L-Valinyl-L-Prolinylbenzylsulfonamid (Verbindung 4.3);
(E)-(S)-6-[(S)-1-((S)-2-Ethylcarbamoyl-pyrrolidin-1-carbonyl)-2-methyl-propylcarbamoyl]-6-(2-piperidin-4-yl-ethylamino)-hex-2-ensäureisopropylester (Verbindung 5.1);
(S)-2-[((S)-1-{(E)-2R,5S)-2-(4-Fluorobenzyl)-9-methansulfonyl-5-[(5-methyl-isoxazol-3-carbonyl)-amino]-4-oxo-non-8-enoyl}-pyrrolidin-2-carbonyl)-amino]-4-methyl-pentansäuremethylester (Verbindung 5.3.b);
(E)-(6R,9S)-9-Benzyloxycarbonylamino-6-[2-(2-ethylcarbamoyl-octahydroindol-1-yl)-1-methyl-2-oxoethylcarbamoyl]-8-oxo-10-phenyl-dec-2-enylsäureisopropylester (Verbindung 5.4);
Piperidin-4-carbonyl-((E)-(S)-5-benzylsulfonyl-1-{2-[2-((S)-2-benzylsulfonylaminocarbonyl-octahydro-indol-1yl)-2-oxo-ethylamino]-acetyl}-4-enyl)-amid (Verbindung 5.6);
(E)-5-(N'-Acetyl-N-carboxy-hydrazino)-[(pent-2-enoyl)-1-ethanoly]-LValinyl-L-Prolinyl-L-Leucinmethylester (Verbindung 5.7).

Die hierin beschriebenen Verbindungen mit mindestens einem akzeptorsubstituierten Olefin bzw. mindestens einem Michael-System aus einer konjugierten Doppelbindung und einer Carbonylfunktion sowie die Verbindungen gemäß allgemeiner Formel [A], [B], [C], [D], [E], [F], (I), (II), (III) und (IIB) eignen sich insbesondere zur Behandlung und Prophylaxe von Zöliakie sowie anderer mit Transglutaminasen assoziierten Erkrankungen.

Zöliakie wird auch als Sprue, nichttropische oder einheimische Sprue, gluteninduzierte Enteropathie, Glutenunverträglichkeit oder intestinaler Infantilismus bezeichnet. Zöliakie ist eine Unverträglichkeit gegen "Gluten", welche zu einer chronischen Erkrankung der Dünndarmschleimhaut führt.

Gluten ist ein Klebereiweiß aus Prolamin und Glutenin, das in vielen Getreidesorten vorkommt wie beispielsweise Weizen, Bulgur (Weizensorte), Dinkel (Weizensorte), Einkorn (Weizensorte), Emmer (Weizensorte), Kamut (Weizensorte), Gerste, Grünkern, Hafer, Roggen, Triticale (Weizen-Roggen-Kreuzung). Diese Getreidesorten besitzen einen Proteingehalt von etwa 7-15%, der zu ca. 90% aus Gluten besteht. Das Prolamin wird bei Weizen als Gliadin bezeichnet und bei Roggen als Secalin, bei Gerste als Hordein und bei Hafer als Avenin.

Zu den anderen mit Transglutaminasen assoziierten Erkrankungen zählen unter anderem Fibrosen, Thrombose, neurodegenerative Erkrankungen, Katarakt, Akne, Psoriasis, Hautalterung und Candiose.

Die wichtigsten Beispiele für neurodegenerative Erkrankungen sind Chorea Huntington, Parkinson (Parkinsonsche Krankheit) und Alzheimer (Alzheimer Erkrankung), wobei jedoch auch Hemiatrophie-Hemiparkinson, Parkinsonsyndrom, Huntington'sche Krankheit, amyotrophe laterale Sklerose, Demenz, Demenz bedingt durch AIDS, Senil-Demenz, Retinitis Pigmentosa, muskuläre Atrophie, muskuläre Atrophie der Wirbelsäule, paraneoplastische zerebellare Degeneration (PCD), zerebellare Atrophie, extrapyramidale Atrophie, Ataxie, Multiple Sklerose, Phakomatosen, FXTAS (fragile X-associated tremor/ataxia syndrome) auch bezeichnet als das fragile X-Syndrom, progressive supranukleäre Lähmung (PSP: progressive supranuclear palsy), striatonigrale Degeneration (SND), olivoponto-zerebellare Degeneration (OPCD), Shy Drager syndrome (SDS), kortiko-basale Degeneration, Lewy-Körperchen-Demenz, Lewy-Körperchen Krankheit, striato-nigrale Degeneration (SND), idiopathische orthostatische Hypotension (IOH), Multisystematrophien frontotemporale Demenz, Lytico-Bodig Krankheit (Parkinsonismus-Demenz-amyotrophe Lateralsklerose), progressive pallidale Atrophie, Hallervorden-Spatz Erkrankung, x-Chromosom verknüpfte Dystonie (Morbus Lubag), mitochondriale Zytopathie mit striataler Nekrose, Neuroakanthocytose, Restless Leg Syndrom, Wilson'sche Krankheit als auch multiple System-Atrophie (MSA), Polyneuropathien, Inflammation Bowel Diseases, Morbus Crohn, Colitis Ulcerosa, Entzündungen, rheumatoide Erkrankungen, ADHS (Attention deficite hyperactivity syndrome) als Beispiele für neurodegenerative Erkrankungen zu nennen sind, welche durch die hierin beschriebenen Verbindungen behandelt werden können.

Die hierin beschriebenen Verbindungen haben größtenteils basische aus auch saure Eigenschaften und liegen zumeist in ihrer Betain-Struktur vor. Somit ist der Einsatz von Salzen der hierin beschriebenen Peptide, Peptidderivate und Peptidomimetika bevorzugt.

Somit können die hierin beschriebenen Verbindungen der Formel (I) oder [A] entsprechend der vorliegenden Erfindung selbst oder in Form eines pharmakologisch wirksamen Salzes verabreicht werden. Da die Verbindungen der allgemeinen Formel [A] oder (I) basische Eigenschaften als auch saure Eigenschaften besitzen können, können nach gängigen Methoden Salze dieser Verbindungen hergestellt werden.

Geeignete Beispiele dieser Salze der Verbindungen der Formeln [A], [B], [C], [D], [E], [F], (I), (II), (III) und (IIB) Säureadditionssalze, Alkalimetallsalze sowie Salze mit Aminen umfassen. So können Alkalimetallsalze wie das Natriumsalz, das Kaliumsalz, das Lithiumsalz oder das Magnesiumsalz, das Calciumsalz, Alkylaminosalze oder Aminosäurensalze, z.B. mit Aminosäuren wie Methionin, Tryptophan, Lysin oder Arginin genannt werden. Als Säuren, welche ein Säureadditionssalz der Verbindungen der Formeln [A], [B], [C], [D], [E], [F], (I), (II), (III) und (IIB) bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-)Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure.

Bei den meisten hierin offenbarten Verbindungen lassen sich auch durch Zugabe von Basen Salze erzeugen. Somit können zum Beispiel Salze mit anorganischen als auch organischen Basen, wie zum Beispiel NaOH, KOH, NH₄OH, Tetraalkylammoniumhydroxid und dergleichen, gebildet werden.

Ferner betrifft die vorliegende Erfindung pharmazeutische Zusammensetzungen umfassend mindestens eine Verbindung gemäß allgemeiner Formel [A], [B], [C], [D], [E], [F], (I), (II), (III), (IIB) und/oder pharmakologisch verträgliche Salze davon und mindestens einen pharmakologisch verträglichen Träger, Hilfsstoff oder zumindest ein pharmakologisch verträgliches Lösungsmittel.

Die pharmazeutischen Zusammensetzungen können in Form von Tropfen, Mundspray, Nasenspray, Pillen, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektionslösungen bereitgestellt werden.

Zudem sind Kombinationspräparate mit anderen Wirkstoffen möglich, wobei die oder der weitere Wirkstoff mit zumindest einer hierin offenbarten Verbindung der allgemeinen Formel [A], [B], [C], [D], [E], [F], (I), (II), (III) oder (IIB) gemischt oder in Kombination verabreicht wird. Bevorzugt werden die Transglutaminase-Blocker zur Unterstützung der Glutenfreien Diät gegeben. Natürlich kann eine Ergänzung mit Vitaminen, Mineralien und Spurenelementen indiziert sein. Auch eine Unterstützung von Enzympräparaten, bei denen beispielsweise Prolylendopeptidasen oder andere Peptidasen verwendet werden ist sinnvoll. Des weiteren kommen Kombinationen mit Entzündungshemmern (steroide und nicht-steroide), mit T-Zell-Silencern oder Cytokinen oder mit monoklonalen Antikörpern oder Zonulin in Frage.

Die pharmazeutischen Zusammensetzungen werden insbesondere für die Behandlung und Prophylaxe von Zöliakie sowie anderer mit Transglutaminasen assoziierten oder durch Transglutaminasen verursachten Erkrankungen eingesetzt.

Die Verbindungen der allgemeinen Formel [A], [B], [C], [D], [E], [F], (I), (II), (III) oder (IIB) können ferner in Form ihrer pharmazeutisch aktiven Salze optional unter Verwendung von im Wesentlichen nicht toxischen pharmazeutisch verträglichen Trägern, Hilfsstoffen oder Verdünnern verabreicht werden. Die Medikationen werden in einem herkömmlichen festen oder flüssigen Träger oder Verdünnern und einem herkömmlichen pharmazeutisch verträglichen Hilfsstoff mit einer geeigneten Dosierung in einer bekannten Weise hergestellt. Die bevorzugten Präparationen sind in einer verabreichbaren Form, die für orale Anwendung geeignet ist wie beispielsweise Pillen, Tabletten, Filmtabletten, beschichtete Tabletten, Kapseln, Pulver, Deposits und Depotformen.

Die bevorzugten pharmazeutischen Formulierungen sind Tabletten, Filmtabletten, beschichtete Tabletten, Gelatinkapseln und opake Kapseln. Jede pharmazeutische Zusammensetzung enthält mindestens eine Verbindung der allgemeinen Formel [A], [B], [C], [D], [E], [F], (I), (II), (III) oder (IIB) und/oder pharmazeutisch verträgliche Salze davon in einer Menge von 5 mg bis 500 mg, bevorzugt 10 mg bis 250 mg und am meisten bevorzugt in einer Menge von 10 bis 100 mg pro Formulierung.

Außerdem schließt der Gegenstand der vorliegenden Erfindung auch pharmazeutische Präparationen für orale, parenterale, dermale, intradermale, intragastralen, intrakutane, intravasale, intravenöse, intramuskuläre, intraperitoneale, intranasale, intravaginale, intrabukkale, perkutane, rektale, subkutane, sublinguale, topische, transdermale oder inhalative Anwendung ein, die zusätzlich zu typischen Vehikeln und Verdünnern eine Verbindung der allgemeinen Formel [A], [B], [C], [D], [E], [F], (I), (II), (III) oder (IIB) und/oder ein pharmazeutisch verträgliches Salz davon als einen aktiven Bestandteil enthalten.

Die pharmazeutischen Zusammensetzungen der vorliegenden Erfindung enthalten eine der hierin offenbarten Peptide oder Peptidomimetika als aktiven Bestandteil, typischerweise in einer Mischung mit geeigneten Trägermaterialien, ausgewählt im Hinblick auf die beabsichtigte Form der Verabreichung, d.h. oral verabreichbare Tabletten, Kapseln (entweder fest gefüllt, halbfest gefüllt oder flüssig gefüllt), Pulver, oral verabreichbare Gele, Elixiere, dispergierbare Granulate, Sirups, Suspensionen und dergleichen in Übereinstimmung mit herkömmlichen pharmazeutischen Praktiken. Zum Beispiel kann für die orale Verabreichung in der Form von Tabletten oder Kapseln die aktive Wirkstoffkomponente mit einem beliebigen oralen nicht toxischen pharmazeutisch verträglichen inerten Träger, wie Laktose, Stärke, Sucrose, Zellulose, Magnesiumstearat, Dikalziumphosphat, Kalziumsulfat, Talkum, Mannitol, Ethylalkohol (flüssige Formen) und dergleichen kombiniert werden. Außerdem können nach Bedarf geeignete Bindemittel, Gleitmittel, Sprengmittel und Färbemittel ebenfalls der Mischung beigefügt werden. Pulver und Tabletten können aus etwa 5Gew.-% bis zu etwa 95Gew.-% der erfinderischen Zusammensetzung aus diesen inerten Trägern bestehen.

Geeignete Bindemittel schließen Stärke, Gelatine, natürliche Zucker, Maissüßstoffe, natürliche und synthetische Gummis, wie Akaziengummi, Natriumalginat, Carboxymethyl-Cellulose, Polyethylenglycol und Wachse ein. Unter den Gleitmitteln können für die Verwendung in diesen Dosierungsformen Borsäure, Natriumbenzoat, Natriumacetat, Natriumchlorid und dergleichen erwähnt werden. Sprengmittel schließen Stärke, Methylcellulose, Guargummi und dergleichen ein. Süßstoffe und Geschmacksstoffe und Konservierungsstoffe können, falls dienlich, ebenfalls eingeschlossen sein. Einige der oben angeführten Ausdrücke, nämlich Sprengmittel, Verdünner, Gleitmittel, Bindemittel und dergleichen werden unten genauer diskutiert.

Zusätzlich können die Zusammensetzungen der vorliegenden Erfindung in einer Form mit verzögerter Freisetzung formuliert werden, um die Geschwindigkeits-gesteuerte Freisetzung einer beliebigen oder mehrerer Komponenten oder aktiven Bestandteile bereitzustellen, um die therapeutischen Wirkungen zu optimieren, d.h. antihistaminische Aktivität und dergleichen. Geeignete Dosierungsformen für eine verzögerte Freisetzung schließen Schichttabletten ein, die Schichten mit variierenden Abbaugeschwindigkeiten oder polymere Matrizen mit gesteuerter Freisetzung enthalten, die mit den aktiven Komponenten imprägniert sind, und in Tablettenform oder Kapseln gestaltet sind, die derartige imprägnierte oder verkapselte poröse polymere Matrizen beinhalten.

Präparationen in flüssiger Form schließen Lösungen, Suspensionen und Emulsionen ein. Als ein Beispiel können Wasser oder Wasser-Propylenglycol-Lösungen für parenterale Injektionen oder der Zusatz von Süßstoffen und Trübungsmitteln für orale Lösungen, Suspensionen und Emulsionen erwähnt werden.

Zur Inhalation geeignete Aerosol-Präparationen können Lösungen und Feststoffe in Pulverform einschließen, die mit einem pharmazeutisch verträglichen Träger, wie ein komprimiertes Inertgas, z.B. Stickstoff, in Kombination sein können.

Für die Zubereitung von Suppositorien wird zuerst ein niedrig schmelzendes Wachs, wie z.B. eine Mischung von Fettsäureglyceriden, wie z.B. Kakaobutter, geschmolzen und der aktive Bestandteil wird darin durch Rühren oder ähnliches Vermischen homogen dispergiert. Die geschmolzene homogene Mischung wird dann in passend bemessene Formen gegossen, man lässt abkühlen und dadurch verfestigen.

Ferner eingeschlossen sind Präparationen in fester Form, die kurz vor der Verwendung zu Präparationen in flüssiger Form für entweder orale oder parenterale Verabreichung umgewandelt werden sollen. Solche flüssigen Formen schließen Lösungen, Suspensionen und Emulsionen ein.

Die Verbindungen der vorliegenden Erfindung können ferner transdermal verabreichbar sein. Die transdermalen Zusammensetzungen können die Form von Cremes, Lotionen, Aerosolen und/oder Emulsionen annehmen.

Der Begriff Kapsel bezieht sich auf einen speziellen Behälter oder Gehäuse, das aus Methylzellulose, Polyvinylalkoholen oder denaturierten Gelatinen oder Stärke hergestellt ist, worin die Wirkstoffe eingeschlossen werden können. Hartmantelkapseln sind typischerweise aus Mischungen von Knochen und Schweinehautgelatinen relativ hoher Gelstärke hergestellt. Die Kapsel selbst kann kleine Mengen von Farbstoffen, Trübungsmitteln, Weichmachern und Konservierungsstoffen enthalten.

Tablette bedeutet komprimierte oder gegossene feste Dosierungsform, die die aktiven Bestandteile mit geeigneten Verdünnern enthält. Die Tablette kann durch Komprimieren von Mischungen oder Granulaten hergestellt werden, die durch Nassgranulierung, Trockengranulierung oder durch Kompaktierung erhalten wurden, die einem Fachmann bekannt sind.

Orale Gele beziehen sich auf die aktiven Bestandteile, die in einer hydrophilen halbfesten Matrix dispergiert oder solubilisiert sind.

Pulver für Zusammensetzungen beziehen sich auf Pulvermischungen, die die aktiven Bestandteile und geeignete Verdünner beinhalten, die in Wasser oder Säften suspendiert werden können.

Geeignete Verdünner sind Substanzen, die für gewöhnlich den Großteil der Zusammensetzung oder Dosierungsform ausmachen. Geeignete Verdünner schließen Zucker, wie Lactose, Sucrose, Mannitol und Sorbitol, von Weizen, Mais, Reis und Kartoffeln abgeleitete Stärken, und Zellulosen, wie mikrokristalline Zellulose ein. Die Menge an Verdünnern in der Zusammensetzung kann sich von etwa 5 bis etwa 95 Gew.-% der gesamten Zusammensetzung, bevorzugt von etwa 25 bis etwa 75 Gew.-% und weiter bevorzugt von etwa 30 bis etwa 60 Gew.-% erstrecken.

Der Ausdruck Sprengmittel bezieht sich auf Materialien, die der Zusammensetzung hinzugefügt wurden, um sie beim Aufbrechen (Zersprengen) und Freigeben der Medikamente zu unterstützen. Geeignete Sprengmittel schließen Stärken, "Kaltwasser-lösliche" modifizierte Stärken, wie Natrium-Carboxymethylstärke, natürliche und synthetische Gummis, wie Johannisbrotkernmehl, Karaya, Guar, Tragacanth und Agar, Cellulosederivate, wie Methylcellulose und Natrium-Carboxymethylcellulose, mikrokristalline Cellulosen und quervernetzte mikrokristalline Cellulosen, wie Natrium-Croscarmellose, Alginate, wie Alginsäure und Natriumalginat, Tonerden, wie Bentonite, und schäumende Mischungen. Die Menge an Sprengmittel in der Zusammensetzung kann sich von etwa 2 bis 20 Gew.-% der Zusammensetzung und weiter bevorzugt von etwa 5 bis etwa 10 Gew.-% erstrecken.

Bindemittel charakterisieren Substanzen, die Pulver miteinander binden oder "verkleben" und somit als "Kleber" in der Formulierung dienen. Bindemittel fügen eine Kohäsionsstärke hinzu, die in den Verdünnern oder dem Aufgehmittel bereits verfügbar ist. Geeignete Bindemittel schließen Zucker, wie Sucrose, von Weizen, Mais, Reis und Kartoffeln abgeleitete Stärken, natürliche Gummis, wie Akaziengummi, Gelatine und Tragacanth, Derivate von Seetang, wie Alginsäure, Natriumalginat und Ammonium-Calcium-Alginat, Zellulosematerialien, wie Methylcellulose und NatriumCarboxymethylcellulose und Hydroxypropyl-methylcellulose, Polyvinylpyrrolidon und anorganische Verbindungen, wie Magnesium-Aluminium-Silicat ein. Die Menge der Bindemittel in der Zusammensetzung kann sich von etwa 2 bis etwa 20 Gew.-% der Zusammensetzung, weiter bevorzugt von etwa 3 bis etwa 10 Gew.-% und noch weiter bevorzugt von etwa 3 bis etwa 6 Gew.-% erstrecken.

Gleitmittel bezieht sich auf eine der Dosierungsform hinzugefügte Substanz, um zu ermöglichen, dass die Tablette, Granulat usw., nachdem sie komprimiert wurden, aus der Gießform oder Pressform durch Verringern der Friktion oder Reibung freigegeben werden. Geeignete Gleitmittel schließen metallische Stearate, wie Magnesiumstearat, Calciumstearat oder Kaliumstearat, Stearinsäure, Wachse mit hohem Schmelzpunkt, und wasserlösliche Gleitmittel, wie Natriumchlorid, Natriumbenzoat, Natriumacetat, Natriumoleat, Polyethylenglycole und D,L-Leucin ein. Gleitmittel werden gewöhnlich bei dem letzten Schritt vor dem Komprimieren hinzugefügt, da sie auf den Oberflächen der Granulate und zwischen ihnen und den Teilen der Tablettenpresse vorhanden sein müssen. Die Menge an Gleitmittel in der Zusammensetzung kann sich von etwa 0,2 bis etwa 5 Gew.-% der Zusammensetzung, bevorzugt von etwa 0,5 bis etwa 2 Gew.-% und weiter bevorzugt von etwa 0,3 Gew.-% bis etwa 1,5 Gew.-% erstrecken.

Gleitmittel sind Materialien, die eine Anbackung verhindern und die Fließcharakteristika von Granulaten verbessern, so dass der Fluss glatt und einheitlich ist. Geeignete Gleitmittel schließen Siliziumdioxid und Talkum ein. Die Menge von Gleitmittel in der Zusammensetzung kann sich von 0,1 bis etwa 5 Gew.-% der gesamten Zusammensetzung und bevorzugt von etwa 0,5 bis etwa 2 Gew.-% erstrecken.

Färbemittel sind Hilfsstoffe, die der Zusammensetzung oder der Dosierungsform eine Färbung bereitstellen. Derartige Hilfsstoffe können Farbstoffe mit Lebensmittelqualität und Farbstoffe mit Lebensmittelqualität einschließen, die auf einem geeigneten Adsorptionsmittel, wie Tonerde oder Aluminiumoxid adsorbiert sind. Die Menge des Färbemittels kann von etwa 0,1 bis etwa 5 Gew.-% der Zusammensetzung und bevorzugt von etwa 0,1 bis etwa 1 Gew.-% variieren.

Wie hierin verwendet ist eine "pharmazeutisch wirksame Menge" eines Transglutaminaseinhibitors die Menge oder die Aktivität, die wirksam ist, um das erwünschte physiologische Ergebnis entweder in in-vitro behandelten Zellen oder in einem in-vivo behandelten Patienten zu erreichen. Spezifisch ist eine pharmazeutisch wirksame Menge eine Menge, die ausreichend ist, um für eine gewisse Zeitspanne ein oder mehrere der klinisch definierten pathologischen Prozesse, die mit der Transglutaminase assoziiert sind, zu inhibieren. Die wirksame Menge kann in Abhängigkeit des spezifischen Inhibitors variieren und ist ferner von einer Vielfalt von Faktoren und Zuständen abhängig, die mit dem zu behandelnden Subjekt und der Schwere der Erkrankung in Beziehung stehen. Wenn zum Beispiel ein Inhibitor in-vivo verabreicht werden soll, dann wären Faktoren, wie das Alter, Gewicht und die Gesundheit des Patienten als auch Dosisreaktionskurven und Toxizitätsdaten, die aus vorklinischen Tierstudien erhalten wurden, unter den zu berücksichtigenden Faktoren. Falls der Inhibitor in Form der hierin beschriebenen Peptide oder Peptidomimetika mit den Zellen in-vivo in Kontakt gebracht werden soll, würde man ferner eine Vielfalt von vorklinischen in-vitro Studien entwerfen, um solche Parameter, wie Aufnahme, Halbwertszeit, Dosis, Toxizität, usw. zu bestimmen. Die Bestimmung einer pharmazeutisch wirksamen Menge für einen gegebenen pharmazeutisch aktiven Wirkstoff gehört zu den normalen Fähigkeiten eines Fachmanns.

Die folgenden Beispiele sollen die Erfindung an ausgewählten Verbindungen veranschaulichen, ohne jedoch den Schutzumfang des vorliegenden Schutzrechtes auf diese konkreten Beispiele zu beschränken. Es ist für einen Fachmann ersichtlich, dass analoge Verbindungen und Verbindungen hergestellt nach analogen Synthesewegen unter den Schutzumfang des vorliegenden Schutzrechtes fallen.

### Figurenbeschreibung

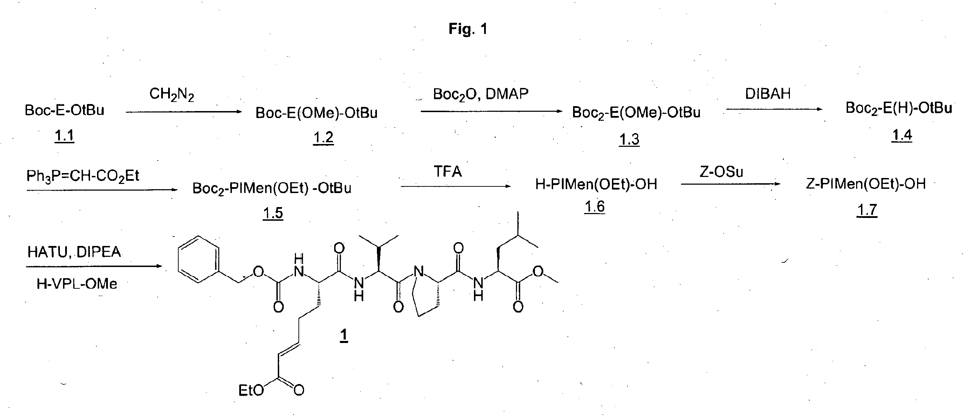

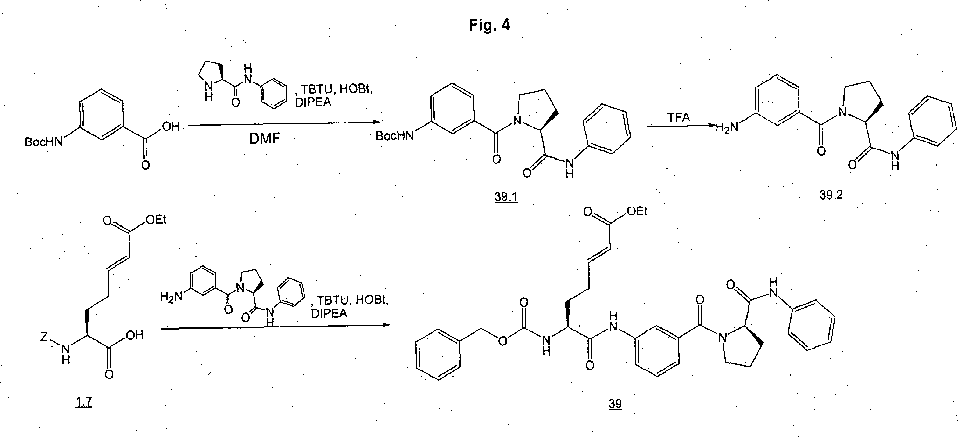

- Figur 1: zeigt das Syntheseschema für die Darstellung von L-2-Amino-hept-5-endicarbonsäurederivate.
- Figur 2: zeigt das Syntheseschema für eine alternative Darstellung von L-2-Amino-hept-5-en-dicarbonsäurederivate.

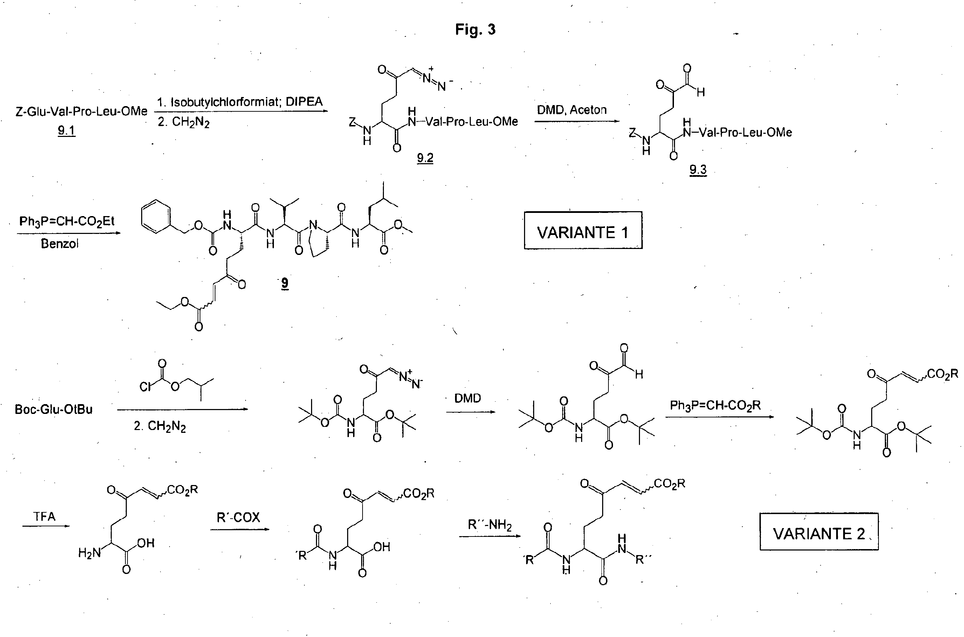

- Figur 3: zeigt zwei Varianten einer allgemeinen Synthese erfindungsgemäßer Verbindungen am Beispiel von L-2-Amino-4-oxo-oct-2-endicarbonsäureethylester, wobei bei Variante 1 die Seitenkettenmodifikation zum essentiellen Michaelsystem (d.h. der akzeptorsubstituierten Doppelbindung) an der Glutaminsäure nach der Synthese des Grundgerüstes am geschützten Grundgerüst durchgeführt wird und bei Variante 2 zuerst ein Aminosäurebaustein mit dem essentiellen Michaelsystem (d.h. der akzeptorsubstituierten Doppelbindung) hergestellt wird und dieser Baustein an seinem C-Terminus und/oder N-Terminus über eine Amidbindung mit weiteren Aminosäuren oder Oligopeptiden verknüpft wird. In Figur 3 haben folgende Abkürzungen folgende Bedeutungen:
EWG: (Elektronen wegziehende Gruppe; elektron withdrawing group)
TBTU: O-(Benzotriazol-1 -yl)-N,N,N',N'-tetramethyluroniumtetrafluoroborat
HOBt: 1-Hydroxybenzotriazol
DIPEA: N-Ethyldiisopropylamin
TFA: Trifluoressigsäure
DMD: Dimethyldioxiran 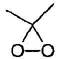
- Figur 4: zeigt die Darstellung von Inhibitoren mit nicht-proteinogenen Aminosäuren. HATU: O-(7-Azabenzotriazol-1-yl)-*N,N,N',N*'-tetramethyluronium hexafluorophosphate

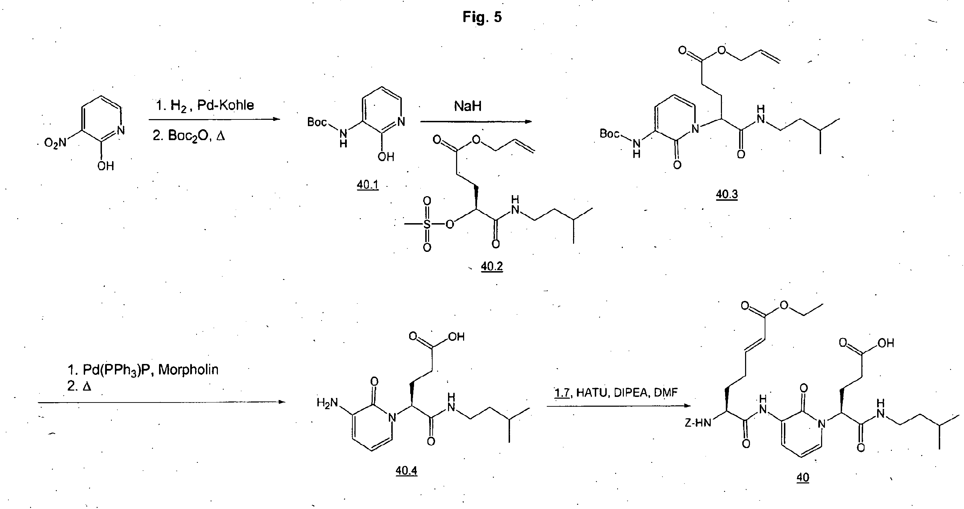

- Figur 5: zeigt die Darstellung von Pyridon enthaltenden Peptiden.

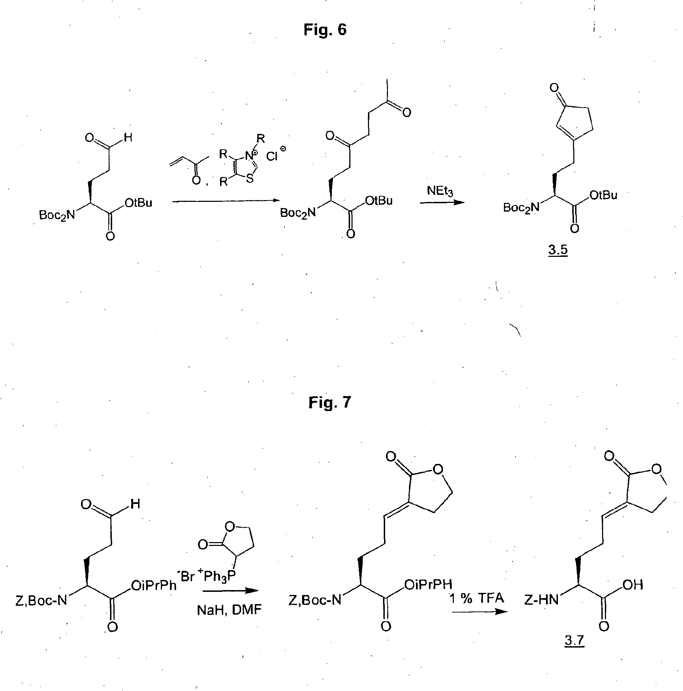

- Figur 6: zeigt die Darstellung von Michaelakzeptorverbindungen mit endocyclischer Doppelbindung.
- Figur 7: zeigt die Darstellung von erfindungsgemäßen Michaelakzeptorverbindungen mit exocyclischer Doppelbindung.

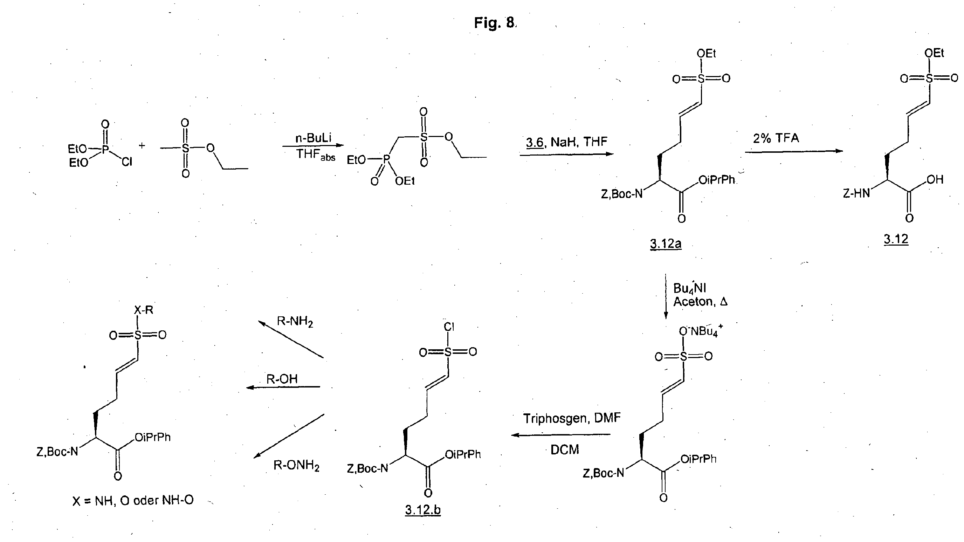

- Figur 8: zeigt die Darstellung erfindungsgemäßer, vinyloger Sulfonsäurederivate.

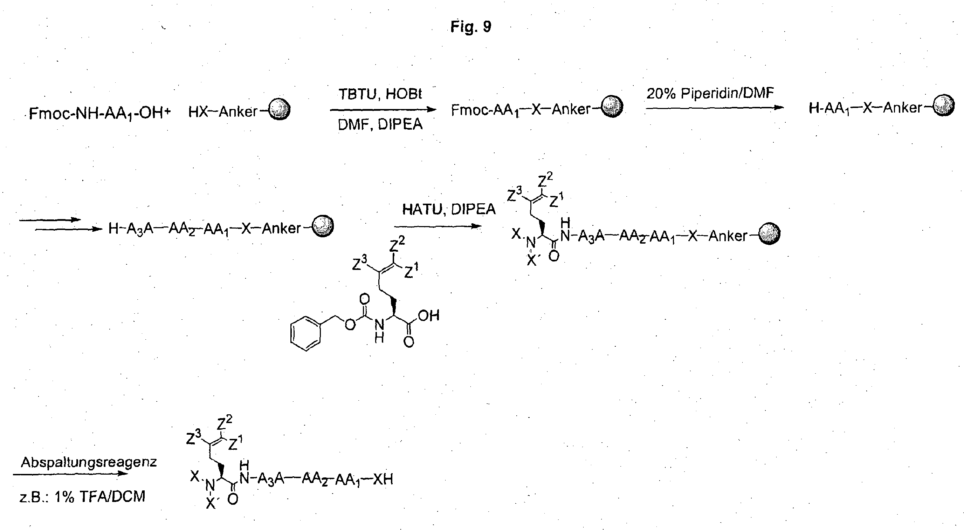

- Figur 9: zeigt die Festphasensynthese erfindungsgemäßer Inhibitoren mit variierendem C-Terminalen Rest. Anker bedeutet beispielsweise Trityl (X=O), 2-Chlortrityl (X=O), Sieber-Amid (X=NH)

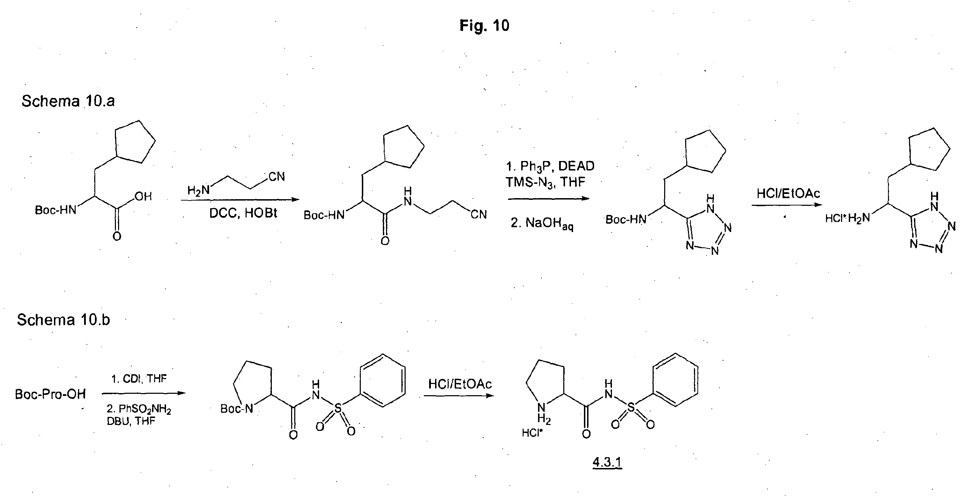

- Figur 10: zeigt Carbonylgruppensurrogate und die Synthese des C-terminalen Restes am Beispiel von Tetrazolen (10a) und Sulfonamiden (10b).

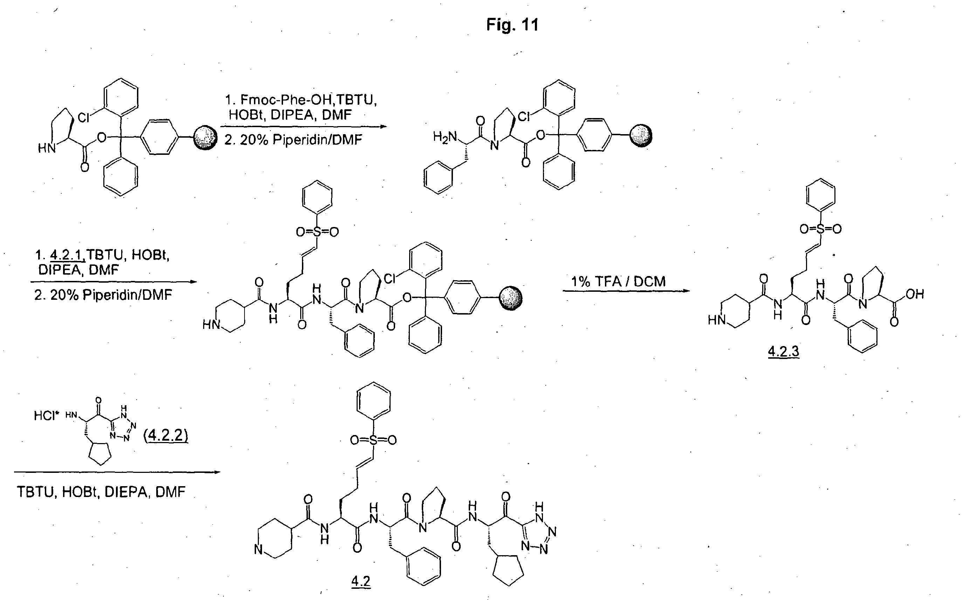

- Figur 11: zeigt die konvergente Synthese erfindungsgemäßer Inhibitoren enthaltend Carbonsäuresurrogate.

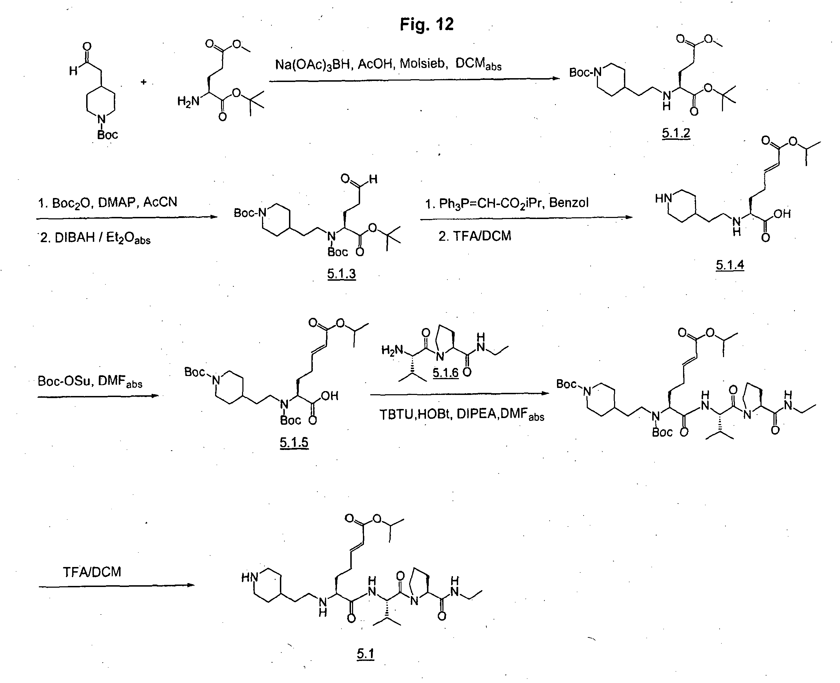

- Figur 12: zeigt die Reaktionsübersicht zur Darstellung von Verbindung: 5.1.

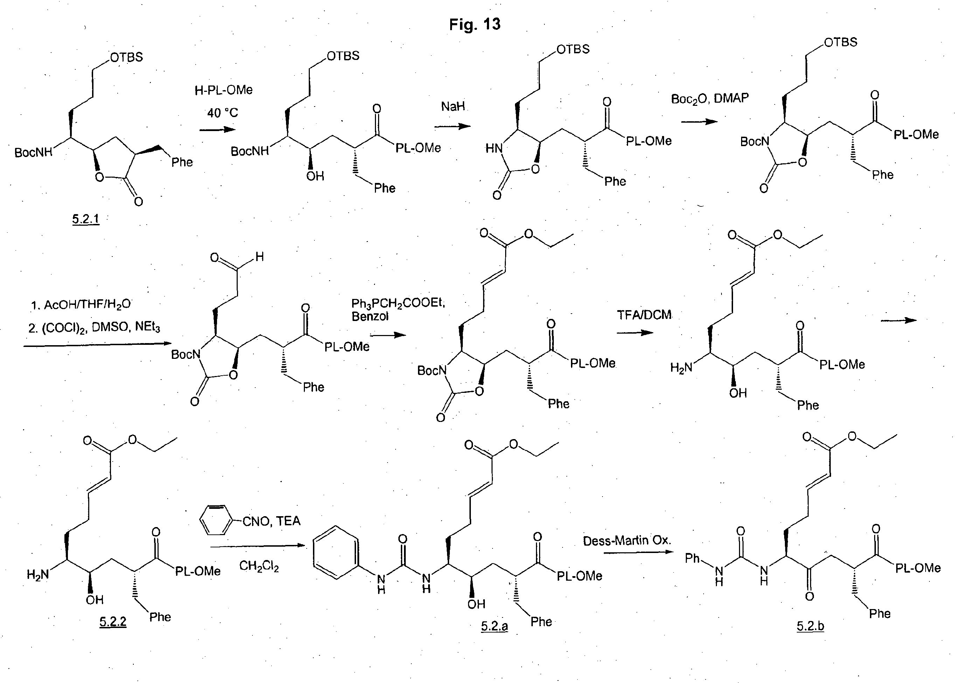

- Figur 13: zeigt die Reaktionsübersicht zur Darstellung erfindungemäßer Inhibitoren mit Hydroxymethylen- (5.2.a) oder Ketomethylenisosteren (5.2.b).

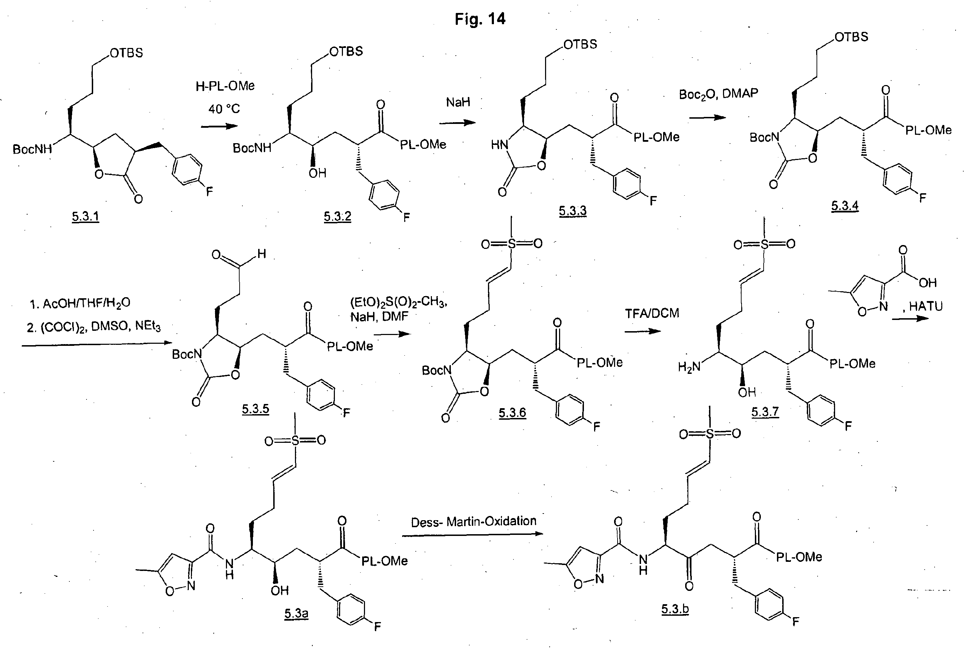

- Figur 14: zeigt die Reaktionsübersicht zur Darstellung der Verbindungen 5.3.a und 5.3.b.

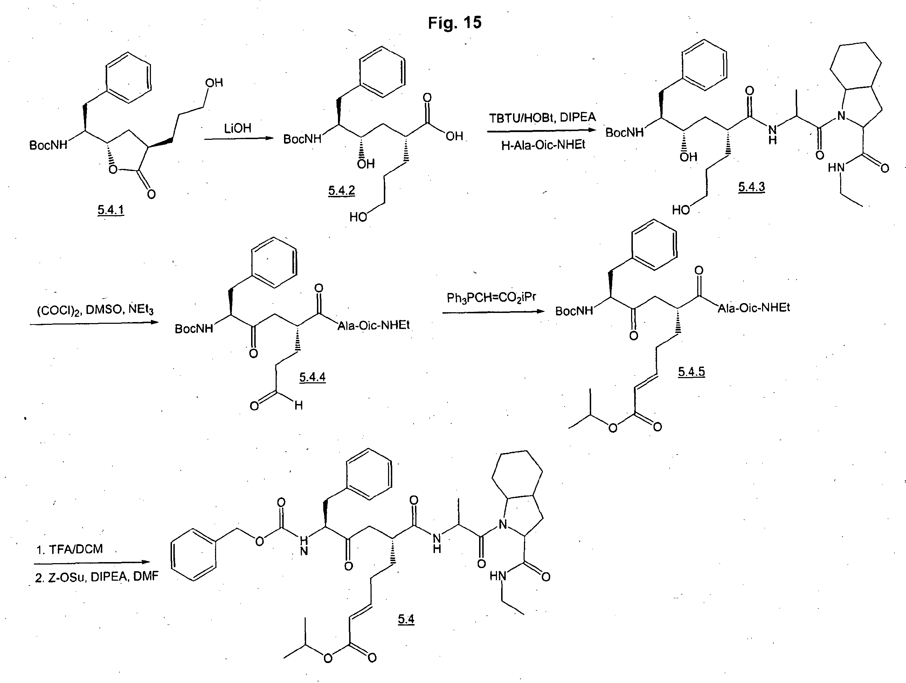

- Figur 15: zeigt die Reaktionsübersicht zur Darstellung von Verbindung 5.4.

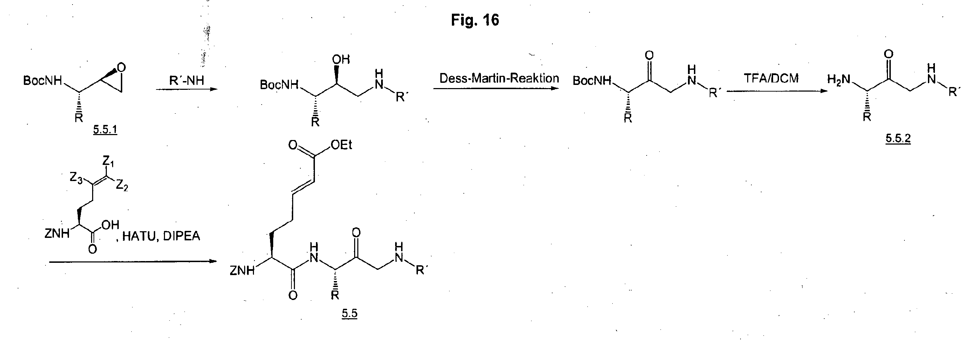

- Figur 16: zeigt ein allgemeines Syntheseschema zur Darstellung erfindungsgemäßer Hydroxyethylamino-isostere.

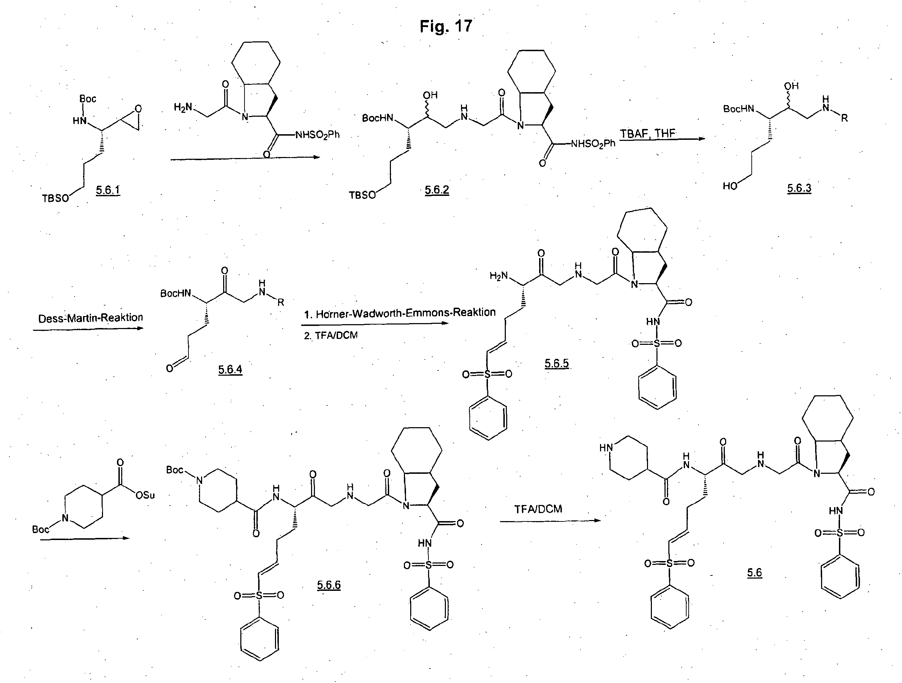

- Figur 17: zeigt die Reaktionsübersicht zur Darstellung von Verbindung: 5.6.

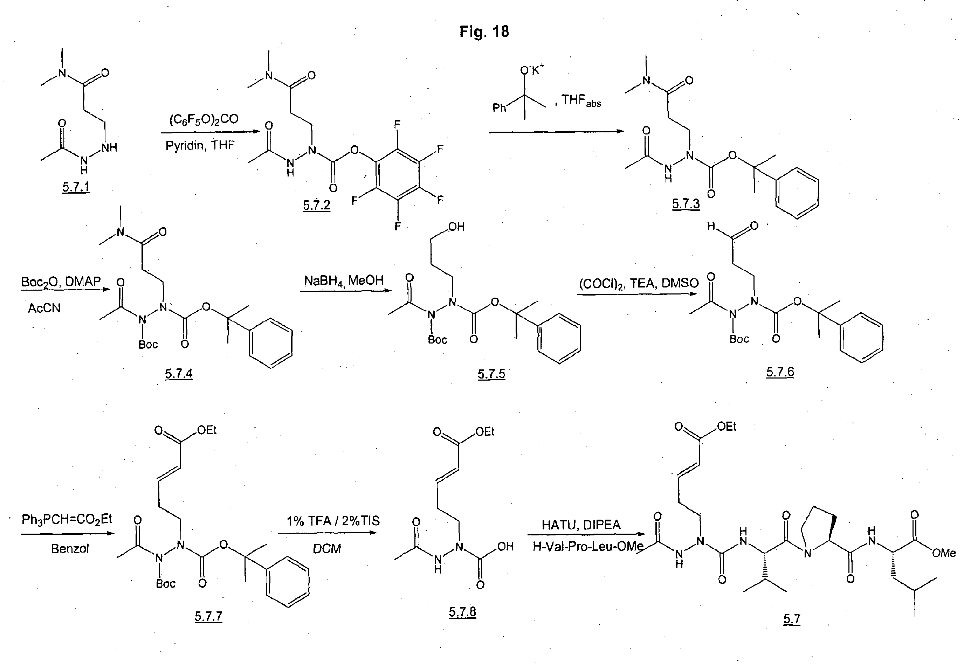

- Figur 18: zeigt die Reaktionsübersicht zur Darstellung von Verbindung 5.7.

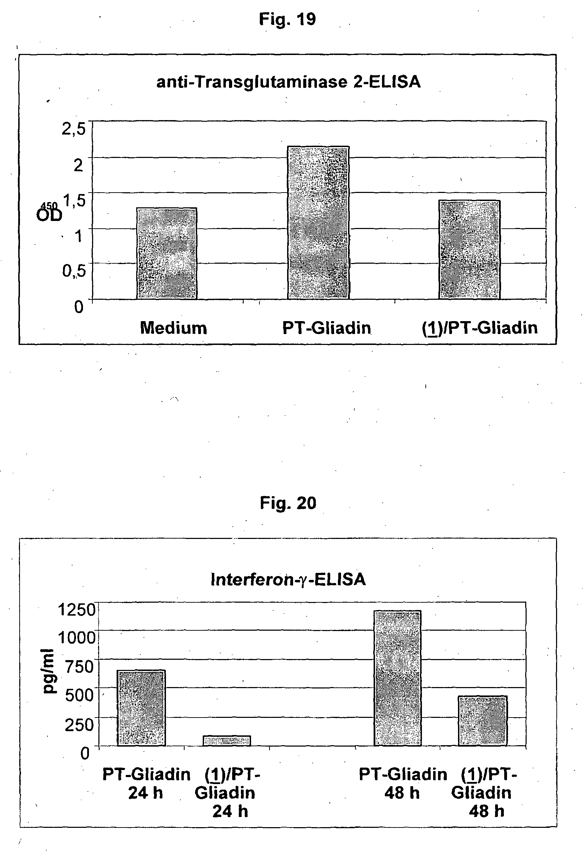

- Figur 19: zeigt die Messwerte der optischen Dichte OD₄₅₀ im anti-TG2-ELISA zwecks Bestimmung der Antikörper- und Autoantikörperkonzentration. Im anti-TG2-ELISA konnte eine OD₄₅₀ von 1,285 für die Mediumkontrolle gemessen werden. Im Überstand der mit PT-Gliadin stimulierten Biopsie wurde eine deutlich erhöhte Antikörperkonzentration (OD₄₅₀ = 2,144) gemessen, während in der mit dem Inhibitor (1) vorinkubierten Biopsie mit OD₄₅₀ = 1,401 nur eine geringfügige Erhöhung der Autoantikörperkonzentration vorhanden war.
Bei Zöliakie-Patienten führt der Verzehr von Gliadin (Getreideprotein, z. B. Brot) zu einer erhöhten Autoantikörperproduktion gegen die TG2. Die im anti-TG2-ELISA gemessene Extinktion (OD₄₅₀) korreliert mit der Autoantikörperkonzentration in der Probe. Die deutlich erhöhte Autoantikörperkonzentration nach Behandlung mit PT-Gliadin zeigt, dass das Biopsie-Modell bzgl. der Autoantikörperprocuktion stimulierbar ist und somit zur Evaluierung von Transglutaminase-Inhibitoren geeignet.
Die geringfügige Erhöhung der OD₄₅₀ und somit der Autoantikörperkonzentration nach Vorinkubation mit Inhibitor (1) zeigt, dass die Inhibitoren im biologischen Modell die gewünschte Wirkung zeigen. Sie reduzieren eindeutig die Stimulation der Autoantikörper-Produktion nach Behandlung mit PT-Gliadin.
- Figur 20: zeigt die Messwerte im Interferon-γ-ELlSA zwecks Bestimmung der Interferon-γ-Werte.
In der mit Inhibitor (1) präinkubierten Biopsie konnten trotz Stimulation nach 24 h nur 86 pg/ml Interferon-γ gemessen werden, nach 48 h waren es lediglich 426 pg/ml.
Mit dem Interferon-γ-ELISA wird die Konzentration von Interferon-γ in einer Probe ermittelt.
Interferon-gamma gehört zu den so genannten Leitzytokinen (Botensstoffe) für Entzündungsreaktionen. Für Zöliakie-Patienten bedeutet dies, dass nach Verzehr von Getreideprotein das Leitzytokin Interferon-γ produziert wird. Erhöhte Interferon-gamma-Konzentrationen führen zur Entzündung der Dünndarmschleimhaut, einem Grundsymptom der Zöliakie.
In Fig.20 ist dargestellt, dass die Inkubation der Biopsien zur Produktion von Interferon-γ führt, wobei eine längere Inkubation erwartungsgemäß eine erhöhte Interferon-Konzentration zur Folge hat. Die Vorinkubation mit Transglutaminase-Inhibitor (1) führt zu einer drastischen Absenkung der Interferon-γ-Konzentration, sowohl nach 24 als auch nach 48 h. Somit konnte im Biopsie-Modell gezeigt werden, dass Transglutaminase-Inhibitor (1) die Produktion des Leitzytokins Interferon-gamma inhibiert.
Gemeinsam mit den Daten aus Beispiel 1 ist somit der Proof-of-Prinziple für die Zöliakie-Therpie mit Transglutaminase-Inhibitoren gezeigt.

### Allgemeine Synthesebeschreibung

### Aufbau des erfindungsgemäßen Michael-Akzeptorsystemes

Michael-Akzeptoren sind Olefine die mit mindestens einem elektronenziehenden Substituenten in Konjugation stehen. Für den Aufbau solcher Michael-Akzeptoren sind deshalb alle die Reaktionen geeignet, die ein solches Olefin generieren. Beispielhaft aber nicht beschränkend seien hierfür Alkenylierungsreaktionen an Metallorganylen, Corey-Winter-Reaktionen, Horner-Wadworth-Emmonsreaktionen, Knoevenagel-Reaktionen, Wittig-Reaktionen, Wittig-Horner-Reaktionen, Julia-Lytgoe-Reaktionen und Peterson-Olefinierungen genannt. Diese und andere olefinbildenden Reaktionen sind dem Fachmann wohlbekannt. Besonders bevorzugt sind dabei die Reaktionen, in denen ein Aldehyd mit einem geeignet substituiertem Phosphory-Ylid oder einem entsprechenden Phosphonat reagiert (Wittig-Reaktion, Wittig-Horner-Reaktion, Horner-Wadworth-Emmonsreaktion). Dragovich et al. zeigen die breite Anwendbarkeit dieses Reaktionstyps zum Aufbau von Michael-Akzeptorsystemen (Dragovich et al., J. Med. Chem., 1998, 41,15, 2806-2818). Die dafür benötigten Reagenzien sind in großem Umfang kommerziell erhältlich (z.B. Sigma-Aldrich) oder in der Literatur beschrieben. Im Folgenden ist eine allgemeine Synthesevorschrift für diese Olefinierungsreaktionen von Aldehyden gegeben. Konkrete Ausführungsbeispiele sind weiter unten aufgeführt.

Ausgegangen wird von einem geeignet substituierten Aldehyd, d.h. von einem Aldehyd der allgemeinen Struktur, worin X ein beliebiger Rest ist:

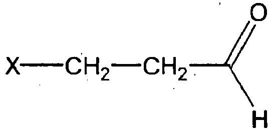

Dieser Aldehyd kann beispielhaft aus Derivaten der Glutaminsäure dargestellt werden wie für Verbindung 1.4 hierin offenbart.

Ein Äquivalent eines Phosphor-Ylides (z.b. Triphenylphosphonium-Ylide) wird in einem geeignetem Lösungsmittel (z.B. Benzol, Toluol oder DMF) gelöst und mit einer Base deprotoniert (z.B. NaH, n-BuLi, NaNH₂). Nach beendeter Reaktion wird ein Äquivalent des jeweiligen Aldehyds zugesetzt. Nach beendeter Reaktion wird das Lösungsmittel i. Vak. entfernt und das erhaltene Olefin durch chromatographische Methoden gereinigt.

### Allgemeine Vorschrift 1:

Allgemeine Synthesevorschrift zu Verbindungen mit einem Alkyloxycarbonylethenyl-Michael-System:

Ausgegangen wird von der Aminosäure Glu (Glutaminsäure), welche am C-Terminus und N-Terminus mit Schutzgruppen versehen wird. Als Schutzgruppen können säurelabile Schutzgruppen wie beispielsweise tert-Butyloxycarbonyl, tert.-Butylester oder 2-Phenylisopropyllester verwendet werden. Mittels Diisobutylaluminiumhydrid wird die Seitenkette selektiv zum Aldehyd reduziert und danach mit einem Phosphoran zwecks Erzeugung des Michael-Systems umgesetzt. Nach säureinduzierter Spaltung beispielsweise mittels Trifluoressigsäure der Schutzgruppen wird an das N-terminale Ende ein Peptidrest oder Peptid-Analogon oder Aminosäure-Analogon oder ein oder zwei Alkylreste eingeführt. Dies erfolgt vorzugsweise mittels einer aktivierten Carbonsäure beispielsweise als Aktivester oder Carbonsäureanhydrid und das C-terminale Ende wird danach mit einer Aminogruppe eines Pepdidrestes oder eines Peptid-Analogons oder eines Aminosäure-Analogons zur Reaktion gebracht oder es wird eine Veresterung durchgeführt. Diese Reaktionen sind universell einsetzbar, dem Fachmann wohl bekannt und daher können an die Aminosäure mit dem Michael-System beliebige peptidische oder peptidähnliche Reste sowohl am C- als auch am N-Terminus hinzugefügt werden.

Anstelle zuerst bei der Aminosäure Glutaminsäure das Michael-System zu erzeugen und danach den N-Terminus und dann den C-Terminus zu modifizieren wie oben geschildert, kann auch zuerst ein gewünschtes Peptid synthetisiert werden, welches danach mit Schutzgruppen versehen wird und woran dann die Modifizierung der Glutaminsäureseitenkette oder der Glutaminsäureseitenketten zum Michael-System oder zu mehreren Michael-Systemen erfolgt. Nach der Erzeugung des oder der Michael-Systeme können die Schutzgruppen teilweise oder vollständig entfernt werden.

### Allgemeine Vorschrift 2:

### Allgemeine Synthesevorschrift zu Verbindungen mit einem Alkyloxycarbonylethenylcarbonyl-Michael-System:

Ausgegangen wird ebenfalls von der Aminosäure Glu (Glutaminsäure), welche am C-Terminus und N-Terminus mit einer Schutzgruppe versehen wird und die Seitenkette wird mittels Diazomethan und danach mittels Dimethyldioxiran in eine vicinale Diketoverbindung (Glyoxal) überführt. Die Diketoseitenketten wird danach mit einem gewünschten Phosphoran zwecks Erzeugung des Michael-Systems umgesetzt. Nach säureinduzierter Spaltung beispielsweise mittels Trifluoressigsäure der Schutzgruppen wird an das N-terminale Ende ein Peptidrest oder Peptid-Analogon oder Aminosäure-Analogon oder ein oder zwei Alkylreste eingeführt. Dies erfolgt vorzugsweise mittels einer aktivierten Carbonsäure beispielsweise als Aktivester oder Carbonsäureanhydrid und das C-terminale Ende wird danach mit einer Aminogruppe eines Pepdidrestes oder eines Peptid-Analogons oder eines Aminosäure-Analogons zur Reaktion gebracht oder es wird eine Veresterung durchgeführt. Diese Reaktionen sind universell einsetzbar, dem Fachmann wohl bekannt und daher können an die Aminosäure mit dem Michael-System beliebige peptidische oder peptidähnliche Reste sowohl am C- als auch am N-Terminus hinzugefügt werden.

Anstelle zuerst bei der Aminosäure Glutaminsäure das Michael-System zu erzeugen und danach den N-Terminus und dann den C-Terminus zu modifizieren wie oben geschildert, kann auch zuerst ein gewünschtes Peptid synthetisiert werden, welches danach mit Schutzgruppen versehen wird und woran dann die Modifizierung der Glutaminsäureseitenkette oder der Glutaminsäureseitenketten zum Michael-System oder zu mehreren Michael-Systemen erfolgt. Nach der Erzeugung des oder der Michael-Systeme können die Schutzgruppen teilweise oder vollständig entfernt werden.

### Beispiele

### Generelle Methode zur Inaktivierung von humaner Gewebetransglutaminase

250 µg lyophilisierte His-getagte rekombinante humane Gewebetransglutaminase (His₆-rh-TG2) werden durch Zusatz von 150 µl Wasser rekonstituiert. (resultierender Puffer 50 mM NaH2PO4, 150 mM NaCl, pH = 8).

Eine 10 mM Inhibitor-Stammlösung in DMSO wird hergestellt und jeweils mit Puffer (50mM Tris-HCl, 10 mM CaCl₂, 5 mM DTT, pH = 7,5) auf das zwanzigfache der im Inhibitionsansatz gewünschten Konzentration verdünnt (mind. jedoch 1/50 Verdünnung resultierend 2% DMSO-Konzentration.).

900 µl einer Assay-Lösung zusammengesetzt aus 55,56 mM Tris, 11,11 mM CaCl₂, 0,11% PEG₈₀₀₀, 5,56 mM DTT, 5,56 mM Glycinmethylester und 50 µM Abz-APE(CAD-DNP)QEA-OH, (Patentnummer: ), pH = 7,5 werden in einer Küvette vorgelegt und in der Messzelle eines Spektrophotometers auf 37°C temperiert. Zu dieser Lösung werden 50 µl der jeweiligen Inhibitorlösung zugesetzt (resultierend eine Konzentration von weniger als 0,2% DMSO im Ansatz).

7 µl der oben rekonstituierten Transglutaminase-Lösung werden mit 51 µl Puffer (50 mM Tris, 100 mM NaCl, 5 mM DTT, pH = 7,5 verdünnt. 50µl dieser Enzymlösung (10µg His₆-rhTG2) werden zu der Assay-Lösung, enthaltend die jeweilige Inhibitorkonzentration, zugesetzt. Es wird 5 min bei 37 °C inkubiert bevor die Messung gestartet wird. (λ_{exc} = 313 nm und λₑₘ = 418 nm,15 min)

Die Auswertung der resultierenden Enzymaktivität erfolgt über die Steigung der durch Zunahme der Fluoreszenz erhaltenen Geraden.

Um die nicht inhibierte Enzymaktivität zu bestimmen, wird DMSO statt Inhibitor-Stammlösung verwendet. IC₅₀-Werte werden bestimmt, indem die resultierende Enzymaktivität gegen den Logarithmus der Inhibitorkonzentration aufgetragen wird. IC₅₀ ist definiert als die Inhibitorkonzentration bei der nach 5 min Vorinkubation 50% Enzymaktivität resultiert.

### Biopsiebeispiel 1

Biopsien mit einem Durchmesser von ca. 2 mm wurden nach Entnahme aus dem tiefen Duodenum von Zöliakie-Patienten maximal 30 min in eiskaltem PBS aufbewahrt. Die einzelnen Biopsien wurden dann in die Vertiefungen einer 24-well Zellkulturschale transferiert und mit 500 µl Trowell T8 Medium überschichtet.

Zur Vorinkubation wurde der Transglutaminaseinhibitor (1) in einer Endkonzentration von 5 µM zu den Biopsien gegeben und 30 min bei 37°C im Brutschrank unter CO₂-Begastung (5%) inkubiert. Im Anschluss erfolgte die Stimulierung mit peptisch-tryptisch verdautem Gliadin (PT-Gliadin) das in einer Endkonzentration von 1 mg/ml eingesetzt wurde. PT-Gliadin wurde nach der Vorschrift von Wieser und Belitz hergestellt (Wieser H und Belitz HD (1992). Z Lebenm Unters Forsch 194:229-234). Durch die Behandlung mit den Proteasen Pepsin und Trypsin wird die Verdauung im Magendarmtrakt simuliert, so dass die entstandenen Gliadin-Peptide denen entsprechen, die auch i m Dünndarm nach Verzehr von Getreideprotein vorkommen. Die Biopsien wurden nun für weitere 48 h unter obigen Bedingungen im Brutschrank inkubiert.

Kontrollen wurden mit inhibitorfreiem Medium vorinkubiert sowie ohne bzw. mit PT-Gliadin inkubiert.

Nach 48 h wurden 50 µl Probe entnommen und mit einem anti-TG2- ELISA-Kit analysiert.

Im anti-TG2-ELISA konnte eine OD₄₅₀ von 1,285 für die Mediumkontrolle gemessen werden. Im Überstand der mit PT-Gliadin stimulierten Biopsie wurde eine deutlich erhöhte Antikörperkonzentration (OD₄₅₀ = 2,144) gemessen, während in der mit dem Inhibitor (1) vorinkubierten Biopsie mit OD₄₅₀ = 1,401 nur eine geringfügige Erhöhung der Autoantikörperkonzentration vorhanden war (s. Fig 19).

### Biopsiebeispiel 2

Biopsien wurden wie in Biopsiebeispiel 1 beschrieben kultiviert und behandelt. Nach 24 und 48 h wurden von der mit PT-Gliadin stimulierten Biopsie als auch von der mit Inhibitor (1) präinkubierten Biopsie, die im Anschluss ebenfalls mit PT-Gliadin stimuliert wurde 50 µl Probe entnommen und mit einem Interferon-γ-ELISA analysiert. In der mit PT-Gliadin stimulierten Biopsie konnte bereits nach 24 h ein sehr hoher Interferon-γ-Wert von 653 pg/ml gemessen werden, der nach 48 h auf 1177 pg/ml anstieg. In der mit Inhibitor (1) präinkubierten Biopsie konnten trotz Stimulation nach 24 h nur 86 pg/ml Interferon-γ gemessen werden, nach 48 h waren es lediglich 426 pg/ml (s. Fig. 20).

### Aufbau benötigter Peptidsequenzen:

Für den Aufbau der nicht-modifizierten Peptidsequenzen werden Standardmethoden verwendet. Grundsätzlich ist für den Peptidaufbau der Einsatz aller in der Literatur bekannten Methoden möglich. (siehe dazu auch: Bodanzky M, Bodanzky A., *The practice of peptide synthesis,* Springer Verlag, 1994) Exemplarisch werden zwei Methoden angegeben, die am häufigsten zum Einsatz kommen.
1. Aufbau einer Peptidsequenz in Lösung: α-Aminofunktion der zu koppelnden Aminosäuren geschützt mit der tert.-Butyloxycarbonyl-Schutzgruppe (Boc-Schutzgruppe), Kopplung an freies Amin mit O-(Benzotriazol-1-yl)-N,N,N',N'-tetramethyluronium-hexafluorborat (TBTU), 1-Hydroxybenzotriazol (HOBt) und N,N-Diethylisopropylamin), Abspaltung der Boc-Schutzgruppe durch Trifluoressigsäure (TFA) in Dichlormethan (DCM). Anschließend Kopplung der nächsten Aminosäure.
2. Aufbau einer Peptidsequenz an fester Phase: Ausgehend von kommerziell erhältlichen, über einen 2-Chlortrityllinker polymer gebundenen Startaminosäuren werden 9-Fluorenylmethyl (Fmoc)-geschütze Aminosäuren der Sequenz entsprechend gekoppelt. Als Reagenzien kommen außerdem TBTU, HOBt und DIPEA zum Einsatz. Dem Fachmann sind die Reaktionsbedingungen bekannt. Für weitergehende Informationen siehe z.B.: *Fmoc Solid Phase Peptide Synthesis, A practical approach, Chan, W.C., White P.D., Oxford University Press*

Nachfolgen sind einige Beispielpeptide angegeben, die zum Aufbau verschiedener Inhibitoren benötigt werden.

### L-Valinyl-L-Prolinyl-Isopentylamid (5.1)

### Boc-L-Prolinyl-Isopentylamid

479 mg (5,5 mmol) Isopentylamid werden in 5 ml DMF gelöst. Zu dieser Lösung wird eine Lösung aus 1,06 g Boc-Prolin (5 mmol), 1,57 g TBTU (4,9 mmol), 675 mg HOBt (5 mmol) und 1,71 ml DIPEA (10 mmol) in 15 ml DMF zugesetzt. Nach einer Stunde wird das Lösungsmittel i. Vak. entfernt und der ölige Rückstand in 200 ml Ethylacetat aufgenommen. Die organische Phase wird je drei Mal mit je 50 ml 10%iger Citronensäure, 10%iger NaHCO₃ und gesättigter NaCl-Lösung gewaschen. Die organische Phase wird über Na₂SO₄ getrocknet und das Lösungsmittel i. Vak. entfernt.
Ausbeute: 1,38 g

### Abspaltung der N^{α}-tert.Butyloxycarbonyl-Schutzgruppe

Die so dargestellte Zwischenverbindung wird in 25 ml Dichlormethan gelöst und 25 ml TFA zugesetzt. Bei Raumtemperatur wird für eine Stunde gerührt. Das Lösungsmittel wird i. Vak. entfernt und Reste von TFA durch codestillieren mit Methanol und Trocknen im Hochvakuum entfernt.

### Boc-L-Valinyl-Prolinyl-Isopentylamid

Das oben erhaltene Trifluoracetatsalz (TFA*Pro-Isopentylamid) wird in DMF gelöst und eine Lösung aus 1,15 g Boc-Valin (5,33 mmol), 1,6 g TBTU (5,1 mmol), 719 mg HOBt (5,33 mmol) und 1,88 ml DIPEA (11 mmol) in 20 ml DMF zugesetzt. Nach einer Stunde wird das Lösungsmittel i. Vak. entfernt und der ölige Rückstand in 200 ml Ethylacetat aufgenommen. Die organische Phase wird je drei Mal mit je 50 ml 10%iger Citronensäure, 10%iger NaHCO₃ und gesättigter NaCl-Lösung gewaschen. Die organische Phase wird über Na₂SO₄ getrocknet und das Lösungsmittel i. Vak. entfernt. Die Abspaltung der *N*^{α}-*tert.Butyloxycarbonyl-Schutzgruppe* liefert Verbindung 5.a Ausbeute: 1,75 g
ESI-MS: 284,2 [M+H]⁺

### L-Serinyl-L-Leucinyl-L-Valinyl-L-Isoleucinyl-Glycinmethylester (8.1)

Die Titelverbindung wird nach der o.g. Standardmethode ausgehend von Glycinmethylester in Lösung aufgebaut.

Exemplarisch wird im Folgenden die erste Kopplung beschrieben. Die nachfolgenden Kopplungen werden unter identischen Bedingungen durchgeführt.

### N^{α}-tert.-Butyloxycarbonyl-L-Isoleucinyl-Glycinmethylester

850 mg N^{α}-tert.-Butyloxycarbonyl-L-Isoleucin (3,68 mmol) werden in 10 ml DMF zusammen mit 1,18 g TBTU (3,68 mmol) und 497 mg HOBt (3,68 mmol) gelöst. Durch Zusatz von 1,9 ml DIPEA (11,1 mmol) wird die Lösung auf pH ∼ 11 eingestellt und der Reaktionsansatz mit einer Lösung aus 461,4 mg Glycinmethylesterhydrochlorid (3,68 mmol) in 5 ml DMF versetzt. Bei Raumtemperatur wird für 45 min gerührt und danach das Lösungsmittel i. Vak. entfernt. Der erhaltene ölige Rückstand wird in 200 ml Ethylacetat aufgenommen und mit jeweils 50 ml 10%iger Citronensäure, 10%iger NaHCO₃ und Wasser je drei Mal gewaschen. Die organische Phase wird mit Na₂SO₄ getrocknet und das Lösungsmittel i. Vak. entfernt. Man erhält einen farblosen Feststoff.
Ausbeute: 1,08 g (97%d. Th)

### Abspaltung der N^{α}-tert.Butyloxycarbonyl-Schutzgruppe

Das geschützte Peptid wird in Dichlormethan gelöst und mit dem gleichen Volumen Trifluoressigsäure versetzt. Nach einer Stunde wird das Lösungsmittel i. Vak. entfernt. Säurereste werden durch mehrmaliges codestilieren mit Methanol entfernt. Das so erhaltene freie Amin kann direkt mit der nächsten Aminosäure gekoppelt werden.

Die Titelverbindung (8.1) wird durch wiederholte Kopplungs- und Entschützungsreaktionen als hellbrauner Feststoff erhalten.
ESI-MS: 502,2 [M+Na]⁺

### N^{α}-Acetyl-L-Leucinyl-Glycinyl-L-Prolinyl-Glycin (8.2)

Die Titelverbindung wird durch Festphasenpeptidsynthese aufgebaut. Ausgehend von kommerziell erhältlichem H-Glycin-2-Chlortritylester (polymer gebunden), werden mit TBTU, HOBt und DIPEA die Fmoc-geschüzten Aminosäuren der Sequenz entsprechend gekoppelt. Dem Fachmann sind die Reaktionsbedingungen bekannt. Für weitergehende Informationen siehe z.B.: Fmoc Solid Phase Peptide Synthesis, A practical appraach, Chan, W.C., White P.D., Oxoford University Press

Nach Abspaltung vom polymeren Träger und Reinigung des erhaltenen Rohproduktes durch Waschen mit Diethylether erhält man die Titelverbindung in reiner Form.
ESI-MS: 407,2 [M+Na]⁺

Im Folgenden werden die Synthesen der Inhibitoren beschrieben. Die dabei verwendeten Peptide werden, soweit nichts Gegenteiliges vermerkt ist, nach einer der oben beschriebenen Methoden hergestellt.

### 1. Darstellung von 6-Amino-hept-2-endicarbonsäure-derivaten

### N^{α}-tert.Butyloxycarbonyl-L-Glutaminsäure-5-methylester-1-tert.-Butylester (1.2)

2,3 g N-tert.Butyloxycarbonyl-L-Glutaminsäure-1-tert.-Butylester (7,58 mmol) werden in 80 ml Methanol gelöst und bei Raumtemperatur eine frisch hergestellten Diazomethanlösung zugetropft (23 mmol Diazald^{®}). Nach einer Stunde wird das Lösungsmittel i. Vak. entfernt. Die Reinigung der Verbindung erfolgt durch Chromatographie an Kieselgel. (Säule: 18,5*4 cm, DCM/MeOH = 99/1, R_{f} = 0,99)
Ausbeute: 1,3 g
ESI-MS: 340,2 [M+Na]⁺

### N,N-Di-(tert.-Butyloxycarbonyl)-L-Glutaminsäure-5-methylester-1-tert.-Butylester (1.3)

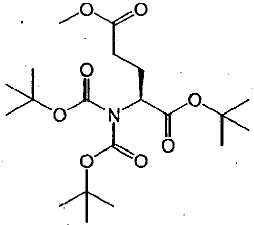

1,3 g N-tert.Butyloxycarbonyl-L-Glutaminsäure-5-methylester-1-tert.-Butylester (1.2, 4,1 mmol) werden in 15 ml Acetonitril gelöst und mit 100 mg N,N-Dimethyl-4-aminopyridin (DMAP) versetzt. Unter Stickstoffatmosphäre wird eine Lösung aus 1,79 g Di-tert.-Butyl-bicarbonat (8,2 mmol) in 7 ml Acetonitril zugesetzt. Nach Rühren über Nacht wird das Lösungsmittel i. Vak. entfernt und das erhaltene Rohprodukt durch Chromatographie an Kieselgel gereinigt.
(Säule: 33*3,5 cm, Petrolether/Ethylacetat = 92/8, R_{f} = 0,32)
Ausbeute: 1,3 g
ESI-MS: 440,3 [M+Na]⁺

### N,N-Di-(tert.-Butyloxycarbonyl)-L-2-Amino-5-Oxopentansäure-tert.-Butylester (1.4)

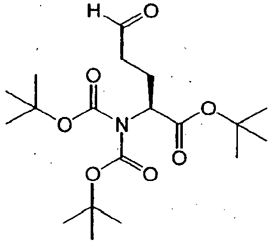

1,31 g N,N-Di-(tert.-Butyloxycarbonyl)-L-Glutaminsäure-5-methylester-1-tert.-Butylester (1.3, 3,14 mmol) werden in 40 ml absolutem Diethylether gelöst und unter Argonatmosphäre auf - 78 °C gekühlt. Bei dieser Temperatur werden langsam 3,45 ml einer Lösung von Diisobutylaluminiumhydrid (1 M in Hexan) zugetropft. Nach beendeter Zugabe wird für weitere 15 min bei - 78 °C gerührt, bevor bei dieser Temperatur der Reaktionsansatz durch Zugabe von 1,5 ml Wasser gequencht wird. Unter starkem Rühren wird auf Raumtemperatur aufgetaut und die trübe Lösung über Celite filtriert. Das Filtrat wird bis zur Trockne eingeengt und Restwasser durch Codestillation mit Toluol entfernt. Die Reinigung erfolgt durch Chromatographie an Kieselgel (Säule: 37*3,2 cm, Petrolether/Ethylacetat = 92/8 auf 90/10)
Ausbeute: 890 mg
500-MHz-¹H-NMR-cosy (DMSO_{d6}):δ[ppm]=9,65 (s, 1H, H-4), 4,63 (dd, 1H, H-1, J_{1/2a} = 4,8 Hz, J_{1/2b} = 9,85 Hz), 2,51-2,50 (m, 1H, H-3ₐ), 2,48-4,40 (m, 1H, H-3_{b}), 2,27-2,20 (m, 1 H, H-2a), 1,98-1,91 (m, 1 H, H-2_{b}), 1,44 (s, 18H, 6*CH₃(Boc)), 1,92 (s, 9H, 3*CH₃(O-tBu)
ESI-MS: 410,4 [M+Na]⁺, 428,4 [M+H₂O+Na]⁺

### N,N-Di-(tert.-Butyloxycarbonyl)-{(E)-(L)-6-Amino-hept-2-endicarbonsäure}-1-ethanoyl-7-tert.Butylester (1.5)

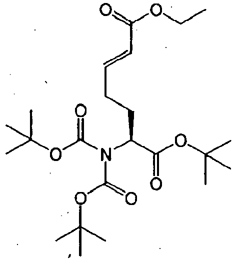

160 mg N, N-Di-(tert.Butyloxycarbonyl)-L-2-Amino-5-Oxopentansäure-1-tert.-Butylester (1.4, 0,413 mmol) werden in 8 ml trockenem Benzol vorgelegt und unter Argonatmosphäre bei Raumtemperatur eine Lösung von 152 mg (Ethoxycarbonylmethylen)-triphenylphosphoran (0,413 mmol) zugesetzt. Nach Rühren über Nacht, wird das Lösungsmittel i. Vak. entfernt und der erhaltene ölige Rückstand durch präparative HPLC gereinigt. (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1%/min).
Rₜ: 98-103,6 min
Ausbeute: 80 mg
ESI-MS: 480,3 [M+Na]⁺

### N^{α}-Benzyloxycarbonyl-{(E)-(L)-6-Amino-hept-2-endicarbonsäure}-1-ethylester (1.7)

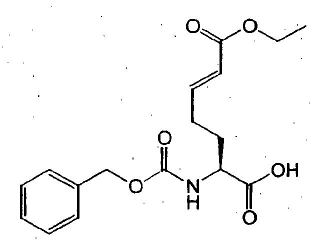

80 mg N,N-Di-(tert.-Butyloxycarbonyl)-{(E)-(L)-6-Amino-hept-2-endicarbonsäure}-1-ethanoyl-7-tert.Butylester (1.5, 0,175 mmol) werden in 5 ml Dichlormethan gelöst und unter Stickstoffatmosphäre auf 0 °C gekühlt. Zu dieser Lösung werden 5 ml TFA langsam zugesetzt. Nach zwei Stunden Rühren bei dieser Temperatur wird das Lösungsmittel i. Vak. entfernt. Der erhaltene braune Rückstand (*L-6-Amino-hept-2-endicarbonsäure-1-ethylester,* 1.6) wird im Hochvakuum von restlichem TFA befreit. Man erhält 64 mg braunen Feststoff (116% bez. auf das TFA-Salz.).

Das so erhaltene Produkt wird direkt weiter umgesetzt, indem es zu einer Lösung aus 65,4 mg N-(Benzyloxycarbonyl)-succinimid (0,262 mmol) in 4 ml DMF zugesetzt wird. Unter Stickstoffatmosphäre wird DIPEA zugesetzt, so dass der pH-Wert bei ca. 6 liegt. Nach einer Stunde wird die erhaltene klare Lösung i. Vak. bis zur Trockne eingeengt und der feste Rückstand durch präparative HPLC gereinigt (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1%/min)
Rₜ: 64,0-66,5 min
Ausbeute: 64 mg
500-MHz-¹H-NMR-cosy (DMSO_{d6}): δ[ppm] =7,62 (d, 1H, NH), 7,37-7,30 (m, 5H, Aryl-H), 6,87 (dt, 1H, H-4, J_{4/3} = 6,9 Hz J_{4/5} = 15,6 Hz), 5,84 (d, 1H, H-5, J_{5/4} = 15,6 Hz), 5,02 (s, 2H, Benzyl-CH₂), 4,11 (q, 2H, H-6ₐ, H-6_{b}, J_{6/7} = 7,1Hz), 4,08-4,00 (m, 1H, H-1), 2,30-2,23 (m, 2H, H-3ₐ, H-3_{b}), 1,90-1,80 (m, 1 H, H-2ₐ, H-2_{b}), 1,20 (t, 3H, CH₃-7)
ESI-MS: 358,2 [M+Na]⁺

### N,N-Di-(tert.-Butyloxycarbonyl)-{(E)-(L)-6-Amino-hept-2-endicarbonsäure}-1-methanoyl-7-tert.-Butylester (1.8)

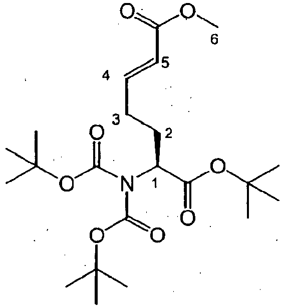

445 mg N,N-Di-(tert.-Butyloxycarbonyl)-L-2-Amino-5-Oxopentansäure-1-tert.-Butylester (1.4, 1,15 mmol) werden in 20 ml trockenem Benzol vorgelegt und unter Argonatmosphäre bei Raumtemperatur eine Lösung von 385 mg (Methoxycarbonylmethylen)-triphenylphosphoran (1,15 mmol) zugesetzt. Nach Rühren über Nacht, wird das Lösungsmittel i. Vak. entfernt und der erhaltene ölige Rückstand durch Chromatographie an Kieselgel gereinigt. (Säule: 29*2,4 cm, Petrolether/Ethylacetat = 99,5/0,5)
Ausbeute: 424 mg
500-MHz-¹H-NMR-cosy (DMSO_{d6}): δ[ppm] = 6,66 (dt, 1H, H-4, J_{4/3} = 6,8 Hz J_{4/5} = 15,9 Hz), 5,64 (d, 1 H, H-5, J_{5/4} = 15,9 Hz), 4,45-4,2 (m, 1H, H-1), 3,44 (s, 3H, CH₃-6), 2,01-1,95 (m, 2-H, H-3ₐ, H-3_{b}), 1,95-1,86 (m, 1H, H-2ₐ), 1,78-1,67 (m, 1H, H-2_{b}), 1,24 (s, 18H, 6*CH₃(Boc)),
ESI-MS: 466,3 [M+Na]⁺

### N^{α}-tert.-Butyloxycarbonyl-{(E)-(L)-6-Amino-hept-2-endicarbonsäure}-1-ethylester (1.9)

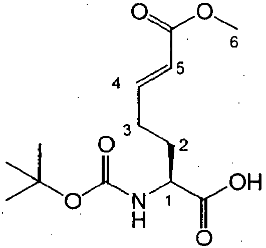

50 mg N,N-Di-(tert.-Butyloxycarbonyl)-{(E)-(L)-6-Amino-hept-2-endicarbonsäure}-1-methanoyl-7-tert.-Butylester (1.8, 0,11 mmol) werden in 5 ml Dichlormethan gelöst und unter Stickstoffatmosphäre auf 0 °C gekühlt. Bei dieser Temperatur werden 5 ml Trifluoresssigsäure zugesetzt und für zwei Stunden bei Raumtemperatur gerührt. Das Lösungsmittel wird i. Vak. entfernt und der erhaltene grünliche Rückstand (*L-6-Amino-hept-2-endicarbonsäure-1-methylester*) mehrfach mit Methanol codestilliert um restliche TFA zu entfernen. Man erhält 88 mg des Trifluoracetatsalzes (139% d.Th.). Der ölige Rückstand wird in 4 ml DMF aufgenommen und mit 36 mg Boc-OSu (1,65 mmol) versetzt. Durch Zusatz von DIPEA wird der pH-Wert auf ca. 6 eingestellt. Nach Rühren bei Raumtemperatur über Nacht, wird das Lösungsmittel i. Vak. entfernt und das Produkt in reiner Form durch präparative HPLC erhalten. (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1%/min).
Rₜ: 53,5-56,5 min
Ausbeute: 23 mg
ESI-MS: 310.1 [M+Na]⁺

### N^{a}-Acetyl-{(E)-(L)-6-Amino-hept-2-endicarbonsäure}-1-methylester (1.10)

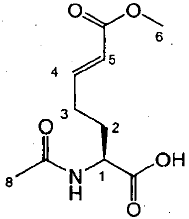

Wie unter 1.9 beschrieben wird zunächst *L-6 Amino-hept-2-endicarbonsäure-1-methylester* hergestellt, 0,1 mmol). Das Zwischenprodukt wird in 10 ml DMF aufgenommen und unter Stickstoffatmosphäre eine Lösung von 68 mg Pentafluorphenylacetat (0,3 mmol) in 4 ml DMF zugesetzt. Bei Raumtemperatur wird über Nacht gerührt bevor das Lösungsmittel i. Vak. entfernt wird. Das reine Produkt wird durch präparative HPLC erhalten..
(Synergie Max, 4µm, 250x21,2 mm, A-Eluent: 0,1 %TFA / Wasser, B-Eluent: 90% AcCN / 10% Wasser / 0,1 %TFA. Gradient: 8 ml/min, 5% B auf 100% B, 1 %/min).
Rt: 54,6-56,8 min
Ausbeute: 19 mg
ESI-MS: 252,1 [M+Na]⁺

### N^{α}-Benzyloxycarbonyl-{(E)-(L)-6-Amino-hept-2-endicarbonsäure}-1-methylester (1.11)

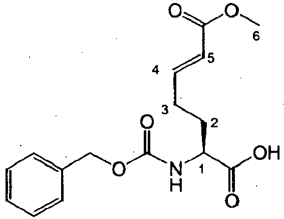

Wie unter 1.10 beschrieben, wird *L-6-Amino-hept-2-endicarbonsäure-1-methylester**TFA (0,2 mmol) hergestellt. Das Zwischenprodukt wird in 20 ml DMF aufgenommen und unter Stickstoffatmosphäre eine Lösung von 75 mg N-(Benzyloxycarbonyl)-succinimid (0,3 mmol) in 7 ml DMF zugesetzt. Bei Raumtemperatur wird über Nacht gerührt bevor das Lösungsmittel i. Vak. entfernt wird. Das reine Produkt wird durch präparative HPLC erhalten (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1 %/min).
Rₜ:38,4-42,4 min
Ausbeute: 25 mg
ESI-MS: 344,1 [M+Na]⁺

### L-2-(4-Chloro-butyrylamino)-pentandicarbonsäure-1-tert.-Butylester-5-methylester (1.12)

933 mg L-Glutaminsäure-5-methylester-1-tert.-Butylester-hydrochlorid (3,68 mmol) werden 20 ml Dichlormethan_{abs}. gelöst und nach Zusatz von 1,28 ml DIPEA (7,5 mmol) auf 0 °C abgekühlt. Zu dieser Lösung werden 518,5 mg 4-Chlorbuttersäurechlorid zugesetzt. Über Nacht wird gerührt während sich der Ansatz allmählich auf Raumtemperatur erwärmt. Die Lösung wird mit Dichlormethan auf 100 ml verdünnt und mit 10%iger Citronensäure sowie gesättigter NaCl Lösung gewaschen (je drei Mal). Die organische Phase wird über Na₂SO₄ getrocknet und das Lösungsmittel i. Vak. entfernt. Man erhält das Produkt in reiner Form als farbloses Öl.
Ausbeute: 1,15 g
ESI-MS: 344,1 [M+Na]⁺

### L-2-(2-Oxo-pyrrolidin-1-yl)-pentandicarbonsäure-1-tert.-Butylester-5-methylester (1.13)

1,15 g 2-(4-Chloro-butyrylamino)-pentandicarbonsäure-1-tert.-Butylester-5-methylester (1.12, 3,58 mmol) werden in 10 ml DMF gelöst. Unter Argonatmosphäre wird auf 0 °C gekühlt und 173 mg Natriumhydrid (60% auf Mineralöl) zugesetzt. Nach 15 min wird das Eisbad entfernt und für vier Stunden bei Raumtemperatur gerührt. Das Lösungsmittel wird im Hochvakuum entfernt und der Rückstand in 200 ml Ethylacetat aufgenommen. Nach Waschen mit 1 N HCl, 10%iger NaHCO₃ und gesättigter NaCl-Lösung wird über Na₂SO₄ getrocknet und das Lösungsmittel erneut i. Vak. entfernt. Man erhält das reine Produkt als blassgelbes Öl.
Ausbeute: 908 mg
ESI-MS: 308,3 [M+Na]⁺, 252,3 [M-t-Bu+Na]⁺

### (E)-(L)-6-(2-Oxo-pyrrolidin-1-yl-)hept-2-endicarbonsäure-1ethylester (1.14)

100 mg 2-(2-Oxo-pyrrolidin-1-yl)-pentandicarbonsäure-1-tert.-Butylester-5-methylester (1.13, 0,35 mmol) werden in 10 ml Diethylether_{abs}. gelöst und unter Argonatmosphäre auf -78 °C gekühlt. Bei dieser Temperatur werden langsam 0,385 ml einer einmolaren Lösung von Diisobutylaluminiumhydrid in Hexan zugetropft. Nach 30 min. wird der Ansatz durch Zusatz von 1 ml Wasser gequencht und anschließend auf Raumtemperatur aufgetaut. Die Reaktionslösung wird über Kieselgut filtriert, zweimal mit Diethylether nachgewaschen und die vereinigten organischen Phasen eingeengt.
Ausbeute: 83 mg
ESI-MS: 278,2 [M+Na]⁺

### (L)-(E)-(L)-6-(2-Oxo-pyrrolidon-1-yl)-hept-2-endicarbonsäure-1-ethylester (1.15)

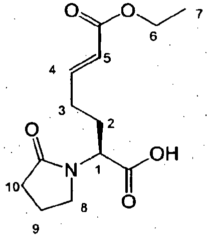

70 mg (E)-6-(2-Oxo-pyrrolidin-1-yl-)hept-2-endicarbonsäure-1ethylester (1.14, 0,27 mmol) werden in 5 ml über Molsieb (4Å) getrocknetem Benzol gelöst. Unter Stickstoffatmosphäre wird eine Lösung von 95 mg Carboxymethylentriphenylphosphoran zugesetzt und bei Raumtemperatur über Nacht gerührt. Das Lösungsmittel wird i. Vak. entfernt und der erhaltene ölige Rückstand in 10 ml Dichlormethan aufgenommen und mit 10 ml Trifluoressigsäure versetzt. Nach einer Stunde wird das Lösungsmittel i. Vak. entfernt und das Rohprodukt durch Chromatographie an Kieselgel gereinigt. (Säule:21x1,2 cm, Petrolether/Ethylacetat: 8/2)
ESI-MS: 292,1 [M+Na]⁺,

### Alternative Schutzgruppenstrategie zur Herstellung von 6-Amino-hept-2-endicarbonsäure

Um eine orthogonale Schutzgruppenstrategie zu ermöglichen, kann folgender Weg beschritten werden: Ein am Stickstoff geschütztes Derivat von Glutaminsäure, beispielhaft Z-Glu(OMe)-OH, wird in Dichlormethan gelöst und nach Zusatz von 2-Phenylisopronanol und N,N-Dimethyl-4-aminopyridin (DMAP) mit 1,5 Äquivalenten Dicyclohexylcarbondiimid versetzt. Nach Rühren bei Raumtemperatur über Nacht, wird vom ausgefallenen Feststoff abfiltriert und das erhaltene Rohprodukt durch Chromatographie an Kieselgel gereinigt. Man erhält das Produkt (Z-Glu(OMe)-OiPrPh) in reiner Form.

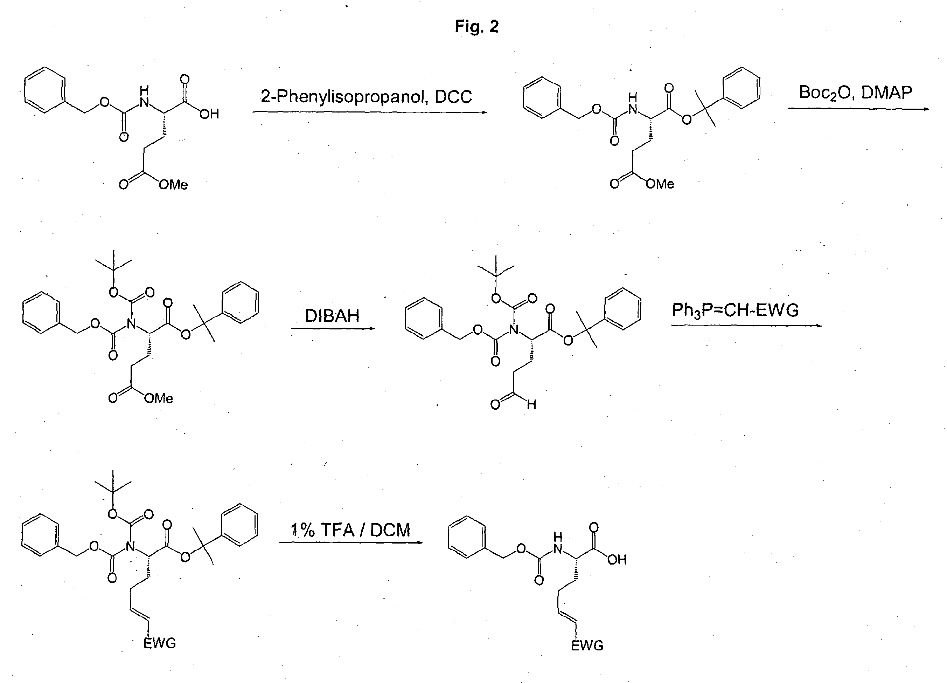

Z-Glu(OMe)-iPrPh wird, wie unter 1.3 beschrieben, am Stickstoff durch eine tert.-Butyloxycarbonyschutzgruppe geschützt und das Produkt zum Aldehyden Z,Boc-Glu(H)-iPrPh reduziert, wie unter 1.4 beschrieben. Die Umsetzung zum Olefin unter den Bedingungen der Wittig-Reaktion wird, wie beispielhaft unter 1.8 beschrieben durchgeführt, bevor durch Behandlung mit 1 % TFA in Dichlormethan die Abspaltung der Caboxylschutzgruppe zusammen mit einer der beiden Aminoschutzgruppen erreicht werden kann. Man erhält auf diesem Weg direkt Verbindungen wie 1.7 oder 1.8, abhängig von der Wahl des Wittig-Reagenzes, die weiteren Reaktionen wie zum Beispiel der Kopplung an ein Amin zugänglich sind (Figur 2). Ein Ausführungsbeispiel ist unter 3.6 beschrieben.

### 2. Darstellung von Inhibitoren mit peptidischer Umgebung der pharmakophoren Gruppe

Eine bevorzugte Ausführungsform erfindungsgemäßer Inhibitoren ist eine Peptidsequenz aus proteinogenen α-Aminosäuren, in die an geeigneter Position eine erfindungsgemäße pharmakophore Gruppe eingeführt wird. Im Folgenden werden eine Reihe solcher peptidischer Inhibitoren beschrieben.

### N^{α}-Benzyloxycarbonyl-{[(E)-(L)-6-Amino-hept-2-endicarbonsäure]-1-ethanoyl}-L-Valinyl-L-Prolinyl-L-Leucinmethylester (Verbindung 1)

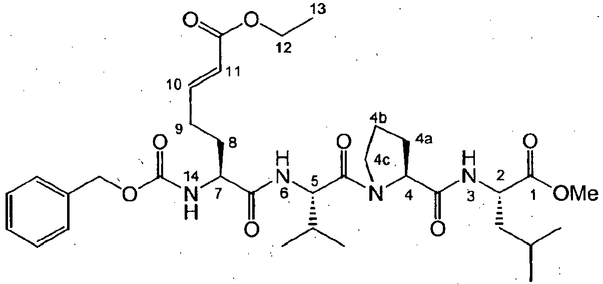

15 mg N^{α}-Benzyloxycarbonyl-{(E)-(L)-6-Amino-hept-2-endicarbonsäure}-1-ethylester (1.7, 44,7 µmol) werden zusammen mit HATU in 5 ml DMF gelöst. Zu dieser Lösung werden 22,8 µl DIPEA (134,2 µl) zugesetzt und die erhaltene gelbe Lösung sofort zu einer Lösung von 20,4 mg (44,7 µmol) des Trifluoracetatsalzes von L-Valinyl-L-Prolinyl-L-Leucinmethylester (hergestellt nach Methode 1) zugesetzt. Der pH-Wert (gemessen mit einem befeuchteten Indikatorstäbchen), wird auf 9 eingestellt. Dazu werden 44,7 µmol zusätzliches DIPEA benötigt. Nach zehn Minuten wird das Lösungsmittel i. Vak. entfernt und der ölige braune Rückstand durch präparative HPLC gereinigt (Synergie Max, 4µm, 250x21,2 mm, A-Eluent: 0,1 %TFA / Wasser, B-Eluent: 90% AcCN / 10% Wasser / 0,1%TFA. Gradient: 8 ml/min, 40% B auf 100% B, 1%/min).
Rₜ: 37,8-40,32 min
Ausbeute: 17 mg
500-MHz-¹H-NMR-cosy (DMSO_{d6}): δ[ppm] = 8,19 (d, 1H, H-3), 7,88 (d, 1H, H-6), 7,47 (d, 1H, H-14), 7,37-7,31 (m, 5H, Aryl-H), 6,87 (dt, 1H, H-10, J_{10/9} = 6,6 Hz, J_{11/10} = 15,4 Hz), 5,81 (d, 1H, H-11, J_{11/10} = 15,4 Hz), 5,02 (s, 2H, Benzyl-CH₂), 4,37 -4,28 (m, 2H, H-5, H-4), 4,28-4,20 (m, 1H, H-2), 4,10 (q, 2H, H-12ₐ, H-12_{b}), 4,08-4,00 (m, 1H, H-7), 3,73-3,67 (m, 1 H, H-4cₐ), 3,61 (s, 3H, OMe), 3,60-3,52 (m, 1 H, H-4c_{b}), 2,27-2,15 (m, 2H, H-9ₐ, H-9_{b}), 2,10-2,00 (m, 1H, H-4_{a/1}), 2,00-1,90 (m, 2H, H-4_{b/1}, Methin-H(Val)), 1,88-1,78 (m, 3H, H-4_{a/2}, H-4_{b/2}), 1,78-1,65 (m, 2H, H-8ₐ, Methin-H (Leu)), 1,65-1,60 (m, 1H, H-8_{b}), 1,58-1,50 (m, 1H, CH₂ₐ-Leu), 1,50-1,43 (m, 1H, CH_{2b}-Leu), 1,22 (t, 3H, CH₃-16), 0,89 (dd, 6H, 2xCH₃-Val), 0,84 (dd, 6H, 2xCH₃-Leu)
ESI-MS: 681,4 {M+Na}⁺

### N^{α}-Benzyloxycarbonyl-{[(E)-(L)-6-Amino-hept-2-endicarbonsäure]-1-ethanoyl}-L-Glutaminyl-L-Prolinyl-L-Leucinmethylester (Verbindung 2)

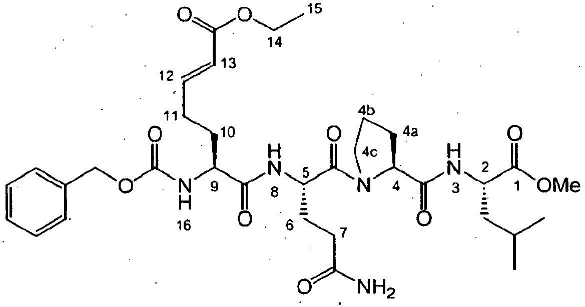

33,5 mg N^{α}-Benzyloxycarbonyl-{(E)-(L)-6-Amino-hept-2-endicarbonsäure}-1-ethylester (1.7, 0,1 mmol) werden in 7,5 ml DMF gelöst und nacheinander mit 38 mg HATU (0,1 mmol) und 51 µl DIPEA (0,3 mmol) versetzt und sofort zu einer Lösung des Trifluoracetatsalzes von 0,1 mmol H-Gln-Pro-Leu-OMe (nach Methode 1 dargestellt) in 7,5 ml DMF zugesetzt. Durch sukzessive Zugabe von DIPEA wird der pH-Wert auf 9 eingestellt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 30% B auf 100% B, 1 %/min).
Rₜ = 33,9-36,1 min
Ausbeute: 28 mg
500-MHz-¹H-NMR-cosy (DMSO_{d6}): δ[ppm] = 8,19 (d, 1H, H-3), 8,11 (d, 1H, H-8), 7,45 (d, 1H, H-16), 7,37-7,28 (m, 5H, Aryl-H), 7,20 (br. s., 1 H, CONH₂), 6,87 (dt, 1H, H-12, J_{12/13} = 15,6 Hz), 6,78 (br. s., 1H, CONH₂), 5,82 (d, 1H, H-11, J_{13/12} = 15,6 Hz), 5,03 (s, 2H, Benzyl-CH₂), 4,50 -4,43 (m, 1 H, H-5), 4,37-4,34 (m, 1 H, H-4), 4,27-4,22 (m, 1H, H-2), 4,10 (q, 2H, H-14ₐ, H-14_{b}), 4,05-4,00 (m, 1 H, H-9), 3,65-3,57 (m, 2H, H-4cₐ, H-4c_{b}), 3,61 (s, 3H, OMe), 2,25-2,19 (m, 2H, H-11ₐ, H-11_{b}), 2,16-2,10 (m, 2H, H-7ₐ, H-7_{b}), 2,10-2,00 (m, 1H, H-4_{a/1}), 1,95-1,70 (m, 5H, H-4_{a/2}, H-4_{b/1}, H-4_{b/2}, H-6ₐ, H-10ₐ), 1,71-1,60 (m, 3H, H-10_{b}, Methin-H (Leu), H-6_{b}), 1,65-1,60 (m, 1H, H-8_{b}), 1,60-1,51 (m, 1H, CH₂ₐ-Leu), 1,51-1,45 (m, 1H, CH_{2b}-Leu), 1,21 (t, 3H, CH₃-16), 0,87 (dd, 6H, 2xCH₃-Leu)
ESI-MS: 710,4 {M+Na}⁺

### N^{α}-Benzyloxycarbonyl-{[(E)-(L)-6-Amino-hept-2-endicarbonsäure]-1-ethanoyl}-L-phenylalaninmethylester (Verbindung 3)

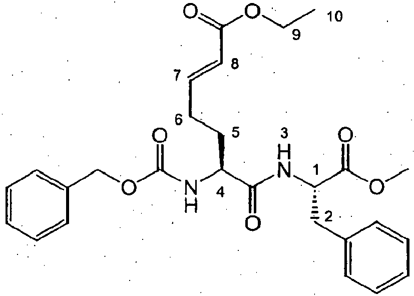

33,5 mg N^{α}-Benzyloxycarbonyl-{(E)-(L)-6-Amino-hept-2-endicarbonsäure}-1-ethylester (1.7, 0,1 mmol) werden in 7,5 ml DMF gelöst und nacheinander mit 38 mg HATU (0,1 mmol) und 51 µl DIPEA (0,3 mmol) versetzt. Die so aktivierte Aminosäure wird zu einer Lösung von 21,5 mg Phenylalaninmethylesterhydrochlorid (kommerziell erhältlich) in 7,5 ml DMF zugesetzt. Durch allmähliche Zugabe von DIPEA wird der pH-Wert auf ca. 9 gebracht. Bei Raumtemperatur wird für 30 min gerührt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 30% B auf 100% B, 1 %/min).
Rₜ = 39,6-42,0 min
Ausbeute: 29 mg
ESI-MS: 519,2 {M+Na}⁺

### N^{α}-Benzyloxycarbonyl-{[(E)-(L)-6-Amino-hept-2-endicarbonsäure]-1-methanoyl}-L-Glutaminyl-L-Prolinmethylester (Verbindung 4)

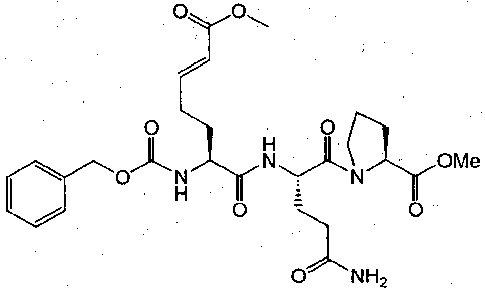

32 mg (1.11, 0,1 mmol) werden in 7 ml DMF gelöst und nacheinander mit 38 mg HATU (0,1 mmol) und 51 µl DIPEA (0,3 mmol) versetzt. Diese Lösung wird zu einer Lösung des Trifluoracetatsalzes von Gln-Pro-OMe zugesetzt. Die weitere Durchführung und Aufarbeitung wird wie für Verbindung 1 beschrieben durchgeführt.
Rₜ = 28,8,9-31,2 min
ESI-MS: 583,3 {M+Na}⁺

### N^{α}-Benzyloxycarbonyl-{[(E)-(L)-6-Amino-hept-2-endicarbonsäure]-1-ethanoyl}-L-Glutaminyl-L-Prolinyl-Isopentylamid (Verbindung 5)

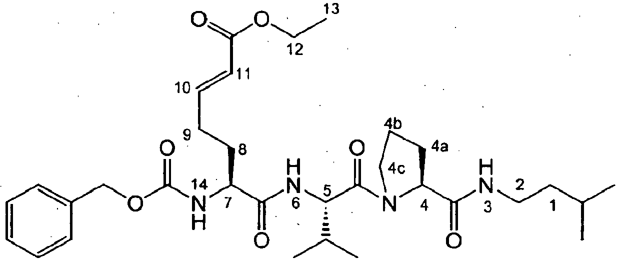

22 mg N^{α}-Benzyloxycarbonyl-{(E)-(L)-6-Amino-hept-2-endicarbonsäure}-1-ethylester (1.7, 0,066 mmol) werden in 5 ml DMF gelöst und nacheinander mit 25 mg HATU (0,066 mmol) und 33,5 µl DIPEA (0,196 mmol) versetzt und sofort zu einer Lösung des Trifluoracetatsalzes von 0,0657 mmol H-Val-Pro-Isopropylamid (5.a) in 5 ml DMF zugesetzt. Durch sukzessive Zugabe von DIPEA wird der pH-Wert auf 9 eingestellt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 30% B auf 100% B, 1%/min).
Rₜ = 37,3-40,3 min
Ausbeute: 20 mg
500-MHz-¹H-NMR-cosy (DMSO_{d6}): δ[ppm] = 7,86 (d, 1H, H-6), 7,69 (t, 1H, H-3), 7,48 (d, 1H, H-14), 7,39-7,28 (m, 5H, Aryl-H), 6,88 (dt, 1H, H-10, J_{10/9} = 7,0 Hz, J_{10/11} = 14,3 Hz), 5,80 (d, 1H, H-11, J_{11/10} = 14,3 Hz), 5,02 (s, 2H, Benzyl-CH₂), 4,33 -4,30 (m, 1H, H-5), 4,30-4,22 (m, 1H, H-4), 4,10 (q, 2H, H-12ₐ, H-12_{b}), 4,08-4,03 (m, 1H, H-7), 3,73-3,67 (m, 1H, H-4cₐ), 3,60-3,54 (m, 1H, H-4c_{b}), 3,14-3,06 (m, 1H, H-2ₐ), 3,05-2,95 (m, 1 H, H-2_{b}) 2,25-2,17 (m, 2H, H-9ₐ, H-9_{b}), 2,04-1,90 (m, 3H, H-4_{a/1}, H-4_{b/1}, Methin-H(Val)), 1,85-1,70 (m, 3H, H-4_{a/2}, H-4_{b/2}, H-10ₐ), 1,68-1,60 (m, 1H, H-10_{b}), 1,60-1,50 (m, 1H, Methin-H(Isopropylamid)), 1,31-1,24 (m, 2H, H-3ₐ, H-3_{b}), 1,21 (t, 3H, CH₃-13), 0,89 (d, 12 H, 4xCH₃)
ESI-MS: 623,5 {M+Na}⁺

### [(E)-(L)-6-(2-Oxo-pyrrolidon-1-yl)-hept-2-endicarbonsäure-1-ethanoyl]-L-Valinyl-L-Prolinmethylester (Verbindung 6)

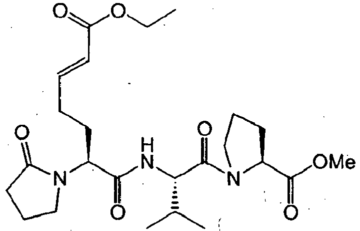

27 mg (L)-(E)-(L)-6-(2-Oxo-pyrrolidon-1-yl)-hept-2-endicarbonsäure-1-ethylester (1.15, 0,1 mmol) werden in 7,5 ml DMF gelöst und nacheinander mit 38 mg HATU (0,1 mmol) und 51 µl DIPEA (0,3 mmol) versetzt. Die so aktivierte Aminosäure wird zu einer Lösung von 0,1 mmol des Trifluoracetatsalzes von H-Val-Pro-OMe in 7,5 ml DMF zugesetzt. Durch allmähliche Zugabe von DIPEA wird der pH-Wert auf ca. 9 gebracht. Bei Raumtemperatur wird für 30 min gerührt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 30% B auf 100% B, 1 %/min).
ESI-MS: 502,3 [M+Na]⁺

### L-Glutamyl-L-Alaninyl-L-Valinmethylester (7.1)

Die Titelverbindung wird nach Standardmethoden der Peptidsynthese hergestellt.
ESI-MS: 332,1 [M+H]⁺

### N^{α}-tert.-Butyloxycarbonyl-{[(E)-(L)-6-Amino-hept-2-endicarbonsäure]-1-methanoyl}-L-Glutamyl-L-Alaninyl-L-Valinmethylester (7.2)

29 mg N^{α}-tert.-Butyloxycarbonyl-{(E)-(L)-6-Amino-hept-2-endicarbonsäure}-1-methylester (1.9, 0,1 mmol) werden zusammen mit 38 mg HATU (0,1 mmol) in 5 ml DMF gelöst. Zu dieser Lösung werden 51 µl DIPEA (0,3 mmol) zugesetzt und die erhaltene gelbe Lösung sofort zu einer Lösung von 44 mg (des Trifluoracetatsalzes von L-Glutamyl-L-Alaninyl-L-Valinmethylester (7.1, 0,1 mmol)) zugesetzt. Durch sukzessive Zugabe von DIPEA wird der pH-Wert auf 9 eingestellt. Nach 30 wird das Lösungsmittel i. Vak. entfernt und der ölige braune Rückstand durch präparative HPLC gereinigt (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 40% B auf 100% B, 1%/min).
Ausbeute: 38 mg
ESI-MS: 623,2 [M+Na]⁺

### N^{α}-Acetyl-L-Asparaginyl-{[(E)-(L)-6-Amino-hept-2-endicarbonsäure]-1-methanoyl}-L-Glutamyl-L-Alaninyl-L-Valinmethylester (Verbindung 7)

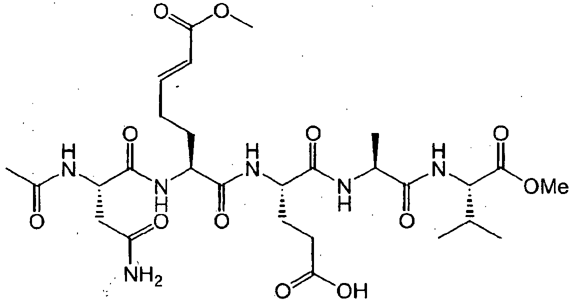

38 mg N^{α}-tert.-Butyloxycarbonyl-{[(E)-(L)-6-Amino-hept-2-endicarbonsäure]-1-methanoyl}}-L-Glutamyl-L-Alaninyl-L-Valinmethylester (7.2, 63 µmol) werden in 5 ml Dichlormethan gelöst, mit dem gleichen Volumen Trifluoressigsäure versetzt und für eine Stunde bei Raumtemperatur gerührt. Nach dieser Zeit wird das Lösungsmittel i. Vak. entfernt. Säurereste werden durch mehrmaliges codestillieren mit Methanol entfernt. Der erhaltene ölige Rückstand wird in 4 ml DMF gelöst. Zu dieser Lösung wird eine Lösung aus 11 mg N^{α}-Acetyl-L-Asparagin, 24 mg HATU und 33 µl DIPEA zugesetzt. Der pH-Wert der resultierenden Lösung wird mit DIPEA auf ca. 7 eingestellt. Nach einer Stunde wird das Lösungsmittel i. Vak. entfernt. Die Reinigung erfolgt durch präparative HPLC. (Synergie Max, 4µm, 250x21,2 mm, A-Eluent: 0,1%TFA / Wasser, B-Eluent: 90% AcCN /10% Wasser / 0,1%TFA. Gradient: 8 ml/min, 40% B auf 100% B, 1 %/min).
Rₜ: 37,0-39,8min
Ausbeute: 27 mg
ESI-MS: 679,3 [M+Na]⁺

### N^{α}-tert.-Butyloxycarbonyl-{[(E)-(L)-6-Amino-hept-2-endicarbonsäure]-1-methanoyl}-L-Serinyl-L-Leucinyl-L-Valinyl-L-Isoleucinyl-Glycinmethylester (8.3)

29 mg N^{α}-tert.-Butyloxycarbonyl-{(E)-(L)-6-Amino-hept-2-endicarbonsäure}-1-methylester (1.9, 0,1 mmol) werden zusammen mit 38 mg HATU (0,1 mmol) in 5 ml DMF gelöst. Zu dieser Lösung werden 51 µl DIPEA (0,3 mmol) zugesetzt und die erhaltene gelbe Lösung sofort zu einer Lösung von 64 mg (0,1 mmol) des Trifluoracetatsalzes von L-Serinyl-L-Leucinyl-L-Valinyl-L-Isoleucinyl-Glycinmethylester (8.1) zugesetzt. Durch sukzessive Zugabe von DIPEA wird der pH-Wert auf 9 eingestellt. Nach 30 wird das Lösungsmittel i. Vak. entfernt und der ölige braune Rückstand durch präparative HPLC gereinigt (Synergie Max, 4µm, 250x21,2 mm, A-Eluent: 0,1 %TFA / Wasser, B-Eluent: 90% AcCN / 10% Wasser / 0.1 %TFA. Gradient: 8 ml/min, 40% B auf 100% B, 1%/min).
Ausbeute: 39 mg
ESI-MS: 807,5 {M+Na}⁺

### N^{α}-Acetyl-L-Leucinyl-Glycinyl-L-Prolinyl-Glycinyl-{[(E)-(L)-6-Amino-hept-2-endicarbonsäure]-1-ethanoyl}-L-Serinyl-L-Leucinyl-L-Valinyl-L-Isoleucinyl-Glycinmethylester (Verbindung 8)

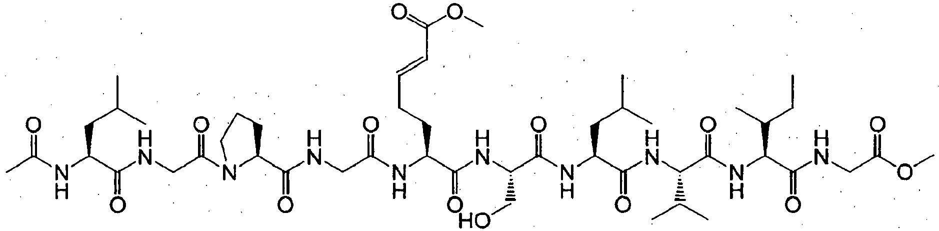

39 mg N^{α}-tert.-Butyloxycarbonyl-{[(E)-(L)-6-Amino-hept-2-endicarbonsäure]-1-ethanoyl}-L-Serinyl-L-Leucinyl-L-Valinyl-L-Isoleucinyl-Glycinmethylester (8.3, 49µmol) werden in 5 ml Dichlormethan gelöst. Nach Zusatz des gleichen Volumens Trifluoressigsäure wird für eine Stunde bei Raumtemperatur gerührt und danach das Lösungsmittel i. Vak. entfernt. Säurereste werden durch mehrmaliges codestillieren mit Methanol entfernt. Der erhaltene ölige Rückstand wird in 4 ml DMF gelöst.

Eine Lösung von 20 mg (50 µmol) N^{α}-Acetyl-L-Leucinyl-Glycinyl-L-Prolinyl-Glycin (8.2) in DMF 19 mg HATU (50 µmol) wird durch Zusatz von 26 µl DIPEA aktiviert und nachfolgend zu der oben dargestellten Lösung des Trifluoracetatsalzes von entschütztem 8.3 zugesetzt Durch sukzessiven Zusatz von DIPEA wird der pH-Wert auf ca. 7 eingestellt. Nach einer Stunde wird das Lösungsmittel i. Vak. entfernt. Die Reinigung erfolgt durch präparative HPLC. (Synergie Max, 4µm, 250x21,2 mm, A-Eluent: 0,1%TFA / Wasser, B-Eluent: 90% AcCN 10% Wasser / 0,1%TFA. Gradient: 8 ml/min, 40% B auf 100% B, 1 %/min).
Rₜ : 13,6-15,4min
Ausbeute: 15 mg
500-MHz-¹H-NMR-cosy (DMSO_{d6}): δ[ppm] = 8,34 (t, 1 H), 8,25-8,16 (m, 2H), 8,09 (d, 1H), 8,01 (d, 1 H), 7,99 (d, 1H), 7,89 (t, 1 H), 7,81-7,73 (m, 2H), 6,88 (dt, 1 H), 5,85 (d, 1H), 4,49-3,65 (mehrere multipletts, 13 H), 3,64 (s, 3H), 3,61 (s, 3H), 3,56 (m, 2H), 3,55-3,42 (m, 2H), 2,18-2,12 (m, 2H), 2,07-1,55 (mehrere multipletts, 7H), 1,84 (s, 3H), 1,51-1,38 (m 4H), 1,13-1,04 (m, 1H), 0,89-0,78 (mehrere multipletts, 18 H)
ESI-MS: 1059,8 [M+Na]⁺

### Darstellung von erfindungemäßen Inhibitoren mit m=1

Überraschenderweise hat sich gezeigt, dass wirksame Inhibitoren auch dann erhalten werden können, wenn die Ethylengruppe als Linker zwischen der elektrophilen Doppelbindung, d.h. der akzeptorsubstituierten Doppelbindung und dem peptidischen Grundgerüst um eine Carbonylgruppe erweitert wird. Diese völlig neuartige pharmakophore Gruppe, d.h. die über eine Ethylencarbonylgruppe verknüpfte elektrophile Doppelbindung ist als essentieller Bestandteil eines Inhibitors zuvor auch für andere Enzyme noch nicht beschrieben worden. Die Synthesesequenz dieser erfindungsgemäßen Inhibitoren ist in Figur 3 dargestellt.

### N^{α}-Benzyloxycarbonyl-L-Glutamyl-L-Valinyl-L-Prolinyl-L-Leucin-methylester (9.1)

Die Titelverbindung wird nach Standardmethoden der Peptidsynthese hergestellt.
ESI-MS: 627,3 [M+Na]

### N^{α}-Benzyloxycarbonyl-(5-Oxo-6-Diazo)-L-norleucinyl-L-Valinyl-L-Prolinyl-Leucinmethylester (9.2)

1,83 g N^{α}-Benzyloxycarbonyl-L-Glutamyl-L-Valinyl-L-Prolinyl-Leucin-methylester (9.1, 2,12 mmol) werden in 40 ml THF_{(abs.)} gelöst und auf -15°C abgekühlt. Zu dieser Lösung werden unter Argonatmosphäre nacheinander 1,8 ml Diisopropylethylamin (10,6 mmol) und 1,43 ml Isobutylchloroformat (10,6 mmol) zugesetzt. Nach 10 Minuten wird eine frisch hergestellte Lösung von Diazomethan in Diethylether (ca. 33 mmol) zugesetzt. Der Reaktionsansatz wird über Nacht gerührt und das Lösungsmittel i. Vak. entfernt.
Reinigung durch Chromatographie an Kieselgel.
ESI-MS: 651,4 [M+Na], 623,4 [M-N₂+Na]

### N^{α}-Benzyloxycarbonyl-(L-7-Amino-1,2-Dioxo-hexanoyl)-L-Valinyl-L-Prolinyl-Leucin-methylester (9.3)

100 mg N^{α}-Benzyloxycarbonyl-(5-Oxo-6-Diazo)-L-Norleucinyl-L-Valinyl-L-Prolinyl-Leucin-methylester (9.2, 0,16 mmol) werden in 20 ml absolutem Aceton gelöst. Unter Argonatmosphäre werden bei 0 °C 3 ml einer frisch hergestellten Dimethyldioxiran-Lösung (~0,1M) versetzt. Nach zehn Minuten wird das Lösungsmittel i. Vak. entfernt, der Rückstand in Dichlormethan_{abs}. aufgenommen und über Na₂SO₄ getrocknet1%/min).
ESI-MS: 639,4 [M+Na], 657,4 [M+H₂O]

### N^{α}-Benzyloxycarbonyl-{[L-7-Amino-4-oxo-oct-2-endicarbonsäure]-1-ethanoyl}-L-Valinyl-L-Prolinyl-Leucin-methylester (Verbindung 9)

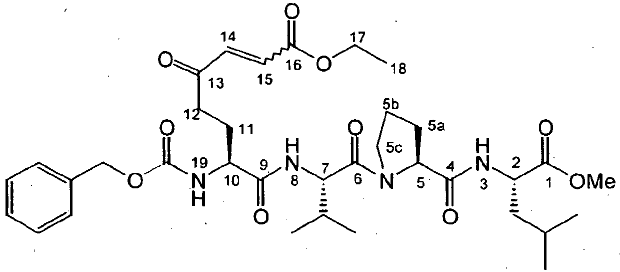

98 mg N^{α}-Benzyloxycarbonyl-(L-2Amino-5,6-Dioxo-hexanoyl)-L-Valinyl-L-Prolinyl-L-Leucinmethylester (9.3, 0,16 mmol) werden in absolutem Benzol gelöst. Zu dieser Lösung werden 52 mg (Ethoxycarbonylmethylen)triphenylphosphoran (0,15 mmol)) zugesetzt. Unter Argonatmosphäre wird für eine Stunde bei Raumtemperatur gerührt. Das Lösungsmittel wird i. Vak. entfernt und der feste Rückstand durch präparative HPLC gereinigt (Synergie Max, 4µm, 250x21,2 mm, A-Eluent: 0,1%TFA / Wasser, B-Eluent: 90% AcCN / 10% Wasser 0,1%TFA. Gradient: 8 ml/min, 40% B auf auf 100% B, 1%/min). Dabei werden zwei Produkte isoliert. Das Lösungsmittel wird i. Vak. entfernt und der Rückstand im Hochvakuum getrocknet.

| | |
|---|---|
| Rₜ: 37-40 min | 7 mg |
| Rₜ: 40-43 min | 27 mg |

Z-Isomer (Fraktion 37-40 min)
500-MHz-¹H-NMR-cosy (DMSO_{d6}): δ[ppm] = 8,24 (d, 1H, H-3), 7,89 (d, 1H, H-8), 7,51 (d, 1H, H-19), 7,43-7,30 (m, 5H, Aryl-H), 6,76 (d, 1H, H-14, J_{14/15} = 12,1 Hz), 6,18 (d, 1H, H-15, J_{14/15} = 12,1 Hz), 5,06 (s, 1 H, Benzyl-CH₂), 4,43-4,34 (m, 2H, H-5, H-7),4,32-4,26 (m, 1H, H-2), 4,15 (q, 2H, H-17ₐ, H-17_{b}), 4,12-3,96 (m, 1H, H-10), 3,76-3,70 (m, 1H, H-5cₐ), 3,65 (s, 3H, OMe), 3,63-3,55 (m, 1H, H-5c_{b}), 2,60-2,66 (m, 2H, H-12ₐ, H-12_{b}), 2,13-2,03 (m, 1H, H-5_{a/1}), 2,03-1,92 (m, 2H, H-5_{b/1}, Methin-H(Val)), 1,92-1,81 (m, 3H, H-5_{a/2}, H-5_{b/2}, H-11ₐ), 1,81-1,70 (m, 2H, H-11_{b}, Methyin-H (Leu), 1,63-1,55 (m, 1H, CH₂ₐ-Leu)), 1,55-1,47 (m, 1H, CH_{2b}-Leu), 1,21 (t, 3H, CH₃-18), 0,94 (d, 6H, 2xCH₃-Val), 0,90 (d, 6H, 2xCH₃-Leu)
ESI-MS: 709,5 {M+Na}⁺
E-Isomer (Fraktion 40-43 min)
500-MHz-¹H-NMR-cosy (DMSO_{d6}): δ[ppm] = 8,22 (d, 1H, H-3), 7,90 (d, 1H, H-8), 7,51 (d, 1H, H-19), 7,43-7,30 (m, 5H, Aryl-H), 6,97 (d, 1H, H-14, J_{14/15} = 16,1 Hz), 6,72 (d, 1H, H-15, J_{14/15} = 16,1 Hz), 5,07 (s, 1 H, Benzyl-CH₂),4,43 -4,33 (m, 2H, H-5, H-7), 4,30-4,28 (m, 1H, H-2), 4,25 (q, 2H, H-17ₐ, H-17_{b}), 4,15-3,97 (m, 1H, H-10), 3,77-3,71 (m, 1H, H-5cₐ), 3,65 (s, 3H, OMe), 3,63-3,58 (m, 1 H, H-5c_{b}), 2,85-2,73 (m, 2H, H-12ₐ, H-12_{b}), 2,13-2,03 (m, 1H, H-5_{a/1}), 2,03-1,92 (m, 2H, H-5_{b/1}, Methin-H(Val)), 1,92-1,81 (m, 3H, H-5_{a/2}, H-5_{b/2}, H-11ₐ), 1,81-1,70 (m, 2H, H-11_{b}, Methyin-H (Leu), 1,63-1,55 (m, 1H, CH₂ₐ-Leu)), 1,55-1,47 (m, 1H, CH_{2b}-Leu), 1,28 (t, 3H, CH₃-18), 0,96 (dd, 6H, 2xCH₃-Val), 0,90 (dd, 6H, 2xCH₃-Leu)
ESI-MS: 709,5 {M+Na}⁺

### N^{α}-Benzyloxycarbonyl-{[(E)-(L)-6-Amino-hept-2-endicarbonsäure]-1-ethanoyl}-L-Phenylalaninyl-L-Prolinyl-L-Leucinmethylester (Verbindung 25)

500-MHz-¹H-NMR-cosy (DMSO_{d6}): δ[ppm] = 8,53 (d, 1H), 8,19 (d, 1H), 7,37-7,17 (m, 11 H), 6,83 (dt, 1H), 5,78 (d, 1 H), 5,01 (s, 1 H), 4,73-4,65 (m, 1 H), 4,42-4,35 (m, 1H) 4,32-4,25 (m, 1 H), 4,10 (q, 2H), 3,99-3,92 (m, 1 H), 3,56 (s, 3H), 3,55-3,43 (m, 2H) 3,00 (dd, 1H), 2,76 (dd, 1 H), 2,21-2,10 (m, 2H), 2,10-2,00 (m, 1H), 1,95-1,75 (m, 3H), 1,72-1,42 (m, 5H), 1,19 (t, 3H), 0,91 (d, 3H), 0,87 (d, 3H)

### N^{α}-Benzyloxycarbonyl-{[(E)-(L)-6-Amino-hept-2-endicarbonsäure]-1-ethanoyl}-Glycinyl-L-Prolinyl-L-Leucinmethylester (Verbindung 26)

500-MHz-¹H-NMR-cosy (DMSO_{d6}): δ[ppm] = 8,20 (d, 1 H), 8,01 (d, 1 H); 7,50 (d, 1H), 7,37-7,25 (m, 5H), 6,87 (dt, 1H), 5,81 (d, 1H), 5,02 (s, 1 H), 4,39-4,35 (m, 1H), 4,28-4,22 (m, 1 H), 4,11 (q, 2H), 4,07-4,03 (m, 1H), 4,01 (dd, 1H), 3,80 (dd, 1 H), 3,61 (s, 3H), 3,61-3,42 (m, 2H), 2,32-2,20 (m, 2H), 2,08-2,01 (m, 1 H), 1,95-1,88 (m, 1 H), 1,88-1,73 (m, 2H), 1,81-1,60 (m, 3H), 1,59-1,45 (m, 2H), 1,21 (t, 3H), 0,88 (d, 3H), 0,83 (d, 3H)

### N^{α}-Benzyloxycarbonyl-{[(E)-(L)-6-Amino-hept-2-endicarbonsäure]-1-ethanoyl}-L-Alaninyl-L-Prolinyl-L-Leucinmethylester (Verbindung 27)

500-MHz-¹H-NMR-cosy (DMSO_{d6}): 5[ppm] = 8,14 (d, 1H), 8,10 (d, 1H); 7,42 (d, 1H), 7,37-7,31 (m, 5H), 6,87 (dt, 1H), 5,82 (d, 1 H), 5,02 (s, 1 H), 4,53-4,45 (m, 1 H), 4,40-4,35 (m, 1H), 4,25-4,18 (m, 1H), 4,10 (q, 2H), 4,05-3,97 (m, 1H), 3,60 (s, 3H), 3,56-3,50 (m, 1H), 2,28-2,18 (m, 2H), 2,09-2,00 (m, 1H), 1,98-1,87 (m, 2H), 1,87-1,73 (m, 2H), 1,73-1,55 (m, 2H), 1,55-1,50 (m, 1H), 1,50-1,49 (m, 1 H), 1,21 (m, 6H), 0,89 (d, 3H), 0,84 (d, 3H)

### 2.1 Darstellung von Inhibitoren mit peptidischer Umgebung der pharmakophoren Gruppe. Reaktionen an Aminofunktionen am Beispiel von (E)-(L)-6-Amino-hept-2-endicarbonsäure

Erfindungsgemäße pharmakophore Gruppen lassen sich beispielhaft aber nicht beschränkend leicht aus Aminosäuren wie Glutaminsäure darstellen. Man erhält so erfindungsgemäße Inhibitoren mit einer α-Aminogruppe, beispielsweise bei der Verwendung von Carboxymethylen-Wittigreagenzien, (E)-(L)-6-Amino-hept-2-endicarbonsäure. Diese Aminogruppe kann durch jede Reaktion die mit einer Aminogruppe durchgeführt werden kann, modifiziert werden.

Überraschenderweise wurde festgestellt, dass potente Inhibitoren hauptsächlich dann erhalten werden können, wenn solche Aminogruppen (-NXX'), sofern sie in unmittelbarer räumlicher Nähe zur pharmakophoren Gruppe positioniert sind, acyliert oder alkyliert sind, insbesondere also nicht als freies primäres Amin vorliegen. Verwendet man zum Beispiel (E)-(L)-6-Amino-hept-2-endicarbonsäurederivate als bioisosteren Pharmakophor, so muss die Aminofunktion durch Austausch mindestens eines der beiden Wasserstoffatome am Stickstoff z.B. durch Acylierung in eine weniger polare Verbindung überführt werden. Nicht beschränkende Beispiele für die Synthese von Inhibitoren mit variierenden unpolaren Gruppen an diesem Stickstoff, werden im Folgenden beschrieben. Darüber hinaus können neben Acylierungsreaktionen auch alle anderen bekannten Reaktionen von Aminogruppen realisiert werden, solange keine hohen pH-Werte oder andere Reaktionsbedingungen vorliegen, die einen Abbau des Michael-Akzeptor-Strukturelementes zur Folge haben.

Allgemeine Synthesebeschreibung für die Modifikation von Aminofunktionen an erfindungsgemäßen Inhibitoren:
Die Umsetzung von Aminogruppen in erfindungsgemäßen Inhibitoren kann in der Regel ohne Schwierigkeiten nach den für solche Modifikationen üblichen Verfahren erfolgen. Ein besonderer Fall ergibt sich dann, wenn wie im Beispiel von (E)-(L)-6-Amino-hept-2-endicarbonsäurederivaten, die Aminogruppe unter den Reaktionsbedingungen bevorzugt selbst mit der pharmakophoren Gruppe reagiert. Ein schneller Zugang zu verschiedenen Derivaten solcher Inhibitoren ist beispielhaft aber nicht beschränkend durch die folgende Synthesesequenz gegeben:
   N^{α}-tert.-Butyloxycarbonyl-{(E)-(L)-6-Amino-hept-2-endicarbonsäure}-1-ethylester, das Ethylesteranalogon zu 1.9 wird unter den hier beschriebenen Methoden an ein geeignetes Amin gebunden. Vom erhaltenen Amid wird acidolytisch die Boc-Schutzgruppe abgespalten. Das Salz ist bei niedrigen pH-Werten stabil. Die weitere Umsetzung der Aminogruppe gelingt dann problemlos, wenn der pH-Wert der Reaktionsmediums nicht über 9 liegt.

### N^{α}-tert.-Butyloxycarbonyl-{[(E)-(L)-6-Amino-hept-2-endicarbonsäure]-1-ethanoyl}-L-Glutaminyl-L-Prolinyl-L-Leucinmethylester (Verbindung 28)

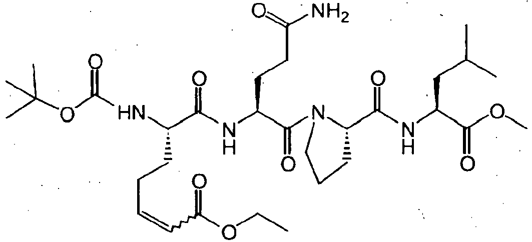

Ausgehend von 283 mg Boc-Gln-Pro-Leu-OMe (0,6 mmol) wird durch Behandlung mit Trifluoressigsäure das , Salz des freien Amines (H-Gln-Pro-Leu-OMe) dargestellt. Das erhaltene gelbe Öl, wird in 10 ml DMF gelöst. N^{α}-tert.-Butyloxycarbonyl-{(E)-(L)-6-Amino-hept-2-endicarbonsäure}-1-ethylester (0,6 mmol) werden in 10 ml DMF gelöst und mit 229 mg HATU (0,6 mmol) und 307 µl DIPEA (1,8 mmol) versetzt. Die erhaltene Lösung wird sofort zu der oben dargestellten Lösung des Amins (H-Gln-Pro-Leu-OMe) zugesetzt und durch sukzessive Zugabe von DIPEA auf pH~9 gebracht.

Nach einer Stunde rühren bei Raumtemperatur wird das Lösungsmittel i. Vak. entfernt und das Produkt durch Chromatographie an Kieselgel gereinigt. (Säule:32,5x3,2 cm, Dichlormethan/Methanol: 95/5)
Ausbeute: 345 mg
500-MHz-¹H-NMR-cosy (DMSO_{d6}): δ[ppm] = 8,10 (d, 1H), 7,86 (d, 1H), 7,07 (s, 1H), 6,78-6,72 (m, 2H), 6,66 (s, 1 H), 5,71 (d, 1 H), 4,42-4,33 (m, 1 H), 4,29-4,21 (m, 1 H), 4,19-4,10 (m, 1H), 4,00 (q, 2H), 3,85-3,79 (m, 1H), 3,57-3,45 (m, 5H), 3,49 (s, 3H), 2,15-2,05 (m, 2H), 2,05-2,01 (m, 2H), 1,95-1,88 (m, 1H), 1,80-1,72 (m, 2H), 1,72-1,65 (m, 2H), 1,65-1,45 (m, 3H), 1,45- 1,37 (m, 1H), 1,37-1,31 (m, 1H), 1,08 (t, 3H), 0,78 (d, 3H), 0,73 (d, 3H)
ESI-MS: 676,4 {M+Na}⁺

### N^{α}-Thiophen-2-carbonyl-{[(E)-(L)-6-Amino-hept-2-endicarbonsäure]-1-ethanoyl}-L-Glutaminyl-L-Prolinyl-L-Leucinmethylester (Verbindung 29)

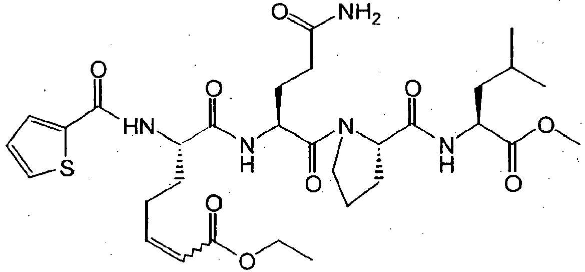

100 mg N^{α}-tert.-Butyloxycarbonyl-{[(E)-(L)-6-Amino-hept-2-endicarbonsäure]-1-ethanoyl}-L-Glutaminyl-L-Prolinyl-L-Leucinmethylester (28, 0,152 mmol ) werden in 5 ml Dichlormethan gelöst und mit 5 ml Trifluoressigsäure versetzt. Bei Raumtemperatur wird für eine Stunde gerührt und nachfolgend das Lösungsmittel i. Vak entfernt. Das erhaltene Amin H-{[(E)-(L)-6-Amino-hept-2-endicarbonsäure]-1-ethanoyl}-L-Glutaminyl-L-Prolinyl-L-Leucinmethylester wird in 5 ml DMF gelöst. Ein Lösung aus 19,6 mg 2-Thiophencarbonsäure (0,153 mmol) und 58,2 mg HATU (0,153 mmol) in 5 ml DMF wird mit 52 µl DIPEA (0,306 mmol) versetzt und sofort zu der oben dargestellten Lösung des Amins gegeben. Durch sukzessive Zugabe von DIPEA wird der pH-Wert auf - 9 eingestellt. Nach 30 Minuten wird das Lösungsmittel i. Vak. entfernt und der erhaltene Rückstand durch präparative HPLC-Chromatographie gereinigt (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 40% B auf 100% B, 1 %/min).
500-MHz-¹H-NMR-cosy (DMSO_{d6}): δ[ppm] = 8,44 (d, 1 H), 8,24 (d, 1 H), 8,22 (d, 1 H), 7,88 (dd, 1H), 7,77 (dd, 1 H), 7,24 (br. s, 1 H), 7,16 (dd, 1H), 6,95-6,87 (m, 1H), 6,80 (br. S., 1 H), 5,83 (d, 1 H), 4,49-4,41 (m, 2H), 4,37-4,33 (m, 1 H), 4,26-4,21 (m, 1 H), 4,08 (q, 2H), 3,65-3,59 (m, 5H), 2,31-2,23 (m, 2H), 2,19-2,11 (m, 2H), 2,09-1,99 (m, 1H), 1,95-1,75 (mehrere multipletts, 6H), 1,73-1,63 (m, 2H), 1,59-1,51 (m, 1 H), 1,51-1,44 (m, 1 H), 1,19 (t, 3H), 0,89 (d, 3H), 0,83 (d, 3H)
ESI-MS: 686,4 {M+Na}⁺

Nach der gleichen Methode wurden folgende Verbindungen dargestellt und gereinigt:

### N^{α}-Furan-3-carbonyl-{[(E)-(L)-6-Amino-hept-2-endicarbonsäure]-1-ethanoyl}-L-Glutaminyl-L-Prolinyl-L-Leucinmethylester (Verbindung 30)

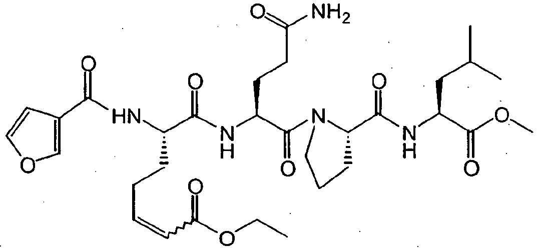

500-MHz-¹H-NMR-cosy (DMSO_{d6}): δ[ppm] = 8,24 (m, 3H), 8,14 (d, 1H), 7,73 (t, 1H), 7,25 (br. s, 1 H), 6,94-6,86 (m, 2H), 6,79 (br. S., 1 H), 5,83 (d, 1H), 4,49-4,41 (m, 2H), 4,37-4,33 (m, 1 H), 4,28-4,21 (m, 1 H), 4,09 (q, 2H), 3,66-3,59 (m, 5H), 2,30-2,23 (m, 2H), 2,18-2,11 (m, 2H), 2,08-2,00 (m, 1 H), 1,95-1,64 (mehrere multipletts, 8H), 1,59-1,51 (m, 1 H), 1,51-1,44 (m, 1H), 1,20 (t, 3H), 0,89 (d, 3H), 0,84 (d, 3H) ESI-MS: 648,5 {M+H}⁺

### N^{α}-Isoxazol-5-carbonyl-{[(E)-(L)-6-Amino-hept-2-endicarbonsäure]-1-ethanoyl}-L-Glutaminyl-L-Prolinyl-L-Leucinmethylester (Verbindung 31)

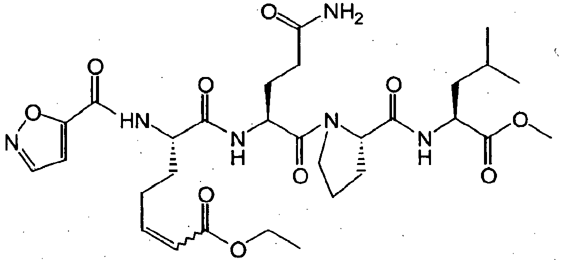

500-MHz-¹H-NMR-cosy (DMSO_{d6}): δ[ppm] = 8,70 (d, 1H), 8,56 (d, 1H), 8,13 (d, 1H), 8,02 (d, 1 H), 7,03 (br. s, 1 H), 6,95 (d, 1 H), 6,71 (dt, 1H), 6,59 (br. S., 1 H), 5,65 (d, 1 H), 4,33-4,24 (m, 2H), 4,18-4,15 (m, 1 H), 4,09-4,04 (m, 1 H), 3,91 (q, 2H), 3,47-3,40 (m, 5H), 2,13-2,04 (m, 2H), 2,00-1,93 (m, 2H), 1,90-1,82 (m, 1H), 1,78-1,60 (mehrere multipletts, 6H), 1,55-1,45 (m, 2H), 1,39- 1,34 (m, 1H), 1,34-1,24 (m, 1 H), 1,01 (t, 3H), 0,71 (d, 3H), 0,65 (d, 3H)
ESI-MS: 671,4 {M+Na}⁺

### N^{α}-(5-Methyl-Isoxazol-4-carbonyl)-{[(E)-(L)-6-Amino-hept-2-endicarbonsäure]-1-ethanoyl}-L-Glutaminyl-L-Prolinyl-L-Leucinmethylester (Verbindung 32)

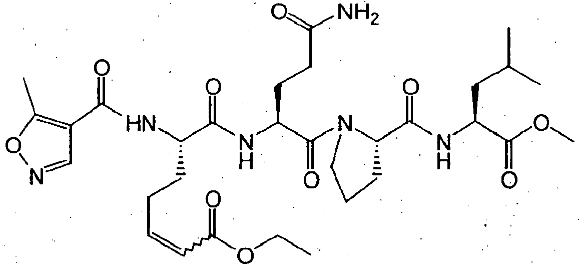

Die eingesetzte 5-Methyl-Isoxazol-4-carbonsäure wird nach Street et al., J. Med. Chem., 2004, 3642-3657) dargestellt.

NMR von 32: 500-MHz-¹H-NMR-cosy (DMSO_{d6}): δ[ppm] = 8,97 (s, 1 H), 8,27 (d, 2H), 8,22 (d, 1H), 7,21 (br. s, 1 H), 6,90 (dt, 1H), 6,77 (br. S., 1H), 5,83 (d, 1 H), 4,50-4,41 (m, 2H), 4,38-4,33 (m, 1 H), 4,28-4,21 (m, 1H), 4,08 (q, 2H), 3,65-3,58 (m, 5H), 2,61 (s, 3H), 2,28-2,20 (m, 2H), 2,18-2,11 (m, 2H), 2,09-2,00 (m, 1H), 1,95-1,77 (mehrere multipletts, 6H), 1,72-1,61 (m, 2H), 1,59- 1,52 (m, 1H), 1,52-1,45 (m, 1 H), 1,19 (t, 3H), 0,89 (d, 3H), 0,83 (d, 3H)
ESI-MS: 685,5 {M+Na}⁺

### N^{α}-(5-Methyl-Isoxazol-3-carbonyl)-{[(E)-(L)-6-Amino-hept-2-endicarbonsäure]-1-ethanoyl}-L-Glutaminyl-L-Prolinyl-L-Leucinmethylester (Verbindung 33)

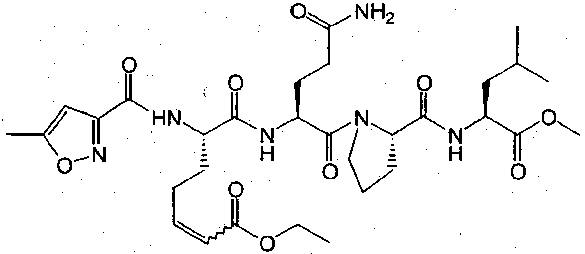

500-MHz-¹H-NMR-cosy (DMSO_{d6}): δ[ppm] = 8,51 (d, 1 H), 8,27 (d, 1H), 8,21 (d, 1 H), 7,21 (br. s, 1H), 6,88 (dt, 1H), 6,77 (br. s., 1H), 5,83 (d, 1 H), 4,50-4,43 (m, 2H), 4,38-4,33 (m, 1 H), 4,27-4,21 (m, 1 H), 4,09 (q, 2H), 3,65-3,59 (m, 5H), 2,50 (s, 3H), 2,28-2,21 (m, 2H), 2,19-2,10 (m, 2H), 2,09-2,00 (m, 1H), 1,95-1,77 (mehrere multipletts, 6H), 1,72-1,61 (m, 2H), 1,59- 1,52 (m, 1 H), 1,52-1,45 (m, 1 H), 1,19 (t, 3H), 0,88 (d, 3H), 0,84 (d, 3H)
ESI-MS: 685,5 {M+Na}⁺

### N^{α}-(trans-3-(3-Thienyl)acryloyl)-{[(E)-(L)-6-Amino-hept-2-endicarbonsäure]-1-ethanoyl}-L-Glutaminyl-L-Prolinyl-L-Leucinmethylester (Verbindung 34)

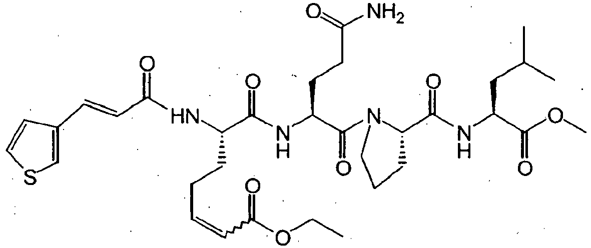

500-MHz-¹H-NMR-cosy (DMSO_{d6}): δ[ppm] = 8,20 (m, 2H), 8,14 (d, 1H), 7,87 (s, 1 H), 7,60 (s, 1 H), 7,42 (d, 1 H), 7,33 (s, 1 H), 7,21 (br. s., 1 H), 6,88 (dt, 1 H), 6,76 (br. s., 1 H),6,56 (d, 1 H), 5,83 (d, 1H), 4,55-4,40 (m, 2H), 4,40-4,31 (m, 1 H), 4,29-4,20 (m, 1 H), 4,08 (q, 2H), 3,70-3,48 (m, 5H), 2,28-2,21 (m, 2H), 2,19-2,10 (m, 2H), 2,09-2,00 (m, 1 H), 1,95-1,77 (mehrere multipletts, 6H), 1,72-1,61 (m, 2H), 1,59- 1,52 (m, 1H), 1,52-1,45 (m, 1H), 1,18 (t, 3H), 0,88 (d, 3H), 0,83 (d, 3H)
ESI-MS: 712,5 {M+Na}⁺

### N^{α}-Acetyl-{[(E)-(L)-6-Amino-hept-2-endicarbonsäure]-1-ethanoyl}-L-Glutaminyl-L-Prolinyl-L-Leucinmethylester (Verbindung 35)

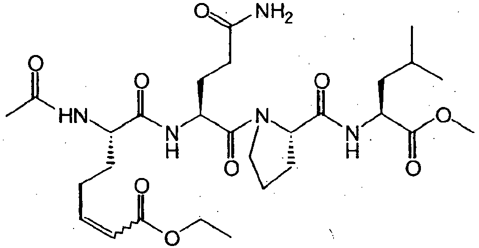

500-MHz-¹H-NMR-cosy (DMSO_{d6}): δ[ppm] = 8,21 (d, 1H), 8,13 (d, 1 H), 7,95 (d, 1H), 7,21 (br. s, 1 H), 6,86 (dt, 1 H), 6,77 (br. s., 1 H), 5,83 (d, 1 H), 4,46-4,42 (m, 2H), 4,37-4,33 (m, 1H), 4,29-4,22 (m, 1H), 4,11 (q, 2H), 3,68-3,59 (m, 5H), 2,25-2,16 (m, 2H), 2,16-2,10 (m, 2H), 2,08-2,00 (m, 1H), 1,94-1,78 (mehrere multipletts, 3H), 1,84 (s, 3H), 1,78-1,45 (mehere multipletts, 6H) 1,20 (t, 3H), 0,89 (d, 3H), 0,84 (d, 3H)
ESI-MS: 618,5 {M+Na}⁺

### N^{α}-(4-Trifluormethoxy-benzolsulfonyl)-{[(E)-(L)-6-Amino-hept-2-endicarbonsäure]-1-ethanoyl}-L-Glutaminyl-L-Prolinyl-L-Leucinmethylester (Verbindung 36)

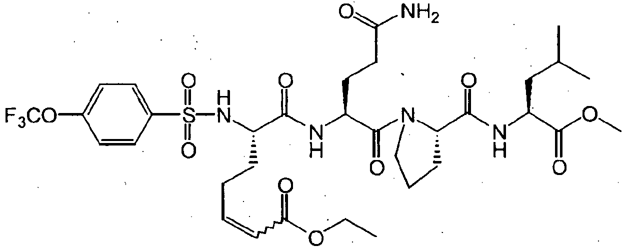

500-MHz-¹H-NMR-cosy (DMSO_{d6}): δ[ppm] = 8,24 (d, 1H), 8,19 (d, 1H), 8,14 (d, 1H), 7,88 (d, 2H), 7,54 (d, 2H), 7,21 (br.s., 1H), 6,80 (br.s.), 6,76 (dt, 1H), 5,70 (d, 1H), 4,34-4,30 (m, 1 H), 4,26-4,20 (m, 1H), 4,18-4,13 (m, 1 H), 4,09 (q, 2H), 3,84-3,79 (m, 1 H), 3,60 (s, 3H), 3,58-3,47 (m, 2H), 2,18-1,97 (mehrere multipletts, 3H), 1,91-1,84 (m, 1 H), 1,84-1,76 (mehrere multipletts, 4H), 1,70-1,44 (mehrere multipletts, 6H), 1,20 (t, 3H), 0,88 (d, 3H), 0,82 (d, 3H)
ESI-MS: 800,5 {M+Na}⁺

### Darstellung von Inhibitoren enthaltend nicht-proteinogene Aminosäuren

Unter dem Begriff "Aminosäure" oder "Aminosäuren" sollen nicht nur die natürlichen Aminosäuren oder deren Derivate verstanden werden, sondern ganz allgemein chemische Verbindungen, die zumindest eine Aminofunktion und zumindest eine Carbonsäurefunktion aufweisen. Diese Aminosäuren im erweiterten Sinn sollten in der Lage sein, eine Betain-Struktur im weiteren Sinne zu bilden und/oder sollten in der Lage sein Amidbindungen auszubilden. Eine besonders bevorzugte Form sind nicht-proteinogene α-Aminosäuren. Die *in vivo* Metabolisierung von Wirkstoffen kann durch solche nicht-proteinogenen Aminosäuren reduziert werden.

Allgemeine Synthesebeschreibung für die Darstellung von Inhibitoren enthaltend nicht-proteinogene Aminosäuren:
Die Aminosäuren können hinsichtlich der Schutzgruppen- und der Kopplungschemie analog den proteinogenen Aminosäuren behandelt werden. Die Synthese folgt im Allgemeinen also den oben beschriebenen Methoden.

Dabei wird die Aminofunktion kommerziell erhältlicher, nicht-natürlicher Aminosäuren entweder mit der Boc- oder mit der Fmoc-Schutzgruppe, nach Methoden die dem Fachmann wohlbekannt sind, geschützt. Der Aufbau des jeweiligen Peptidrestes geschieht in Lösung oder an fester Phase. Das Peptidfragment wird anschließend von seiner Aminoschutzgruppe befreit und mit einer pharmakophor-tragenden Säure gekoppelt.

Falls eine weitere Verlängerung am N-Terminus beabsichtigt ist, wird das Derivat 1.9 eingesetzt. Das so erhaltene Produkt kann anschließend wie in den Beispielen 7 und 8 beschrieben weiter verlängert werden.

Durch Verwendung von Derivaten wie 1.10 oder 1.11 können kürzere Inhibitoren mit stabilen Endgruppen erhalten werden.

Im Folgenden werden mit 37 und 38 zwei Ausführungsbeispiele solcher, erfindungsgemäßer Inhibitoren, enthaltend nicht-proteinogene α-Aminosäuren gegeben.

### N^{α}-Benzyloxycarbonyl-{[(E)-(L)-6-Amino-hept-2-endicarbonsäure]-1-ethanoyl}-L-Cyclohexylglycin-L-Prolinyl-L-Leucinmethylester (Verbindung 37)

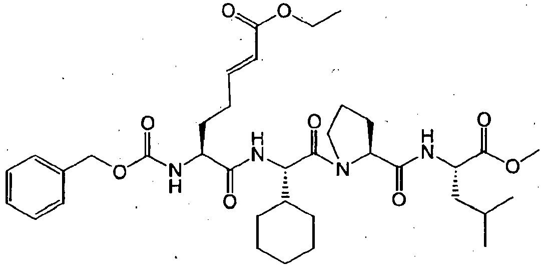

500-MHz-¹H-NMR-cosy (DMSO_{d6}): δ[ppm] = 8,19 (d, 1H), 7,90 (d, 1H), 7,45 (d, 1H), 7,36-7,30 (m 5H), 6,86 (dt, 1 H), 5,81 (d, 1 H), 5,02 (s, 2H), 4,37-4,28 (m, 2H), 4,28-4,18 (m, 1 H), 4,08 (q, 2H), 4,06-4,00 (m, 1 H), 3,54 (s, 3H), 3,56-3,44 (m, 2H), 2,28-2,15 (m, 2H), 2,09-1,98 (m, 1 H), 1,98-1,87 (m, 1H), 1,87-1,56 (mehrere multipletts, 11 H), 1,56-1,51 (m, 1H), 1,51-1,45 (m, 1H)1,20 (t, 3H), ,1,08-1,02 (m, 3H), 1,01-0,95 (m, 2H), 0,89 (d, 3H), 0,83 (d, 3H)
ESI-MS: 721,6 {M+Na}⁺

### N^{α}-Benzyloxycarbonyl-{[(E)-(L)-6-Amino-hept-2-endicarbonsäure]-1-ethanoyl}-L-Valinyl-L-Homoprolinyl-L-Leucinmethylester (Verbindung 38)

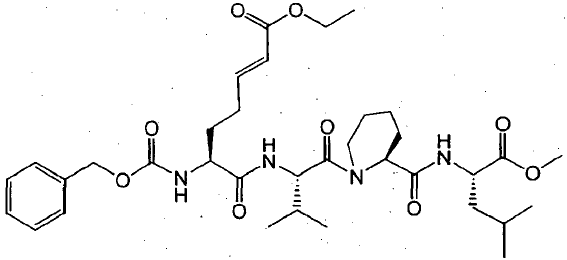

500-MHz-¹H-NMR-cosy (DMSO_{d6}): δ[ppm] = 8.06 (d, 1H), 7,86 (d, 1H), 7,54 (d, 1 H), 7,37,25 (m 5H), 6,86 (dt, 1H), 5,81 (d, 1 H), 5,11-5,05 (m, 1 H), 5,02 (,s, 2H), 4.72-4,66 (m, 1H), 4,32-4,24 (m, 1 H), 4,09 (q, 2H), 4,09-4,01 (m, 1H), 3,92-3,83 (m, 1 H), 3,61 (s, 3H), 3,45-3,37 (m, 1H), 2,31-2,18 (m, 2H), 2,18-2,09 (m, 1H), 2,07-1,99 (m, 1H), 1,83-1,70 (m, 1H), 1,70-1,46 (mehrere multipletts, 6H), 1,32-1,27 (m, 1H), 1,20 (t, 3H), 0,93-0,74 (m, 12H)
ESI-MS: 695,6 {M+Na}⁺

Eine weitere bevorzugte Form erfindungsgemäßer Inhibitoren enthaltend nicht-natürliche Aminosäuren kann dargestellt werden, indem aromatische oder aliphatische Moleküle verwendet werden, deren Carbonsäure- und Aminofunktion nicht am gleichen Kohlenstoffatom, positioniert sind, die also keine α-Aminosäuren sind.

Überraschenderweise verfügen auch solche, strukturell hoch diversen Inhibitoren über gute bis hohe inhibitorische Potenz.

Als Ausführungsbeispiel für diese Klasse erfindungsgemäßer Inhibitoren ist Verbindung 39 gegeben. In Verbindung 39 ist m-Aminobenzoesäure an die pharmakophortragende Aminosäure 1.9 gebunden. Die Synthese für erfindungsgemäße Inhibitoren enthaltend "nicht α-Aminosäuren" folgt dem gleichen Weg, wie er für Inhibitoren enthaltend proteinoge Aminosäuren hierin beschrieben ist (Figur 4). Auf Seite 17, 18 und 19 der Beschreibung sind weitere Aminosäureanaloga sowie Dipeptidmimetika offenbart, welche entsprechend der hier beschriebenen allgemeinen Synthesevorschrift umgesetzt werden können bzw. in das nicht-proteinogene Grundgerüst eingebaut werden können.

### (E)-(S)-6-Benzyloxycarbonylamino-6-[3-((R)-2-phenylcarbamoyl-pyrrolidin-1carbonyl)-phenylcarbamoyl]-hex-2-ensäureethylester(Verbindung 39)

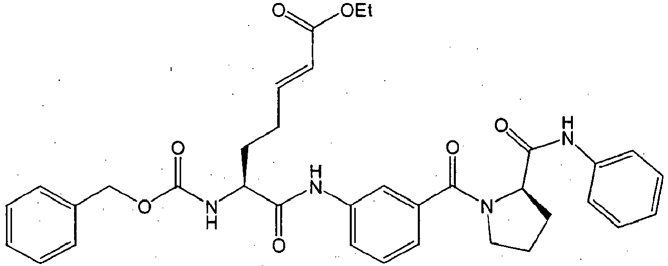

215 mg Boc-Prolin (1 mmol), 135 mg HOBt (1 mmol) und 305 mg TBTU werden in 10 ml DMF gelöst. Die Lösung wird mit 342 µl DIPEA (2 mmol) versetzt und sofort zu einer Lösung von 93 mg Anilin in 5 ml DMF zugesetzt. Nach einer Stunde rühren bei Raumtemperatur wird das Lösungsmittel i. Vak entfernt und der erhaltene Rückstand in 200 ml Ethylacetat aufgenommen. Die Lösung wird je dreimal mit je 30 ml 10%ige Citronensäure-Lösung, ges. NaHCO₃-Lösung und Wasser gewaschen. Die organische Phase wird über Na₂SO₄ getrocknet und das Lösungsmittel i. Vak entfernt. Man erhält 213 mg N^{α}-tert.-Butyloxycarbonyl-L-prolinylanilid. Nach Behandlung mit 10 ml einer Lösung von TFA in Dichlormethan (1/1) für eine Stunde, entfernen des Lösungsmittels i. Vak und Behandlung des öligen Rückstandes mit Diethylether erhält man 111 mg L-Prolinylanilid als weißen Feststoff.

100 mg L-Prolinylanilid (0,52 mmol) werden in DMF gelöst. Zu dieser Lösung wird eine Lösung aus:118 mg Boc-3-Aminobenzoesäure (0,5mmol), 71 mg HOBt (0,5 mmol), 157 mg TBTU (0,5 mmol) und 170 µl DIPEA (1 mmol) zugesetzt. Bei Raumtemperatur wird für eine Stunde gerührt und anschließend das Lösungsmittel i. Vak entfernt. Der Rückstand wird in 200 ml Ethylacetat aufgenommen. Die Lösung wird je dreimal mit je 30 ml 10%ige Citronensäure-Lösung, ges. NaHCO₃-Lösung und Wasser gewaschen. Die organische Phase wird über Na₂SO₄ getrocknet und das Lösungsmittel i. Vak entfernt. Man erhält 174 mg der Verbindung 39.1 als weißen Feststoff.

Von 160 mg der Verbindung 39.1 (0,39 mmol) wird die Boc-Schutzgruppe durch Behandlung mit TFA in Dichlormethan (s.o.) abgespalten. Nach der Behandlung des Rückstandes mit Diethylether erhält man 145 mg des TFA-Salzes des primären Amins 39.2.

42 mg 39.2 (0,1 mmol) werden in 3 ml DMF gelöst. 29 mg N^{α}-tert.-Butyloxycarbonyl-{(E)-(L)-6-Amino-hept-2-endicarbonsäure}-1-methylester (1.9, 0,1 mmol) werden zusammen mit 38 mg HATU (0,1 mmol) in 5 ml DMF gelöst. Zu dieser Lösung werden 51 µl DIPEA (0,3 mmol) zugesetzt und die erhaltene Lösung sofort zu der Lösung von 39.2 gegeben. Durch sukzessive Zugabe von DIPEA wird der pH-Wert auf 9 eingestellt. Nach 30 wird das Lösungsmittel i. Vak. entfernt und der ölige braune Rückstand durch präparative HPLC gereinigt (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 40% B auf 100% B, 1 %/min).
Ausbeute: 43 mg
ESI-MS: 649,3 {M+Na}⁺

Beispiel 40 enthält ein Pyridon als peptidomimetisches Strukturelement. Pyridon ist ein anderes nicht-beschränkendes Beispiel für eine nicht-proteinoge Aminosäure. Der Syntheseweg verläuft parallel zu dem z.B. von Dragovich et al. publizierten (Dragovich P.S. et al., J. Med. Chem. 2002, 45, 16071623).

Allgemeine Synthesebeschreibung Darstellung Pyridon enthaltener Peptidomimetika (siehe Figur 5): Die Pyridongruppe wird in Form eines Dipeptidanalogons aufgebaut. Der Schlüsselschritt dabei ist die Bildung einer C-N-Bindung zwischen dem Stickstoff eines Pyridinderivates (z.B. 40.1) und einer geeignet substituierten α-Hydroxycarbonsäure (z.B. 40.2). Das erhaltene Dipeptidanalogon (z.B. 40.3) kann in nachfolgenden, oder vorausgegangenen Reaktionen an der Carbonsäure- sowie an der Aminofunktion weiter modifiziert werden. Dabei können alle hierin enthaltenen sowie literaturbekannten Methoden und Reaktionen zur Anwendung kommen.

### {E)-(S)-6-Benzyloxycarbonylamino-6-{1-[(S)-3-carboxy-1-(3-methyl-butylcarbamoyl)-propyl]-2-oxo-1,2-dihydro-pyridin-3-ylcarbamoyl}-hex-2-enoylsäureisopropylester (Verbindung 40)

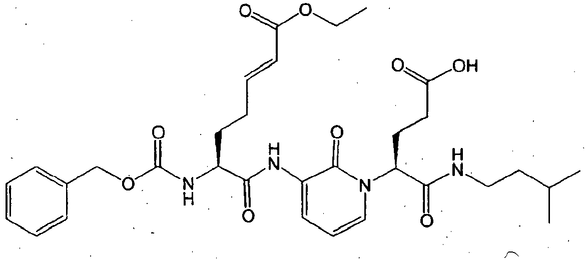

Die Synthese verläuft konvergent. Verbindung 40.1 (siehe Schema 5) wird wie in der Literatur beschrieben ausgehend von kommerziell erhältlichem 2-Hydroxy-3-nitropyridin dargestellt. Man erhält N-Boc-(2-Hydroxy-3-aminopyridin) (40.1). Die Darstellung von 40.2 gelingt wie folgt: 671 mg kommerziell erhältlicher D-N^{α}-tert.Butyloxycarbonyl-Glutaminsäure-5-allyllester (Boc-Glu(OAllyl)-OH, 2,34 mmol) werden, in 10 ml DMF gelöst und nacheinander mit 738 mg TBTU (2,3 mmol), 316 mg HOBT (2,34 mmol) und 605 mg (800 µl) DIPEA versetzt. Diese Lösung wird sofort zu einer Lösung von 224 mg Isopentylamin (2,57 mmol) in 5 ml DMF zugesetzt. Bei Raumtemperatur wird eine Stunde gerührt, bevor das Lösungsmittel i. Vak. entfernt wird. Der ölige Rückstand wird in 200 ml Dichlormethan aufgenommen und mit 10%iger Citronensäurelösung, 10%iger NaHCO₃-Lösung und ges. NaCl-Lösung gewaschen. Nach Trocknung der organischen Phase und entfernen des Lösungsmittels i. Vak. erhält man 589 mg Boc-Glu(OAllyl)-Isoperitylamid in reiner Form. Durch Behandlung mit TFA in Dichlormethan wird die Boc-Schutzgruppe abgespalten und die so freigesetzte Aminogruppe wie in der Literatur beschrieben (Winitz et al., J. Am. Soc. Chem., 1956, 78, 2423-2428), durch Diazotierung in eine Hydroxygruppe umgewandelt. Nach Reinigung durch Chromatographie an Kieselgel (Säule: 18*2,3 cm, DCM/Methanol = 98/2) erhält man 221 mg (D)-4-Hydroxy-4-(3-methylbutylcarbamoyl)-buttersäureallylester.

200 mg des erhaltenen (D)-4-Hydroxy-4-(3-methylbutylcarbamoyl)-buttersäure-allylesters (0,77 mmol) und 140 mg DIPEA (1,08 mmol) werden in Dichlormethan gelöst und unter Argonatmosphäre auf -10°C gekühlt. Zu dieser Lösung werden 132,9 mg Methansulfonylchlorid (0,92 mmol) tropfenweise zugesetzt. Nach 30 Minuten wird die Lösung mit Dichlormethan verdünnt und mit ges. NaCl-Lösung gewaschen. Die organische Phase wird über Na₂SO₄ getrocknet und das Lösungsmittel i. Vak. entfernt. Man erhält 183 mg des Mesylesters 40.2, der ohne weitere Reinigung umgesetzt werden kann.

237 mg des oben beschriebenen Hydroxypyridins 40.1 (1,13 mmol) werden in 10 ml absolutem THF gelöst. 43 mg NaH (60%ig auf Mineralöl, 1,08 mmol) werden zugesetzt und bei Raumtemperatur unter Stickstoffatmosphäre für 20 Minuten gerührt. Zu dieser Lösung werden 165 mg des Mesylates 40.2 (0,49 mmol) zugesetzt und die Reaktionsmischung für 48 Stunden am Rückfluss gekocht. Die Reaktionsmischung wird anschließend mit 200 ml Diethylether verdünnt und zweimal mit 10%iger KHSO₄-Lösung sowie zweimal mit ges. NaCl-Lösung gewaschen. Der erhaltene Rückstand wird durch Chromatographie an Kieselgel gereinigt. (Säule: 22*1,8 cm, DCM/Methanol = 99/1). Man erhält 138 mg der Verbindung 40.3 in reiner Form.

125 mg 40.3 (0,28 mmol) werden in THF_{abs} gelöst. Unter Argonatmophäre werden 242 mg Morpholin (2,8 mmol) und anschließend 32 mg Tetrakis(triphenylphosphin)palladium zugesetzt. Bei Raumtemperatur wird für 45 Minuten gerührt. Das Lösungsmittel wird i. Vak entfernt und die erhaltene Carbonsäure durch präparative HPLC gereinigt gereinigt (Synergie Max, 4µm, 250x21,2 mm, A-Eluent: 0,1 %TFA / Wasser, B-Eluent: 90% AcCN / 10% Wasser / 0,1 %TFA. Gradient: 8 ml/min, 5% B auf 100% B, 1%/min). Nach Abspaltung der Boc-Schutzgruppe durch Behandlung mit TFA in Dichlormethan erhält man 101 mg der freien Aminosäure 4.4 in Form ihres TFA-Salzes.

35,8 mg der Carbonsäure 1.7 (0,1 mmol) werden zusammen mit 38mg HATU (0,1 mmol) in 5 ml DMF gelöst. Zu dieser Lösung werden 51 µl DIPEA (0,3 mmol) zugesetzt und die erhaltene gelbe Lösung sofort zu einer Lösung von 42 mg der Aminosäure 4.4 (0,1 mmol) zugesetzt. Durch sukzessive Zugabe von DIPEA wird der pH-Wert auf 9 eingestellt. Nach 30 wird das Lösungsmittel i. Vak. entfernt und der ölige braune Rückstand durch präparative HPLC gereinigt (Synergie Max, 4µm, 250x21,2 mm, A-Eluent: 0,1 %TFA / Wasser, B-Eluent: 90% AcCN / 10% Wasser / 0,1 %TFA. Gradient: 8 ml/min, 40% B auf 100% B, 1%/min).
Ausbeute: 23 mg
ESI-MS: 649,2 [M+Na]⁺

### 3. Darstellung von Inhibitoren mit alternativen vinylogen Elektronenakzeptorgruppen

Erfindungsgemäße Inhibitoren enthalten eine vinyloge Elektronenakzeptor-Gruppe. Solche Michael-Akzeptor-Systeme können erhalten werden, indem ein geeigneter Aldehyd (z.B. 1.4) in einer Wittig-Reaktion oder einer Horner-Wadsworth-Emmons Reaktion (J. Am. Chem. Soc, 1961, 83, 1733) mit einem geeignetem Phosphonium-Ylid umgesetzt wird.

Andere nicht-beschränkende Beispiele zur Darstellung erfindungsgemäßer vinyloger Elektronenakzeptor Verbindungen (Michael-Systeme) nach denen ein erfindungsgemäßes Olefin dargestellt werden kann, sind: Deacylierungsreaktionen (Tetrahedron 2004, 2337), Knoevenagel-Reaktioneh (J. Chem. Soc. Perkin Trans. 1986, 2137) oder Peterson-Ölefinierungen (J. Chem. Soc. Perkin. Trans1, 1985, 1949) Besonders bevorzugt sind Reaktionen mit Phosphonium-Yliden. Geeignet substituierte Phosphonium-Ylide sind entweder kommerziell erhältlich, oder können leicht selbst dargestellt werden (z.B. J. Chem. Soc., Perkin Trans1, 1985, 1481; J. Med. Chem., 1987, 1995). Nicht beschränkende Synthesebeispiele für die entsprechenden Vorstufen erfindungsgemäßer Inhibitoren nach Formel G mit variierendem Z und Z' sind im Folgenden aufgeführt. Die weitere Umsetzung dieser Vorstufen kann nach den unter 1.7 oder 1.9 sowie den Beispielen 1 bis 38 beschriebenen Verfahren durchgeführt werden.

### 3.1 Darstellung der pharmakophor-tragenden Aminosäuren

### N,N-Di-(tert.-Butyloxycarbonyl)-[(E)-(L)-6-Amino-hept-2-endicarbonsäure]-7-tert.-Butylester (Verbindung 3.1)

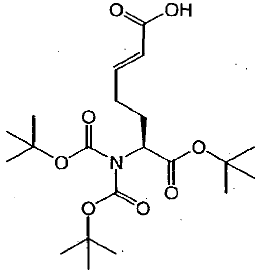

100 mg N,N-Di-(tert.-Butyloxycarbonyl)-{(E)-(L)-6-Amino-hept-2-endicarbonsäure}-1-ethanoyl-7-tert.-Butylester (1.8, 0,22 mmol) werden in einem Milliliter Ethanol gelöst und mit 503 µl einer einmolaren Natriumhydroxyd-Lösung versetzt. Bei Raumtemperatur wird für drei Stunden gerührt. Die Lösung wird durch Zusatz von je 20 ml Wasser und Diethylether verdünnt und anschließend mit 2 molare HCl-Lösung auf einen pH-Wert von ~ 2 gebracht. Die wässrige Phase wird noch zweimal mit je 20 ml Ethylacetat extrahiert und die vereinigten organischen Phasen über Na₂SO₄ getrocknet. Nach Entfernen des Lösungsmittels im Vakuum erhält man die freie Carbonsäure als farbloses Öl.
Ausbeute: 94 mg
ESI-MS: 452,4 {M+Na}⁺

### N,N-Di-(tert.-Butyloxycarbonyl)-[(E)-(L)-6-Amino-hept-2-endicarbonsäure]-1-pentylamido-7-tert.-Butylester (Verbindung 3.2)

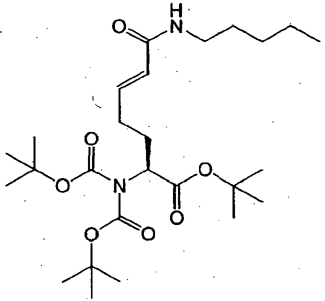

90 mg des unter 3.2 erhaltenen Produktes werden in 3,5 ml trockenem Dichlormethan gelöst. Nacheinander werden 42 mg Dicyclohexylcarbodiimid und 30 mg 1-Aminopentan zugesetzt.

Bei Raumtemperatur wird über Nacht gerührt. Anschließend wird das Lösungsmittel i. Vak. entfernt und der erhaltene Rückstand durch Chromatographie an Kieselgel gereinigt (Säule: 21*1,8 cm, Petrolether/Ethylacetat = 9/1).
Ausbeute: 80 mg
ESI-MS: 521,2 {M+Na}⁺

### N,N-Di-(tert.-Butyloxycarbonyl)-[(E)-(L)-6-Amino-hept-2-endicarbonsäure]-1-isopropanoyl-7-tert.-Butylester (Verbindung 3.3)

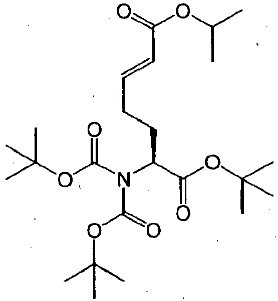

100 mg N,N-Di-(tert.-Butyloxycarbonyl)-L-2-Amino-5-Oxopentansäure-1-tert.-Butylester (1.4, 0,258 mmol), werden in 3 ml trockenem Benzol gelöst und mit 93,5 mg (0,258 mmol) (Isopropoxycarbonylmethylen)-triphenylphosphoran (dargestellt nach üblichen literaturbekannten Verfahren) versetzt. Bei Raumtemperatur wird für fünf Stunden gerührt. Anschließend wird das Lösungsmittel i. Vak. entfernt und der erhaltene ölige Rückstand durch Chromatographie an Kieselgel gereinigt.
(Säule: 29*2,3 cm, DCM/Methanol = 99,5/0,5).
Ausbeute: 91 mg
ESI-MS:494,4 {M+Na}⁺

### N,N-Di-(tert.-Butyloxycarbonyl)-[(E)-(L)-6-Amino-hept-2-endicarbonsäure]-1-benzoyl-7-tert.-Butylester (Verbindung 3.4)

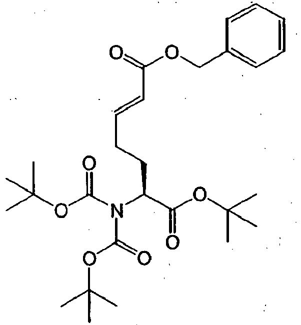

100 mg N,N-Di-(tert.-Butyloxycarbonyl)-L-2-Amino-5-Oxopentansäure-1-tert.-Butylester (1.4, 0,258 mmol), werden in 3 ml trockenem Benzol gelöst und mit 109,2 mg (0,258 mmol) (Benzyloxycarbonylmethylen)-triphenylphosphoran (Sigma Aldrich, 0,258 mmol) versetzt. Bei Raumtemperatur wird für 18 Stunden gerührt. Anschließend wird das Lösungsmittel i. Vak. entfernt und der erhaltene ölige Rückstand durch Chromatographie an Kieselgel gereinigt.
(Säule: 28*2,3 cm, DCM/Methanol = 99/1).
Ausbeute: 94 mg
ESI-MS: 542,4 {M+Na}⁺

Erfindungsgemäße Michaelakzeptoren können auch in Form endo- und exocyclischen Systemen dargestellt werden. Figur 6 zeigt die Darstellung eines Cyclopentenons (3.5, Nóvak et al. Liebigs Ann. Chem. 1986, 509-524).

### N,N-Di-(tert.-Butyloxycarbonyl)-[((L)-2-Amino-4-(3)-oxo-cyclopent1enyl)-buttersäure]-1-tert.-Butylester (Verbindung 3.5)

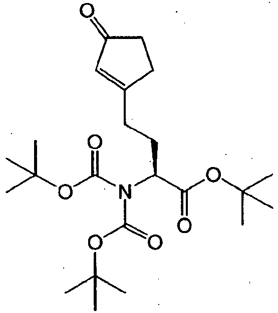

Die Darstellung erfolgt wie in der Literatur beschrieben.
Ausbeute: 126 mg
ESI-MS: 493,3

Die Abspaltung der Schutzgruppen und das Einführen einer Benzyloxycarbonylschutzgruppe wird durchgeführt, wie unter 1.7 beschrieben.

Einige der dargestellten vinylogen Elektronenakzeptorverbindungen stellten sich im Syntheseverlauf als nicht ausreichend stabil gegenüber starken Säuren heraus. Die Bedingungen die unter 1.7 oder 1.9 beschrieben werden, sind deshalb bei der Synthese solcher Michael-Akzeptoren nicht anwendbar. Als alternative Syntheseroute kann in einem solchen Fall, der exemplarisch für die Verbindungen 3.7, bis 3.9 und 3.13 dargestellt ist, erfolgreich eine Variation der unter 1.16 beschriebenen Synthese durchgeführt werden (3.6). Man erhält Verbindungen deren weitere Umsetzung nach den hier beschriebenen Methoden (Figur 2) zu den säurelabileren Michael-Akzeptoren führt. Figur 7 zeigt die Darstellung eines Synthesebausteines mit exocyclischer Doppelbindung (3.7). Das dabei benötigte Triphenylphosphoniumsalz ist kommerziell nicht erhältlich, kann aber wie in der Literatur beschrieben hergestellt werden (Baldwinn, J.E., J. Org. Chem., 1971, 10, 1441-1443).

### N^{α}-tert.-Butyloxycarbonyl, N^{α}-Benzyloxycarbonyl-[L-2-Amino-5-Oxopentansäure]-1-2-Phenyl-2-propylester (Verbindung 3.6)

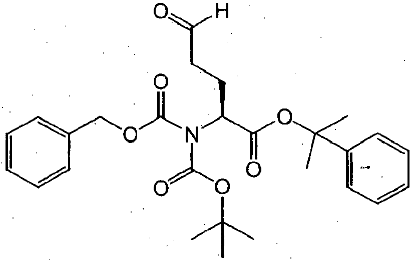

2,95 g Z-Glu(OMe)-OH (10 mmol) werden in 200 ml absolutem Dichlormethan gelöst und unter Stickstoffatmosphäre auf 0°C gekühlt. Zu dieser Lösung werden 2,88 g Dicyclohexylcarbodiimid (14mmol) und 122 mg N,N-Dimethylaminopyridin (1 mmol) zugesetzt. Es wird für 15 Minuten bei 0°C gerührt und anschließend werden 1,63 g 2-Phenylisopropanol (12 mmol) zugegeben. Nach weiteren 30 Minuten wird das Eisbad entfernt und die Reaktionslösung über Nacht bei Raumtemperatur weiter gerührt. Vom ausgefallenen Feststoff wird abfiltriert und das erhaltene Rohprodukt durch Chromatographie an Kieselgel gereinigt (Ausbeute: 1,53 g).

Der so erhaltene Isopropylphenylester (1,53 g, 3,7 mmol) wird in 10 ml Acetonitril gelöst und nacheinander mit 90 mg N,N-Dimethylaminopyridin (0,7 mmol) sowie 1,58 ml Di-tert.Butyldicarbonat (Boc₂O, 7,4 mmol) versetzt. Unter Stickstoffatmosphäre wird über Nacht bei Raumtemperatur gerührt. Das Lösungsmittel wird i. Vak. entfernt und der erhaltene ölige Rückstand durch Chromatographie an Kieselgel gereinigt. Man erhält das reine Zwischenprodukt (Z,Boc-Glu(OMe)-OiPrPh) als farbloses, zähes Öl.

1,1 g Z,Boc-Glu(OMe)-OiPrPh (2,14mmol) werden in 25 ml absolutem Diethylether gelöst und unter Argonatmosphäre auf -78°C gekühlt. Zu dieser Lösung werden langsam 2,36 ml einer 1 molaren Lösung von Diisobutylaluminumhydrid in n-Hexan zugetropft und anschließend für 30 Minuten gerührt. Die Reaktion wird durch Zusatz von 1 ml Wasser gequencht und anschließend auf Raumtemperatur aufgetaut. Der Ansatz wird über Kieselgur filtriert und das Filtrat zur Trockne eingeengt. Chromatographie an Kieselgel liefert den reinen Aldehyd (Z,Boc-Glu(H)-OiPrPh, 3.6 Ausbeute: 667 mg.
ESI-MS: 506,2 [M+Na]⁺, 406 [M-Boc+Na]⁺

### (L)-2-Benzyloxycarbonylamino-5-[2-oxo-dihydrofuran-(3E)-yliden]-pentansäure (Verbindung 3.7)

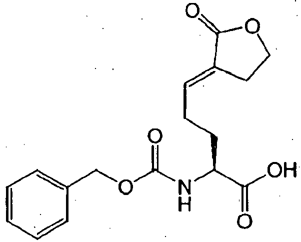

12,4 mg NaH (60 %ig in Mineralöl, 0,3 mmol) werden unter Argonatmosphäre vorgelegt und eine Lösung aus 133 mg α-Triphenylphosphoniumbutyrolacton-bromid (Baldwin, 1971) in 3,5 ml DMF_{abs} zugesetzt. Bei Raumtemperatur wird bis zur Beendigung der Gasentwicklung gerührt und anschließend 150 mg des Aldehyds 3.6 (0,3 mmol) gelöst in 0,5 ml DMF_{abs}, zugesetzt. Nach Rühren über Nacht wird das Lösungsmittel i. Vak. entfernt und der erhaltene Rückstand durch Chromatographie an Kieselgel gereinigt (Ausbeute: 112 mg).

Durch die Behandlung mit 2%iger TFA in Dichlormethan werden die stark säurelabilen Schutzgruppen entfernt und man erhält durch Chromatographie an Kieselgel (Säule: 29,5x2,3 cm, DCM/Methanol = 8/2) die Titelverbindung in reiner Form.
Ausbeute: 65 mg
ESI-MS: 356,0 [M+Na]⁺

### N^{α}-Benzyloxycarbonyl)-[(E)-(L)-7-Amino-2-oxo-oct-3-endicarbonsäure]-1-ethylester (Verbindung 3.8)

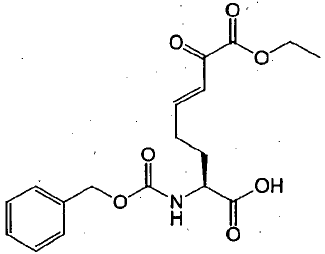

556 mg N^{α}-tert.-Butyloxycarbonyl, N-Benzyloxycarbonyl-L-2-Amino-5-Oxopentansäure-1-(2-Phenylisopropylester, 3.6, 1,15 mmol) werden in 20 ml trockenem Xylol vorgelegt und unter Argonatmosphäre bei Raumtemperatur eine Lösung von 432 mg Ethyl- (triphenylphosphoranyliden)pyrovat (Sigma-Aldrich, 1,15 mmol) zugesetzt. Nach Rühren bei 115 °C für vier Stunden, wird das Lösungsmittel i. Vak. entfernt und der erhaltene ölige Rückstand durch Chromatographie an Kieselgel gereinigt. (Säule: 29*2,4 cm, Petrolether/Ethylacetat = 99/1).
Verbindung 3.8 wird dargestellt, indem man das oben erhaltene Zwischenprodukt in Dichlormethan mit 1-2 % TFA für eine Stunde bei Raumtemperatur rührt. Die Reinigung erfolgt durch Chromatographie an Kieselgel.
Ausbeute: 324 mg
ESI-MS: 386,2 {M+Na}⁺

### N^{α}-Benzyloxycarbonyl-[(Z)-(L)-2-Amino-7-oxo-oct-5-ensäure] (Verbindung 3.9)

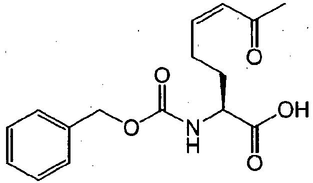

125 mg 3.6 (0,258 mmol), werden in 3 ml trockenem Benzol gelöst. Zu dieser Lösung werden 82 mg (0,258 mmol) Acetylen-triphenylphosphoran zugesetzt. Bei 60°C wird 2 Stunden gerührt. Das Lösungsmittel wird i. Vak. entfernt und der erhaltene Rückstand durch Chromatographie an Kieselgel gereinigt. Anschließendes Entfernen der Schutzgruppen durch Verwendung von 2% TFA in Dichlormethan und Reinigung mittels präparative HPLC liefert Verbindung 3.9.

(Synergie Max, 4µm, 250x21,2 mm, A-Eluent: 0,1 %TFA / Wasser, B-Eluent: 90% AcCN /10% Wasser/0,1%TFA. Gradient: 8 ml/min, 25% B auf 100% B, 1%/min).
Ausbeute: 58 mg
ESI-MS: 328,1 {M+Na}⁺

### N^{α}-Benzyloxycarbonyl-(Z)-(L)-2-Amino-6-cyano-hex-5-ensäure (Verbindung 3.10)

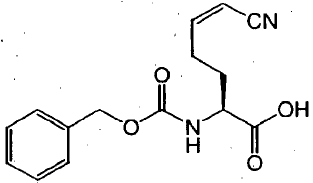

125 mg 3.6 (0,258 mmol), werden in 3 ml trockenem Benzol gelöst. Zu dieser Lösung werden 78 mg (Triphenylphophoranylidin)acetonitril (Sigma-Aldrich, 0,258 mmol) zugesetzt. Bei 70°C wird 4 Stunden gerührt. Das Lösungsmittel wird i. Vak. entfernt und der erhaltene Rückstand durch Chromatographie an Kieselgel gereinigt. Anschließendes Entfernen der Schutzgruppen durch Verwendung von 2% TFA in Dichlormethan und Reinigung mittels präparativer HPLC liefert Verbindung 3.13
Ausbeute: 78 mg
ESI-MS: 311,2 {M+Na}⁺

Durch Einsatz der entsprechenden Phosphonate oder Phosphoniumsalze können über die Horner-Wadsworth-Emmons-Reaktion auch Vinylsulfone dargestellt werden (beispielhaft: 3.11, 3.12a und 3.13). Von besonderem Interesse sind dabei solche vinylogen Sulfone, die durch nachfolgende Reaktionen weiter diversifiziert werden können. Roush et al. beschreiben einen solchen Syntheseweg für Inhibitoren gegen Cruazin und Papain (Roush W.R., Bioorg. Med. Chem. Lett., 2001, 11, 2759) (und darin zitierte Referenzen); Roush W.R., J. Am. Chem. Soc., 1998, 120, 10994). Dabei wird, ausgehend von einem vinylogen Sulfonsäureester (siehe z.B. 3.12a in Schema 8) ein Sulfonsäurechlorid (3.12.b) erzeugt und dieses anschließend in ein vinyloges Sulfonamid, ein N-Alkoxysulfonamid oder einen Sulfonsäurester mit einem anderen Rest überführt (Schema 8). Beispielhaft sind gegeben: 3.14 bis 3.16.

Überraschenderweise hat sich herausgestellt, dass solche Michael-Akzeptoren, mit Sulfonylresten als elektronenwegziehende Gruppe, auch bei Transglutaminasen potente Inhibitoren darstellen. Ein Ausführungsbeispiel ist mit 3.2.1 gegeben.

### N^{α}-Benzyloxycarbonyl-(E)-(L)-2-Amino-6-methansulfonyl-hex-5-ensäure (Verbindung 3.11)

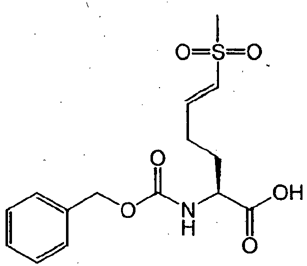

Die Darstellung der Verbindung 3.11 gelingt nach den unter 3.7 beschriebenen Methoden. Das zur Darstellung des vinylogen Sulfons benötigte Reagenz ((EtO)₂P(=O)SO₂Me) kann nach dem Fachmann bekannten Verfahren durch Oxidation, z.B. mit Metachlorperbenzoesäure, des kommerziell erhältlichen ((EtO)₂P(=O)SMe, Sigma-Aldrich), erhalten werden.

### Detaillierte Beschreibung der Synthese:

10 mg NaH (60%ig in Mineralöl, 0,258 mmol) werden in 5 ml DMF_{abs} gelöst und unter Stickstoffatmosphäre 59 mg (EtO)₂P(=O)SO₂Me (0,258 mmol) zugesetzt. Nach beendeter Gasentwicklung werden 130 mg 3.6 (0,258 mmol) zugesetzt und die Lösung bei Raumtemperatur über Nacht gerührt. Das Lösungsmittel wird i. Vak. entfernt und der erhaltene Rückstand mit einer Lösung von 1 % TFA in Dichlormethan übergossen. Nach 30 Minuten wird das Lösungsmittel i. Vak. eingeengt und der erhaltene Rückstand durch Chromatographie an Kieselgel gereinigt. (Säule:29x2,3 cm, Dichlormethan/Methanol: 99/1).
Ausbeute : 89 mg
ESI-MS: 364,2 [M+Na]⁺

### N^{α}-Benzyloxycarbonyl,N^{α}-tert.Butyloxycarbonyl-[(E)-(L)-2-Amino-6-ethoxysulfonyl-hex-5-ensäure]-1-(2-phenyl-2-propylester) (Verbindung 3.12a)

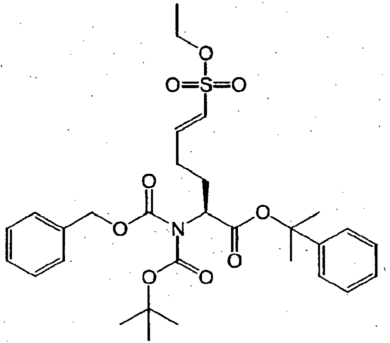

119,8 mg NaH (3 mmol) werden vorgelegt und mit einer Lösung von 780 mg (3 mmol) Ethyldiethylphophorylmethansulfonat (Carretero et al., 1987) in 20 ml DMF_{abs} versetzt. Nach beendeter Gasentwicklung wird eine Lösung von 1,45 g 3.6 (3 mmol) gelöst in 10 ml DMF_{abs} werden zugesetzt. Bei Raumtemperatur wird über Nacht gerührt. Das Lösungsmittel wird i. Vak. entfernt und der erhaltene Rückstand durch präparative HPLC gereinigt. Man erhält so, nach Abtrennung des Z-Isomeres, Verbindung 3.12.a in reiner Form (siehe Schema 8). (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 40% B auf 100% B, 1 %/min).
Ausbeute: 1,5 g
ESI-MS: 512,2 {M-Boc+Na}⁺

### N^{α}-Benzyloxycarbonyl,N-tert.Butyloxycarbonyl-[(E)-(L)-2-Amino-6-chlorosulfonyl-hex-5-ensäure]-1-(2-phenyl-2-propylester) (Verbindung 3.12b)

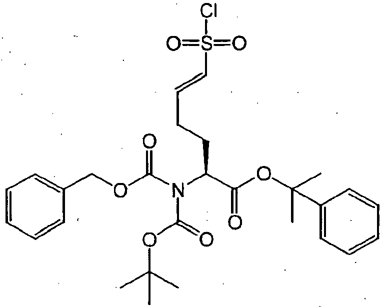

1,47 g von Verbindung 3.12a (2,5 mmol) werden in 100 ml Aceton gelöst und mit 941 mg Tetrabutylammoniumiodid (2,55 mmol) versetzt. Die Lösung wird über Nacht am Rückfluss zu sieden erhitzt. Anschließend wird das Lösungsmittel i. Vak entfernt und das Tetrabutylammoniumsalz (siehe Schema 8) durch Chromatographie an Kieselgel gereinigt (Säule: 2,5x27 cm, DCM/MeOH/TEA = 8/2/0,1). Das so erhaltene Salz (1,27 g) wird in trockenem Dichlormethan gelöst und diese Lösung mit einem Tropfen DMF_{abs} versetzt. Anschließend werden 235 mg Triphosgen (0,8 mmol) zugesetzt und der Ansatz unter Stickstoffatmosphäre für eine Stunde bei Raumtemperatur gerührt. Die Lösung wird zweimal mit je 30 ml Wasser gewaschen, über NaSO₄ getrocknet und das Lösungsmittel i. Vak. entfernt. Man erhält so die Titelverbindung in hinreichend reiner Form, um ohne weitere Reinigung die folgenden Reaktionen durchführen zu können.
Ausbeute: 803 mg
ESI-MS: 502,1 {M-Boc+Na}⁺

Exemplarische Arbeitsvorschrift zur Darstellung vinyloger Vinylsulfone mit alterierendem Rest.

Durch Einsatz von ((EtO)₂P(=O)SO₂Phe), kann auf dem gleichen Syntheseweg wie für Verbindung 3.11 beschrieben, Verbindung 3.13 erhalten werden.

### N^{α}-Benzyloxycarbonyl-(E)-(L)-2-Amino-6-phenylsulfonyl-hex-5-ensäure (Verbindung 3.13)

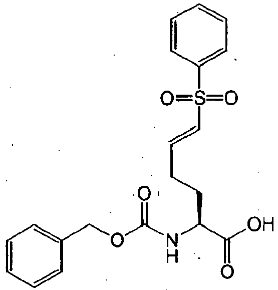

ESI-MS: 426,1 [M+Na]⁺

### N^{α}-Benzyloxycarbonyl-((E)-(L)-2-Amino-6-benzyloxysulfonyl)-hex-5-ensäure (Verbindung 3.14)

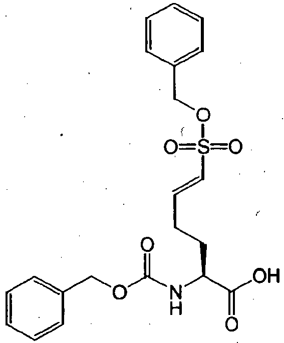

150 mg N^{α}-Benzyloxycarbonyl,N^{α}-tert.Butyloxycarbonyl-[(E)-(L)-2-Amino-6-chlorosulfonyl-hex-5-ensäure]-1-(2-phenyl-2-propylester) (3.12b, 0,25 mmol) werden in 10 ml DCM_{abs} gelöst. Unter Argonatmosphäre werden nacheinander 30 mg Benzylalkohol und 42 mg 1,8-Diazabicyclo[5,4,0]-undec-7-en (DBU, 0,27 mmol) zugesetzt.

Bei Raumtemperatur wird über Nacht gerührt. Der Lösung werden 200µl TFA zugesetzt und für weitere 30 Minuten gerührt. Das Lösungsmittel wird i. Vak. entfernt und der erhaltene ölige Rückstand durch präparative HPLC gereinigt. (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 40% B auf 100% B, 1%/min).
Ausbeute: 60,6 mg
ESI-MS: 456,2 {M+Na}⁺

### N^{α}-Benzyloxycarbonyl-[(E)-(L)-2-Amino-6-dimethylsulfamoyl]-hex-5-ensäure (Verbindung 3.15)

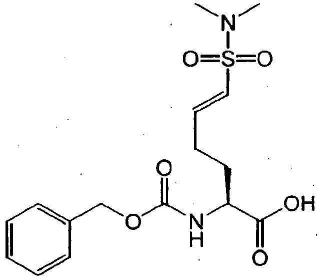

150 mg N^{α}-Benzyloxycarbonyl,N^{α}-tert.Butyloxycarbonyl-[(E)-(L)-2-Amino-6-chlorosulfonyl-hex-5-ensäure]-1-(2-phenyl-2-propylester) (3.12b, 0,25 mmol) werden in 10 ml DCM_{abs} gelöst. Unter Argonatmosphäre werden nacheinander 42 mg 1,8-Diazabicyclo[5,4,0]-undec-7-en (DBU, 0,27 mmol) und 138 µl einer zweimolaren Lösung von Dimethylamin (0,27 mmol) in THF zugesetzt.

Bei Raumtemperatur wird über Nacht gerührt. Der Lösung werden 200µl TFA zugesetzt und für weitere 30 Minuten gerührt. Das Lösungsmittel wird i. Vak. entfernt und der erhaltene ölige Rückstand durch präparative HPLC gereinigt. (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 40% B auf 100% B, 1%/min).
Ausbeute: 66 mg
ESI-MS: 393,12 {M+Na}⁺

### N^{α}-Benzyloxycarbonyl-((E)-(L)-2-Amino-6-benzyloxysulfamoyl)-hex-5-ensäure (Verbindung 3.16)

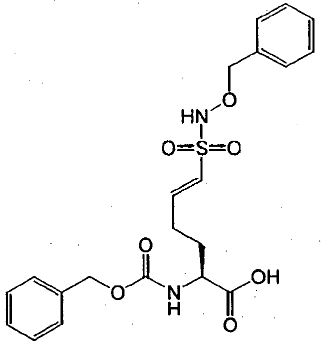

Nach dem unter 3.14 und 3.15 beschriebenem Verfahren wird die Titelverbindung durch Einsatz von 44 mg O-Benzylhydroxylamin-Hydrochlorid (0,27 mmol) als Nucleophil dargestellt.
Ausbeute: 53 mg
ESI-MS: 393,12 {M+Na}⁺

Nach den unter 1. und 2. beschriebenen Methoden können aus den unter 3. beschriebenen pharmakophor-tragenden Aminosäuren die in Tabelle 2 aufgeführten Inhibitoren dargestellt werden. Zusätzlich sind einige Ausführungsbeispiele im Folgenden (3.2) gegeben.

### 3.2 Exemplarische Darstellung von Inhibitoren enthaltend alternative vinyloge Elektronenakzeptorgruppen

### N^{α}-Benzyloxycarbonyl-{[(E)-(L)-2-Amino-6-methansulfonyl]-hex-5-enyl}-L-Glutaminyl-L-Prolinyl-L-Leucinmethylester (Verbindung 3.2.1)

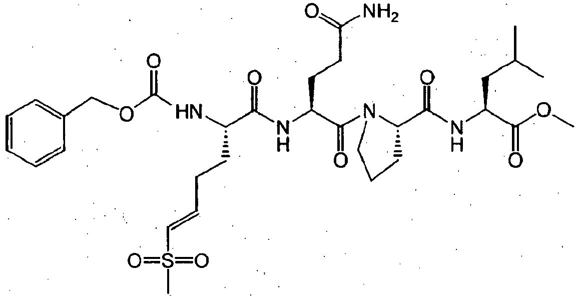

27 mg N^{α}-Benzyloxycarbonyl-(E)-(L)-2-Amino-6-methansulfonyl-hex-5-ensäure (3.11, 79 µmol) werden in 2,5 ml DMF gelöst und nacheinander mit 30 mg HATU (79 µmol) und 2.7µl DIPEA (158 µmol) versetzt und sofort zu einer Lösung des Trifluoracetatsalzes von 79 µmol H-Gln-Pro-Leu-OMe (nach Methode 1 dargestellt) in 5 ml DMF zugesetzt. Durch sukzessive Zugabe von DIPEA wird der pH-Wert auf 9 eingestellt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 30% B auf 100% B, 1 %/min).
Ausbeute: 40 mg
ESI-MS: 716,5 {M+Na}⁺

### N^{α}-Benzyloxycarbonyl-[(E)-(L)-2-Amino-6-dimethylsulfamoyl)-hex-5-enyl]-L-Glutaminyl-L-Prolinyl-L-Leucinmethylester (Verbindung 3.2.2)

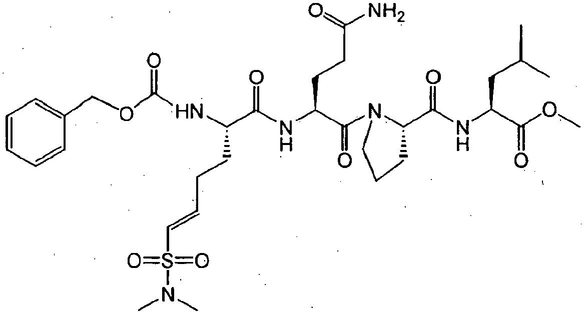

33 mg N^{α}-Benzyloxycarbonyl-[(E)-(L)-2-Amino-6-dimethylsulfamoyl]-hex-5-ensäure (3.15, 89 µmmol) werden in 2,5 ml DMF gelöst und nacheinander mit 34 mg HATU (89 µmol) und 30 µl DIPEA (158 µmol) versetzt und sofort zu einer Lösung des Trifluoracetatsalzes von 89 µmol H-Gln-Pro-Leu-OMe (nach Methode 1 dargestellt) in 5 ml DMF zugesetzt. Durch sukzessive Zugabe von DIPEA wird der pH-Wert auf 9 eingestellt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 35% B auf 100% B, 1 %/min).
Ausbeute: 42 mg
ESI-MS: 745,3 {M+Na}⁺

### N^{α}-Benzyloxycarbonyl-[(L)-2-Amino-4-(3)-oxo-cyclopent-1-enyl]-butyryl-L-Glutaminyl-L-Prolinyl-L-Leucinmethylester (Verbindung 3.2.3)

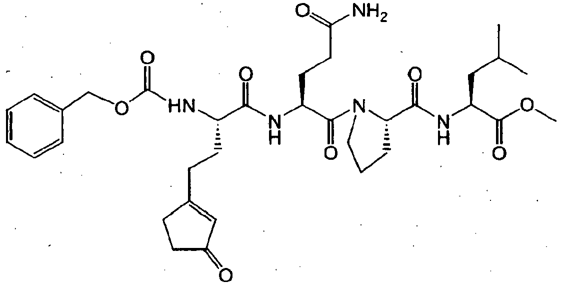

Verbindung 3.5 wird, wie unter 1.7 beschrieben, entschützt und in das N^{α}-Benzyloxycarbonyl-geschützte Derivat überführt. 30,3 mg N^{α}-Benzyloxycarbony-[(L)-2-Amino-4-(3)-oxo-cyclopent1-enyl]-buttersäure (0,1 mmol) werden in 7,5 ml DMF gelöst und nacheinander mit 38 mg HATU (0,1 mmol) und 51 µl DIPEA (0,3 mmol) versetzt und sofort zu einer Lösung des Trifluoracetatsalzes von 0,1 mmol H-Gln-Pro-Leu-OMe (nach Methode 1 dargestellt) in 7,5 ml DMF zugesetzt. Durch sukzessive Zugabe von DIPEA wird der pH-Wert auf 9 eingestellt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1%/min).
Ausbeute: 26 mg
ESI-MS: 692,2 {M+Na}⁺

### N^{α}-Benzyloxycarbonyl[(L)-2-Amino-5-(2-oxo-dihydrofuran-(3E)-yliden)]-pentanoyl-L-Glutaminyl-L-Prolinyl-L-Leucinmethylester (Verbindung 3.2.4)

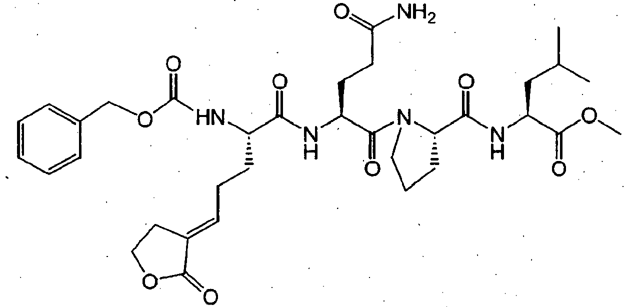

58 mg (L)-2-Benzyloxycarbonylamino-5-[-2-oxo-dihydrofuran-(3E)-yliden]-pentansäure (3.7, 0,17 mmol) werden in 10 ml DMF gelöst und nacheinander mit 64 mg HATU (0,17 mmol) und 87 µl DIPEA (0,5 mmol) versetzt und sofort zu einer Lösung des Trifluoracetatsalzes von 0,17 mmol H-Gln-Pro-Leu-OMe (nach Methode 1 dargestellt) in 10 ml DMF zugesetzt. Durch sukzessive Zugabe von DIPEA wird der pH-Wert auf 9 eingestellt. Die Aufarbeitung erfolgt wie für Verbindung 1 beschrieben: (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 25% B auf 100% B, 1%/min).
Ausbeute: 46 mg
ESI-MS: 708,4 {M+Na}⁺.

### 4. Variation des C-Terminus

Der Einsatz erfindungsgemäßer Transglutaminaseinhibitoren als Wirkstoffe zur Behandlung von Krankheiten im Menschen ist natürlich auch davon abhängig, wie ausgeprägt Bioverfügbarkeit und Metabolismus der Verbindung sind. Die Metabolisierung von peptidischen Wirkstoffen verläuft oft schnell. Damit wird die effektiv wirksame Dosis verringert. Um diesem Prozess entgegen zu wirken, werden peptidische Wirkstoffe meist in Peptidomimetika umgewandelt. Am C-Terminus können diese Mimetika neben den oben zumeist beschriebenen Methylestern auch andere Ester, prim., sek. oder tert. Amide, sowie freie Carbonsäuren als C-terminale Endgruppe als nicht-peptidische funktionelle Gruppe enthalten Die Festphasensynthese erfindungsgemäßer Inhibitoren ist in Figur 9 gezeigt. Eine umfassende Zusammenstellung allgemeiner Synthesevorschriften für die Festphasensynthese von Peptiden findet man beispielsweise in Fmoc Solid Phase Peptide Synthesis, A practical approach, Chan, W.C., White P.D., Oxford University Press*.* Als konkretes Ausführungsbeispiel ist mit Verbindung 4.1 die Synthese eines C-terminalen Amides gegeben. Sekundäre Amide können durch den Einsatz entsprechender Amine im ersten Kopplungsschritt erhalten werden. Siehe dazu beispielsweise die Synthese der Verbindungen 5 bzw. 5.1

Als Carbonylsurrogate können beispielhaft Tetrazole oder Sulfonamide dargestellt werden (Johanson A. et al., Bioorg. & Med. Chem., 11, 2003, 2551-68, siehe Figur 10). Überraschenderweise sind die dabei entstehenden, zum Teil sehr polaren Moleküle, hoch potente Inhibitoren der Transglutaminasen.

Einige konkrete Ausführungsbeispiele sind im Folgenden beschrieben.

### Exemplarische Festphasensynthese eines erfindungsgemäßen Inhibitors

### N^{α}-Benzyloxycarbonyl-{[(E)-(L)-6-Amino-hept-2-endicarbonsäure]-1-ethanoyl}-L-Valinyl-L-(Octahydroindol-2-carboxyl)-L-Leucinylamid (Verbindung 4.1)

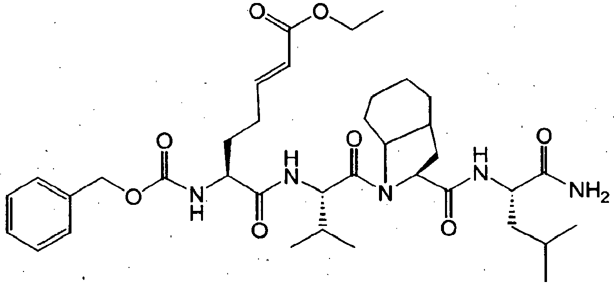

364 mg Sieber-Amid Harz (0,2 mmol, Nova Biochem) werden nach literaturbekannten Methoden durch Behandlung mit 20%Piperidin in DMF von der Fmoc-Schutzgruppe befreit und die erhaltende Aminogruppe mit 141 mg Fmoc-Leu-OH (0,4 mmol, Fluka), 126 mg TBTU (0,39 mmol, Fluka), 54 mg (0,4 mmol) und 136 µl DIPEA (0,8mmol) nach dem Fachmann bekannten Methoden mit Fmoc-Leu-OH gekoppelt. Nach einer Stunde wird das Reaktionsgemisch abfiltriert und wiederholt gründlich mit DMF gewaschen. Die Vollständigkeit der Kopplungsreaktion wird durch Kaiser-Test nachgewiesen. Die Fmoc-Schutzgruppe wird durch Behandlung des polymeren Trägers mit 20% Piperidin in DMF abgespalten. Die Reaktionslösung wird abfiltriert und der Harz mit DMF gründlich gewaschen. Die nun freie Aminogruppe des polymer gebundenen Leucins, wird mit 157 mg Fmoc-L-octahydroindole-2-Carbonsäure (Fmoc-Oic-OH, 0,4 mmol) unter den oben angegebenen Bedingungen acyliert. Durch den Kaiser-Test wird die Vollständigkeit der Reaktion nachgewiesen und anschließend, die Reaktionslösung abfiltriert und der polymere Träger mit DMF gründlich gewaschen. Nach Abspaltung der Fmoc-Schutzgruppe von Oic und Auswaschen der Reaktionslösung, wird die den Pharmakophor tragende Aminosäure N^{α}-Benzyloxycarbonyl-{(E)-(L)-6-Amino-hept-2-endicarbonsäure}-1-ethylester (1.7) an die freigesetzte Aminogruppe gekoppelt. Dafür werden 134 mg 1.7 (0,4 mmol) in DMF gelöst und nacheinander mit 150 mg HATU (0,39 mmol) und 136 µl DIPEA versetzt und zu dem polymer gebundenen Amin zugesetzt. Nach einer Stunde wird die Reaktionslösung abfiltriert und der Harz gründlich mit DMF gewaschen. Durch Kaiser-Test wird die Vollständigkeit der Reaktion nachgewiesen.

Die Abspaltung der Titelverbindung vom polymeren Träger erfolgt durch 30 minütige Behandlung des Trägermateriales mit einer Lösung von 1% TFA in Dichlormethan. Die erhaltene Produktlösung wird i. Vak. eingeengt und der Rückstand durch präparative HPLC gereinigt. (Synergie Max, 4µm, 250x21,2 mm, A-Eluent: 0,1%TFA / Wasser, B-Eluent: 90% AcCN /10% Wasser / 0,1 %TFA. Gradient: 8 ml/min, 5% B auf 100% B, 1%/min).
Ausbeute: 78 mg
ESI-MS: 720,3 {M+Na}⁺

### N^{α}-(Piperidinyl-4-carbonyl)-{[(E)-(L)-2-Amino-6-phenylsulfonyl]-hex-5-enyl}-L-Phenylalaninyl-L-Prolinyl-L-1-Cyclopentylmethyl-2-oxo-2-(1H-tetrazol-5-yl)-ethylamid (Verbindung 4.2)

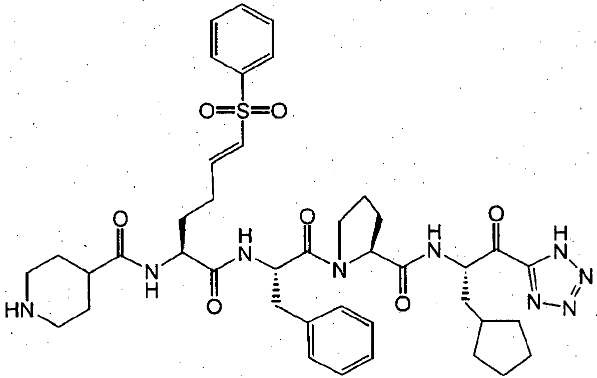

Die Synthese verläuft konvergent. (Übersicht: Siehe Schema 11). Die Pharmakophor tragende Aminosäure (N^{α}-(N-Boc-(Piperidinyl-4-carbonyl))-{[(E)-(L)-2-Amino-6-phenylsulfonyl]-hex-5-ensäure, 4.2.1) wird wie unter 3.11 beschrieben dargestellt. Anstelle von Z-OSu wird N-Boc-(Piperidinyl-4-carbonsäure)-OSu eingesetzt, das aus kommerziell erhältlicher Piperidinyl-4-carbonsäure nach dem Fachmann bekannten Methoden dargestellt werden kann. L-1-Cyclopentylmethyl-2-oxo-2-(1H-tetrazol-5-yl)-ethylamid (4.2.2) kann ausgehend von N^{α}-tert.Butyloxycarbonyl-β-Cyclopentylalanin (Bachem) nach literaturbekannten Methoden (Johanson A. et al., Bioorg. & Med. Chem., 11, 2003, 2551-68) dargestellt werden Nach Abspaltung der Boc-Schutzgruppe erhält man mit HCl / Diehtyhlether-Lösung das Hydrochlorid des Amins 4.2.2.

Der Aufbau der Titelverbindung erfolgt zunächst an fester Phase:
0,5 g H-Pro-2ClTrt-resin (Fluka, 0,45 mmol) werden nach dem Fachmann bekannten und unter 4.1 skizzierten Methoden mit 455 mg Fmoc-Phe-OH (1,35 mmol) und 424 mg TBTU (1,32 mmol), 182 mg HOBt (1,35 mmol) und 483 µl DIPEA (2,7 mmol) gekoppelt. Nach Kaiser-Test und Auswaschen der Reaktionslösung mit DMF, wird die Fmoc-Schutzgruppe mit 20% Piperidin in DMF abgespalten und anschließend 342 mg (0,9 mmol) der pharmakophor-tragenden Aminosäure 4.2.1 mit 283 mg TBTU (0,88 mmol) und 121 mg HOBt (0,9 mmol) gekoppelt. Das Zwischenprodukt N^{α}-(Piperidinyl-4-carbonyl)-{[(E)-(L)-2-Amino-6-phenylsulfonyl]-hex-5-enyl}-L-Phenylalaninyl-L-Prolin (4.2.3) wird vom polymeren Träger abgespalten, indem dieser für 30 Minuten mit einer Lösung von 1 % TFA in Dichlormethan behandelt wird. Das Filtrat wird eingeengt und der erhaltene Rückstand wiederholt mit Methanol codestilliert (Ausbeute: 134 mg).

Eine Lösung von 72 mg 4.2.3 (0,12 mmol) in 5 ml DMF wird nacheinander mit 38 mg TBTU (0,12 mmol), 16mg HOBt (0,12 mmol) und 62 µl DIPEA (0,36 mmol) versetzt und sofort zu einer Lösung von 29,3 mg des oben dargestellten Hydrochlorides der Verbindung 4.2.2 in 2 ml DMF zugesetzt. Nach einer Stunde rühren bei Raumtemperatur, wird das Lösungsmittel i. Vak entfernt. Vom erhaltenen Rückstand wird durch Behandlung mit TFA / DCM (1/1) die Boc-Schutzgruppe abgespalten und die Titelverbindung durch präp. HPLC-Chromatographie gereinigt. (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 5% B auf 100% B, 1 %/min).
Ausbeute: 68 mg
ESI-MS: 816,3 {M+H}⁺

### N^{α}-Benzyloxycarbonyl-[(E)-(L)-2-Amino-6-benzyloxysulfonyl-hex-5-enyl]-L-Valinyl-L-Prolinylbenzylsulfonamid (Verbindung 4.3)

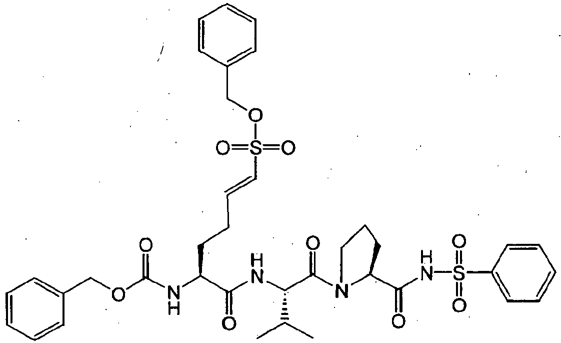

290 mg der Verbindung 4.3.1 (1 mmol, dargestellt nach literaturbekannten Methoden (Johanson A. et al., Bioorg. & Med. Chem., 11, 2003, 2551-2568, siehe Schema 10b)), wird mit Boc-Valin nach der literaturbekannten und oben beschriebenen Methode unter Zuhilfenahme von TBTU, HOBt und DIPEA in DMF gekoppelt. Zur Reinigung des Produktes wird der erhaltene Rückstand in Ethylacetat gelöst und nacheinander mit 10%iger Citronensäure, ges. NaHCO₃-Lösung und ges. NaCl-Lösung gewaschen und anschließend über Na₂SO₄ getrocknet. (Ausbeute: 342 mg). Von 100 mg des Zwischenproduktes(0,22 mmol) wird die Boc-Gruppe abgespalten und das erhaltene Amin wie oben beschrieben mit 98 mg (0,22 mmol) der pharmakophortragende Aminosäure 3.14 gekoppelt.

Das Lösungsmittel wird i. Vak. entfernt und die Titelverbindung durch präp. HPLC gereinigt (Synergie Max, 4µm, 250x21,2 mm, A-Eluent: 0,1%TFA / Wasser, B-Eluent: 90% AcCN / 10% Wasser/0,1%TFA. Gradient: 8 ml/min, 5% B auf 100% B, 1%/min).
Ausbeute: 100 mg
ESI-MS: 791,3 {M+Na}⁺

### 5. Umwandlung erfindungsgemäßer Inhibitoren in Peptidomimetika

Peptidische Wirkstoffe unterliegen in vivo oft einem schnellen enzymatischen Abbau, oder anderen Metabolisierungsreaktionen. Die dabei entsehenden Abbauprodukte weisen meist eine geringere inhibitorische Aktivität und oder eine geringere Selektivität gegenüber dem Target auf. Dieser Abbau findet überwiegend am Peptidbackbone selbst statt, da für die Hydrolyse peptidischer Bindungen *in vivo* zahlreiche Enzyme existieren. Um diesem Prozess entgegen zu wirken, d.h. um den *in vivo*-Abbau der dargestellten peptischen Inhibitoren zu verringern, werden in der Regel peptidähnliche bzw. bioisostere Aminosäurebausteine dargestellt. Unter einem Isoster oder Bioisoster versteht man *"Gruppen oder Moleküle die chemische und physikalische Eigenschaften aufweisen, die ähnliche biologische Effekte auslösen"* (siehe Thornber C.W., Chem. Soc. Rev., 8, 563-580). Grundsätzlich kann jede Gruppe in einem potenten peptidischen Inhibitor ausgetauscht werden, wenn eine *in vivo* stabilere und bioisostere Gruppe gefunden werden kann.

Im Folgenden werden deshalb einige nicht beschränkende Beispiele für Peptidomimetika und die Synthese einiger häufig zum Einsatz kommender bioisosterer Gruppen beschrieben.

### Ersatz einer Amidbindung durch ein sekundäres Amin

### (E)-(S)-6-[(S)-1-((S)-2-Ethylcarbamoyl-pyrrolidin-1-carbonyl)-2-methyl-propylcarbamoyl]-6-(2-piperidin-4-yl-ethylamino)-hex-2-ensäureisopropylester (Verbindung 5.1)

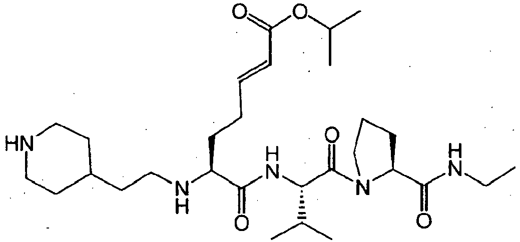

Reaktionsübersicht: siehe Schema 12

### Detaillierte Beschreibung der Synthese:

Die beiden Teilmoleküle 5.1.5 und 5.1.6 werden unabhängig voneinander aufgebaut.

Die Synthese des Amins 5.1.6 erfolgt, ausgehend von einer 70%igen wässrigen Lösung von Ethylamin in Wasser und Boc-Pro-OH, wie für Verbindung 5.a beschrieben.

Das Teilmolekül 5.1.5 wird wie folgt dargestellt.

Zu einer Lösung von 1 g N-Boc-4-piperidinacetaldehyd (Aldrich, 4,4 mmol) in 30 ml Dichlormethan_{abs} werden 1,92 g H-Glu(OMe)-OtBu (Bachem, 8,8 mmol) sowie 4 g Molsieb (4Å) zugesetzt. Eine Lösung aus 467 µl Essigsäure (8,8 mmol) und 2,84 g Natriumtrisacetoxyborhydrid (13,2 mmol), Aldrich) in Dichlormethan_{abs} wird zugetropft und die Reaktionslösung bei Raumtemperatur unter Schutzgas für 36 Stunden gerührt. Nach dieser Zeit sind laut Dünnschichtchromatogramm keine Edukte mehr in der Reaktionsmischung vorhanden. Die leicht trübe Lösung wird über Kieselgur filtriert. Das Filtrat wird mit 100 ml Dichlormethan verdünnt und mit 40 ml 1 N NaOH gewaschen. Die organische Phase wird abgetrennt und die wässrige Phase noch dreimal mit je 20 ml Dichlormethan reextrahiert. Die vereinigten organischen Phasen werden über Na₂SO₄ getrocknet und das Lösungsmittel i. Vak entfernt. Das erhaltene Rohprodukt wird ohne weitere Reinigung in Acetonitril gelöst und unter der Annahme vollständiger Umsetzung mit Boc₂O und DMAP zu Verbindung 5.1.3 umgesetzt. Die Reaktionsführung erfolgt analog zum Vorgehen bei der Synthese zu 1.3. Die Reinigung erfolgt durch Chromatographie an Kieselgel (Säule:30x2,3 cm, Petrolether/Ethylacetat: 9/1, Ausbeute: 977 mg).

950 mg des Methylesters (1,8 mmol) werden in trockenem Diethylether gelöst und bei - 78°C, wie bei der Synthese von 1.4 beschrieben, zum Aldehyd 5.1.3 reduziert. Die Umsetzung ist vollständig und das Rohprodukt kann ohne weitere chromatographische Reinigung unter den Bedingungen der Wittig-Reaktion zum Michael-Akzeptor umgesetzt werden. Dafür wird das Rohprodukt des Aldehyds 5.1.3 in 20 ml Benzol gelöst und bei Raumtemperatur mit 651 mg (2-Propoxycarbonylmethylen)-triphenylphosphoran (1,8 mmol) versetzt. Bei dieser Temperatur wird über Nacht gerührt, bevor das Lösungsmittel i. Vak. entfernt wird. Die Reinigung der Verbindung erfolgt durch Chromatographie an Kieselgel (Säule:22x2,3 cm, Petrolether/Ethylacetat: 9/1, Ausbeute: 522 mg). Die Abspaltung aller drei Schutzgruppen erfolgt wie beschrieben durch Behandlung mit TFA / DCM (1/1) bei Raumtemperatur. Die nachfolgende Reinigung kann durch präparative HPLC erfolgen. Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1%/min). Man erhält 211 mg der freien Aminosäure 5.1.4.

200 mg 5.1.4 (0,55 mmol) werden in DMF bei Raumtemperatur gelöst und 248 mg Boc-OSu (1,15 mmol) zugesetzt. Mit DIPEA wird der pH-Wert auf ca. 8 bis 9 eingestellt und über Nacht bei Raumtemperatur gerührt. Nach Entfernen des Lösungsmittels i. Vak. und Chromatographie an Kieselgel (Säule:20x1,3 cm, Dichlormethan/Methanol: 95/5) erhält man 178 mg der gereinigten Verbindung 5.1.5.

### Darstellung von Beispiel 5.1

Die Kopplung der Carbonsäure 5.1.5 an das Amin 5.1.6 gelingt durch Verwendung von TBTU in Gegenwart von HOBt glatt. Dazu werden 50 mg der Carbonsäure (95 µmol) in DMF gelöst. Zu dieser Lösung werden nacheinander 29 mg TBTU, 13 mg HOBt und 40 µl DIPEA zugesetzt. Unmittelbar nach Zugabe der Base, wird die Lösung zu einer Lösung von 33 mg des Trifluoracetatsalzes von 5.1.6 (95 µmol) zugegeben. Der pH-Wert wird mit DIPEA auf ca. 10 eingestellt und nach einer Stunde rühren bei Raumtemperatur wird das Lösungsmittel i. Vak. entfernt. Der ölige Rückstand wird in 50 ml Ethylacetat aufgenommen und zweimal mit je 10 ml 10%iger wässriger Citronensäure sowie gesättigter NaHCO₃-Lösung gewaschen. Vom erhaltenen Produkt werden die beiden Boc-Schutzgruppen durch Behandlung mit TFA in Dichlormethan abgespalten. Die Titelverbindung 5.1 kann durch präparative HPLC in reiner Form erhalten werden (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1 %/min).
Ausbeute: 19 mg
ESI-MS: 550,4 {M+H}⁺

### Ersatz einer Amidbindung gegen eine Ketomethylen oder Hydroxymethylengruppe

Eine weitere Möglichkeit das Peptidrückgrat in vivo zu stabilisieren besteht darin, das Stickstoffatom der Amidbindung durch ein Kohlenstoffatom zu ersetzen. Die resultierenden Verbindungen werden *Hydroxymethylen-* oder *Ketomethylen-Isostere zur Amidbindung* genannt. Die Synthese erfolgt meist über Dipeptidanaloga, die separat dargestellt und anschließend in jeweilige die Molekülumgebung eingebaut werden. Derartige Mimetika und _Analoga sind auf den Seiten 17 bis 19 der Beschreibung ausführlich diskutiert. Überraschenderweise können erfindungsgemäße Inhibitoren in dieser Weise verändert werden, ohne an inhibitorischer Potenz zu verlieren.

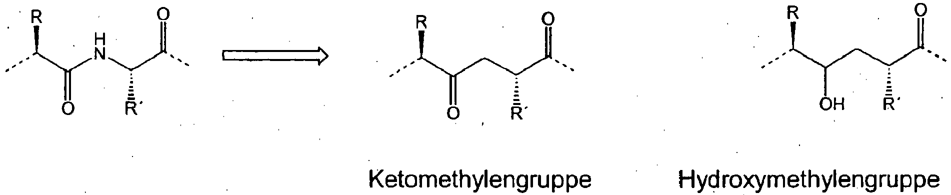

Beispielhaft aber nicht beschränkend für diese petidomimetischen Modifikationen erfindungsgemäßer Inhibtoren, ist die Synthese der Verbindungen 5.2 bis 5.4 in den Figuren 11 bis 13 gegeben. Die Darstellung der erfindungsgemäßen Inhibitoren 5.2 (Figur 13) und 5.3 (jeweils a und b) (Figur 14) erfolgt ausgehend von einem jeweils geeigneten Isoxazol als Dipeptidanalogon.

Potente Inhibitoren werden bevorzugt erhalten, wenn der Rest A ein sekundär substituiertes Atom, vorzugsweise ein Kohlenstoffatom darstellt, woran die olefinische Seitenkette gebunden ist. An dieses vorzugsweise sekundär substituierte Kohlenstoffatom ist vorzugsweise ein Stickstoffatom gebunden. Überraschenderweise wurde gefunden, dass potente Inhibitoren auch dann erhalten werden können, wenn dieses Stickstoffatom durch andere Atome, vorzugsweise durch ein Kohlenstoffatom, ersetzt wird, wie beipielsweise durch Formel (II) mit E=CH₂ dargestellt wird.

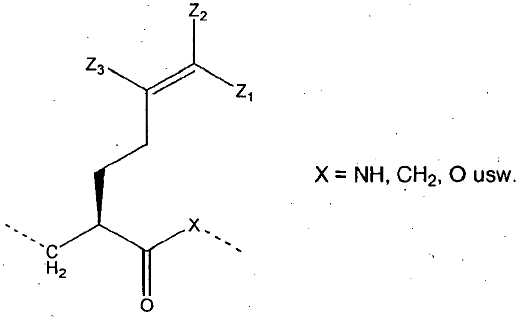

Eine Synthesemöglichkeit für peptidomimetische Inhibitoren, bei denen dieser Stickstoff ausgetauscht wurde, ist in Figur 15 beschrieben. Als nicht beschränkendes Ausführungsbeispiel ist Verbindung 5.4 gegeben.

Als konkrete Ausführungsbeispiele für die Darstellung von Hydroxymethylen- sowie Ketomethylenisosteren in verschiedenen Molekülpositionen erfindungsgemäßer Inhibitoren, sind die Synthesen der Verbindungen 5.3a & b und 5.4 gegeben.

### Allgemeine Synthesebeschreibung für Hydroxymethylen- und Ketomethylenbioisostere (Schemata 11 und 12):

Die Darstellung der Hydroxymethylen- oder Ketomethylenstrukturelemente kann beispielsweise über die von Haug *et al.* und Litera *et al.* beschriebene Verbindung 5.2.1 erfolgen. (Haug, B. E., et al.; Org. Lett., 2004, 6, 4783-4786, Litera, J.; et al.. Collet. Czech, Chem. Commun., 1998, 63, 231-244., siehe Schema 13).

Die beispielhafte Synthesesequenz in Schema 13 beginnt mit dem, in der Literatur beschriebenen, Lacton 5.2.1. Dieses Lacton wird vorzugsweise mit einem Amin, bevorzugt einer peptischen Aminosäure oder einem Peptid, unter Erwärmung in DMF zum Hydroxyethylen Analogon geöffnet. Die nachfolgende Cyclisierung zum Oxazolidinon erfolgt mit Natriumhydrid. Die Amidfunktion des Oxazolidinons wird, wie unter 1.3 beschriebenen, mit einer weiteren Boc-Gruppe geschützt. Die Freisetzung des primären Alkohols kann nach dem Fachmann bekannten Methoden mit Tetrabutylammoniumfluorid (TBAF), oder im Falle von Racemisierungsreaktionen auch durch Essigsäure in einem THF/Wasser Gemisch erfolgen. Die nachfolgende Oxidation zum Aldehyd wird durch die Variante nach Swern ermöglicht (Omura, K.; Swern, D. Tetrahedron, 1978, 34, 1651-1660.). Erfindungsgemäße Michael-Akzeptor-Systeme werden erhalten, indem ein geeigneter Aldehyd in einer Wittig-Reaktion oder einer Horner-Wadworth-Emmons Reaktion (JACS, 1961, 83, 1733) mit einem geeigneten Phosphonium-Ylid umgesetzt wird. Als nicht beschränkendes Beispiel wird analog zur Verbindung 1.5 im Schema 11 das (Ethoxycarbonylmethylen)-triphenylphosphoran verwendet. Die Öffnung des Oxazolidinons erfolgt' mit TFA und der freigesetzte β-Aminoalkohol (5.2.2) wird am Stickstoffatom derivatisiert.

Erfindungsgemäße Inhibitoren können daraus auch erhalten werden, indem Isocyanate mit der Aminofunktion zu Harnstoffderivaten umgesetzt werden (siehe Schema 11, Verbindung 5.2.a, Durchführungsvorschrift: z.B.: Davies, J.S., J. Chem. Soc. Perkin Trans 2, 1992, 1225-1231). Mit geeigneten Aktivestern (wie z.B. Z-OSu) lässt sich die freie Aminofunktion in 5.2.2 und analogen Verbindungen unter Ausbildung von Carbamaten oder, wie unter 3. beschrieben, mit Carbonsäure unter Bildung einer Amidbindung derivatisieren. Nicht beschränkende Beispiele für die Synthese von Ketomethylen-Analoga können aus den Hydroxymethylenderivaten durch eine nachfolgende Oxidation nach der Methode von Dess-Martin (Dess, D. B; Martin, J. C. JOC, 1983, 48, 4155) dargestellt werden.

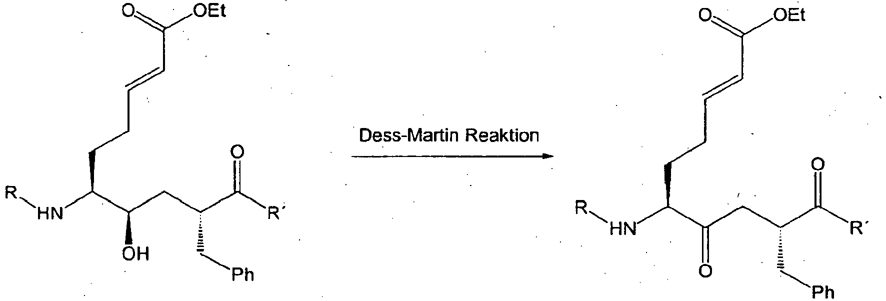

### Allgemeine Synthesebeschreibung für Hydroxymethylen- und Ketomethylenbioisostere (Figur 15):

Die Darstellung der erfindungsgemäßen Inhibitoren mit E=CH₂ erfolgte über den von Ghosh *et al.* und Brady *et al.* beschriebene Verbindung 5.4.1 (Ghosh, A. K; JACS, 2000, 122, 3522; Brady, S. F., Bioorg & Med. Chem. Lett., 2004, 14, 601). Als nicht beschränkendes Beispiel ist in Figur 15 eine Synthesesequenz aufgezeigt.

### Allgemeine Synthesebeschreibung (siehe Figur 15)

Die Synthesesequenz beginnt mit dem in der Literatur beschriebenen Lacton 5.4.1. (Ghosh, A. JACS, 2000, 122, 3522. Brady; S. F. Bioorg & Med. Chem. Lett., 2004, 14, 601). Dieses Lacton wird mit LiOH zum Hydroxyethylen Analogon geöffnet. Die nachfolgende Derivatisierung der C-terminalen Carboxlyfunktion wurde vorzugsweise mit Aminen durchgeführt. Die nachfolgende Oxidation der sekundären Hydroxyfunktion als auch des primären Alkohols wird durch die Variante nach Swern ermöglicht. Michael-Akzeptor-Systeme werden wiederum erhalten, indem ein geeigneter Aldehyd in einer Wittig-Reaktion oder einer Horner-Wadworth-Emmons Reaktion mit einem geeigneten Phosphonium-Ylid umgesetzt wird. Nach Spaltung der Boc-Gruppe am N-Terminus können an diesem Stickstoff die gleichen Folgereaktionen durchgeführt werden, die hierin an anderer Stelle beschrieben sind.

### Ausführungsbeispiele

### (S)-2-[((S)-1-{(E)-2R,5S)-2-(4-Fluorobenzyl)-9-methansulfonyl-5-[(5-methyl-isoxazol-3-carbonyl)-amino]-4-oxo-non-8-enoyl}-pyrrolidin-2-carbonyl)-amino]-4-methyl-pentansäuremethylester (Verbindung 5.3.b)

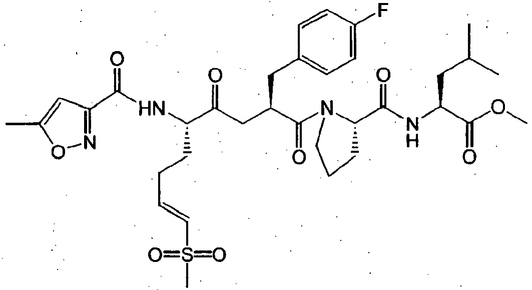

750 mg [(1*S*)-1-[(4*R*)-4-(4-Fluoro)-benzyl-5-oxo-tetrahydro-furan-(2*R*)-2-yl]-4-(*tert*-butyl-dimethylsilanyloxy)-butyl]-carbamidsäure-tert-butylester (5.3.1, siehe Schema 14 Hergestellt nach Haug et al., Org. Lett., 2004, 6, 4783-4786, 1,51 mmol) werden in 20 ml THF gelöst und 759 mg H-Pro-Leu-OMe (3,14 mmol) zugesetzt. Die Reaktionsmischung wird durch DIPEA-Zusatz auf pH-11 eingestellt und für vier Stunden auf 40°C erwärmt. Das Lösungsmittel wird i. Vak. entfernt und der erhaltene ölige Rückstand in 200 ml Ethylacetat aufgenommen. Die organische Phase wird mit NaHCO₃-Lösung und ges. NaCl-Lösung gewaschen und über Na₂SO₄ getrocknet. Man erhält 1,0 g Rohprodukt (5.3.2), das für die weitere Umsetzung ohne zusätzliche Reinigung verwendet werden kann.

Zu einer Lösung von 1 g 5.3.2 (1,36 mmol) in 8 ml DMF_{abs} werden unter Rühren 271 mg NaH (60% auf Mineralöl) zugesetzt. Nach drei Stunden rühren bei Raumtemperatur, werden 5 ml gesättigte NaCl-Lösung zugesetzt und die Mischung dreimal mit je 20 ml Ethylacetat extrahiert. Die vereinigten organischen Phasen werden über Na₂SO₄ getrocknet und das Lösungsmittel i. Vak entfernt. Das Produkt (5.3.3) wird durch Chromatographie an Kieselgel gereinigt (Säule:21x2,3 cm, Dichlormethan/Methanol: 95/5, Ausbeute: 685 mg).

670 mg 5.3.3 (1 mmol), werden in Acetonitril gelöst und der Stickstoff wie für Verbindung 1.3 beschrieben durch die tert.-Butyloxycarbonylgruppe geschützt. Die Reinigung des Produktes (5.3.4) erfolgt wiederum durch Chromatographie an Kieselgel (Säule:26x2,3 cm, Dichlormethan/Methanol: 99/1, Ausbeute: 596 mg).

590 mg des Silylethers 5.3.4 (0,77 mmol), werden in 25 ml THF gelöst und bei Raumtemperatur 10 Milliliter einer Lösung von Essigsäure in THF und Wasser (10/2/6) zugesetzt. Nach einer Stunde Rühren bei Raumtemperatur, wird das Lösungsmittel i. Vak. entfernt und der erhaltene feste Rückstand durch Chromatographie an Kieselgel gereinigt (Säule:21x1,2 cm, Dichlormethan/Methanol: 9/1, Ausbeute: 465 mg). Der so erhaltene primäre Alkohol wird in einer Swern-Oxidation zum Aldehyd 5.3.5 oxidiert. Dazu werden 100 mg Oxalylchlorid (0,786 mmol) in 5 ml Dichlormethan gelöst. Unter Stickstoffatmosphäre wird diese Lösung auf -60°C gekühlt, bevor eine Lösung von 132 mg DMSO (1,7 mmol) in 2 ml Dichlormethan langsam zugetropft wird. Es wird für zehn Minuten bei -60°C weiter gerührt und anschließend eine Lösung von 460 mg (0,715 mmol) des oben dargestellten Alkohols in 2 ml Dichlormethan zugesetzt. Nach weiteren 15 Minuten Rühren bei dieser Temperatur werden 3,5 g Triethylamin (35 mmol) zugesetzt. Es wird für fünf Minuten bei -60°C gerührt, anschließend das Kältebad entfernt. Die Lösung taut dabei innerhalb von 30 Minuten auf ca. 0°C auf. Bei dieser Temperatur werden 10 ml Wasser zugesetzt und der Ansatz für zehn Minuten stark gerührt. Die organische Phase wird abgetrennt und die wässrige Phase noch zweimal mit je 10 ml Dichlormethan gewaschen. Die vereinigten organischen Phasen werden über Na₂SO₄ getrocknet und i. Vak. zur Trockne eingeengt. Der erhaltene Aldehyd 5.3.5 kann ohne weitere Reinigung in einer Horner-Wadsworth-Emmons-Reaktion wie sie für Verbindung 3.11 beschrieben ist, zum vinylogen Sulfon 5.3.6 umgesetzt werden. Die Reinigung erfolgt durch präparative HPLC-Chromatographie. (Synergie Max, 4µm, 250x21,2 mm, A-Eluent: 0,1%TFA-/ Wasser, B-Eluent: 90% AcCN / 10% Wasser / 0,1%TFA. Gradient: 8 ml/min, 30% B auf 100% B, 1%/min). Ausbeute (5.3.6): 419 mg.

Die Behandlung von 400 mg des Oxazolidinons 5.3.6 (0,55 mmol) mit 15 ml einer Lösung von TFA/DCM (1/1) liefert den Aminoalkohol 5.3.7 als TFA-Salz in quantitativer Ausbeute (400 mg). Dieser ist ohne weitere Reinigung verwendbar.

8,7 mg 5-Methyl-Isoxazol-4-carbonsäure (69 µM, Darstellung: Street et al., J. Med. Chem., 2004, 3642-3657) werden 2 ml DMF gelöst und mit 26 mg HATU (138 mmol) sowie 23 µl DIPEA (140 µmol) versetzt. Diese Lösung wird sofort zu einer Lösung von 50 mg des Aminoalkohols 5.3.7 (69 µmol) in 3 ml DMF zugesetzt. Nach 30 Minuten rühren bei Raumtemperatur ist die Reaktion beendet. Das Lösungsmittel wird i. Vak. entfernt und das Produkt durch präparative HPLC gereinigt. (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 5% B auf 100% B, 1 %/min).
Ausbeute (5.3.a): 38 mg.
ESI-MS: 729,4 {M+Na}⁺

19 mg 5.3.a (27 µmol) werden in 2 ml Dichlormethan gelöst und zu einer Lösung von 12,5 mg 1,1,1-Tris(acetyloxy)-1,1-dihydro-1,2-benziodoxol-3-(1H)-one (Dess-Martin Periodan, 29,7 µmol) in Dichlormethan zugesetzt. Bei Raumtemperatur wird für 20 Minuten bei Raumtemperatur gerührt. Das Lösungsmittel wird i. Vak. entfernt und die Titelverbindung durch präparative HPLC isoliert. (Synergie Max, 4µm, 250x21,2 mm, A-Eluent: 0,1%TFA /Wasser, B-Eluent: 90% AcCN / 10% Wasser / 0,1%TFA. Gradient: 8 ml/min, 30% B auf 100% B, 1%/min).
Ausbeute (5.3.b): 11 mg
ESI-MS: 727,4

### (E)-(6R,9S)-9-Benzyloxycarbonylamino-6-[2-(2-ethylcarbamoyl-octahydroindol-1-yl)-1-methyl-2-oxoethylcarbamoyl]-8-oxo-10-phenyl-dec-2-enylsäure-isopropylester (Verbindung 5.4)

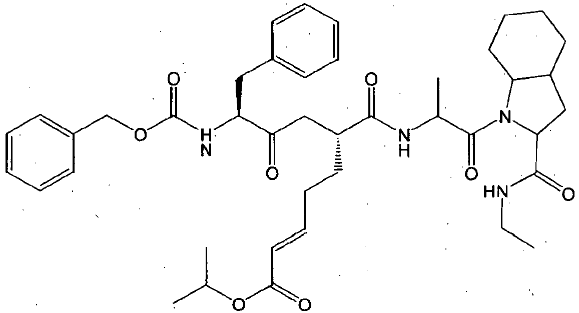

Die Synthese geht von dem literaturbekannten Lacton 5.4.1 (Ghosh, A. JACS, 2000, 122, 3522. Brady, S. F. Bioorg & Med. Chem. Lett., 2004, 14, 601) aus. Dieses wurde wie dort beschrieben dargestellt. 363 mg 5.4.1 (1 mmol) werden in 5 ml Methanol gelöst und bei Raumtemperatur mit 1,1 ml 1N LiOH-Lösung über Nacht gerührt. Anschließend wird der pH-Wert mit 1N HCl auf 3-4 gebracht und das Lösungsmittel i. Vak. entfernt. Der erhaltene ölige Rückstand wird in 100 ml Dichlormethan aufgenommen und zweimal mit 10%iger Citronensäure und zweimal mit ges. NaCl-Lösung gewaschen. Nach Trocknen der organischen Phase über Na₂SO₄ und entfernen des Lösungsmittels i. Vak. erhält man 350 mg der Carbonsäure 5.4.2 in reiner Form.

335 mg der Carbonsäure 5.4.2 (0,87 mmol) werden in 5 ml DMF gelöst und nacheinander mit 276 mg TBTU (0,86 mmol), 118 mg HOBt und 451 µl DIPEA versetzt und diese Lösung sofort im Anschluss zu einer Lösung von 334 mg des Trifluoracetates des Amins H-Ala-Oic-NHEt (0,87 mmol) zugesetzt. Nach einer Stunde rühren bei Raumtemperatur, wird das Lösungsmittel i. Vak entfernt und der erhaltene feste Rückstand durch präparative HPLC gereinigt (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 30% B auf 100% B, 1%/min). Man erhält 409 mg des Diols 5.4.3.

118 µl Oxalylchlorid (1,38 mmol) werden in 10 ml Dichlormethan gelöst. Unter Stickstoffatmosphäre wird diese Lösung auf -60°C gekühlt, bevor eine Lösung von 212 µl DMSO (3 mmol) in 5 ml Dichlormethan langsam zugetropft wird. Es wird für zehn Minuten bei -60°C weiter gerührt und anschließend eine Lösung von 398 mg des oben dargestellten Diols 5.4.3 (0,63 mmol) in 2 ml Dichlormethan zugesetzt. Nach weiteren 15 Minuten Rühren bei dieser Temperatur werden 6,2 g Triethylamin (60 mmol) zugesetzt. Es wird für fünf Minuten bei -60°C gerührt, anschließend das Kältebad entfernt. Die Lösung taut dabei innerhalb von 30 Minuten auf ca. 0°C auf. Bei dieser Temperatur werden 10 ml Wasser zugesetzt und der Ansatz für zehn Minuten stark gerührt. Die organische Phase wird abgetrennt und die wässrige Phase noch zweimal mit je 10 ml Dichlormethan gewaschen. Die vereinigten organischen Phasen werden über Na₂SO₄ getrocknet und i. Vak. zur Trockne eingeengt. Der erhaltene Aldehyd 5.4.4 ist laut HPLC und DC rein und wird ohne weitere Reinigung weiter umgesetzt (Ausbeute: 318 mg).

300 mg Aldehyd 5.4.4 (0,48 mmol) werden in 15 ml Benzol gelöst und analog der Vorschrift zur Darstellung der Verbindung 3.3 zum Olefin 5.4.5 umgesetzt. Die Reinigung erfolgt durch Chromatographie an Kieselgel ((Säule:20x2,3 cm, Dichlormethan/Methanol: 98/2, Ausbeute: 227 mg). 50 mg des Olefins 5.4.5 (70 µmol) werden in 3 ml Dichlormethan gelöst und mit 1,5 ml Trifluoressigsäure versetzt. Nach 45 min Rühren bei Raumtemperatur wird die Lösung mit 5 ml Methanol versetzt und das Reaktionsgemisch i. Vak. zur Trockne eingeengt. Der erhaltene Rückstand wird in 5 ml DMF gelöst, mit 17,4 mg N-(Benzyloxycarbonyloxy)-succinimid (Z-OSu, 70 µmol, Fluka) versetzt und die Lösung durch Zusatz von DIPEA auf pH~8-9 eingestellt. Bei Raumtemperatur wird für zwei Stunden gerührt und anschließend das Lösungsmittel i. Vak. entfernt. Durch Reinigung mittels präparative HPLC (Synergie Max, 4µm, 250x21,2 mm, A-Eluent: 0,1%TFA / Wasser, B-Eluent: 90% AcCN / 10% Wasser /0,1%TFA. Gradient: 8 ml/min, 30% B auf 100% B, 1%/min) erhält man die Titelverbindung in reiner Form.
Ausbeute: 30 mg
ESI-MS: 767,4 {M+Na}⁺

### Ersatz einer Amidbindung gegen eine Hydroxyethylaminogruppe

Die oben dargestellten Peptidomimetika sind dadurch gekennzeichnet, dass jeweils ein Atom der Amidbindung entfernt oder ausgetauscht wird, und so eine neue chemische Struktur geschaffen wird, die keinem, oder zumindest einem wesentlich langsameren Metabolismus unterliegt als die ursprüngliche Peptidbindung. Diese Strategie nur möglichst geringe Unterschiede zwischen einer chemischen Struktur und einer bioisosteren Struktur zuzulassen, sichert den Erhalt einer Verbindung, die weiterhin einen potenten erfindungsgemäßen Inhibitor darstellt. Überraschenderweise wurde festgestellt, dass auch weitergehende strukturelle Veränderungen weiterhin potente Inhibitoren der Transglutaminasen ergeben. Ein nicht-beschränkendes Beispiel für eine solche Substitution ist die Hydroxyethylaminogruppe.

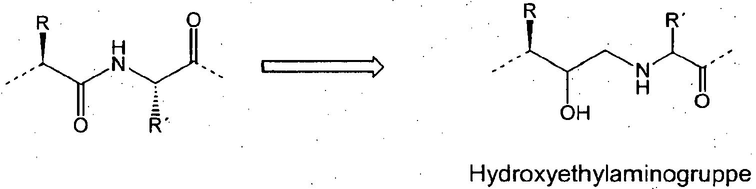

### Allgemeine Synthesebeschreibung für Hydroxyethylamino-Bioisostere

Ein nicht beschränkendes Beispiel für die Synthese von Hydroxyethalamino-Isosteren wird in Figur 16 beschrieben. Als Ausführungsbeispiel ist die Synthese der Verbindungen 5.6 (siehe auch Figur 17) gegeben.

Zur Darstellung von Hydroxyethylamino-Analoga erfindungsgemäßer Inhibitoren werden geeignet substituierte Epoxide wie z.B. Verbindung 5.5.1 (Aldrich) vorzugsweise mit Aminen umgesetzt. Bevorzugt sind diese Amine C-terminal geschützte Aminosäuren oder besonders bevorzugt Peptide oder Peptidomimetika. Insbesondere kann das verwendete Amin bereits die erfindungsgemäße pharmakophore Gruppe, d.h. die in Konjugation mit einer elektronenwegziehenden Gruppe stehende Doppelbindung beinhalten. Die sekundäre Hydroxylgruppe im resultierenden Hydroxylethylamino-Analogon kann durch die Dess-Martin-Reaktion oder ähnliche Reaktionen zum Keton oxidiert werden. Falls dies im weiteren Syntheseverlauf notwendig wird, kann auf dieser Stufe die neu gebildete sekundäre Aminofunktion orthogonal zur Boc-Schutzgruppe geschützt werden. Nach Abspaltung der Boc-Schutzgruppe erhält man Amine vom Typ 5.5.2. Befindet sich die erfindungsgemäße pharmakophore Gruppe nicht in unmittelbarer Umgebung zu dem Hydroxyethylamino-Bioisoster, so kann der Pharmakophor wie oben beschrieben, in Form des Amines welches zur Epoxid-Öffnung verwendet wird, oder an der primären Aminofunktion wie in Schema 16 beispielhaft aber nicht beschränkend dargestellt, eingebracht werden. Die pharmakophor-tragende Seitenkette erfindungsgemäßer Inhibitoren kann aber an jeder Position des Moleküls stehen. In Schema 17 ist die Synthese des Ausführungsbeispieles 5.6 gegeben. In diesem tritt ein Hydroxyethylamino-Bioisoster in unmittelbarer Umgebung der erfindungsgemäßen pharmakophoren Gruppe auf.

### Piperidin-4-carbonyl-((E)-(S)-5-benzylsulfonyl-1-{2-[2-((S)-2-benzylsulfonylaminocarbonyl-octahydro-indol-1yl)-2-oxo-ethylamino]-acetyl}-4-enyl)-amid (Verbindung 5.6)

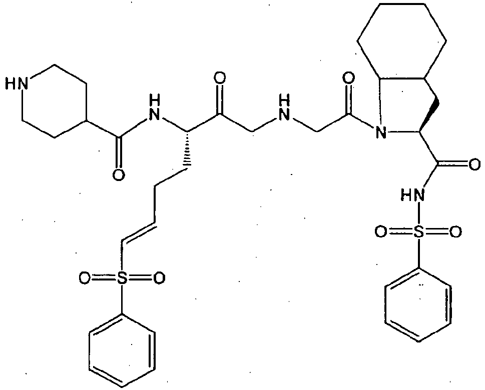

Das benötigte Epoxid 5.6.1 (siehe Schema 17) wird wie in der Literatur beschrieben dargestellt (Pico A. et al., J. Org. Chem., 2003, 68,. 5075-5083). Die Aminkomponente (N[(S)-1-(2-Amino-acetyl)octahydro-indol-2-carbonyl]-benzylsulfonamid), wird analog zum Vorgehen bei der Synthese der Verbindung 4.3.1 und der bereits in der Literatur beschriebenen Syntheseroute (Johanson A. et al., Bioorg. & Med. Chem., 11, 2003, 2551-2568) dargestellt.

980 mg 5.6.1 (2,83 mmol) werden in 20 ml Methanol gelöst und nacheinander 1,03 g N[(S)-1-(2-Amino-acetyl)octahydro-indol-2-carbonyl]-benzylsulfonamid (2,83 mmol) sowie 100 mg Triethylamin (1 mmol) zugesetzt. Die Lösung wird für fünf Stunden am Rückfluss gekocht. Das Lösungsmittel wird i. Vak. entfernt und der erhaltene feste Rückstand durch Chromatographie an Kieselgel gereinigt (Säule:32x3,5 cm, Dichlormethan/Methanol: 95/5) Man erhält 1,12 g der Verbindung 5.6.2 in reiner Form als weißen Feststoff. 1,1 g der Verbindung 5.6.2 (1,54 mmol) werden in THF gelöst und bei Raumtemperatur 2 ml einer 1M Lösung von Tetrabutylammoniumfluorid (TBAF) in THF zugesetzt. Nach 30 Minuten rühren bei dieser Temperatur, wird das Lösungsmittel i. Vak. entfernt und das Produkt (5.6.3) durch Chromatographie an Kieselgel gereinigt (Säule:26x2,8 cm, Dichlormethan/Methanol: 90%1, Ausbeute: 781 mg).

760 mg des Diols 5.6.3 (1,27 mmol) werden in 15 ml Dichlormethan gelöst und eine Lösung von 1,18 g 1,1,1-Tris(acetyloxy)-1,1-dihydro-1,2-benziodoxol-3-(1*H*)-one (Dess-Martin Periodan, 2,79 mmol) in Dichlormethan zugesetzt. Bei Raumtemperatur wird für 30 Minuten bei Raumtemperatur gerührt. Das Lösungsmittel wird i. Vak. entfernt und das Zwischenprodukt 5.6.4 durch Chromatographie an Kieselgel gereinigt (Säule:30x2,8 cm, Dichlormethan/Methanol: 98/2, Ausbeute: 474 mg).

257 mg (EtO)₂P(=O)SO₂Phe (0,85 mmol) werden in 10 ml DMF_{abs} gelöst und unter Stickstoffatmosphäre bei Raumtemperatur 34 mg NaH (60%ig auf Mineralöl) zugesetzt.

Nach beendeter Gasentwicklung wird eine Lösung von 460 mg 5.6.4 (0,77 mmol) in 10 ml DMF_{abs} zugesetzt und bei Raumtemperatur über Nacht gerührt. Das Lösungsmittel wird i. Vak entfernt. Die Reinigung des Produktes erfolgt durch präparative HPLC (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 25% B auf 100% B, 1%/min). Ausbeute: 498 mg.

Die Abspaltung der Boc-Schutzgruppe durch Behandlung 10 ml einer Lösung aus TFA/DCM = 1/1 gelingt glatt und das erhaltene primäre Amin (5.6.5) kann ohne weitere Reinigung verwendet werden. Das Rohprodukt der Verbindung 5.6.5 wird in 10 ml DMF gelöst und mit 195 mg N-Boc-Piperidin-4-carbonsäure-N-Hydroxysuccinimid (0,6 mmol) versetzt. Durch Zusatz von DIPEA wird der pH-Wert auf ca. 8 bis 9 eingestellt. Nach einer Stunde Rühren bei Raumtemperatur ist die Reaktion beendet. Das Lösungsmittel wird i. Vak. entfernt und der erhaltene ölige Rückstand ohne weitere Aufarbeitung direkt in 10 ml einer Lösung aus TFA/DCM = 1/1 gelöst. Nach dreißig Minuten Rühren bei Raumtemperatur ist die Reaktion beendet. Nach Entfernen des Lösungsmittels kann die Titelverbindung durch präparative HPLC gereinigt werden.
Ausbeute: 402 mg
ESI-MS: 742,3 {M+H}⁺

Eine bevorzugte Form der akzeptorsubstituierten Doppelbindung ist ein Michael-System aus einer Carbonylfunktion in Konjugation mit einer Doppelbindung, welches die folgende allgemeine Struktur [G] hat:

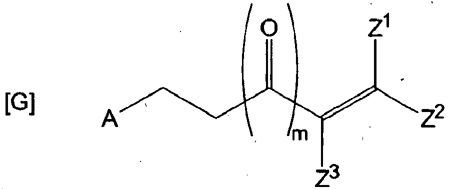

In einer Ausführungsform ist das Atom mit dem A an den erfindungsgemäßen Pharmakophor gebunden ist, ein Stickstoffatom. An dieses vorzugsweise tertiär substituierte Stickstoffatom ist vorzugsweise eine Carbonylgruppe (C=O) gebunden.

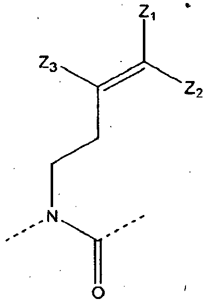

Überraschenderweise wurde gefunden, dass auch derart substituierte, erfindungsgemäße Verbindungen, Transglutaminasen potent inhibieren.

### Allgemeine Synthesebeschreibung:

Als nicht beschränkendes Beispiel ist in Figur 18 eine Synthesesequenz aufgezeigt.

Die Darstellung der erfindungsgemäßen Inhibitoren gelingt ausgehend von der von Hill *et al..* publizierten Verbindung 5.7.1. (Hill, R. D.; Vederas, J. C., JOC, 1999, 64, 9538-9546). Durch Reaktion mit Bis-(Pentafluorphenyl)carbonat in Gegenwart von Pyridin, wird der Pentaflourphenylester 5.7.2 gebildet, der mit Alkoholaten zu unterschiedlich geschützten Estern wie beispielsweise 5.7.3 reagiert. Bevor ein Aldehyd wie beispielsweise 5.7.6 dargestellt werden kann, beispielsweise über einen Alkohol wie 5.7.5, muss der zweite Hydrazinstickstoff, wie hierin beschrieben, geschützt werden. Beispielhaft kann diese Schutzgruppe durch Reaktion mit Di-tert.-Butylbicarbonat in Gegenwart von DMAP eingeführt werden. Nach erfolgreicher Reduktion des Dimethylamides 5.7.4 zum Alkohol 5.7.5 kann dieser, beispielsweise durch eine Swern-Oxidation, zum Aldehyd 5.7.6 oxidiert werden. Die weitere Synthese erfindungsgemäßer Inhibitoren kann ausgehend von 5.7.6 nach den hierin beschriebenen Methoden erfolgen (siehe z.B. Verbindung 3.11). Ein Ausführungsbeispiel ist mit Verbindung 5.7 gegeben.

### (E)-5-(N'-Acetyl-N-carboxy-hydrazino)-[(pent-2-enoyl)-1-ethanoly]-LValinyl-L-Prolinyl-L-Leucinmethylester (Verbindung 5.7

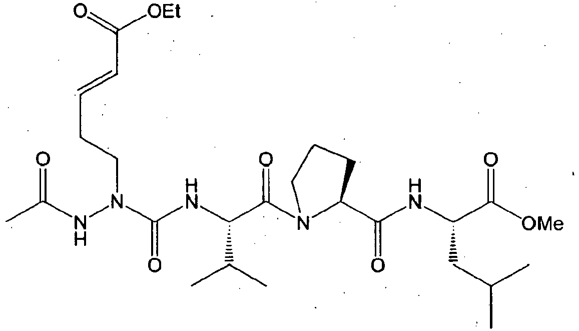

693 mg des Hydrazids 5.7.1 (4 mmol, Schema 18; Hill, R. D.; Vederas, J. C. JOC, 1999, 64, 9538-95469 werden in 20 ml THF_{abs} gelöst und unter Argonatmosphäre 326 µl Pyridin (4 mmol) zugesetzt. Nach zwei Minuten werden 1,43 g Bis-(Pentafluorphenyl)carbonat (3,62 mmol, Fluka) zugesetzt und die Lösung über Nacht bei Raumtemperatur gerührt. Das Lösungsmittel wurde i. Vak. entfernt und der Rückstand durch Chromatographie an Kieselgel gereinigt (Säule:39x2,8 cm, Dichlormethan/Methanol: 95/5, Ausbeute: 1,24 g).

1,22 g des Pentaflourphenylesters 5.7.2 (3,18 mmol) werden erneut in 35 ml THF_{abs} gelöst. Zu dieser Lösung werden 540 mg Kalium-2-Phenyl-2-propanolat (3,1 mmol) zugesetzt. Bei Raumtemperatur wird für zwei Stunden gerührt bevor das Lösungsmittel i. Vak. entfernt. Der erhaltene feste Rückstand wird in 200 ml Ethylacetat aufgenommen und mit 10%iger Na₂CO₃-Lösung sowie gesättigter NaCl-Lösung gewaschen. Die organische Phase wird über Na₂SO₄ getrocknet. Nach Entfernen des Lösungsmittels i. Vak erhält man 628 mg des 2-Phenyl-2-propanolesters 5.7.3. Das Rohprodukt ist ohne weitere Reinigung für die nachfolgende Synthese verwendbar. 628 mg 5.7.3 (1,87 mmol) werden in 20 ml Acetonitril gelöst und mit 817 mg Di-tert.-Butylbicarbonat (3,74 mmol) und 46 mg DMAP ((0,37 mmol) zugesetzt. Bei Raumtemperatur wird über Nacht gerührt und anschließend das Lösungsmittel i. Vak. entfernt. Die anschließende Reinigung von Verbindung 5.4.1 erfolgt durch Chromatographie an Kieselgel (Säule:32x2,2 cm, Dichlormethan/Methanol: 99/1, Ausbeute: 521 mg).

510 mg 5.4.1 (1,17 mmol), werden in 20 ml Methanol gelöst und bei 0°C 110 mg Natriumborhydrid (2,95 mmol) zugesetzt. Bei dieser Temperatur wird über Nacht gerührt. Die Reaktionslösung wird anschließend mit 56 ml einer 1%igen Citronensäure-Lösung versetzt und auf Raumtemperatur aufgetaut. Die wässrige Phase wird fünf Mal mit je 100 ml Diethylether extrahiert und die vereinigten organischen Phasen über Na₂SO₄ getrocknet. Man erhält 388 mg des Alkohols 5.7.5, der für die Folgereaktion (Swern-Oxidation zum Aldehyd 5.7.6) hinreichend rein ist. Dazu werden 137 mg Oxalylchlorid (1,07 mmol) in 5 ml Dichlormethan gelöst. Unter Stickstoffatmosphäre wird diese Lösung auf -60°C gekühlt, bevor eine Lösung von 180 mg DMSO (2,33 mmol) in 2 ml Dichlormethan langsam zugetropft wird. Es wird für zehn Minuten bei -60°C weiter gerührt und anschließend eine Lösung von 388 mg (0,983 mmol) des oben dargestellten Alkohols in 5 ml Dichlormethan zugesetzt. Nach weiteren 15 Minuten Rühren bei dieser Temperatur werden 4,8 g Triethylamin (48 mmol) zugesetzt. Es wird für fünf Minuten bei -60°C gerührt, anschließend das Kältebad entfernt. Die Lösung taut dabei innerhalb von 30 Minuten auf ca. 0°C auf. Bei dieser Temperatur werden 15 ml Wasser zugesetzt und der Ansatz für zehn Minuten stark gerührt. Die organische Phase wird abgetrennt und die wässrige Phase noch zweimal mit je 20 ml Dichlormethan gewaschen. Die vereinigten organischen Phasen werden über Na₂SO₄ getrocknet und i. Vak. zur Trockne eingeengt. Nach Entfernen des Lösungsmittels i. Vak. erhält man 354mg 5.7.6. Der Aldehyd ist ohne weitere Reinigung in der nachfolgenden Wittig-Reaktion einsetzbar. Dafür werden 340 mg 5.7.6 (0,866 mmol) in 15 ml Benzol gelöst und bei Raumtemperatur mit 301 mg (0,866 mmol) (Ethoxycarbonylmethylen)-triphenylphosphoran zugesetzt. Bei Raumtemperatur wird über Nacht gerührt und anschließend das Lösungsmittel i. Vak. entfernt. Nach Reinigung mittels präparativer HPLC (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 25% B auf 100% B, 1 %/min) erhält man 300 mg des reinen Olefins 5.7.7. Vom gereinigten Produkt werden die säurelabilen Schutzgruppen abgespalten indem 290 mg von Verbindung 5.7.7 (0,63 mmol) in 10 ml Dichlormethan gelöst wird und nacheinander 100 µl Triisopropylsilan und 100 µl Trifluoressigsäure zugesetzt werden. Nach 30 Minuten wird das Lösungsmittel i. Vak. entfernt und der erhaltene Rückstand durch präparative HPLC gereinigt (Synergie Max, 4µm, 250x21,2 mm, A-Eluent: 0,1%TFA / Wasser, B-Eluent: 90% AcCN / 10% Wasser / 0,1%TFA. Gradient: 8 ml/min, 5% B auf 100% B, 1%/min). Man erhält so 131 mg der freien Carbonsäure 5.7.8.

50 mg 5.7.8 (0,2 mmol) werden in 3 ml DMF gelöst. Zu dieser Lösung werden 77 mg (0,2 mmol) HATU und 68 µl DIPEA (0,4 mmol) zugesetzt. Diese Lösung wird sofort zu einer Lösung von 91 mg des Trifluoracetatsalzes von H-Val-Pro-Leu-OMe in 2 ml DMF zugesetzt. Nach einer Stunde Rühren bei Raumtemperatur wird das Lösungsmittel i. Vak. entfernt und die Titelverbindung durch präparative HPLC in reiner Form isoliert (Synergie Max, 4µm, 250x21,2 mm, A-Eluent: 0,1%TFA / Wasser, B-Eluent: 90% AcCN / 10% Wasser / 0,1 %TFA. Gradient: 8 ml/min, 5% B auf 100% B, 1%/min).
Ausbeute: 78 mg
ESI-MS: 590,1 {M+Na}⁺

### (Verbindung 42)

Verbindung 42 wurde gemäß Reaktionsschema der Figur 3 Variante 2 hergestellt und belegt, dass Grundgerüste aus vollständig nicht-proteinogenen Aminosäuren erfindungsgemäß hergestellt werden können.

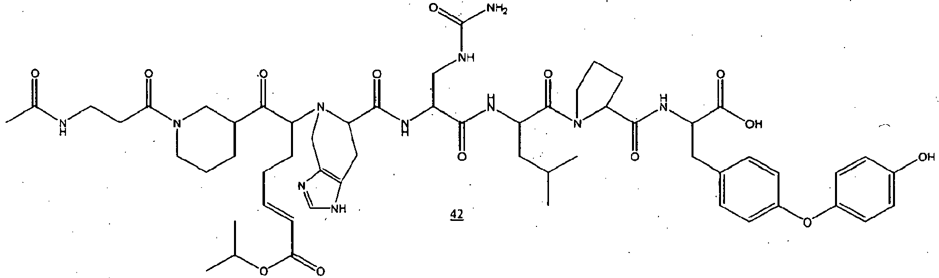

### (Verbindung 43)

Verbindung 43 wurde gemäß Reaktionsschema der Figur 3 Variante 1 hergestellt und belegt, dass Grundgerüste aus vollständig nicht-proteinogenen Aminosäuren erfindungsgemäß hergestellt werden können.

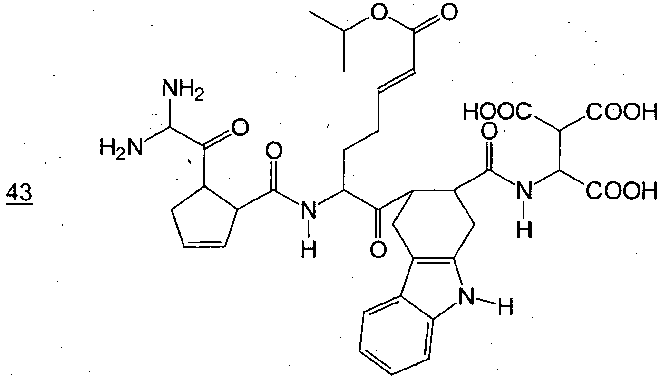

### (Verbindung 44)

Verbindung 44 wurde gemäß Reaktionsschema der Figur 3 Variante 2 hergestellt und belegt, dass Grundgerüste mit 13 Aminosäuren problemlos erfindungsgemäß hergestellt werden können.

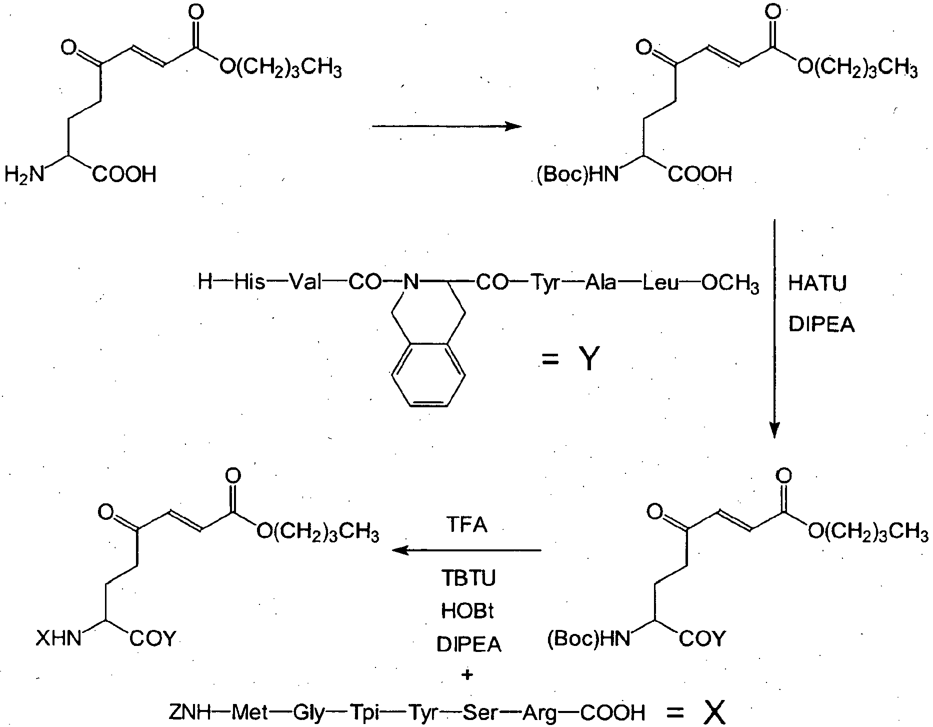

Verbindung 43 und 44 zeigten einen IC₅₀-Wert bei der TG2 von 5µM bzw. 15 µM.

### In-vivo Versuchsbeispiel am Irish Setter

Ein 5 Wochen alter Irish Setter Hund wurde mit Weizen-haltiger Diät gefüttert. Das Tier zeigte Durchfallerscheinungen und eine geringe Gewichtszunahme. Durch eine jejunale Biopsie wurde der partielle Abbau der Darmzotten und eine erhöhte Anzahl an intraepithelialen Lymphozyten festgestellt.

Dem Hund wurden nun vor jeder Mahlzeit 75 mg/kg Körpergewicht Transglutaminase-Inhibitor (1) in einer säurestabilen Formulierung verabreicht. Infolgedessen wurde ein Rückgang des Durchfalls und eine Zunahme des Körpergewichts beobachtet. Nach 2 Monaten wurde per Biopsie festgestellt, dass die Dünndarmzotten die normale Länge aufwiesen und die Anzahl der intraepithelialen Lymphozyten deutlich reduziert war.

### Beispiel: Rekombinante Herstellung von TG6 und TG7

Die Gene für die TG6 und die TG7 wurden aus TG6-cDNA bzw. TG7-cDNA (beide Stand der Technik gemäß US-Patent 7,052,890) mittels PCR nach Standardverfahren amplifiziert. Durch die verwendeten Primer wurden am 5'-Ende eine *Nde*I-Schnittstelle und sechs Histidin-Codons und am 3'-Ende eine *Bgl*II-Schnittstelle (TG6) und eine Hindlll-Schnittstelle (TG7) eingefügt.

Das TG6-PCR-Produkt wurde mit den Restriktionsendonukleasen *Nde*I und *Bg*/II behandelt und in den *Nde*I und *Bam*H I-geschnittenen Vektor pET 3a eingefügt. Das TG7-PCR-Produkt wurde mit den Restriktionsendonukleasen *Nde*I und *Hind*III behandelt und in den ebenso geschnittenen Vektor pET 28b eingefügt.

Der Stamm *E. coli* BL21 (DE3) (Novagen, Darmstadt) wurde mit den erhaltenen Plasmiden transformiert. Eine Kultur je eines Stammes wurde mit IPTG induziert und geerntet. Nach Zellaufschluss durch Hochdruckhomogenisation wurden die Zellaufschlüsse zentrifugiert und die Überstände über lonenmetallaffinitätschromatographie an HiTrap-Chelating HP-Säulen (GE-Healthcare) gereinigt. Die gereinigten Proteine TG6 und TG7 wurden durch SDS-PAGE mit Coomassie-Färbung analysiert. Sämtliche angewendeten Verfahren zur Herstellung der TG6 und TG7 sind dem Fachmann wohlbekannt.

Die weiteren Beispiele 10 bis 43 von Verbindungen gemäß allgemeiner Formel [A] werden analog den obigen Versuchsbeschreibungen hergestellt und weisen folgende Substitutionsmuster auf:

**Tabelle 1**

| Verbindung Nr. | -NXX' | Y | Z | E/Z;m | ESI-MS [M+Na]⁺ | IC₅₀-Wert [TG2] | IC₅₀-Wert [FXIII] | IC₅₀-Wert [TG1] |
|---|---|---|---|---|---|---|---|---|
| 1 | Z*-NH | CO-Val-Pro-Leu-OMe | OCH₂CH₃ | 0;0 | 681,4 | 30 nM | >50µm | n.b. |
| 2 | Z*-NH | CO-Gln-Pro-Leu-OMe | OCH₂CH₃ | 0;0 | 710,4 | 20 nM | >50µM | n.b. |
| 3 | Z*-NH | CO-Phe-OMe | OCH₂CH₃ | 0;0 | 519,2 | 8µM | 2,5µM | 200nM |
| 4 | Z*-NH | Gln-Pro-Leu-OMe | OCH₃ | 0;0 | 583,3 | 350nM | >50µM | n.b. |
| 5 | Z*-NH | Gln-Pro-Isopropylamid | OCH₂CH₃ | 0;0 | 623,5 | 125nM | >50µM | n.b. |
| 6 | (E)-(L)-6-(2-Oxo-pyrrolidin-1-yl-) | Val-Pro-OMe | OCH₂CH₃ | 0;0 | 502,3 | 15µM | >50µM | n.b. |
| 7 | Ac-Asn | Glu-Ala-Valin-OMe | OCH₃ | 0;0 | 679,3 | >50µM | 2µM | >50µM |
| 8 | Ac-Leu-Gly-Pro-Gly | Ser-Leu-Val-Ile-Gly-OMe | OCH₂CH₃ | 0;0 | 1073,7 | >50µM | 500nM | >50µM |
| 9 | Z*-NH | Val-Pro-Leu-OMe | OCH₂CH₃ | 0;1 | 709,5 | 18µM | n.b. | n.b. |
| 10 | Ac-NH | CO-Gln-Glu-Ala-OMe | OCH₃ | 0;0 | 594,3 | 330nM | n.b. | n.b. |
| 11 | Ac-NH | CO-Gln-Glu-OMe | OCH₃ | 0;0 | 523,2 | 2,1µM | n.b. | n.b. |
| 12 | Ac-NH | CO-Phe-Pro-Leu-OMe | OCH₃ | 0;0 | 623,3 | 1,8µM | n.b. | n.b. |
| 13 | Z*-NH | CO-Phe-Pro-Leu-OMe | OCH₃ | 0;0 | 715,3 | 1,5µM | n.b. | n.b. |
| 14 | Ac-NH | CO-(p-Flour-Phe)-Pro-Leu-OMe | OCH₃ | 0;0 | 641,2 | 1,1µM | n.b. | n.b. |
| 15 | Z*-NH | CO-(p-Flour-Phe)-Pro-Leu-OMe | OCH₃ | 0;0 | 733,4 | 880nM | n.b. | n.b. |
| 16 | (E)-(L)-6-(2-Oxo-pyrrolidin-1-yl-) | CO-Val-Pip-Leu-OMe | OCH₂CH₃ | 0;0 | 615,4 | 22µM | n.b. | n.b. |
| 17 | (E)-(L)-6-(2-Oxo-pyrrolidin-1-yl-) | CO-Chg-Pip-Leu-OMe | OCH₂CH₃ | 0;0 | 655,4 | 35µM | n.b. | n.b. |
| 18 | (E)-(L)-6-(2-Oxo-pyrrolidin-1-yl-) | CO-Chg-Pro-Leu-OMe | OCH₂CH₃ | 0;0 | 641,5 | 28µM | n.b. | n.b. |
| 19 | Z*-NH | CO-Gln-Pro-Tyr-OMe | OCH₂CH₃ | 0;0 | 760,3 | 2,4µM | n.b. | n.b. |
| 20 | Z*-Phe-NH | CO-Gln-Pro-Leu-OMe | OCH₂CH₃ | 0;0 | 857,5 | 2µM | n.b. | n.b. |
| 21 | Z*-Gln-NH | CO-Leu-Pro-Gln-OMe | OCH₂CH₃ | 0;0 | 838,3 | 3,2µM | n.b. | n.b. |
| 22 | Ac-NH | CO-Gln-Pro-Leu-OMe | OCH₂CH₄ | 1;0 | 646,2 | 70nM | n.b. | n.b. |
| 23 | 5-Methylisoxazol-3-carbonsäure | CO-Gln-Pro-Leu-OMe | O-CH(CH₃)₂ | 1;0 | 727,4 | 56nM | n.b. | n.b. |
| 24 | 2-Flourbenzoesäure | CO-Val-4-Fluoroprolin-O-CH(CH₃)₂ | OCH₃ | 1;0 | 616,2 | 8µM | n.b. | n.b. |
| 41 | Z-Pro-Gln-Pro- | CO-Gln-Tic-Leu-OMe | O-CH₂Ph | 0;0 | 1156,6 | 215nM | n.b. | n.b. |
| 42 | Ac-β-Ala-(S)-Piperidin-3-carbonyl | CO-L-Spinacinyl-L-Albizzinyl-Val-Pro-Thyronin-OH | O-CH(CH₃)₂ | 0;0 | 1140,4[M+H]⁺ | 4µM | n.b. | n.b. |
| 43 | Agl-Dhp- | CO-Gln-Tpi-Gla-OH | O-CH(CH₃)₂ | 0;0 | 740,3[M+H]⁺ | 1,9µM | n.b. | n.b. |

Die Beispiele 3.2.1 bis 3.2.11 von Verbindungen gemäß allgemeiner Formel [G] mit m = 0 werden aus den Verbindungen 3.1 bis 3.12 nach den unter 1. , 2. und 3.2 beschriebenen Methoden hergestellt und weisen folgende Substitutionsmuster auf:

**Tabelle 2**

| Verbindung Nr. | -NXX' | Y | Z₁ | Z₂ | Z₃ | E/Z | ESI-MS [M+Na]⁺ | IC₅₀-Wert [TG2] | IC₅₀-Wert [FXIII] | IC₅₀-Wert [TG1] |
|---|---|---|---|---|---|---|---|---|---|---|
| 3.2.1 | Z*-NH | CO-Gln-Pro-Leu-OMe | SO₂Me | H | H | 0 | 716,5 | 54 nM | >100 >µM | 250 nM |
| 3.2.2 | Z*-NH | CO-Gln-Pro-Leu-OMe | SO₂N(CH₃)₂ | H | H | 0 | 745,3 | 760 nM | >100 µM | 1,3 µM |
| 3.2.3 | Z*-NH | CO-Gln-Pro-Leu-OMe | Z₁-Z₃ | H | Ring-struktur (siehe Spalte Z₁) | 0' | 692,2 | 18 µM | >100 µM | 14 µM |
| | | | COCH₂CH₂ | | | | | | | |
| 3.2.4 | Z*-NH | CO-Gln-Pro-Leu-OMe | Z₁-Z₂ | Ringstruktur (siehe Spalte Z₁) | H | 0 | 708,4 | 8,9 µM | > 100 µM | 400 nM |
| | | | CO₂CH₂CH₂ | | | | | | | |
| 3.2.5 | Z*-NH | CO-Gln-Pro-Leu-OMe | CO₂H | H | H | 0 | 682,3 | 23 µM | >100 µM | n.b. |
| 3.2.6 | Ac-NH | CO-Gln-Asp-Pro-OMe | CONH-C₅H₁₁ | H | H | 0 | 661,4 | >100 µM | >100 µM | 23 µM |
| 3.2.7 | Z*-NH | CO-(p-Fluor-Phe)-Pro-OH | CO₂-iPr | H | H | 0 | 634,4 | 1,5 µM | >100 µM | 3,7 µM |
| 3.2.8 | Z*-NH | CO-Phe-Pip-Leu-NH₂ | CO₂-Bn | H | H | 0 | 790,3 | 130 nM | >100 µM | 825 nM |
| 3.2.9 | Z*-NH | CO-Phe-OMe | COCO₂Et | H | H | 0 | 547,3 | 3,8 µM | >100 µM | 530 nM |
| 3.2.10 | Z*-NH | CO-Gln-Pro-Leu-OH | CO-CH₃ | H | H | 1 | 666,2 | 56 µM | n.b. | n.b. |
| 3.2.11 | Ac-NH | CO-Gln-Pro-Leu-OMe | CN | H | H | 1 | 663,3 | 43 µM | n.b. | n.b. |

| Inhibitoren der Transglutaminasen 3,6 und7 | | | | | | | | | | |
|---|---|---|---|---|---|---|---|---|---|---|
| | | | | | | | | IC₅₀-Wert [TG3] | IC₅₀-Wert [TG6] | IC₅₀-Wert [TG7] |
| 3.2.12 | 4-Methoxybenzoesäure | CO-Leu-Pro-Tyr-NH-Et | 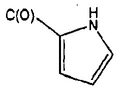 | H | H | 0 | 780,2 | 53µM | 87µM | 12µM |
| 3.2.13 | Ac-Aib-Cph- | CO-Gln-Ile-Val-OH | C(O)-N(CH₃)₂ | H | H | 0 | 905,3 | >100µM | 4µM | 46µM |
| 3.2.14 | Ac-Gln-Gln- | CO-Val-Hci-Gln-(4-Hydroxy)-Tic-NH₂ | NO₂ | H | H | 0 | 1067,6 | 2,3µM | >100µM | >100µM |
| 3.2.15 | GABA-Gly- | CO-Asp-Pro-Val-Tci-Gly-NH-CH₂-CH₂-SO₃H | CO₂Ph | H | H | 0 | 943,2 [M+H]⁺ | 1,5µM | 16µM | 14µM |
| 3.2.16 | Ac-β-Chloralanin-Thioprolin- | CO-Gln-Cha-4-Aminobenzoesäure | C(O)-Ph(4-NO₂) | H | H | 0 | 963,3 | 48µM | 2µM | 3,4µM |

Legende zu Tabelle 1:
Agl: α-Aminoglycin
Dhp: 3,4-Dehydroprolin
Gla: γ-Carboxyglutaminsäure
Chg: Cyclohexylglycin
Tpi: 1,2,3,4-Tetrahydronorharman-3-carbonsäure
Tic: 1,2,3,4-Tetrahydroisochinolin-3-carbonsäure nur bei Verbindung 9 ist m = 1, sonst ist m = 0
Die angegeben Werte beziehen sich für E/Z = 0 immer auf das jeweilige E-Isomer und für E/Z = 1 auf das Z-Isomer
Pip: Hormoprolin
Z*: Benzyloxycarbonyl
n.b.: nicht bestimmt 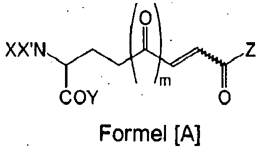
Legende zu Tabelle 2:
Aib: α-Aminoisobuttersäure
Cph: 2,4-Dichlorophenylalanin
Hci: Homocitrullin
Tci: Thiocitrullin
Cha: Cyclohexylalanin n.b.: nicht bestimmt
Z*: Benzyloxycarbonyl

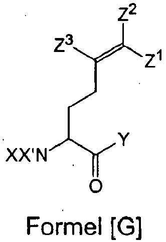

## Patentansprüche

1. Peptid oder Peptidomimetikum der folgenden allgemeinen Formel (I), (II) oder (III): 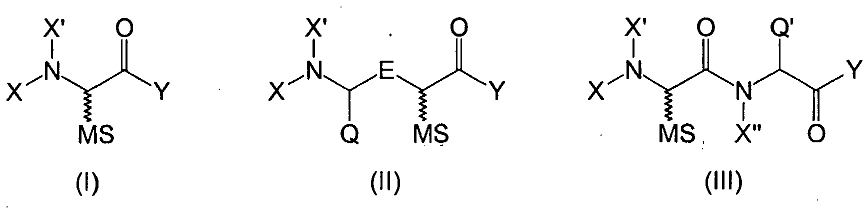 worin
MS das akzeptorsubstituierte Olefin folgender Struktur ist: 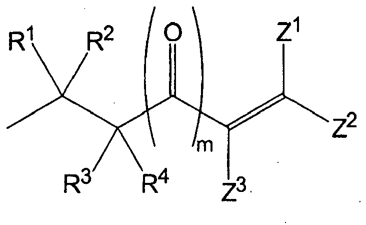 E folgende Gruppe bedeutet -CH₂-, -CF₂-, -C₂H₄-, -CH₂-CF₂-, -CF₂-CH₂-, -CH=CH-, -CH(OH)-CH₂-, -C(=O)-CH₂-, -CH₂-NH-, -CH₂-O-, -CH(OH)-CH₂-NH-, -P(=O)(OH)-NH-, -P(=O)(OH)-O-, -P(=O)(OH)-S-, -P(=O)(OH)-CH₂-, -CH(OH)-CH₂-NH-, -C(=O)-NH-, -C(=O)-O- oder -C(=O)-NX"-;
**m** für 0 oder 1 steht;
die Reste **Z¹, Z², Z³** unabhängig voneinander für folgende Gruppen stehen: -H, -CO-(C₁-C₆-Alkyl), -CO-R⁶, -CO-R⁷, -CO-(C₁-C₆-Halogenalkyl), -CO-(C₃-C₁₀-Heteroaryl), -CO-(C₆-C₁₅-Aryl), -COO-(C₁-C₆-Halogenalkyl), -COO-(C₃-C₁₀-Heteroaryl), -COO-(C₆-C₁₅-Aryl), -COO-(C₁-C₆-Alkyl), -COO-R⁸, -COO-R⁹, -CN, -COOH, -CO-NH(C₁-C₆-Alkyl), -CO-N(C₁-C₆-Alkyl)(C₁-C₆-Alkyl), -CO-NR¹⁰R¹¹, -CO-NH₂, -NO₂, -CO-N(CR¹²R¹³R¹⁴)(CR¹⁵R¹⁶R¹⁷), -CS-(C₁-C₆-Alkyl), -CS-R¹⁸, -CS-R¹⁹, -CS-O-(C₁-C₆-Alkyl), -CS-O-R²⁰, -CS-N(C₁-C₆-Alkyl)(C₁-C₆-Alkyl), -CS-O-R²¹, -CS-NR²²R²³, -CS-NH₂, -CS-N(CR²⁴R²⁵R²⁶)(CR²⁷R²⁸R²⁹), -SO-R³⁰, -SO-R³¹, -SO₂-R³², -SO₂-R³³, -SO-CR³⁴R³⁵R³⁶, -SO-CR³⁷R³⁸R³⁹, -SO₂-CR⁴⁰R⁴¹R⁴², -SO₂-CR⁴³R⁴⁴R⁴⁵, -SO-N(C₁-C₆-Alkyl)(C₁-C₆-Alkyl), -SO-NR⁴⁶R⁴⁷, -SO-NH₂, -SO-N(CR⁴⁸R⁴⁹R⁵⁰)(CR⁵¹R⁵²R⁵³), -SO₂-N(C₁-C₆-Alkyl)(C₁-C₆-Alkyl), -SO₂-NR⁵⁴R⁵⁵, -SO₂-NH₂, -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, wobei zumindest einer der Reste Z¹, Z², Z³ von Wasserstoff verschieden ist;
die Reste **Z¹** und **Z²** zusammen auch einen Rest -CO-O-CO-CH₂-, -CO-O-CH₂-CH₂- bedeuten können,
die Reste **Z²** und **Z³** zusammen auch einen Rest -CO-Z'-CH₂-, -CO-O-CH₂-, -CO-CH₂-CH₂-, -CO-O-CO- oder -CO-NH-CObedeuten können, worin
**Z'** für eine der folgenden Gruppen steht: -CH₂-, -CF₂-, -C₂H₄-, -CF₂-CH₂-, -CH₂-CH₂-, -O-, -O-CH₂-, -NH- oder -NH-CH₂-;
**Q** und **Q'** unabhängig voneinander einen Seitenkettenrest einer natürlichen Aminosäure darstellen, oder **Q** zusammen mit **X'** einen Propylenylrest bilden, oder **Q'** zusammen mit **X"** einen Propylenylrest bilden;
**Y** eine Hydroxygruppe, Aminogruppe, C₁-C₆-Alkylaminogruppe, C₁-C₆-Dialkylaminogruppe, C₁-C₆-Alkoxygruppe, C₆-C₁₉-Aryloxygruppe, C₁-C₆-Alkylgruppe, C₁-C₆-Halogenalkylgruppe, C₃-C₁₀-Heteroarylgruppe oder eine C₆-C₁₅-Arylgruppe bedeutet oder Y für einen über eine Amidbindung gebundenen Peptidrest aus bis zu 6 Aminosäuren steht, dessen C-terminale Carbonylfunktion eine Hydroxygruppe, Aminogruppe, C₁-C₆-Alkylaminogruppe, C₁-C₆-Dialkylaminogruppe, C₁-C₆-Alkoxygruppe, C₁-C₆-Alkylgruppe, C₁-C₆-Halogenalkylgruppe, C₃-C₁₀-Heteroarylgruppe oder eine C₆-C₁₅-Arylgruppe trägt oder Y für einen peptidomimetischen Rest aus bis zu 60 Kohlenstoffatomen steht und
**X"** für Wasserstoff oder eine C₁-C₆-Alkylgruppe steht; und
**-NXX'** eine Aminogruppe, -NH-CHO, C₁-C₁₀-Alkylaminogruppe, C₁-C₈-Alkyloxycarbonylaminogruppe, C₆-C₁₂-Aralkyloxycarbonylaminogruppe, C₁-C₁₀-Dialkylaminogruppe, C₂-C₆-Stickstoffheterocyclus oder eine C₃-C₅-Stickstoffheteroarylgruppe bedeutet oder die Gruppe -NXX' Teil eines peptidomimetischen Restes aus bis zu 60 Kohlenstoffatomen ist
oder
**X'** für Wasserstoff oder eine C₁-C₆-Alkylgruppe steht; und
**X** für einen über eine Amidbindung gebundenen Peptidrest aus bis zu 6 Aminosäuren steht, dessen N-Terminus eine Aminogruppe, -NH-CHO, C₁-C₁₀-Alkylaminogruppe, C₁-C₈-Alkyloxycarbonylaminogruppe, C₆-C₁₂-Aralkyloxycarbonylaminogruppe, C₁-C₁₀-Dialkylaminogruppe, C₂-C₆-Stickstoffheterocyclus oder eine C₃-C₅-Stickstoffheteroarylgruppe; wobei jede der C₁-C₆-Alkoxygruppen, C₁-C₆-Alkylgruppen, C₁-C₁₀-Alkylaminogruppen, C₁-C₈-Alkyloxycarbonylaminogruppe, C₆-C₁₂-Aralkyloxycarbonylaminogruppe, C₁-C₁₀-Dialkylaminogruppen, C₂-C₆-Stickstoffheterocyclen sowie C₃-C₅-Stickstoffheteroarylgruppen unabhängig voneinander mit bis zu 5 Resten ausgewählt aus R⁸⁰, R⁸¹, R⁸², R⁸³, R⁸⁴ substituiert sein kann,
**R¹ - R⁴** Wasserstoff bedeuten,
und die Reste **R⁵ - R⁶⁹** und **R⁸⁰ - R⁸⁴** unabhängig voneinander folgende Gruppen bedeuten:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -CN, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅. -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂. -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(≡NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)-CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH ;
sowie stereoisomere Formen, E/Z-Isomere, Enantiomere, Enantiomerengemische, Diastereomere, Diastereomerengemische, Racemate, Tautomere, Anomere, Keto-Enol-Formen, Betainformen, Solvate, Hydrate als auch pharmakologisch verträgliche Salze der vorgenannten Verbindungen, ausgenommen die Verbindungen gemäß allgemeiner Formel (I) worin X und X' Wasserstoff bedeuten und Y eine Hydroxygruppe darstellt sowie die Verbindung H-Phenylalanyl-[2-(4-ethoxycarbonyl-3-buten)-glycyl]-leucin-amid.

2. Peptid oder Peptidomimetikum gemäß Anspruch 1, der allgemeinen Formel (IIB) 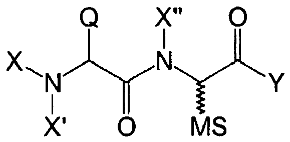 worin
die Reste MS, Q, X, X', X" und Y die Bedeutung wie in Anspruch 1 haben.

3. Peptid oder Peptidomimetikum gemäß eines der vorherigen Ansprüche, worin MS folgende Struktur aufweist: 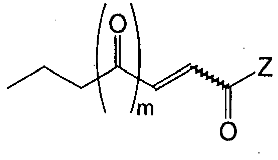 worin
**Z** eine Hydroxygruppe, Aminogruppe, C₁-C₆-Alkylaminogruppe, C₁-C₆-Dialkylaminogruppe, C₁-C₆-Alkoxygruppe, C₁-C₆-Alkylgruppe, C₁-C₆-Halogenalkylgruppe, C₃-C₁₀-Heteroarylgruppe oder eine C₆-C₁₅-Arylgruppe bedeutet.

4. Peptid oder Peptidomimetikum gemäß Anspruch 1 ausgewählt aus der Gruppe bestehend aus:
N^{α}-Benzyloxycarbonyl-{[(E)-(L)-6-Amino-hept-2-endicarbonsäure]-1-ethanoyl}-LValinyl-L-Prolinyl-L-Leucinmethylester (Verbindung 1);
N^{α}-Benzyloxycarbonyl-{[(E)-(L)-6-Amino-hept-2-endicarbonsäure]-1-ethanoyl}-L-Glutaminyl-L-Prolinyl-L-Leucinmethylester (Verbindung 2);
N^{α}-Benzyloxycarbonyl-{[(E)-(L)-6-Amino-hept-2-endicarbonsäure]-1-ethanoyl}-L-phenylalaninmethylester (Verbindung 3);
N^{α}-Benzyloxycarbonyl-{[(E)-(L)-6-Amino-hept-2-endicarbonsäure]-1-methanoyl}-L-Glutaminyl-L-Prolinmethylester (Verbindung 4);
N^{α}-Benzyloxycarbonyl-{[(E)-(L)-6-Amino-hept-2-endicarbonsäure]-1-ethanoyl}-L-Glutaminyl-L-Prolinyl-Isopentylamid (Verbindung 5);
[(E)-(L)-6-(2-Oxo-pyrrolidon-1-yl)-hept-2-endicarbonsäure-1-ethanoyl]-L-Valinyl-L-Prolinmethylester (Verbindung 6);
N^{α}-Acetyl-L-Asparaginyl-{[(E)-(L)-6-Amino-hept-2-endicarbonsäure]-1-methanoyl}-L-Glutamyl-L-Alaninyl-L-Valinmethylester (Verbindung 7);
N^{α}-Acetyl-L-Leucinyl-Glycinyl-L-Prolinyl-Glycinyl-{[(E)-(L)-6-Amino-hept-2-endicarbonsäure]-1-ethanoyl}-L-Serinyl-L-Leucinyl-L-Valinyl-L-Isoleucinyl-Glycinmethylester (Verbindung 8);
N^{α}-Benzyloxycarbonyl-{[L-7-Amino-4-oxo-oct-2-endicarbonsäure]-1-ethanoyl}-L-Valinyl-L-Prolinyl-Leucin-methylester (Verbindung 9);
N^{α}-Acetyl-{[(E)-(L)-6-Amino-hept-2-endicarbonsäure]-1-methanoyl}-L-Glutaminyl-L-Glutamyl-L-Alaninnmethylester (Verbindung 10);
N^{α}-Acetyl-{[(E)-(L)-6-Amino-hept-2-endicarbonsäure]-1-methanoyl}-L-Glutaminyl-L-Glutamylmethylester (Verbindung 11);
N^{α}-Acetyl-{[(E)-(L)-6-Amino-hept-2-endicarbonsäure]-1-methanoyl}-L-Phenylalaninyl-L-Prolinyl-L-Leucinmethylester (Verbindung 12);
N^{α}-Benzyloxycarbonyl-{[(E)-(L)-6-Amino-hept-2-endicarbonsäure]-1-methanoyl}-L- Phenylalaninyl-L-Prolinyl-L-Leucinmethylester (Verbindung 13);
N^{α}-Acetyl-{[(E)-(L)-6-Amino-hept-2-endicarbonsäure]-1-methanoyl}-L-(p-Fluoro)-Phenylalaninyl-L-Prolinyl-L-Leucinmethylester (Verbindung 14);
N^{α}-Benzyloxycarbonyl-{[(E)-(L)-6-Amino-hept-2-endicarbonsäure]-1-methanoyl}-L-(p-Fluoro)-Phenylalaninyl-L-Prolinyl-L-Leucinmethylester (Verbindung 15);
[(E)-(L)-6-(2-Oxo-pyrrolidon-1-yl)-hept-2-end icarbonsäu re-1-ethanoyl]-L-Valinyl-L-Homoprolinyl-L-Leucinmethylester (Verbindung 16);
[(E)-(L)-6-(2-Oxo-pyrrolidon-1-yl)-hept-2-endicarbonsäure-1-ethanoyl]-L-Cyclohexylglycinyl-L-Homoprolinyl-L-Leucinmethylester (Verbindung 17);
[(E)-(L)-6-(2-Oxo-pyrrolidon-1-yl)-hept-2-endicarbonsäure-1-ethanoyl]-L-Cyclohexylglycinyl-L-Prolinyl-L-Leucinmethylester (Verbindung 18);
N^{α}-Benzyloxycarbonyl-{[(E)-(L)-6-Amino-hept-2-endicarbonsäure]-1-ethanoyl}-L-Glutaminyl-L-Prolin-L-Tyrosinmethylester (Verbindung 19);
N^{α}-Benzyloxycarbonyl-L-Penylalaninyl-{[(E)-(L)-6-Amino-hept-2-endicarbonsäure]-1-ethanoyl}-L-Glutaminyl-L-Prolinyl-L-Leucinmethylester (Verbindung 20);
N^{α}-Benzyloxycarbonyl-L-Glutaminyl-{[(E)-(L)-6-Amino-hept-2-endicarbonsäure]-1-ethanoyl}-L-Leucinyl-L-Prolinyl-L-Glutaminmethylester (Verbindung 21);
N^{α}-Acetyl-{[L-7-Amino-4-oxo-oct-2-endicarbonsäure]-1-ethanoyl}-L-Glutaminyl-L-Prolinyl-L-Leucinmethylester (Verbindung 22);
N^{α}-(5-Methylisoxazol-3-carbonyl)-{[L-7-Amino-4-oxo-oct-2-endicarbonsäure]-1-isopropanoyl}-L-Valinyl-L-Prolinyl-Leucin-methylester (Verbindung 23);
N^{α}-(2-Fluorbenzoyl)-{[L-7-Amino-4-oxo-oct-2-endicarbonsäure]-1-methanoyl}-L-Valinyl-L-4-Fluoroprolinyl-Leucin-isopropylester (Verbindung 24);
N^{α}-Benzyloxycarbonyl-{[(E)-(L)-6-Amino-hept-2-endicarbonsäure]-1-ethanoyl}-L-Phenylalaninyl-L-Prolinyl-L-Leucinmethylester (Verbindung 25);
N^{α}-Benzyloxycarbonyl-{[(E)-(L)-6-Amino-hept-2-endicarbonsäure]-1-ethanoyl}-Glycinyl-L-Prolinyl-L-Leucinmethylester (Verbindung 26);
N^{α}-Benzyloxycarbonyl-{[(E)-(L)-6-Amino-hept-2-endicarbonsäure]-1-ethanoyl}-L-Alaninyl-L-Prolinyl-L-Leucinmethylester (Verbindung 27);
N^{α}-tert.-Butyloxycarbonyl-{[(E)-(L)-6-Amino-hept-2-endicarbonsäure]-1-ethanoyl}-L-Glutaminyl-L-Prolinyl-L-Leucinmethylester (Verbindung 28);
N^{α}-Thiophen-2-carbonyl-{[(E)-(L)-6-Amino-hept-2-endicarbonsäure]-1-ethanoyl}-L-Glutaminyl-L-Prolinyl-L-Leucinmethylester (Verbindung 29);
N^{α}-Furan-3-carbonyl-{[(E)-(L)-6-Amino-hept-2-endicarbonsäure]-1-ethanoyl}-L-Glutaminyl-L-Prolinyl-L-Leucinmethylester (Verbindung 30);
N^{α}-Isoxazol-5-carbonyl-{[(E)-(L)-6-Amino-hept-2-endicarbonsäure]-1-ethanoyl}-L-Glutaminyl-L-Prolinyl-L-Leucinmethylester (Verbindung 31);
N^{α}-(5-Methyl-Isoxazol-3-carbonyl)-{[(E)-(L)-6-Amino-hept-2-endicarbonsäure]-1-ethanoyl}-L-Glutaminyl-L-Prolinyl-L-Leucinmethylester (Verbindung 33);
N^{α}-(trans-3-(3-Thienyl)acryloyl)-{[(E)-(L)-6-Amino-hept-2-endicarbonsäure]-1-ethanoyl}-L-Glutaminyl-L-Prolinyl-L-Leucinmethylester (Verbindung 34);
N^{α}-Acetyl-{[(E)-(L)-6-Amino-hept-2-endicarbonsäure]-1-ethanoyl}-L-Glutaminyl-L-Prolinyl-L-Leucinmethylester (Verbindung 35);
N^{α}-(4-Trifluormethoxy-benzolsulfonyl)-{[(E)-(L)-6-Amino-hept-2-endicarbonsäure]-1-ethanoyl}-L-Glutaminyl-L-Prolinyl-L-Leucinmethylester (Verbindung 36);
N^{α}-Benzyloxycarbonyl-{[(E)-(L)-6-Amino-hept-2-endicarbonsäure]-1-ethanoyl}-L-Cyclohexylglycin-L-Prolinyl-L-Leucinmethylester (Verbindung 37);
N^{α}-Benzyloxycarbonyl-{[(E)-(L)-6-Amino-hept-2-endicarbonsäure]-1-ethanoyl}-L-Valinyl-L-Homoprolinyl-L-Leucinmethylester (Verbindung 38);
(E)-(S)-6-Benzyloxycarbonylamino-6-[3-((R)-2-phenylcarbamoyl-pyrrolidin-1 carbonyl)-phenylcarbamoyl]-hex-2-ensäureethylester (Verbindung 39);
(E)-(S)-6-Benzyloxycarbonylamino-6-{1-[(S)-3-carboxy-1-(3-methyl-butylcarbamoyl)-propyl]-2-oxo-1,2-dihydro-pyridin-3-ylcarbamoyl}-hex-2-enoylsäureisopropylester (Verbindung 40);
N^{α}-Benzyloxycarbonyl-{[(E)-(L)-2-Amino-6-methansulfonyl]-hex-5-enyl}-L-Glutaminyl-L-Prolinyl-L-Leucinmethylester (Verbindung 3.2.1);
N^{α}-Benzyloxycarbonyl-[(E)-(L)-2-Amino-6-dimethylsulfamoyl)-hex-5-enyl]-L-Glutaminyl-L-Prolinyl-L-Leucinmethylester (Verbindung 3.2.2);
N^{α}-Benzyloxycarbonyl-[(L)-2-Amino-4-(3)-oxo-cyclopent-1-enyl]-butyryl-L-Glutaminyl-L-Prolinyl-L-Leucinmethylester (Verbindung 3.2.3);
N^{α}-Benzyloxycarbonyl[(L)-2-Amino-5-(2-oxo-dihydrofuran-(3E)-yliden)]-pentanoyl-L-Glutaminyl-L-Prolinyl-L-Leucinmethylester (Verbindung 3.2.4);
N^{α}-Benzyloxycarbonyl-[(E)-(L)-6-Amino-hept-2-endicarbonsäure]-L-Glutaminyl-L-Prolinyl-L-Leucinmethylester (Verbindung 3.2.5);
N^{α}-Acetyl-{[(E)-(L)-6-Amino-hept-2-endicarbonsäure]-1-pentylamido}-L-Glutaminyl-L-Asparatyl-L-Prolinmethylester (Verbindung 3.2.6);
N^{α}-Benzyloxycarbonyl-{[(E)-(L)-6-Amino-hept-2-endicarbonsäure]-1-isopropanoyl}-L-(p-Fluor-phenylalaninyl)-L-Prolin (Verbindung 3.2.7);
N^{α}-Benzyloxycarbonyl-{[(E)-(L)-6-Amino-hept-2-endicarbonsäure]-1-benzoyl}-L-Phenylalaninyl-L-Homoprolinyl-L-Leucinylamid (Verbindung 3.2.8);
N^{α}-Benzyloxycarbonyl-{[(E)-(L)-7-Amino-2-oxo-oct-3-endicarbonsäure]-1-ethanoyl}-LPhenylalaninmethylester (Verbindung 3.2.9);
N^{α}-Benzyloxycarbonyl-[(Z)-(L)-2-Amino-7-oxo-oct-5-ensäure]-L-Glutaminyl-L-Prolinyl-L-Leucin-methylester (Verbindung 3.2.10);
N^{α}-Benzyloxycarbonyl-[(Z)-(L)-2-Amino-6-cyano-hex-5-ensäure]-L-Glutaminyl-L-Prolinyl-L-Leucin-methylester (Verbindung 3.2.11);
N^{α}-Benzyloxycarbonyl-{[(E)-(L)-6-Amino-hept-2-endicarbonsäure]-1-ethanoyl}-L-Valinyl-L-(Octahydroindol-2-carboxyl)-L-Leucinylamid (Verbindung 4.1);
N^{α}-(Piperidinyl-4-carbonyl)-{[(E)-(L)-2-Amino-6-phenylsulfonyl]-hex-5-enyl}-L-Phenylalaninyl-L-Prolinyl-L-1-Cyclopentylmethyl-2-oxo-2-(1H-tetrazol-5-yl)-ethylamid (Verbindung 4.2);
N^{α}-Benzyloxycarbonyl-[(E)-(L)-2-Amino-6-benzyloxysulfonyl-hex-5-enyl]-L-Valinyl-L-Prolinylbenzylsulfonamid (Verbindung 4.3);
(E)-(S)-6-[(S)-1-((S)-2-Ethylcarbamoyl-pyrrolidin-1-carbonyl)-2-methyl-propylcarbamoyl]-6-(2-piperidin-4-yl-ethylamino)-hex-2-ensäureisopropylester (Verbindung 5.1);
(S)-2-[((S)-1-{(E)-2R,5S)-2-(4-Fluorobenzyl)-9-methansulfonyl-5-[(5-methyl-isoxazol-3-carbonyl)-amino]-4-oxo-non-8-enoyl}-pyrrolidin-2-carbonyl)-amino]-4-methyl-pentansäuremethylester (Verbindung 5.3.b);
(E)-(6R,9S)-9-Benzyloxycarbonylamino-6-[2-(2-ethylcarbamoyl-octahydroindol-1-yl)-1-methyl-2-oxoethylcarbamoyl]-8-oxo-10-phenyl-dec-2-enylsäure-isopropylester (Verbindung 5.4);
Piperidin-4-carbonyl-((E)-(S)-5-benzylsulfonyl-1-{2-[2-((S)-2-benzylsulfonylaminocarbonyl-octahydro-indol-1yl)-2-oxo-ethylamino]-acetyl}-4-enyl)-amid (Verbindung 5.6);
(E)-5-(N'-Acetyl-N-carboxy-hydrazino)-[(pent-2-enoyl)-1-ethanoly]-LValinyl-L-Prolinyl-L-Leucinmethylester (Verbindung 5.7).

5. Verbindung gemäß einem der Ansprüche 1 - 4 zur Verwendung in der Medizin.

6. Verwendung einer Verbindung zur Herstellung eines Arzneimittels zur Behandlung oder Prophylaxe von Zöliakie, Fibrosen, Thrombose, neurodegenerative Erkrankungen, Chorea Huntington, Parkinson, Alzheimer, Katarakt, Akne, Psoriasis oder Candiose der allgemeinen Struktur [TGI1]:
akzeptorsubstituierte Doppelbindung-(CO)ₘ-C₂H₄-Grundgerüst [TGI1]
wobei
m für 0 oder 1 steht und
die akzeptorsubstituierte Doppelbindung mindestens einen zur Konjugation befähigten elektronenziehenden Rest aus der Gruppe C=O, C=S, C=N, P=O, P=S, N=O, S=O, N=N, C=N mit einer Elektronegativität ≥ 2,20 trägt und
das Grundgerüst mindestens eine Amidbindung aufweist und das peptidische oder peptidähnliche Grundgerüst aus zwei bis 20 Aminosäuren oder Mimetika für Aminosäuren besteht, welche über Peptidbindungen, Esterbindungen, Carbonatbindungen, Urethanbindungen, Carbamatbindungen und/oder Harnstoffbindungen miteinander verknüpft sind und das Michael-System tragende Grundgerüst mindestens über eine zum Kohlenstoffatom, das die Seitenkette mit dem Michael-System trägt, vicinale Carbonylgruppe verfügt.

7. Verwendung einer Verbindung gemäß Anspruch 6 der allgemeinen Struktur [A]: 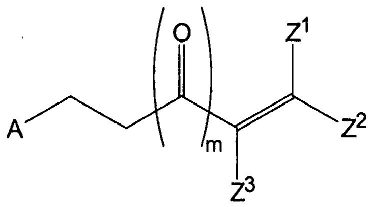 wobei die Verbindung mindestens ein akzeptorsubstituiertes Olefin mit den Resten Z¹, Z² und Z³ aufweist, welches über eine Ethylengruppe oder über eine Carbonylethylengruppe an eine mindestens sekundär substituierte Gruppe A gebunden ist, wobei
**A** einen Peptidrest aus 2 bis 20 Aminosäuren oder einen peptidomimetischen Rest aus 2 bis 20 Aminosäuren darstellt, wobei im Peptidrest die Aminosäuren auch nicht-proteinogene Aminsäuren ausgewählt aus Thiocarbonylaminosäuren, β-Aminosäuren, γ-Aminosäuren, δ-Aminosäuren, 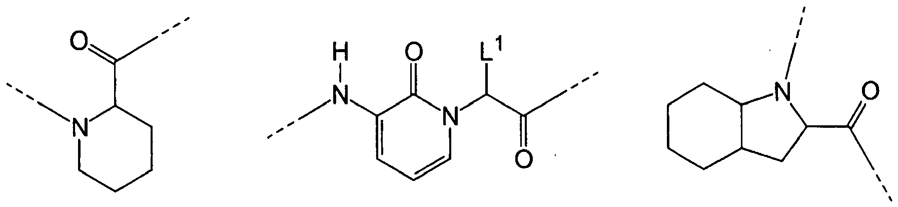 umfassen und im peptidomimetischen Rest 1 bis 3 Aminosäuremimetika ausgewählt aus 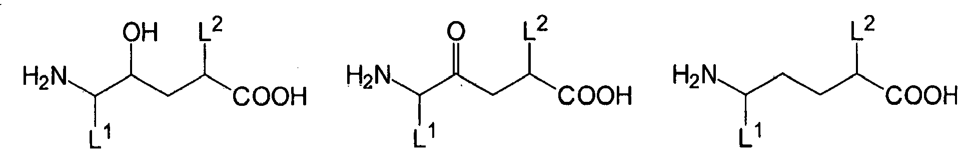 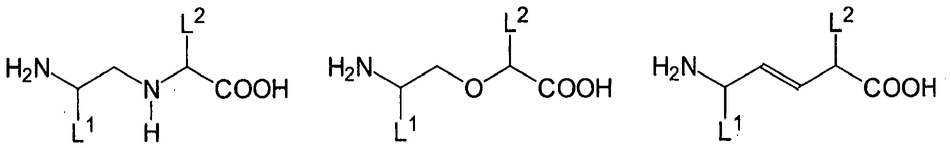 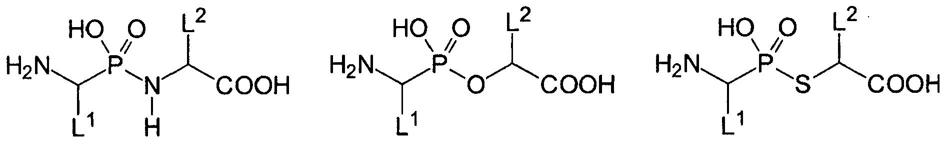 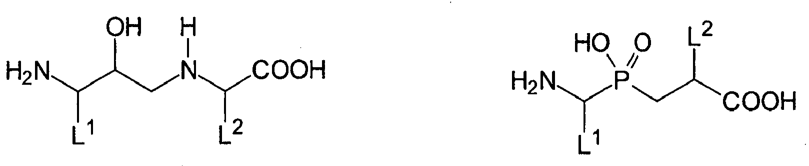 enthalten sein können, worin L¹ und L² unabhängig voneinander einen Seitenkettenrest einer natürlichen Aminosäure oder einen Rest -R⁵⁷, -R⁵⁹, -R⁶⁰, -R⁶¹, -CR⁶²R⁶³R⁶⁴, -CR⁶⁵R⁶⁶-CR⁶⁷R⁶⁸R⁶⁹, -CR⁷⁰R⁷¹-CR⁷²R⁷³-CR⁷⁴R⁷⁵R⁷⁶ bedeuten;
m für 0 oder 1 steht;
die Reste **Z¹, Z², Z³** unabhängig voneinander für folgende Gruppen stehen: -H, -CO-(C₁-C₆-Alkyl), -CO-R⁶, -CO-R⁷, -CO-(C₁-C₆-Halogenalkyl), -CO-(C₃-C₁₀-Heteroaryl), -CO-(C₆-C₁₅-Aryl), -COO-(C₁-C₆-Halogenalkyl), -COO-(C₃-C₁₀-Heteroaryl), -COO-(C₆-C₁₅-Aryl), -COO-(C₁-C₆-Alkyl), -COO-R⁸, -COO-R⁹, -CN, -F, -Cl, -Br, -COOH, -CO-NH(C₁-C₆-Alkyl), -CO-N(C₁-C₆-Alkyl)(C₁-C₆-Alkyl), -CO-NR¹⁰R¹¹, -CO-NH₂, -CO-N(CR¹²R¹³R¹⁴)(CR¹⁵R¹⁶R¹⁷), -CF₃, -OCF₃, -NO₂, -CS-(C₁-C₆-Alkyl), -CS-R¹⁸, -CS-R¹⁹, -CS-O-(C₁-C₆-Alkyl), -CS-O-R²⁰, -CS-N(C₁-C₆-Alkyl)(C₁-C₆-Alkyl), -CS-O-R²¹, -CS-NR²²R²³, -CS-NH₂, -CS-N(CR²⁴R²⁵R²⁶)(CR²⁷R²⁸R²⁹), -SO-R³⁰, -SO-R³¹, -SO₂-R³², -SO₂-R³³, -SO-CR³⁴R³⁵R³⁶, -SO-CR³⁷R³⁸R³⁹, -SO₂CR⁴⁰R⁴¹R⁴², -SO₂-CR⁴³R⁴⁴R⁴⁵, -SO-N(C₁-C₆-Alkyl)(C₁-C₆-Alkyl), -SO-NR⁴⁶R⁴⁷, -SO-NH₂, -SO-N(CR⁴⁸R⁴⁹R⁵⁰)(CR⁵¹R⁵²R⁵³), -SO₂-N(C₁-C₆₋Alkyl)(C₁-C₆-Alkyl), -SO₂-NR⁵⁴R⁵⁵, -SO₂NH₂, -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, wobei zumindest einer der Reste Z¹, Z², Z³ von Wasserstoff verschieden ist;
die Reste **Z¹** und **Z²** zusammen auch einen Rest -CO-O-CO-CH₂-, -CO-O-CH₂-CH₂- bedeuten können,
die Reste **Z²** und **Z³** zusammen auch einen Rest -CO-Z'-CH₂-, -CO-O-CH₂-, -CO-CH₂-CH₂-, -CO-O-CO- oder -CO-NH-CO- bedeuten können, worin
**Z'** für eine der folgenden Gruppen steht: -CH₂-, -CF₂-, -C₂H₄-, -CF₂-CH₂-, -CH₂-CH₂-, -O-, -O-CH₂-, -NH- oder -NH-CH₂-;
die Reste **R⁶ - R⁷⁶** unabhängig voneinander folgende Gruppen bedeuten:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -CN, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, ;
sowie stereoisomere Formen, E/Z-Isomere, Enantiomere, Enantiomerengemische, Diastereomere, Diastereomerengemische, Racemate, Tautomere, Anomere, Keto-Enol-Formen, Betainformen, Solvate, Hydrate als auch pharmakologisch verträgliche Salze der vorgenannten Verbindungen.

8. Verwendung gemäß Anspruch 7, worin die Verbindung folgende allgemeine Struktur [B] aufweist: 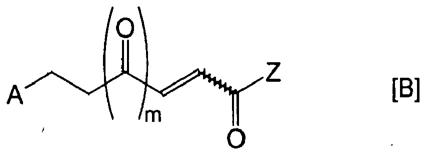 worin
**Z** eine Hydroxygruppe, Aminogruppe, C₁-C₆-Alkylaminogruppe, C₁-C₆-Dialkylaminogruppe, C₁-C₆-Alkoxygruppe, C₁-C₆-Alkylgruppe, C₁-C₆-Halogenalkylgruppe, C₃-C₁₀-Heteroarylgruppe oder eine C₆-C₁₅-Arylgruppe bedeutet und
m für 0 oder 1 steht, und
**A** einen Peptidrest, ein Peptidderivat oder einen peptidomimetischen Rest darstellt.

9. Verwendung gemäß Anspruch 8, worin die Verbindung folgende allgemeine Struktur [C], [D], [E] oder [F] aufweist:

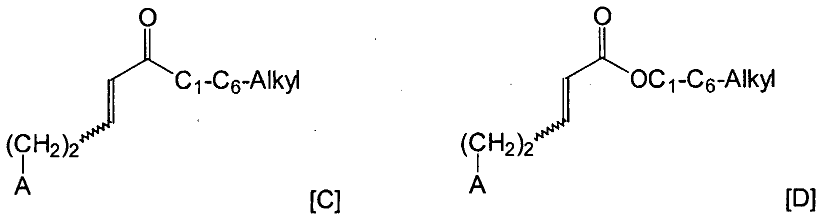

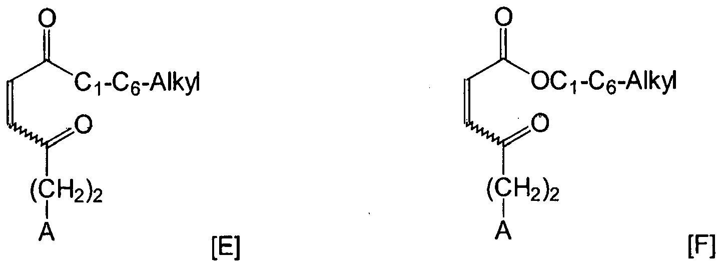

10. Verwendung gemäß eines der Ansprüche 7 - 9, wobei die Verbindung eine der folgenden allgemeinen Formel (I), (II) oder (III) hat: 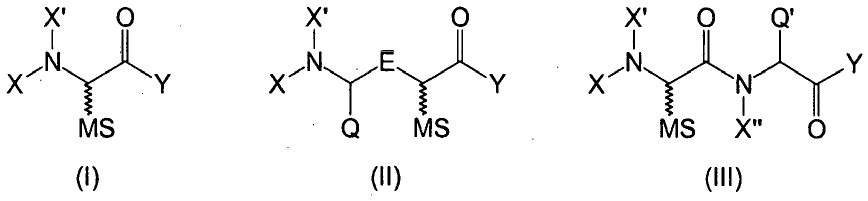 worin
MS das Michael-System folgender Struktur ist 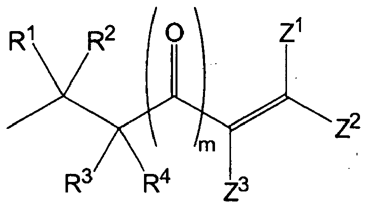 und worin m, E, R¹, R², R³, R⁴, Q, Q', X, X', X", Y, Z¹, Z² und Z³ die Bedeutung wie in Anspruch 1 haben.

11. Pharmazeutische Zusammensetzung umfassend mindestens eine Verbindung gemäß allgemeiner Formel (I), (II), (III), (IIB), [A], [B], [C], [D], [E], [F] und/oder pharmakologisch verträgliche Salze davon und mindestens einen pharmakologisch verträglichen Träger, Hilfsstoff oder Lösungsmittel optional zusammen mit einen Wirkstoff ausgewählt aus der Gruppe umfassend Vitamine, Mineralien, Spurenelemente, Peptidase, Cytokine, monoklonale Antikörper und Zonulin.

12. Verfahren zur Herstellung der Peptide oder Peptidomimetika gemäß Anspruch 1 mit m = 0 nach folgendem Syntheseschema: 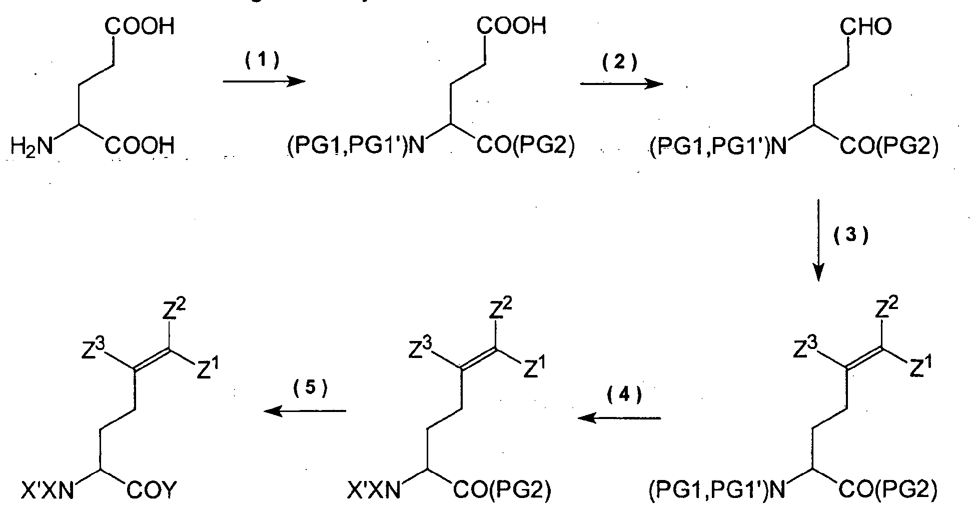
(0) Bereistellung von Glutaminsäure,
(1) versehen der Glutaminsäure am C-Terminus und N-Terminus mit einer Schutzgruppe (PG1 und PG2),
(2) Reduktion der Carboxylfunktion der Seitenkette der Glutaminsäure zum Aldehyd,
(3) Überführung des erhaltenen Aldehyds in eine akzeptorsubstituierte elektrophile Doppelbindung,
(4) Entfernung der Schutzgruppen, und
(5) Verlängerung des C-Terminus und/oder des N-Terminus mit einem Peptidfragment oder Peptidomimetikum.
oder
gemäß folgendem Syntheseschema: 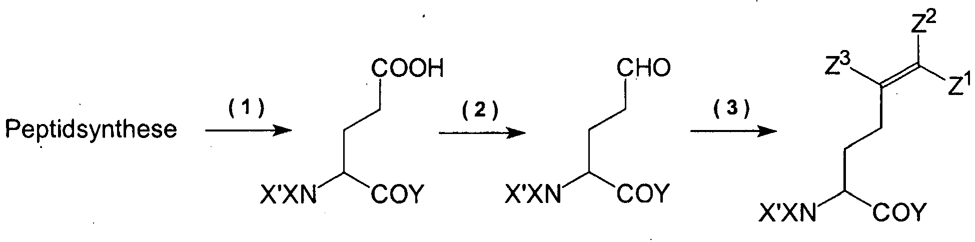
(1) Herstellung eines C-terminal und N-terminal geschützten Peptids oder Peptidomimetikums enthaltend Glutaminsäure,
(2) Reduktion der Carboxylfunktion der Seitenkette der Glutaminsäure zum Aldehyd,
(3) Überführung des erhaltenen Aldehyds in eine akzeptorsubstituierte elektrophile Doppelbindung, und
(4) optional Entfernung der Schutzgruppen.
oder
Verfahren zur Herstellung der Peptide oder Peptidomimetika gemäß Anspruch 1 mit m = 1 nach folgendem Syntheseschema: 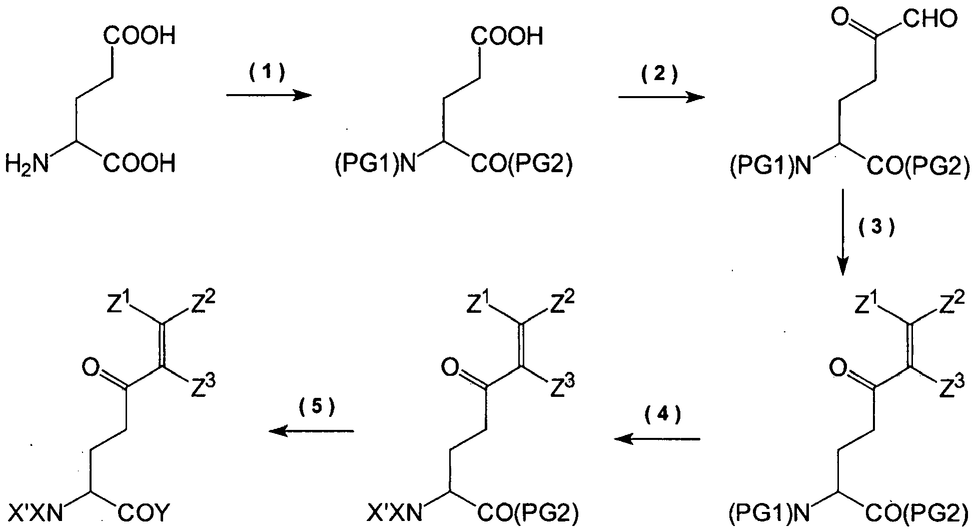
(0) Bereistellung von Glutaminsäure,
(1) versehen der Glutaminsäure am C-Terminus und N-Terminus mit einer Schutzgruppe (PG1 und PG2),
(2) Überführung der Carboxylfunktion der Seitenkette der Glutaminsäure in eine Diketogruppe,
(3) Überführung der endständigen Carbonylfunktion der erhaltenen Diketogruppe in eine akzeptorsubstituierte elektrophile Doppelbindung,
(4) Entfernung der Schutzgruppen, und
(5) Verlängerung des C-Terminus und/oder des N-Terminus mit einem Peptidfragment oder Peptidomimetikum.
oder
gemäß folgendem Syntheseschema: 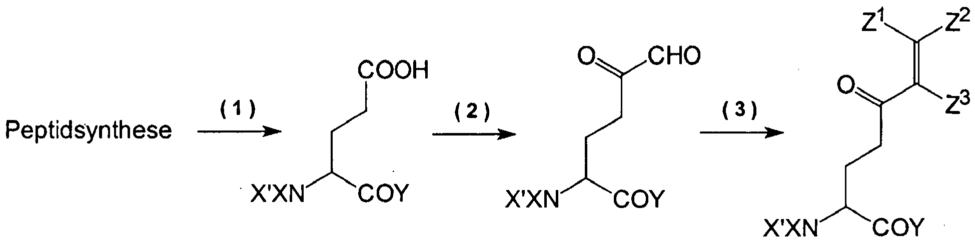
(1) Herstellung eines C-terminal und N-terminal geschützten Peptids oder Peptidomimetikums enthaltend Glutaminsäure,
(2) Überführung der Carboxylfunktion der Seitenkette der Glutaminsäure in eine Diketogruppe,
(3) Überführung der endständigen Carbonylfunktion der erhaltenen Diketogruppe in eine akzeptorsubstituierte elektrophile Doppelbindung, und
(4) optional Entfernung der Schutzgruppen.

## Claims

1. Peptide or peptidomimetic of the following general formula (I), (II) or (III): 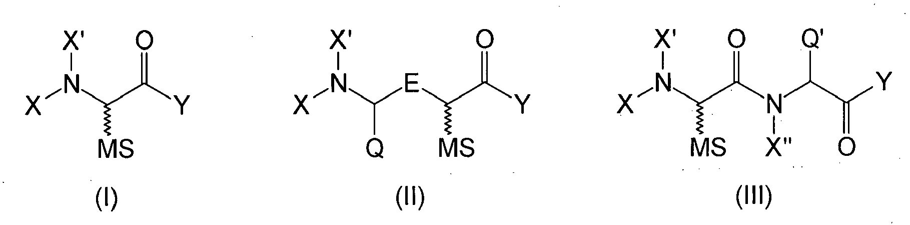 wherein
MS is the acceptor-substituted olefin of the following structure: 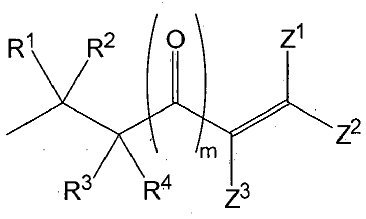 E represents the following group -CH₂-, -CF₂-, -C₂H₄-, -CH₂-CF₂-, -CF₂-CH₂-, -CH=CH-, -CH(OH)-CH₂-, -C(=O)-CH₂-, -CH₂-NH-, -CH₂-O-, -CH(OH)-CH₂-NH-, -P(=O)(OH)-NH-, -P(=O)(OH)-O-, -P(=O)(OH)-S-, -P(=O)(OH)-CH₂-, -CH(OH)-CH₂-NH-, -C(=O)-NH-, -C(=O)-O- or -C(=O)-NX"-;
m is 0 or 1;
the residues **Z¹, Z², Z³** represent independently of each other the following groups: -H, -CO-(C₁-C₆-alkyl), -CO-R⁶, -CO-R⁷, -CO-(C₁-C₆-halogenalkyl), -CO-(C₃-C₁₀-heteroaryl), -CO-(C₆-C₁₅-aryl), -COO-(C₁-C₆-halogenalkyl), -COO-(C₃-C₁₀-heteroaryl), -COO-(C₆-C₁₅-aryl), -COO-(C₁-C₆-alkyl), -COO-R⁸, -COO-R⁹, -CN, -COOH, -CO-NH(C₁-C₆-alkyl), -CO-N(C₁-C₆-alkyl)(C₁-C₆-alkyl), -CO-NR¹⁰R¹¹, -CO-NH₂, -NO₂, -CO-N(CR¹²R¹³R¹⁴)(CR¹⁵R¹⁶R¹⁷), -CS-(C₁-C₆-alkyl), -CS-R¹⁸, -CS-R¹⁹, -CS-O-(C₁-C₆-alkyl), -CS-O-R²⁰, -CS-N(C₁-C₆-alkyl)(C₁-C₆-alkyl), -CS-O-R²¹, -CS-NR²²R²³, -CS-NH₂, -CS-N(CR²⁴R²⁵R²⁶)(CR²⁷R²⁸R²⁹), -SO-R³⁰, -SO-R³¹, -SO₂-R³², -SO₂-R³³, -SO-CR³⁴R³⁵R³⁶, -SO-CR³⁷R³⁸R³⁹, -SO₂-CR⁴⁰R⁴¹R⁴², -SO₂-CR⁴³R⁴⁴R⁴⁵, -SO-N(C₁-C₆-alkyl)(C₁-C₆-alkyl), -SO-NR⁴⁶R⁴⁷, -SO-NH₂, -SO-N(CR⁴⁸R⁴⁹R⁵⁰)(CR⁵¹R⁵²R⁵³), -SO₂-N(C₁-C₆-alkyl)(C₁-C₆₋alkyl), -SO₂-NR⁵⁴R⁵⁵, -SO₂-NH₂, -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, wherein at least one of the residues Z¹, Z², Z³ is different from hydrogen;
the residues **Z¹** and **Z²** together may also represent a residue -CO-O-CO-CH₂-, -CO-O-CH₂-CH₂-,
the residues **Z²** and **Z³** together may also represent a residue -CO-Z'-CH₂-, -CO-O-CH₂-, -CO-CH₂-CH₂, -CO-O-CO- or -CO-NH-CO-, wherein
**Z'** represents one of the following groups: -CH₂-, -CF₂-, -C₂H₄-, -CF₂-CH₂-, -CH₂-CH₂-, -O-, -O-CH₂-, -NH- or -NH-CH₂-;
**Q** and **Q'** represent independently of each other a side chain residue of a natural amino acid; or **Q** forms together with **X'** a propylenyl residue; or **Q'** forms together with **X"** a propylenyl residue;
**Y** represents a hydroxy group, amino group, C₁-C₆-alkylamino group, C₁-C₆-dialkylamino group, C₁-C₆-alkoxy group, C₆-C₁₉-aryloxy group, C₁-C₆-alkyl group, C₁-C₆-halogenalkyl group, C₃-C₁₀-heteroaryl group or a C₆-C₁₅-aryl group; or Y represents a peptide residue of up to 6 amino acids and bound via an amide bond, the C-terminal carbonyl function of said peptide residue carries a hydroxy group, amino group, C₁-C₆-alkylamino group, C₁-C₆-dialkylamino group, C₁-C₆-alkoxy group, C₁-C₆-alkyl group, C₁-C₆-halogenalkyl group, C₃-C₁₀-heteroaryl group or a C₆-C₁₅-aryl group; or Y represents a peptidomimetic residue of up to 60 carbon atoms and
**X"** represents hydrogen or a C₁-C₆-alkyl group; and
**-NXX'** is an amino group, -NH-CHO, C₁-C₁₀-alkylamino group, C₁-C₈-alkyloxycarbonylamino group, C₆-C₁₂-aralkyloxycarbonylamino group, C₁-C₁₀-dialkylamino group, C₂-C₆-nitrogen heterocycle or a C₃-C₅-nitrogen heteroaryl group; or the group -NXX' is part of a peptidomimetic residue of up to 60 carbon atoms
or
**X'** represents hydrogen or a C₁-C₆-alkyl group; and
**X** represents a peptide residue of up to 6 amino acids and bound via an amide bond, the N-terminus of said peptide residue carries an amino group, -NH-CHO, C₁-C₁₀-alkylamino group, C₁-C₈-alkyloxycarbonylamino group C₆-C₁₂-aralkyloxycarbonyl amino group, C₁-C₁₀-dialkylamino group, C₂-C₆-nitrogen heterocycle or a C₃-C₅-nitrogen heteroaryl group;
wherein each of the C₁-C₆-alkoxy groups, C₁-C₆-alkyl groups, C₁-C₁₀-alkylamino groups, C₁-C₈-alkyloxycarbonylamino groups, C₆-C₁₂-aralkyloxycarbonyl amino group, C₁-C₁₀-dialkylamino groups, C₂-C₆-nitrogen heterocycles as well as C₃-C₅-nitrogen heteroaryl groups can be substituted independently of eacht other with up to 5 residues selected from R⁸⁰, R⁸¹, R⁸², R⁸³, R⁸⁴,
**R¹ - R⁴** represent hydrogen,
and the residues **R⁵ - R⁶⁹** and **R⁸⁰ - R⁸⁴** represent independently of each other the following groups:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -CN, -CHO, -COCH₃, -COC₂H₅, -COC₃H₇, -CO-cyclo-C₃H₅, -COCH(CH₃)₂, -COC(CH3)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -NH-CO=NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(-NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃) -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H4-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH=CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH;
and stereoisomeric forms, E/Z isomers, enantiomers, enantiomeric mixtures, diastereomers, diastereomeric mixtures, racemates, tautomers, anomers, keto-enol forms, betaine forms, solvates, hydrates as well as pharmacologically acceptable salts of the aforementioned compounds,
with the exception of the compounds of the general formula (I), wherein X and X' represent hydrogen and Y represents a hydroxy group and the compound H-phenylalanyl-[2-(4-ethoxycarbonyl-3-butene)-glycyl]-leucin-amide.

2. Peptide or peptidomimetic according to claim 1 of the following general formula (IIB) 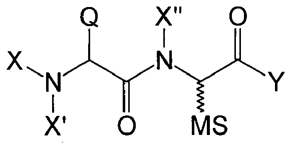 wherein
the residues MS, Q, X, X', X" and Y have the same meaning as in claim 1.

3. Peptide or peptidomimetic according to one of the preceding claims, wherein MS has the following structure: 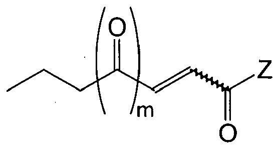 wherein
Z represents a hydroxy group, amino group, C₁-C₆-alkylamino group, C₁-C₆-dialkylamino group, C₁-C₆-alkoxy group, C₁-C₆-alkyl group, C₁-C₆-halogenalkyl group, C₃-C₁₀-heteroaryl group or a C₆-C₁₅-aryl group.

4. Peptide or peptidomimetic according to claim 1 selected from the group consisting of:
N^{α}-benzyloxycarbonyl-{[(E)-(L)-6-amino-hept-2-ene-dicarboxylic acid]-1-ethanoyl}-L-valinyl-L-prolinyl-L-leucine methyl ester (compound 1);
N^{α}-benzyloxycarbonyl-{[(E)-(L)-6-amino-hept-2-ene-dicarboxylic acid]-1-ethanoyl}-L-glutaminyl-L-prolinyl-L-leucine methyl ester (compound 2);
N^{α}-benzyloxycarbonyl-{[(E)-(L)-6-amino-hept-2-ene-dicarboxylic acid]-1-ethanoyl}-L-phenylalanine methyl ester (compound 3);
N^{α}-benzyloxycarbonyl-{[(E)-(L)-6-amino-hept-2-ene-dicarboxylic acid]-1-methanoyl}-L-glutaminyl-L-proline methyl ester (compound 4);
N^{α}-benzyloxycarbonyl-{[(E)-(L)-6-amino-hept-2-ene-dicarboxylic acid]-1-ethanoyl}-L-glutaminyl-L-prolinyl-L-isopentyl amide (compound 5);
[(E)-(L)-6-(2-oxo-pyrrolidon-1-yl)-hept-2-ene-dicarboxylic-acid-1-ethanoyl]-L-valinyl-L-proline methyl ester (compound 6);
N^{α}-acetyl-L-asparaginyl-{[(E)-(L)-6-amino-hept-2-ene dicarboxylic acid]-1-methanoyl}-L-glutamyl-L-alaninyl-L-valine methyl ester (compound 7);
N^{α}-acetyl-L-leucinyl-glycinyl-L-prolinyl-glycinyl-{[(E)-(L)-6-amino-hept-2-ene-dicarboxylic acid]-1-ethanoyl}-L-serinyl-L-leucinyl-L-valinyl-L-isoleucinyl-glycine methyl ester (compound 8);
N^{α}-benzyloxycarbonyl-{[L-7-amino-4-oxo-oct-2-ene-dicarboxylic acid]-1-ethanoyl}-L-valinyl-L-prolinyl-leucine-methyl ester (compound 9);
N^{α}-Acetyl-{[(E)-(L)-6-amino-hept-2-ene-dicarboxylic acid]-1-methanoyl}-L-glutaminyl-L-glutamyl-L-alanine methyl ester (compound 10);
N^{α}-acetyl-{[(E)-(L)-6-amino-hept-2-ene-dicarboxylic acid]-1-methanoyl}-L-glutaminyl-L-glutamyl methyl ester (compound 11);
N^{α}-acetyl-{[(E)-(L)-6-Amino-hept-2-ene-dicarboxylic acid]-1-methanoyl}-L-phenylalaninyl-L-prolinyl-L-leucine methyl ester (compound 12);
N^{α}-benzyloxycarbonyl-{[(E)-(L)-6-amino-hept-2-ene dicarboxylic acid]-1-methanoyl}-L-phenylalaninyl-L-prolinyl-L-leucine methyl ester (compound 13);
N^{α}-acetyl-{[(E)-(L)-6-amino-hept-2-ene-dicarboxylic acid]-1-methanoyl}-L-(p-fluoro)-phenylalaninyl-L-prolinyl-L-leucine methyl ester (compound 14);
N^{α}-benzyloxycarbonyl-{[(E)-(L)-6-amino-hept-2-ene-dicarboxylic acid]-1-methanoyl}-L-(p-fluoro)-phenylalaninyl-L-prolinyl-L-leucine methyl ester (compound 15);
[(E)-(L)-6-(2-oxo-pyrrolidon-1-yl)-hept-2-ene-dicarboxylic acid 1-ethanoyl]-L-valinyl-L-homoprolinyl-L-leucine methyl ester (compound 16);
[(E)-(L)-6-(2-oxo-pyrrolidon-1-yl)-hept-2-ene-dicarboxylic acid-1-ethanoyl]-L-cyclohexylglycinyl-L-homoprolinyl-L-leucine methyl ester (compound 17);
[(E)-(L)-6-(2-oxo-pyrrolidon-1-yl)-hept-2-ene-dicarboxylic acid-1-ethanoyl]-L-cyclohexylglycinyl-L-prolinyl-L-leucine methyl ester (compound 18);
N^{α}-benzyloxycarbonyl-{[(E)-(L)-6-amino-hept-2-ene-dicarboxylic acid]-1-ethanol}-L-glutaminyl-L-proline-L-tyrosine methyl ester (compound 19);
N^{α}-benzyloxycarbonyl-{[(E)-(L)-6-amino-hept-2-ene-dicarboxylic acid]-1-ethanoyl}-L-glutaminyl-L-prolinyl-L-leucine methyl ester (compound 20);
N^{α}-benzyloxycarbonyl-{[(E)-(L)-6-amino-hept-2-ene-dicarboxylic acid]-1-ethanoyl}-L-leucinyl-L-prolinyl-L-glutamine methyl ester (compound 21);
N^{α}-acetyl-{[L-7-Amino-4-oxo-oct-2-ene-dicarboxylic acid]-1-ethanoyl}-L-glutaminyl-L-prolinyl-L-leucine methyl ester (compound 22);
N^{α}-(5-methylisoxazol-3-carbonyl)-{[L-7-amino-4-oxo-oct-2-ene-dicarboxylic acid]-1-isopropanoyl}-L-valinyl-L-prolinyl-leucine methyl ester (compound 23);
N^{α}-(2-fluorobenzoyl)-{[L-7-amino-4-oxo-oct-2-ene-dicarboxylic acid]-1-methanoyl}-L-valinyl-L-4-fluoroprolinyl-leucine isopropyl ester (compound 24);
N^{α}-benzyloxycarbonyl-{[(E)-(L)-6-amino-hept-2-ene-dicarboxylic acid]-1-ethanoyl}-L-phenylalaninyl-L-prolinyl-L-leucine methyl ester (compound 25);
N^{α}-benzyloxycarbonyl-{[(E)-(L)-6-amino-hept-2-ene-dicarboxylic acid]-1-ethanoyl}-glycinyl-L-prolinyl-L-leucine methyl ester (compound 26);
N^{α}-benzyloxycarbonyl-{[(E)-(L)-6-amino-hept-2-ene-dicarboxylic acid]-1-ethanoyl}-L-alaninyl-L-prolinyl-L-leucine methyl ester (compound 27);
N^{α}-tert. butyloxycarbonyl-{[(E)-(L)-6-amino-hept-2-ene-dicarboxylic acid]-1-ethanoyl}-L-glutaminyl-L-prolinyl-L-leucine methyl ester (compound 28);
N^{α}-thiophene-2-carbonyl-{[(E)-(L)-6-amino-hept-2-ene-dicarboxylic acid]-1-ethanoyl}-L-glutaminyl-L-prolinyl-L-leucine methyl ester (compound 29);
N^{α}-furan-3-carbonyl-{[(E)-(L)-6-amino-hept-2-ene-dicarboxylic acid]-1-ethanoyl}-L-glutaminyl-L-prolinyl-L-leucine methyl ester (compound 30);
N^{α}-isoxazole-5-carbonyl-{[(E)-(L)-6-amino-hept-2-ene-dicarboxylic acid]-1-ethanoyl}-L-glutaminyl-L-prolinyl-L-leucine methyl ester (compound 31);
N^{α}-(5-methyl-isoxazole-3-carbonyl)-{[(E)-(L)-6-amino-hept-2-ene-dicarboxylic acid]-1-ethanoyl}-L-glutaminyl-L-prolinyl-L-leucine methyl ester (compound 33);
N^{α}-(trans-3-(3-thienyl) acryloyl)-{[(E)-(L)-6-amino-hept-2-ene-dicarboxylic acid]-1-ethanoyl}-L-glutaminyl-L-prolinyl-L-leucine methyl ester (compound 34);
N^{α}-acetyl-{[(E)-(L)-6-amino-hept-2-ene-dicarboxylic acid]-1-ethanoyl}-L-glutaminyl-L-prolinyl-L-leucine methyl ester (compound 35);
N^{α}-(4-trifluoromethoxy-benzenesulfonyl)-{[(E)-(L)-6-amino-hept-2-ene-dicarboxylic acid]-1-ethanoyl}-L-glutaminyl-L-prolinyl-L-leucine methyl ester (compound 36);
N^{α}-benzyloxycarbonyl)-{[(E)-(L)-6-amino-hept-2-ene-dicarboxylic acid]-1-ethanoyl}-L-cyclohexyl glycine-L-prolinyl-L-leucine methyl ester (compound 37);
N^{α}-benzyloxycarbonyl)-{[(E)-(L)-6-amino-hept-2-ene-dicarboxylic acid]-1-ethanoyl}-L-valinyl-L-homoprolinyl-L-leucin methyl ester (compound 38);
(E)-(S)-6-benzyloxycarbonyl amino-6-[3-((R)-2-phenylcarbamoyl-pyrrolidine-1-carbonyl)-phenylcarbamoyl]-hex-2-ene acid ethyl ester (compound 39);
(E)-(S)-6- benzyloxycarbonyl amino-6-{1-[(S)-3-carboxy-1-(3-methyl-butylcarbamoyl)-propyl]-2-oxo-1,2-dihydropyridine-3-ylcarbamoyl}-hex-2-enoyl acid isopropyl ester (compound 40);
N^{α}-benzyloxycarbonyl)-{[(E)-(L)-2-amino-6-methansulfonyl]-hex-5-enyl}-L-glutaminyl-L-prolinyl-L-leucine methyl ester (compound 3.2.1);
N^{α}-benzyloxycarbonyl)-[(E)-(L)-2-amino-6-dimethylsulfamoyl)-hex-5-enyl]-L-glutaminyl-L-prolinyl-L-leucine methyl ester (compound 3.2.2);
N^{α}-benzyloxycarbonyl)-[(L)-2-amino-4-(3-oxo-cyclopent-1-enyl]-butyryl-L-glutaminyl-L-prolinyl-L-leucine methyl ester (compound 3.2.3);
N^{α}-benzyloxycarbonyl)-[(L)-2-amino-5-(2-oxo-dihydrofurane-(3E)-ylidene)]-pentanoyl-L-glutaminyl-L-prolinyl-L-leucine methyl ester (compound 3.2.4);
N^{α}-benzyloxycarbonyl)-[(E)-(L)-6-amino-hept-2-ene-dicarboxylic acid]-L-glutaminyl-L-prolinyl-L-leucine methyl ester (compound 3.2.5);
N^{α}-acetyl-{[(E)-(L)-6-amino-hept-2-ene-dicarboxylic acid]-1-pentylamido}-L-glutaminyl-L-asparatyl-L-proline methyl ester (compound 3.2.6);
N^{α}-benzyloxycarbonyl)-{[(E)-(L)-6-amino-hept-2-ene-dicarboxylic acid]-1-isopropanoyl}-L-(p-fluoro-phenylalaninyl)-L-proline (compound 3.2.7);
N^{α}-benzyloxycarbonyl)-{[(E)-(L)-6-amino-hept-2-ene-dicarboxylic acid]-1-benzoyl}-L-phenylalaninyl-L-homoprolinyl-L-leucinyl amide (compound 3.2.8);
N^{α}-benzyloxycarbonyl)-{[(E)-(L)-7-amino-2-oxo-oct-3-ene-dicarboxylic acid]-1-ethanoyl}-L-phenylalanine methyl ester (compound 3.2.9);
N^{α}-benzyloxycarbonyl)-[(Z)-(L)-2-amino-7-oxo-oct-5-ene- carboxylic acid]-L-glutaminyl-L-prolinyl-L-leucine methyl ester (compound 3.2.10);
N^{α}-benzyloxycarbonyl)-[(Z)-(L)-2-amino-6-cyano-hex-5-ene- carboxylic acid]-L-glutaminyl-L-prolinyl-L-leucine methyl ester (compound 3.2.11);
N^{α}-benzyloxycarbonyl)-{[(E)-(L)-6-amino-hept-2-ene-dicarboxylic acid]-1-ethanoyl}-L-valinyl-L-(octahydroindol-2-carboxyl)-L-leucinyl amide (compound 4.1);
N^{α}-(piperidinyl-4-carbonyl)-{[(E)-(L)-2-amino-6-phenylsulfonyl]-hex-5-enyl}-L-phenylalaninyl-L-prolinyl-L-1-cyclopentylmethyl-2-oxo-2-(1H-tetrazole-5-yl)-ethyl amide (compound 4.2);
N^{α}-benzyloxycarbonyl)-[(E)-(L)-2-amino-6-benzyloxysulfonyl-hex-5-enyl]-L-valinyl-L-prolinylbenzyl sulfonamide (compound 4.3);
(E)-(S)-6-[(S)-1-((S)-2-ethylcarbamoyl pyrrolidine-1-carbonyl)-2-methyl-propylcarbamoyl]-6-(2-piperidine-4-yl-ethylamino)-hex-2-ene-carboxylic acid isopropyl ester (compound 5.1);
(S)-2-[((S)-1-{(E)-2R,5S)-2-(4-fluorobenzyl)-9-methansulfonyl-5-[(5-methyl-isoxazole-3-carbonyl)-amino]-4-oxo-non-8-enoyl}pyrrolidine-2-carbonyl)-amino]-4-methyl-valeric acid methyl ester (compound 5.3.b);
(E)-(6R,9S)-9-benzyloxycarbonylamino-6-[2-(2-ethylcarbamoyl-octahydroindole-1-yl)-1-methyl-2-oxoethylcarbamoyl]-8-oxo-10-phenyl-dec-2-enyl acid-isopropyl ester (compound 5.4);
Piperidine-4-carbonyl-((E)-(S)-5-benzylsulfonyl-1-{2-[2-((S)-2-benzylsulfonylaminocarbonyl-octahydro-indole-1-yl)-2-oxo-ethylamino]-acetyl}-4-enyl) amide (compound 5.6);
(E)-5-(N'-acetyl-N-carboxy-hydrazino)-[pent-2-enoyl)-1-ethanoly]-L-valinyl-L-prolinyl-L-leucine methyl ester (compound 5.7);

5. Compound according to one of the claims 1 - 4 for use in medicine.

6. Use of a compound of the general structure [TGI1] for the manufacture of a pharmaceutical for the treatment or prevention of celiac disease, fibroses, thrombosis, neurodegenerative diseases, Huntington's chorea, Parkinson's disease, Alzheimer's disease, cataract, acne, psoriasis or candidiasis:
acceptor-substituted double bond-(CO)ₘ-C₂H₄-backbone [TGI1]
wherein m is 0 or 1 and
the acceptor-substituted double bond carries at least one electron-withdrawing residue capable to conjugate from the group of C=O, C=S, C=N, P=O, P=S, N=O, S=O, N=N, C=N with an electronegativity ≥ 2.20 and
the backbone has at least one amide bond and the peptidic or peptide-like backbone consists of 2 to 20 amino acids or mimetics for amino acids, which are linked to each other via peptide bonds, ester bonds, carbonate bonds, urethane bonds, carbamate bonds and/or urea bonds and the backbone carrying the Michael system has at least one carbonyl group vicinal to the carbon atom, which carries the side chain with the Michael system.

7. Use of a compound according to claim 6 of the general structure [A]: 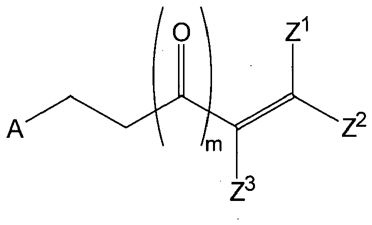 wherein the compound has at least one acceptor-substituted olefin with the residues Z¹, Z² and Z³, which is bound via an ethylene group or via a carbonylethylene group to an at least secondary substituted group A, wherein
**A** represents a peptide residue of 2 to 20 amino acids or a peptidomimetic residue of 2 to 20 amino acids, wherein the amino acids in the peptide residue also comprise non-proteinogenic amico acids selected from thiocarbonyl amino acids, β-amino acids, γ-amino acids, δ-amino acids, 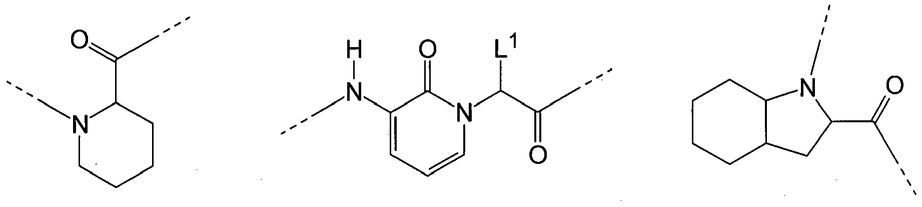 and 1 to 3 amino acid mimetics selected from 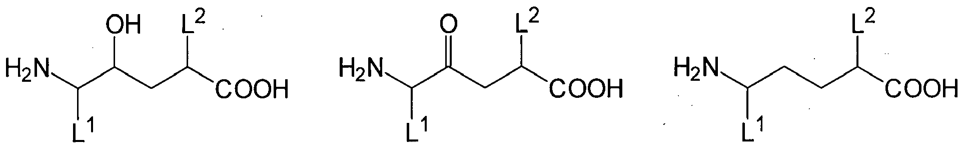 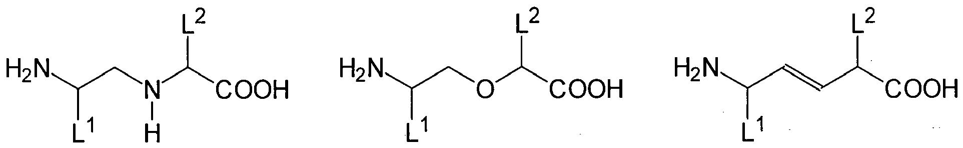 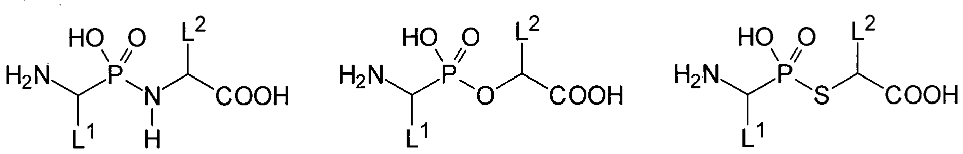 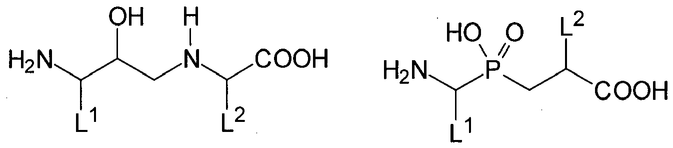 can be contained in the peptidomimetic residue, wherein L¹ and L² represent independently of each other a side chain residue of a natural amino acid or a residue -R⁵⁷, -R⁵⁹, -R⁶⁰, -R⁶¹, -CR⁶²R⁶³R⁶⁴, -CR⁶⁵R⁶⁶-CR⁶⁷R⁶⁸R⁶⁹, -CR⁷⁰R⁷¹-CR⁷²R⁷³-CR⁷⁴R⁷⁵R⁷⁶;
**m** is 0 or 1;
the residues **Z¹**, **Z²**, **Z³** represent independently of each the following groups: -H, -CO-(C₁-C₆-alkyl), -CO-R⁶, -CO-R⁷, -CO-(C₁-C₆-halogenalkyl), -CO-(C₃-C₁₀-heteroaryl), -CO-(C₆-C₁₅-aryl), -COO-(C₁-C₆-halogenalkyl), -COO-(C₃-C₁₀-heteroaryl), -COO-(C₆-C₁₅-aryl), -COO-(C₁-C₆-alkyl), -COO-R⁸, -COO-R⁹, -CN, -F, -Cl, -Br, -COOH, -CO-NH(C₁-C₆-alkyl), -CO-N(C₁-C₆-alkyl)(C₁-C₆-alkyl), -CO-NR¹⁰R¹¹, -CO-NH₂, -CO-N(CR¹²R¹³R¹⁴)(CR¹⁵R¹⁶R¹⁷), -CF₃, -OCF₃, -NO₂, -CS-(C₁-C₆-alkyl), -CS-R¹⁸, -CS-R¹⁹, -CS-O-(C₁-C₆-alkyl), -CS-O-R²⁰, -CS-N(C₁-C₆-alkyl)(C₁-C₆-alkyl), -CS-O-R²¹, -CS-NR²²R²³, -CS-NH₂, -CS-N(CR²⁴R²⁵R²⁶)(CR²⁷R²⁸R²⁹), -SO-R³⁰, -SO-R³¹, -SO₂-R³², -SO₂-R³³ -SO-CR³⁴R³⁵R³⁶, -SO-CR³⁷R³⁸R³⁹, -SO₂-CR⁴⁰R⁴¹R⁴², -SO₂-CR⁴³R⁴⁴R⁴⁵, -SO-N(C₁-C₆-alkyl)(C₁-C₆-alkyl), -SO-NR⁴⁶R⁴⁷, -SO-NH₂, -SO-N(CR⁴⁸R⁴⁹R⁵⁰)(CR⁵¹R⁵²R⁵³), -SO₂-N(C₁-C₆-alkyl)(C₁-C₆-alkyl), -SO₂-NR⁵⁴R⁵⁵, -SO₂-NH₂, -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, wherein at least one of the residues Z¹, Z², Z³ is different from hydrogen;
the residues **Z¹** and **Z²** together may also represent a residue -CO-O-CO-CH₂-, -CO-O-CH₂-CH₂-,
the residues **Z²** and **Z³** together may also represent a residue -CO-Z'-CH₂-, -CO-O-CH₂-, -CO-CH₂-CH₂-, -CO-O-CO- or -CO-NH-CO, wherein
**Z'** represents one of the following groups: -CH₂-, -CF₂-, -C₂H₄-, -CF₂-CH₂-, -CH₂-CH₂-, -O-, -O-CH₂-, -NH- or -NH-CH₂-;
the residues **R⁶ - R⁷⁶** represent independently of each other the following groups: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -CN, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH2, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆=CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, =C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, ;
and stereoisomeric forms, E/Z isomers, enantiomers, enantiomeric mixtures, diastereomers, diastereomeric mixtures, racemates, tautomers, anomers, ketoenol forms, betaine forms, solvates, hydrates as well as pharmacologically acceptable salts of the aforementioned compounds.

8. Use according to claim 7, wherein the compound has the following general structure [B]: 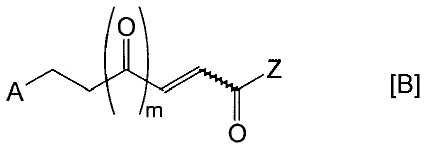 wherein
**Z** represents a hydroxy group, amino group, C₁-C₆-alkylamino group, C₁-C₆-dialkylamino group, C₁-C₆-alkoxy group, C₁-C₆-alkyl group, C₁-C₆-halogenalkyl group, C₃-C₁₀-heteroaryl group or a C₆-C₁₅-aryl group and
**m** is 0 or 1, and
**A** represents a peptide residue, a peptide derivative or a peptidomimetic residue.

9. Use according to claim 8, wherein the compound has the following general structure [C], [D], [E] or [F]:

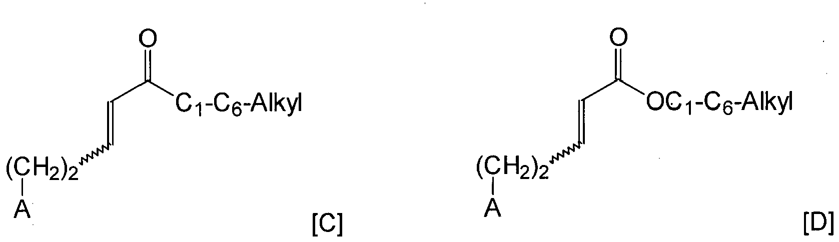

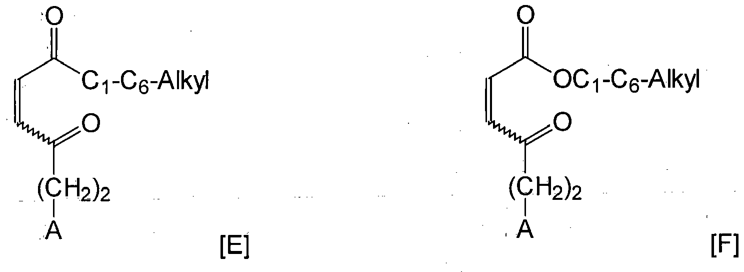

10. Use according to any one of the claims 7 - 9, wherein the compound has one of the following general formulas (I), (II) or (III): 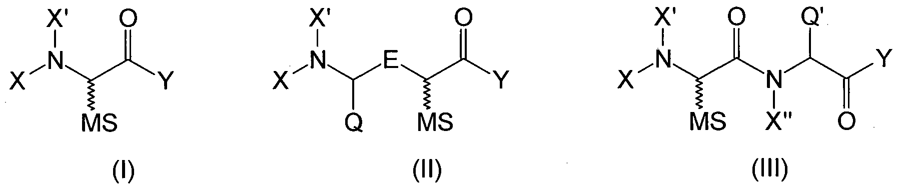 wherein
MS is the Michael system of the following structure 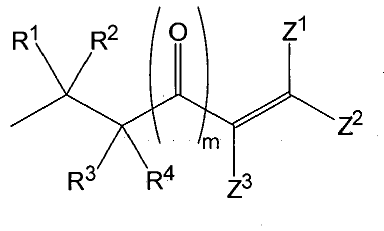 and wherein m, E, R¹, R², R³, R⁴, Q, Q', X, X', X", Y, Z¹, Z² and Z³ have the same meaning as in claim 1.

11. Pharmaceutical composition comprising at least one compound according to the general formula (I), (II), (III), (IIB), [A], [B], [C], [D], [E], [F] and/ or pharmacologically acceptable salts thereof and at least one pharmacologically acceptable carrier, excipient or solvent, optionally together with an active agent selected from the group comprising vitamins, minerals, trace elements, peptidase, cytokines, monoclonal antibodies and zonulin.

12. Method for the preparation of the peptides or peptidomimetics according to claim 1 with m= 0 according to the following synthesis scheme: 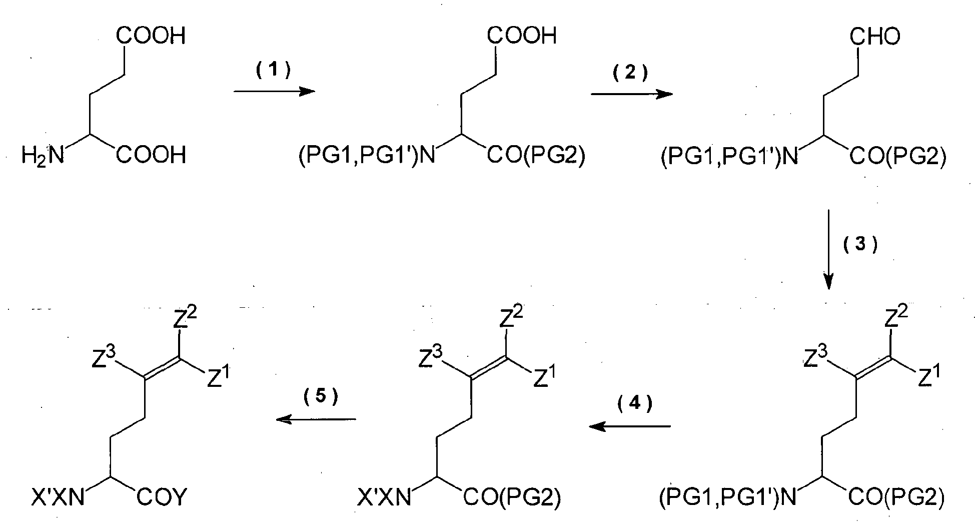
(0) Provision of glutamic acid,
(1) Attaching a protective group (PG1 and PG2) at the C-terminus and N-terminus of the glutamic acid,
(2) Reduction of the carboxylic function of the side chain of the glutamic acid to the aldehyde,
(3) Conversion of the resulting aldehyde to an acceptor-substituted electrophilic double bond,
(4) Removal of the protective groups, and
(5) Extension of the C-terminus and/or of the N-terminus with a peptide fragment or a peptidomimetic.
or according to the following synthesis scheme: 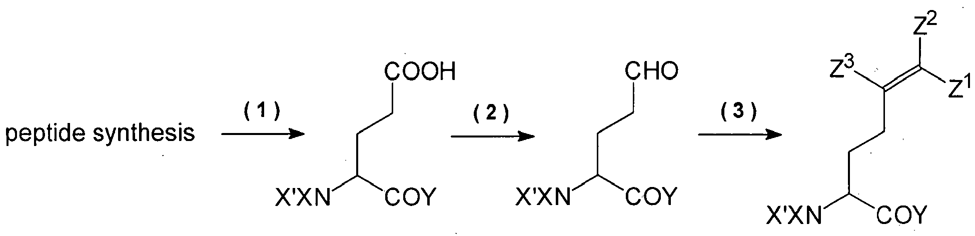
(1) Preparation of a C-terminal and N-terminal protected peptide or peptidomimetic containing glutamic acid,
(2) Reduction of the carboxylic function of the side chain of the glutamic acid to the aldehyde,
(3) Conversion of the resulting aldehyde to an acceptor-substituted electrophilic double bond, and
(4) Optional removal of the protective groups
or
method for the preparation of the peptides or peptidomimetics according to claim 1 with m= 1 according to the following synthesis scheme: 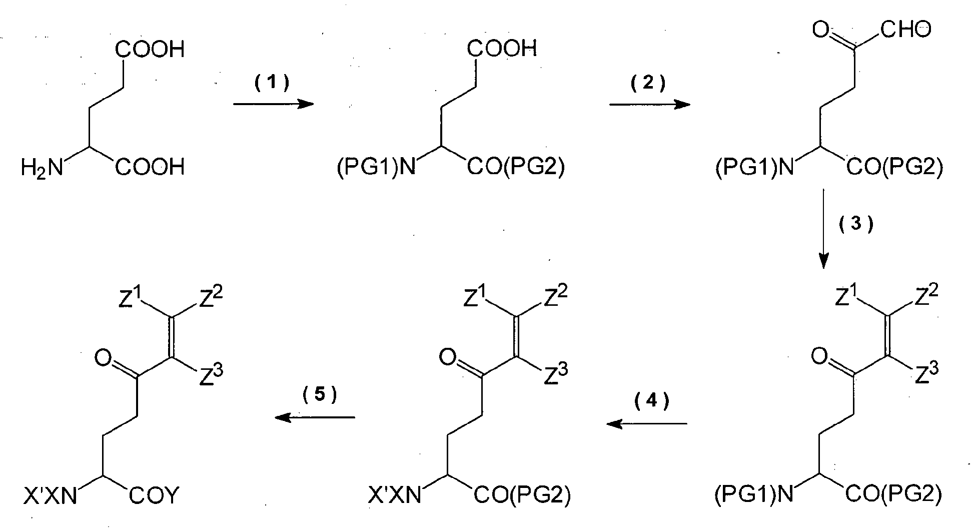
(0) Provision of glutamic acid,
(1) Attaching a protective group (PG1 and PG2) at the C-terminus and N-terminus of the glutamic acid,
(2) Conversion of the carboxylic function of the side chain of the glutamic acid to a diketo group,
(3) Conversion of the terminal carbonyl function of the resulting diketo group to an acceptor-substituted electrophilic double bond
(4) Removal of the protective groups, and
(5) Extension of the C-terminus and/or the N-terminus with a peptide fragment or a peptidomimetic.
or
according to the following synthesis scheme: 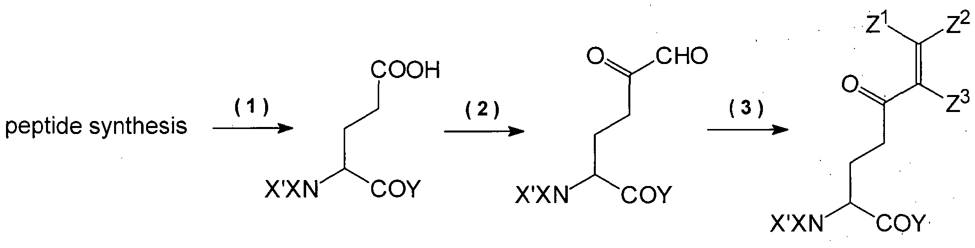
(1) Preparation of a C-terminal and N-terminal protected peptide or peptidomimetic containing glutamic acid,
(2) Conversion of the carboxylic function of the side chain of the glutamic acid to a diketo group,
(3) Conversion of the terminal carbonyl function of the resulting diketo group to an acceptor-substituted electrophilic double bond, and
(4) Optional removal of the protective groups.

## Revendications

1. Un peptide ou un peptidomimétique de formule générale suivante (I), (II) ou (III): 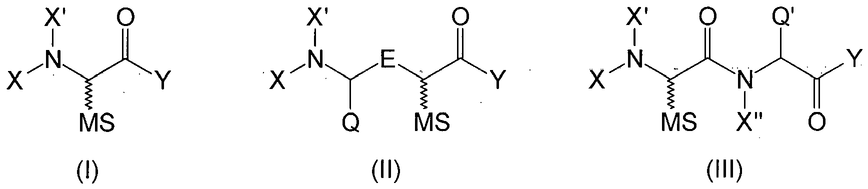 où
MS est une oléfine substituée par un accepteur ayant la structure suivante: 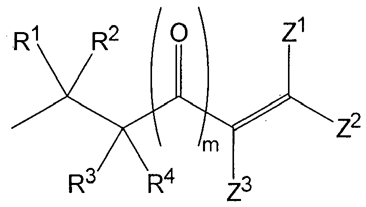 E représente le groupement suivant -CH₂-, -CF₂-, -C₂H₄-, -CH₂-CF₂-, -CF₂-CH₂-, -CH=CH-, -CH(OH)-CH₂-, -C(=O)-CH₂-, -CH₂-NH-, -CH₂-O-, -CH(OH)-CH₂-NH-, -P(=O)(OH)-NH-, -P(=O)(OH)-O-, -P(=O)(OH)-S-, -P(=O)(OH)-CH₂-, -CH(OH)-CH₂-NH-, -C(=O)-NH-, -C(=O)-O- ou -C(=O)-NX"-;
m est 0 ou 1;
les résidus Z¹, Z², Z³ sont indépendamment l'un de l'autre les groupements suivants: -H, -CO-(C₁-C₆-alkyle), -CO-R⁶, -CO-R⁷, -CO-(C₁-C₆-halogénoalkyle), -CO-(C₃-C₁₀-hétéroaryle), -CO-(C₆-C₁₅-aryle), -COO-(C₁-C₆-halogénoalkyle), -COO-(C₃-C₁₀-hétéroaryle), -COO-(C₆-C₁₅-aryle), -COO-(C₁-C₆-alkyle), -COO-R⁸, -COO-R⁹, -CN, -COOH, -CO-NH(C₁-C₆-alkyle), -CO-N(C₁-C₆-alkyle)(C₁-C₆-alkyle), -CO-NR¹⁰R¹¹, -CO-NH₂, -NO₂, -CO-N(CR¹²R¹³R¹⁴)(CR¹⁵R¹⁶R¹⁷), -CS-(C₁-C₆-alkyle), -CS-R¹⁸, -CS-R¹⁹, -CS-O-(C₁-C₆-alkyle), -CS-O-R²⁰, -CS-N(C₁-C₆-alkyle)(C₁-C₆-alkyle), -CS-O-R²¹, -CS-NR²²R²³, -CS-NH₂, -CS-N(CR²⁴R²⁵R²⁶)(CR²⁷R²⁸R²⁹), -SO-R³⁰, -SO-R³¹, -SO₂-R³², -SO₂-R³³ -SO-CR³⁴R³⁵R³⁶, -SO-CR³⁷R³⁸R³⁹, -SO₂-CR⁴⁰R⁴¹R⁴², -SO₂-CR⁴³R⁴⁴R⁴⁵, -SO-N(C₁-C₆-alkyle)(C₁-C₆-alkyle), -SO-NR⁴⁶R⁴⁷, -SO-NH₂, -SO-N(CR⁴⁸R⁴⁹R⁵⁰)(CR⁵¹R⁵²R⁵³), -SO₂-N(C₁-C₆-alkyle)(C₁-C₆-alkyle), -SO₂-NR⁵⁴R⁵⁵ -SO₂-NH₂, -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, où au moins un des résidus **Z¹**, **Z²**, **Z³** est différent de l'hydrogène;
les résidus **Z¹** et **Z²** peuvent aussi former ensemble un résidu -CO-O-CO-CH₂-, -CO-O-CH₂-CH₂-,
les résidus **Z²** et **Z³** peuvent aussi former ensemble un résidu -CO-Z'-CH₂-, -CO-O-CH₂-, -CO-CH₂-CH₂-, -CO-O-CO- ou -CO-NH-CO-, où
**Z'** est un des groupements suivants: -CH₂-, -CF₂-, -C₂H₄-, -CF₂-CH₂-, -CH₂-CH₂-, -O-, -O-CH₂-, -NH- ou -NH-CH₂-;
**Q** et **Q'** représentent indépendamment l'un de l'autre une chaîne latérale d'un acide aminé naturel, ou **Q** et **X'** forment ensemble un résidu propylènyle, ou **Q'** et **X"** forment ensemble un résidu propylènyle;
**Y** est un groupement hydroxyle, un groupement amine, un groupement C₁-C₆-alkylamine, un groupement C₁-C₆-dialkylamine, un groupement C₁-C₆-alcoxy, un groupement C₆-C₁₉-aryloxy, un groupement C₁-C₆-alkyle, un groupement C₁-C₆-halogénoalkyle, un groupement C₃-C₁₀-hétéroaryle ou un groupement C₆-C₁₅-aryle ou **Y** est un résidu peptidique connecté par liaison amide et ayant jusqu'à 6 acides aminés, dont la fonction carbonyle C-términale a un groupement hydroxyle, un groupement amine, un groupement C₁-C₆-alkylamine, un groupement C₁-C₆-dialkylamine, un groupement C₁-C₆-alcoxy, un groupement C₁-C₆-alkyle, un groupement C₁-C₆-halogénoalkyle, un groupement C₃-C₁₀-hétéroaryle ou un groupement C₆-C₁₅-aryle ou **Y** est un résidu peptidomimétique ayant jusqu'à 60 atomes de carbone, et
**X"** est un hydrogène ou un groupement C₁-C₆-alkyle; et
-**NXX'** est un groupement amine, -NH-CHO, un groupement C₁-C₁₀-alkylamine, un groupement C₁-C₈-alkyloxycarbonylamine, un groupement C₆-C₁₂-aralkyloxycarbonylamine, un groupement C₁-C₁₀-dialkylamine, un C₂-C₆-N-hétérocycle ou un groupement C₃-C₅-N-hétéroaryle ou le groupement -**NXX'** est une partie d'un résidu peptidomimétique ayant jusqu'à 60 atomes de carbone
ou
**X'** est un hydrogène ou un groupement C₁-C₆-alkyle; et
**X** est un résidu peptidique connecté par liaison amide et ayant jusqu'à 6 acides aminés, dont le *N*-terminus est un groupement amine, -NH-CHO, un groupement C₁-C₁₀-alkylamine, un groupement C₁-C₈-alkyloxycarbonylamine, un groupement C₆-C₁₂-aralkyloxycarbonylamine, un groupement C₁-C₁₀-dialkylamine, un C₂-C₆-N-hétérocycle ou un groupement C₃-C₅-N-hétéroaryl;
où chacun des groupements C₁-C₆-alcoxy, C₁-C₆-alkyle, C₁-C₁₀-alkylamine, C₁-C₈-alkyloxycarbonylamine, C₆-C₁₂-aralkyloxycarbonylamine, C₁-C₁₀-dialkylamine, des C₂-C₆-N-hétérocycles, et les groupements C₃-C₅-N-hétéroaryle peut être indépendamment de l'autre substitué par jusqu'à 5 résidus sélectionnés à partir de R⁸⁰, R⁸¹, R⁸², R⁸³, R⁸⁴,
**R¹ - R⁴** représentent un hydrogène,
et les résidus **R⁵ - R⁶⁹** et **R⁸⁰ - R⁸⁴** représentent indépendamment l'un de l'autre un des groupements suivants:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -CI, -Br, -I, -CN, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NN[CH(CH₃)_{2]}, -CF₂Cl, -NH-C(=NH)-NH[C(CH₃)₃], -NH-C(=NH)-N(CH₃)₂, -NH-C(=NH)-N(C₂H₅)₂, -NH-C(=NH)₋N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)_{3]}, -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -C=CH, -C=C-CH₃, -CH₂-C=CH, -C₂H₄-C=CH, -CH₂-C=C-CH₃, -C=C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH ;
ainsi que des formes stéréoisomériques, des isomères E/Z, des enantiomères, des mélanges énantiomériques, des diastéréoisomères, des mélanges diastéréoisomériques, des racémates, des tautomères, des anomères, des formes céto-énoliques, des formes bétainiques, des solvates, des hydrates, ainsi que des sels pharmaceutiquement acceptables des composés précités,
à l'exception des composés de formule générale (I), où **X** et **X**' représentent un hydrogène et **Y** est un groupement hydroxyle, ainsi que le composé H-phénylalanyl-[2-(4-éthoxycarbonyl-3-butèn)-glycyl]-léucin-amide.

2. Le peptide ou le peptidomimétique selon la revendication 1, de formule générale (IIB) 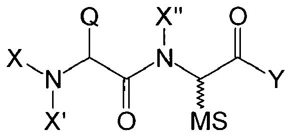 où
les résidus MS, Q, X, X', X" et Y ont les significations précitées dans la revendication 1.

3. Le peptide ou le peptidomimétique selon une des revendications précédentes, où MS a la structure suivante : 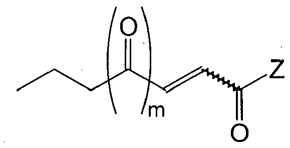 où
**Z** est un groupement hydroxyle, un groupement amine, un groupement 3C₁-C₆-alkylamine, un groupement C₁-C₆-dialkylamine, un groupement C₁-C₆-alcoxy, un groupement C₁-C₆-alkyle, un groupement C₁-C₆-halogénoalkyle, un groupement C₃-C₁₀-hétéroaryle ou un groupement C₆-C₁₅-aryle.

4. Le peptide ou le peptidomimétique selon la revendication 1 sélectionné à partir du groupe consistant en:
N^{α}-Benzyloxycarbonyl-{[(E)-(L)-6-amino-hept-2-ène-acide dicarboxylique]-1-éthanoyl}-L-valinyl-L-prolinyl-L-léucin-méthyl-ester (composé 1);
N^{α}-Benzyloxycarbonyl-{[(E)-(L)-6-amino-hept-2-ène-acide dicarboxilique]-1-éthanoyl}-L-glutaminyl-L-prolinyl-L-léucin-méthyl-ester (composé 2);
N^{α}-Benzyloxycarbonyl-{[(E)-(L)-6-amino-hept-2-ène-acide dicarboxylique]-1-éthanoyl}-L-phénylalanin-méthyl-ester (composé 3);
N^{α}-Benzyloxycarbonyl-{[(E)-(L)-6-amino-hept-2-ène- acide dicarboxylique]-1-méthanoyl}-L-glutaminyl-L-prolin-méthyl-ester (composé 4);
N^{α}-Benzyloxycarbonyl-{[(E)-(L)-6-amino-hept-2-ène-acide dicarboxylique]-1-éthanoyl}-L-glutaminyl-L-prolinyl-isopéntylamide (composé 5);
[(E)-(L)-6-(2-Oxo-pyrrolidon-1-yl)-hept-2-ène acide dicarboxylique-1-éthanoyl]-L-valinyl-L-prolin-méthyl-ester (composé 6);
N^{α}-Acétyl-L-asparaginyl-{[(E)-(L)-6-Amino-hept-2-ène-acide dicarboxylique]-1-méthanoyl}-L-glutamyl-L-alaninyl-L-valin-méthyl-ester (composé 7);
N^{α}-Acétyl-L-léucinyl-glycinyl-L-prolinyl-glycinyl-{[(E)-(L)-6-amino-hept-2-éne-acide dicarboxylique]-1-éthanoyl}-L-sérinyl-L-léucinyl-L-valinyl-L-isoléucinyl-glycin-méthyl-ester (composé 8);
N^{α}-Benzyloxycarbonyl-{[L-7-amino-4-oxo-oct-2-ène- acide dicarboxylique]-1-éthanoyl}-L-valinyl-L-prolinyl-léucin-méthyl-ester (composé 9);
N^{α}-Acétyl-{[(E)-(L)-6-amino-hept-2-ène-acide dicarboxylique]-1-méthanoyl}-L-glutaminyl-L-glutamyl-L-alanin-méthyl-ester (composé 10);
N^{α}-Acétyl-{[(E)-(L)-6-amino-hept-2-ène- acide dicarboxylique]-1-méthanoyl}-L-glutaminyl-L-glutamyl-méthyl-ester (composé 11);
N^{α}-Acétyl-{[(E)-(L)-6-amino-hept-2-ène- acide dicarboxylique]-1-méthanoyl}-L-phénylalaninyl-L-prolinyl-L-léucin-méthyl-ester (composé 12);
N^{α}-Benzyloxycarbonyl-{[(E)-(L)-6-amino-hept-2-ène- acide dicarboxylique]-1-méthanoyl}-L- phénylalaninyl-L-prolinyl-L-léucin-méthyl-ester (composé 13);
N^{α}-Acétyl-{[(E)-(L)-6-amino-hept-2-ène- acide dicarboxylique]-1-méthanoyl}-L-(p-fluoro)-phénylalaninyl-L-prolinyl-L-léucin-méthyl-ester (composé 14);
N^{α}-Benzyloxycarbonyl-{[(E)-(L)-6-amino-hept-2-ène- acide dicarboxylique]-1-méthanoyl}-L-(p-fluoro)-phénylalaninyl-L-prolinyl-L-léucin-méthyl-ester (composé 15);
[(E)-(L)-6-(2-Oxo-pyrrolidon-1-yl)-hept-2-ène- acide dicarboxylique -1-éthanoyl]-L-valinyl-L-homoprolinyl-L-léucin-méthylester (composé 16);
[(E)-(L)-6-(2-Oxo-pyrrolidon-1-yl)-hept-2-ène- acide dicarboxylique -1-éthanoyl]-L-cyclohéxylglycinyl-L-homoprolinyl-L-léucin-méthyl-ester (composé 17);
[(E)-(L)-6-(2-Oxo-pyrrolidon-1-yl)-hept-2-ène- acide dicarboxylique -1-éthanoyl]-L-cyclohéxylglycinyl-L-prolinyl-L-léucin-méthylester (composé 18);
N^{α}-Benzyloxycarbonyl-{[(E)-(L)-6-amino-hept-2-ène- acide dicarboxylique]-1-éthanoyl}-L-glutaminyl-L-prolin-L-tyrosin-méthyl-ester (composé 19);
N^{α}-Benzyloxycarbonyl-L-phénylalaninyl-{[(E)-(L)-6-amino-hept-2-ène- acide dicarboxylique]-1-éthanoyl}-L-glutaminyl-L-prolinyl-L-leucin-méthylester (composé 20);
N^{α}-Benzyloxycarbonyl-L-glutaminyl-{[(E)-(L)-6-amino-hept-2-ène- acide dicarboxylique]-1-éthanoyl}-L-léucinyl-L-prolinyl-L-glutamin-méthyl-ester (composé 21);
N^{α}-Acétyl-{[L-7-amino-4-oxo-oct-2-ène- acide dicarboxylique]-1-éthanoyl}-L-glutaminyl-L-prolinyl-L-léucin-méthyl-ester (composé 22);
N^{α}-(5-Méthylisoxazol-3-carbonyl)-{[L-7-amino-4-oxo-oct-2-ène- acide dicarboxylique]-1-isopropanoyl}-L-valinyl-L-prolinyl-léucin-méthylester (composé 23);
N^{α}-(2-Fluorobenzoyl)-{[L-7-amino-4-oxo-oct-2-ène- acide dicarboxylique]-1-méthanoyl}-L-valinyl-L-4-fluoroprolinyl-léucin-isopropyl-ester (composé 24);
N^{α}-Benzyloxycarbonyl-{[(E)-(L)-6-amino-hept-2- ène- acide dicarboxylique]-1-éthanoyl}-L- phénylalaninyl-L-prolinyl-L-léucin-méthyl-ester (composé 25);
N^{α}-Benzyloxycarbonyl-{[(E)-(L)-6-amino-hept-2-ène- acide dicarboxylique]-1-éthanoyl}-glycinyl-L-prolinyl-L-léucin-méthyl-ester (composé 26);
N^{α}-Benzyloxycarbonyl-{[(E)-(L)-6-amino-hept-2-ène- acide dicarboxylique]-1-éthanoyl}-L-alaninyl-L-prolinyl-L-léucin-méthyl-ester (composé 27);
N^{α}-*tert*-Butyloxycarbonyl-{[(E)-(L)-6-amino-hept-2-ène- acide dicarboxylique]-1-éthanoyl}-L-glutaminyl-L-prolinyl-L-léucin-méthyl-ester (composé 28);
N^{α}-Thiophèn-2-carbonyl-{[(E)-(L)-6-amino-hept-2-ène- acide dicarboxylique]-1-éthanoyl}-L-glutaminyl-L-prolinyl-L-léucin-méthyl-ester (composé 29);
N^{α}-Furan-3-carbonyl-{[(E)-(L)-6-amino-hept-2-ène- acide dicarboxylique]-1-éthanoyl}-L-glutaminyl-L-prolinyl-L-léucin-méthyl-ester (composé 30);
N^{α}-Isoxazol-5-carbonyl-{[(E)-(L)-6-amino-hept-2-ène- acide dicarboxylique]-1-èthanoyl}-L-glutaminyl-L-prolinyi-L-léucin-méthyl-ester (composé 31);
N^{α}-(5-Méthyl-isoxazol-3-carbonyl)-{[(E)-(L)-6-amino-hept-2-ène- acide dicarboxylique]-1-éthanoyl}-L-glutaminyl-L-prolinyl-L-léucin-methylester (composé 33);
N^{α}-(trans-3-(3-Thiényl)acryloyl)-{[(E)-(L)-6-amino-hept-2-ène- acide dicarboxylique]-1-éthanoyl}-L-glutaminyl-L-prolinyl-L-léucin-méthyl-ester (composé 34);
N^{α}-Acetyl-{[(E)-(L)-6-amino-hept-2-ène- acide dicarboxylique]-1-éthanoyl}-L-glutaminyl-L-prolinyl-L-léucin-méthyl-ester (composé 35);
N^{α}-(4-Trifluormethoxy-benzolsulfonyl)-{[(E)-(L)-6-amino-hept-2-ène- acide dicarboxylique]-1-éthanoyl}-L-glutaminyl-L-prolinyl-L-léucin-méthyl-ester (composé 36);
N^{α}-Benzyloxycarbonyl-{[(E)-(L)-6-amino-hept-2-ène- acide dicarboxylique]-1-éthanoyl}-L-cyclohéxylglycin-L-prolinyl-L-léucin-méthyl-ester (composé 37);
N^{α}-Benzyloxycarbonyl-{[(E)-(L)-6-amino-hept-2-ène- acide dicarboxylique]-1-éthanoyl}-L-valinyl-L-homoprolinyl-L-léucin-méthyl-ester (composé 38);
(E)-(S)-6-Benzyloxycarbonylamino-6-[3-((R)-2-phénylcarbamoyl-pyrrolidin-1-carbonyl)-phénylcarbamoyl]-héx-2-ene-éthyl-ester (composé 39);
(E)-(S)-6-Benzyloxycarbonylamino-6-{1-[(S)-3-carboxy-1-(3-méthyl-butylcarbamoyl)-propyl]-2-oxo-1,2-dihydro-pyridin-3-ylcarbamoyl}-héx-2-énoyl-isopropyl-ester (composé 40);
N^{α}-Benzyloxycarbonyl-{[(E)-(L)-2-amino-6-méthansulfonyl]-héx-5-ènyl}-L-glutaminyl-L-prolinyl-L-léucin-méthyl-ester (composé 3.2.1);
N^{α}-Benzyloxycarbonyl-[(E)-(L)-2-amino-6-diméthylsulfamoyl)-héx-5-ènyl]-L-glutaminyl-L-prolinyl-L-léucin-méthyl-ester (composé 3.2.2);
N^{α}-Benzyloxycarbonyl-[(L)-2-amino-4-(3)-oxo-cyclopént-1-ènyl]-butyryl-L-glutaminyl-L-prolinyl-L-léucin-méthyl-ester (composé 3.2.3);
N^{α}-Benzyloxycarbonyl[(L)-2-amino-5-(2-oxo-dihydrofuran-(3E)-ylidèn)]-péntanoyl-L-glutaminyl-L-prolinyl-L-léucin-méthyl-ester (composé 3.2.4);
N^{α}-Benzyloxycarbonyl-[(E)-(L)-6-amino-hept-2-ène- acide dicarboxylique]-L-glutaminyl-L-prolinyl-L-léucin-méthyl-ester (composé 3.2.5);
N^{α}-Acétyl-{[(E)-(L)-6-amino-hept-2-ène- acide dicarboxylique]-1-péntylamido}-L-glutaminyl-L-asparatyl-L-prolin-méthyl-ester (composé 3.2.6);
N^{α}-Benzyloxycarbonyl-{[(E)-(L)-6-amino-hept-2-ène- acide dicarboxylique]-1-isopropanoyl}-L-(p-fluoro-phénylalaninyl)-L-proline (composé 3.2.7);
N^{α}-Benzyloxycarbonyl-{[(E)-(L)-6-amino-hept-2-ène- acide dicarboxylique]-1-benzoyl}-L-phénylalaninyl-L-homoprolinyl-L-léucinylamide (composé 3.2.8);
N^{α}-Benzyloxycarbonyl-{[(E)-(L)-7-amino-2-oxo-oct-3-ène- acide dicarboxylique]-1-éthanoyl}-L-phénylalanin-méthylester (composé 3.2.9);
N^{α}-Benzyloxycarbonyl-[(Z)-(L)-2-amino-7-oxo-oct-5-ène-acide carboxylique]-L-glutaminyl-L-prolinyl-L-léucin-méthyl-ester (composé 3.2.10);
N^{α}-Benzyloxycarbonyl-[(Z)-(L)-2-amino-6-cyano-hèx-5-ène- acide carboxylique]-L-glutaminyl-L-prolinyl-L-léucin-méthyl-ester (composé 3.2.11);
N^{α}-Benzyloxycarbonyl-{[(E)-(L)-6-amino-hépt-2-ène-acide dicarboxylique]-1-éthanoyl}-L-valinyl-L-(octahydroindol-2-carboxyl)-L-léucinylamide (composé 4.1);
N^{α}-(Pipéridinyl-4-carbonyl)-{[(E)-(L)-2-amino-6-phénylsulfonyl]-héx-5-ènyl}-L-phénylalaninyl-L-prolinyl-L-1-cyclopéntylméthyl-2-oxo-2-(1H-tétrazol-5-yl)-éthylamide (composé 4.2);
N^{α}-Benzyloxycarbonyl-[(E)-(L)-2-amino-6-bénzyloxysulfonyl-héx-5-ènyl]-L-valinyl-L-prolinylbenzylsulfonamide (composé 4.3);
(Isopropyl)-(E)-(S)-6-[(S)-1-((S)-2-éthylcarbamoyl-pyrrolidin-1-carbonyl)-2-méthyl-propylcarbamoyl]-6-(2-pipéridin-4-yl-éthylamino)-héx-2-èn-oate (composé 5.1);
(Méthyl)-(S)-2-[((S)-1-{(E)-2R,5S)-2-(4-fluorobenzyl)-9-méthansulfonyl-5-[(5-méthyl-isoxazol-3-carbonyl)-amino]-4-oxo-non-8-énoyl}-pyrrolidin-2-carbonyl)-amino]-4-méthyl-pentanoate (composé 5.3.b);
(Isopropyl)-(E)-(6R,9S)-9-benzyloxycarbonylamino-6-[2-(2-éthylcarbamoyloctahydroindol-1-yl)-1-méthyl-2-oxoéthylcarbamoyl]-8-oxo-10-phényl-dec-2-ènoate (composé 5.4);
Pipéridin-4-carbonyl-((E)-(S)-5-benzylsulfonyl-1-{2-[2-((S)-2-benzylsulfonylaminocarbonyl-octahydro-indol-1 yl)-2-oxo-éthylamino]-acétyl}-4-ényl)-amide (composé 5.6);
(E)-5-(N'-Acétyl-N-carboxy-hydrazino)-[(pént-2-énoyl)-1-éthanoyl]-L-valinyl-L-prolinyl-L-léucin-méthylester (composé 5.7).

5. Le composé selon une des revendications 1 - 4 destiné à être utilisé en médicine.

6. Utilisation d'un composé de structure générale [TGI1] pour la préparation d'un médicament pour le traitement où la prophylaxie de la maladie coeliaque, des fibroses, de la thrombose, des maladies neurodégénératives, de la chorée de Huntington, de la maladie de Parkinson, de la maladie d'Alzheimer, de la cataracte, de l'acné, du psoriasis ou de la candidose:
liaison double substituée par accepteur-(CO)m-C₂H₄-squelette [TGI1]
où,
m est 0 ou 1, et
la liaison double substituée par accepteur porte au moins un groupement électroattracteur par conjugaison choisi parmi le groupe C=O, C=S, C=N, P=O, P=S, N=O, S=O, N=N, C=N et ayant une électronégativité≥ 2,20 et
le squelette a au moins une liaison amide et le squelette peptidique ou peptidomimétique consiste en deux jusqu'à 20 acides aminés ou mimétiques d'acides aminés, qui sont couplés les uns aux autres par des liaisons peptide, des liaisons ester, des liaisons carbonate, des liaisons uréthane, des liaisons carbamate et/ou des liaisons urée et le squelette portant le système de Michael a au moins un groupement carbonyle qui est vicinal à l'atome de carbone portant la chaîne latérale avec le système de Michael.

7. L'utilisation d'un composé selon la revendication 6 de structure générale [A] : 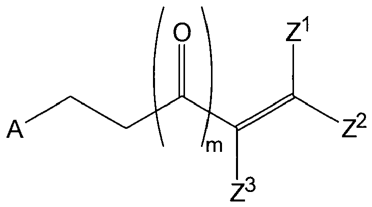 où le composé a au moins une oléfine substituée par un accepteur avec les résidus Z¹, Z² et Z³, qui est connectée à un groupement A à travers un groupement éthylène ou groupement carbonyléthylène, où
A est un résidu peptidique ayant de 2 à 20 acides aminés ou un résidu peptidomimétique ayant de 2 à 20 acides aminés, où les acides aminés comprenant aussi des acides aminés non-protéinogènes sont sélectionnés à partir des acides aminés thiocarbonylés, des ß-amino-acides, des γ-amino-acides, des δ-amino-acides, 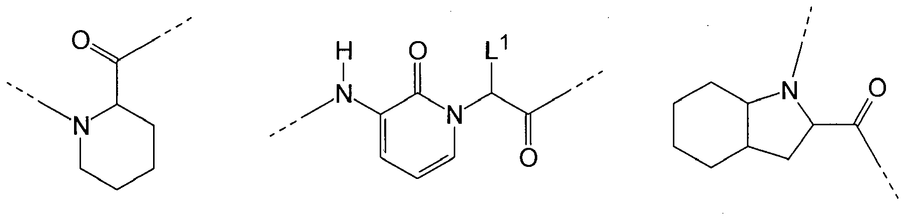 et le résidu peptidomimétique peut comprendre de 1 jusqu'à 3 mimétiques d'acides aminés sélectionnés à partir de : 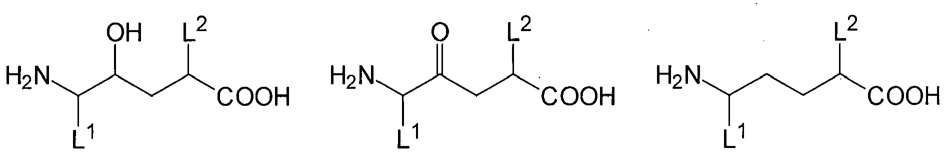 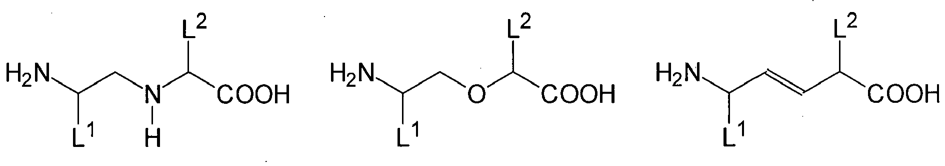 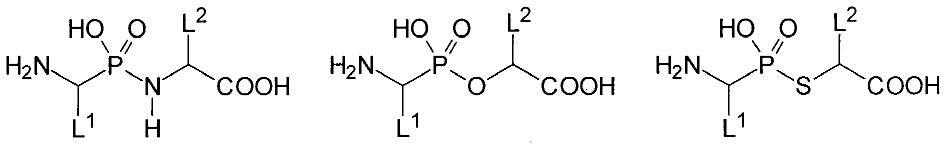 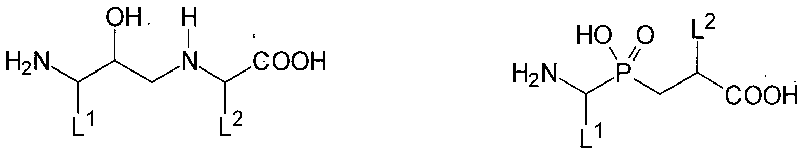 où,
L¹ et L² sont indépendamment l'un de l'autre une chaine latérale d'un acide aminé naturel où un résidu -R⁵⁷, -R⁵⁹, -R⁶⁰, -R⁶¹, -CR⁶²R⁶³R⁶⁴, -CR⁶⁵R⁶⁶-CR⁶⁷R⁶⁸R⁶⁹, -CR⁷⁰R⁷¹-CR⁷²R⁷³-CR⁷⁴R⁷⁵R⁷⁶.
m est 0 ou 1;
les résidus Z¹, Z², Z³ représentent indépendamment l'un de l'autre les groupements suivants : -H, -CO-(C₁-C₆-alkyle), -CO-R⁶, -CO-R⁷, -CO-(C₁-C₆-halogénoalkyle), -CO-(C₃-C₁₀-hétéroaryle), -CO-(C₆-C₁₅-aryle), -COO-(C₁-C₆-halogénoalkyle), -COO-(C₃-C₁₀-hétéroaryle), -COO-(C₆-C₁₅-aryle), -COO-(C₁-C₆-alkyle), -COO-R⁸, -COO-R⁹, -CN, -F, -Cl, -Br, -COOH, -CO-NH(C₁-C₆-alkyle), -CO-N(C₁-C₆-alkyle)(C₁-C₆-alkyle), -CO-NR¹⁰R¹¹, -CO-NH₂, -CO-N(CR¹²R¹³R¹⁴)(CR¹⁵R¹⁶R¹⁷), -CF₃, -OCF₃, -NO₂, -CS-(C₁-C₆-alkyle), -CS-R¹⁸, -CS-R¹⁹, -CS-O-(C₁-C₆-alkyle), -CS-O-R²⁰, -CS-N(C₁-C₆-alkyle)(C₁-C₆-alkyle), -CS-O-R²¹, -CS-NR²²R²³, -CS-NH₂, -CS-N(CR²⁴R²⁵R²⁶)(CR²⁷R²⁸R²⁹), -SO-R³⁰, -SO-R³¹, -SO₂-R³², -SO₂-R³³, -SO-CR³⁴R³⁵R³⁶, -SO-CR³⁷R³⁸R³⁹, -SO₂-CR⁴⁰R⁴¹R⁴², -SO₂-CR⁴³R⁴⁴R⁴⁵, -SO-N(C₁-C₆-alkyle)(C₁-C₆-alkyle), -SO-NR⁴⁶R⁴⁷, -SO-NH₂, -SO-N(CR⁴⁸R⁴⁹R⁵⁰)(CR⁵¹R⁵²R⁵³), -SO₂-N(C₁-C₆-alkyle)(C₁-C₆-alkyle), -SO₂-NR⁵⁴R⁵⁵, -SO₂-NH₂ -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, où au moins un des résidus Z¹, Z², Z³ est différent de l'hydrogène;
les résidus **Z¹** et **Z²** peuvent aussi former ensemble un résidu -CO-O-CO-CH₂-, -CO-O-CH₂-CH₂-,
les résidus **Z²** et **Z³** peuvent aussi former ensemble un résidu -CO-Z'-CH2-, -CO-O-CH₂-, -CO-CH₂-CH₂-, -CO-O-CO- ou -CO-NH-CO-, où
**Z'** représente un des groupements suivants: -CH₂-, -CF₂-, -C₂H₄-, -CF₂-CH₂-, -CH₂-CH₂-, -O-, -O-CH₂-, -NH- ou -NH-CH₂-;
les résidus **R⁶** - **R⁷⁶** représentent indépendamment l'un de l'autre les groupements suivants:
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -CN, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -OC₆H₄-OCH₃, -NH-C(=NH)-NH-cyclo-C₃H₅, -NH-C(=NH)-NH[CH(CH₃)₂], -CF₂Cl, -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -OC₆H₄-CH₃, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂SH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH₂-CH₂SH, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH₂-CH₂-CH₂SH, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₆H₄-OCH₃, -C₆H₄-OH, -CH₂-CH₂-OCH₃, -CH₂-CH₂OH, -CH₂-OCH₃, -CH₂-C₆H₄-OCH₃, -CH₂-C₆H₄-OH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH;
ainsi que des formes stéréoisomériques, des isomères E/Z, des énantiomères, des mélanges énantiomériques, des diastéréoisomères, des mélanges diastéréoisomériques, des racémates, des tautomères, des anomères, des formes céto-énoliques, des formes bétainiques, des solvates, des hydrates, ainsi que des sels pharmaceutiquement acceptables des composés précités.

8. Utilisation selon la revendication 7, où le composé a la suivante formule générale [B]: 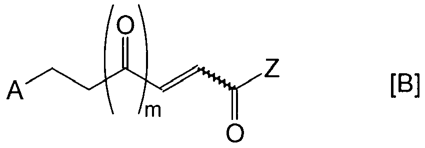 où
**Z** est un groupement hydroxyle, un groupement amine, un groupement C₁₁-C₆-alkylamine, un groupement C₁-C₆-dialkylamine, un groupement C₁-C₆-alcoxy, un groupement C₁-C₆-alkyle, un groupement C₁-C₆-halogénoalkyle, un groupement C₃-C₁₀-hétéroaryle, ou un groupement C₆-C₁₅-aryle, et
**m** est 0 ou 1, et
**A** est un résidu peptidique, un dérivé peptidique ou un peptidomimétique.

9. L'utilisation selon la revendication 8, où le composé a la suivante formule générale [C], [D], [E] ou [F]:

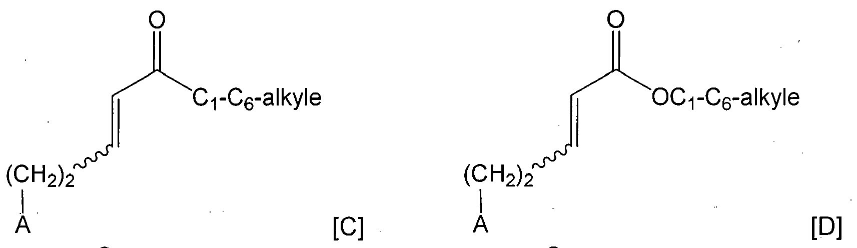

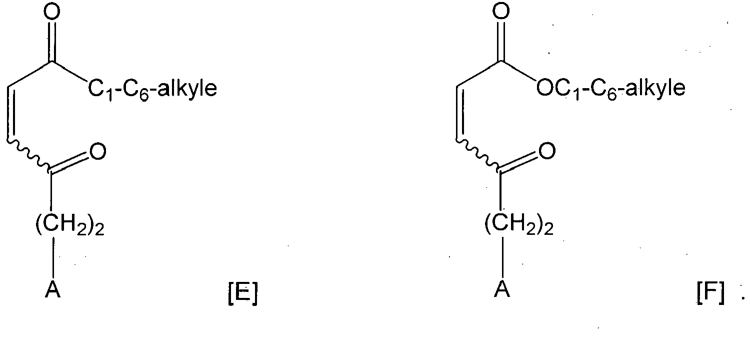

10. L'utilisation selon une des revendications 7 - 9, où le composé a une des suivantes formules générales (I), (II) ou (III): 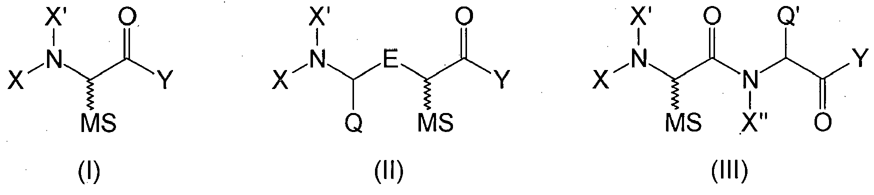 où
MS est le système de Michael de structure suivante 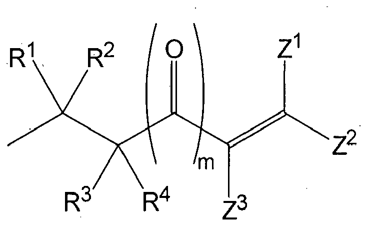 et où m, E, R¹, R², R³, R⁴, Q, Q', X, X', X", Y, Z¹, Z² et Z³ ont les significations précitées dans la revendication 1.

11. Une composition pharmaceutique comprenant au moins un composé selon la formule générale (I), (II), (III), (IIB), [A], [B], [C], [D], [E], [F] et/ou des sels pharmaceutiquement acceptables de ceux-ci et au moins un véhicule, excipient ou solvant pharmaceutiquement acceptable, optionnellement avec une substance sélectionnée parmi le groupe comprenant des vitamines, des minéraux, des oligo-éléments, des peptidases, des cytokines, des anticorps monoclonaux et de la zonuline.

12. Un procédé de préparation du peptide ou du peptidomimétique selon la revendication 1 avec m = 0 suivant le schéma synthétique suivant: 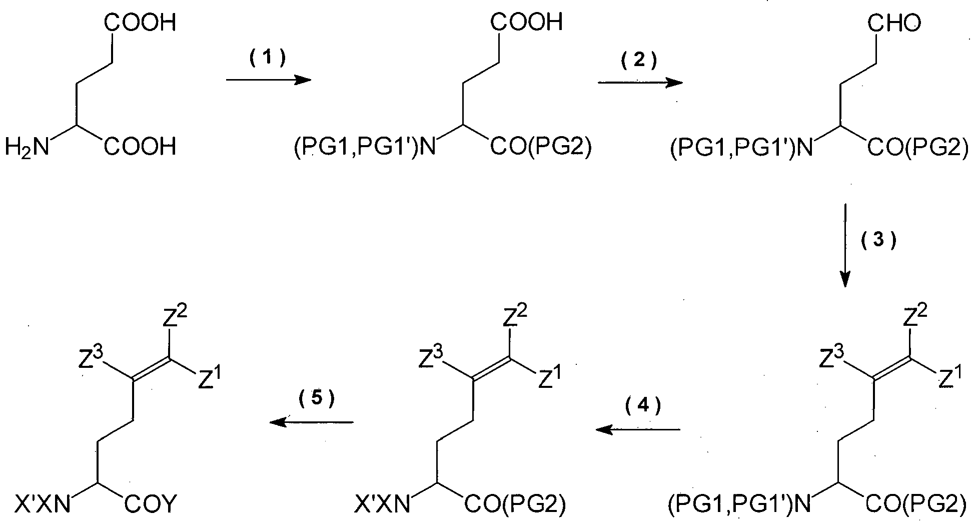
(0) fournir l'acide glutamique,
(1) attacher un groupement protecteur (PG1 ou PG2) au C-terminus ou N-terminus de l'acide glutamique,
(2) réduire la fonction acide carboxylique de la chaîne latérale de l'acide glutamique à l'aldéhyde,
(3) convertir l'aldéhyde obtenu en une double liaison électrophile substituée par accepteur,
(4) enlever les groupements protecteurs, et
(5) élonger le C-terminus et/ou le N-terminus par un fragment peptidique ou par un peptidomimétique,
ou
selon le schéma synthétique suivant: 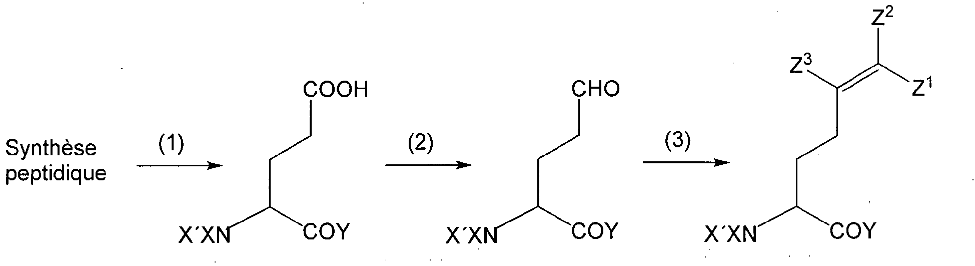
(1) préparer un peptide ou un peptidomimétique comprenant l'acide glutamique, ledit peptide ou peptidomimétique étant protégé au C-terminus et au N-terminus,
(2) réduire la fonction acide carboxylique de la chaîne latérale de l'acide glutamique à l'aldéhyde,
(3) convertir l'aldéhyde obtenu en une double liaison électrophile substituée par accepteur, et
(4) enlever optionnelement les groupements protecteurs,
ou
un procédé de préparation du peptide ou du peptidomimétique selon la revendication 1 avec m = 1 suivant le schéma synthétique suivant: 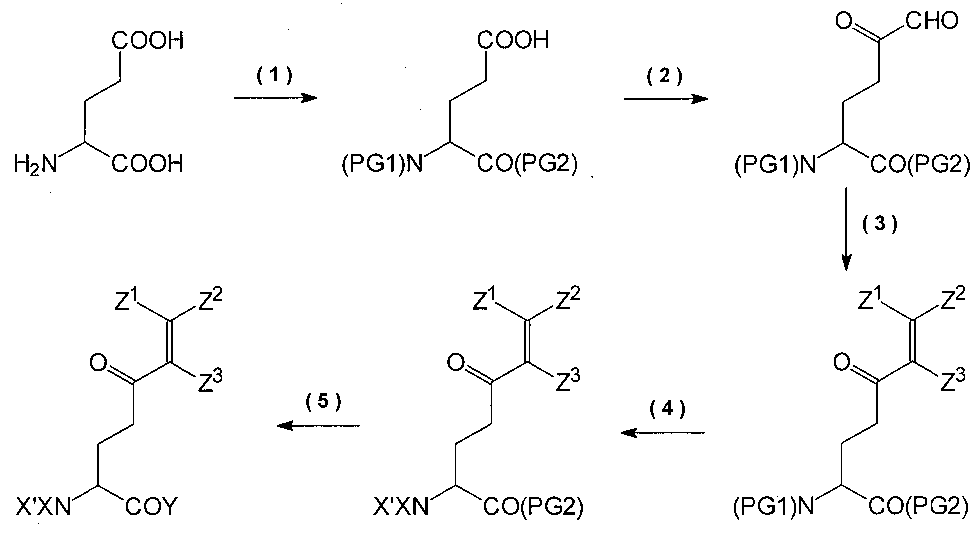
(0) préparer l'acide glutamique,
(1) attacher un groupement protecteur (PG1 et PG2) au C-terminus ou N-terminus de l'acide glutamique,
(2) convertir la fonction acide carboxylique de la chaine latérale en un groupement dicéto,
(3) convertir la fonction carbonyle terminal du groupement dicéto obtenu en une liaison double électrophile substituée par un accepteur,
(4) enlever les groupements protecteurs, et
(5) élonger le C-terminus et/ou le N-terminus par un fragment peptidique ou par un peptidomimétique,
ou
selon le schéma synthétique suivant : 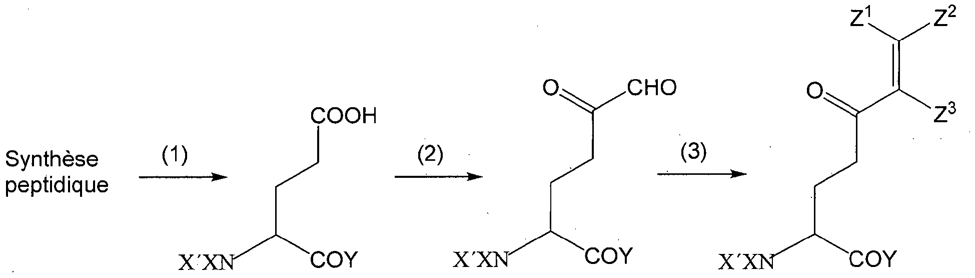
(1) préparer un peptide ou un peptidomimétique comprenant l'acide glutamique, ledit peptide ou peptidomimétique étant protégé au C-terminus et au N-terminus,
(2) convertir la fonction acide carboxylique de la chaîne latérale de l'acide glutamique en un groupement dicéto,
(3) convertir la fonction carbonyl terminal du groupement dicéto obtenu en une liaison double électrophile substituée par un accepteur, et
(4) enlever optionnellement les groupements protecteurs.
